# EUROPEAN PATENT SPECIFICATION

(11) **EP 2 403 868 B1**
(45) Date of publication and mention of the grant of the patent: **11.05.2016**
(21) Application number: 10715674.7
(22) Date of filing: 03.03.2010
(51) Int. Cl.: C07K 14/435, A61K 39/35

(54) **CLONING AND RECOMBINANT PRODUCTION OF VESPULA VENOM PROTEASE AND METHODS OF USE THEREOF**
KLONIERUNG UND REKOMBINANTE PRODUKTION VON WESPENGIFTPROTEASE UND ZUGEHÖRIGE VERWENDUNGSVERFAHREN
Clonage et production recombinante de protéase de venin de Vespula et ses procédés d'utilisation

(30) Priority: 03.03.2009 EP 09003033
(43) Date of publication of application: 11.01.2012
(73) Proprietor: PLS-Design GmbH, 20255 Hamburg (DE)
(72) Inventor: SELSMANN, Henning, 23863 Nienwohld (DE); BRAREN, Ingke, 22303 Hamburg (DE); GRUNWALD, Thomas, 22459 Hamburg (DE)
(74) Representative: Seuss, Thomas
(86) International application number: PCT/EP2010/001329
(87) International publication number: WO 2010/099956

(56) References cited:
- WO-A-99/38978
- HAN ET AL: "An anticoagulant serine protease from the wasp venom of Vespa magnifica" TOXICON, ELMSFORD, NY, US, vol. 51, no. 5, 12 January 2008 (2008-01-12), pages 914-922, XP022520443 ISSN: 0041-0101 cited in the application & DATABASE EMBL [Online] 16 December 2008 (2008-12-16), "Vespa magnifica anticoagulant serine protease mRNA, complete cds." retrieved from EBI accession no. EMBL:EU267370 Database accession no. EU267370 & DATABASE UniProt [Online] 10 February 2009 (2009-02-10), "SubName: Full=Anticoagulant serine protease;" retrieved from EBI accession no. UNIPROT:B7SD94 Database accession no. B7SD94
- WINNINGHAM K M ET AL: "Hymenoptera venom protease allergens" JOURNAL OF ALLERGY AND CLINICAL IMMUNOLOGY, MOSBY - YEARLY BOOK, INC, US, vol. 114, no. 4, 1 October 2004 (2004-10-01), pages 928-933, XP004631445 ISSN: 0091-6749 & DATABASE EMBL [Online] 21 May 2003 (2003-05-21), "Polistes dominulus venom serine protease precursor, mRNA, complete cds." retrieved from EBI accession no. EMBL:AY285998 Database accession no. AY285998 & DATABASE UniProt [Online] 1 October 2003 (2003-10-01), "SubName: Full=Venom serine protease; Flags: Precursor;" retrieved from EBI accession no. UNIPROT:Q7Z269 Database accession no. Q7Z269
- LTTLER ET AL: "Allergenic components of bald-faced hornet (V. maculata) venom" TOXICON, ELMSFORD, NY, US, vol. 24, no. 7, 1 January 1986 (1986-01-01), page 738, XP025528872 ISSN: 0041-0101 [retrieved on 1986-01-01]
- HEMMER W ET AL: "Identification by immunoblot of venom glycoproteins displaying immunoglobulin E-binding N-glycans as cross-reactive allergens in honeybee and yellow jacket venom." CLINICAL AND EXPERIMENTAL ALLERGY, vol. 34, no. 3, March 2004 (2004-03), pages 460-469, XP002556175 ISSN: 0954-7894 cited in the application
- HANCOCK ET AL: "False positive reactivity of recombinant, diagnostic, glycoproteins produced in High Five(TM) insect cells: Effect of glycosylation" JOURNAL OF IMMUNOLOGICAL METHODS, ELSEVIER SCIENCE PUBLISHERS B.V.,AMSTERDAM, NL LNKD- DOI:10.1016/J.JIM.2007.08.002, vol. 330, no. 1-2, 26 January 2008 (2008-01-26), pages 130-136, XP022435275 ISSN: 0022-1759 cited in the application
- AKDIS C A ET AL: "Differential regulation of human T cell cytokine patterns and IgE and IgG4 responses by conformational antigen variants." EUROPEAN JOURNAL OF IMMUNOLOGY MAR 1998, vol. 28, no. 3, March 1998 (1998-03), pages 914-925, XP002554131 ISSN: 0014-2980
- SHREFFLER W G ET AL: "Microarray immunoassay (MIA) for the parallel IgE and IgG4 epitope mapping of milk allergens" JOURNAL OF ALLERGY AND CLINICAL IMMUNOLOGY, MOSBY - YEARLY BOOK, INC, US, vol. 113, no. 2 Supplement, 1 February 2004 (2004-02-01), page S238, XP002449103 ISSN: 0091-6749
- TE PIAO KING ET AL: "STRUCTURE AND BIOLOGY OF STINGING INSECT VENOM ALLERGENS" INTERNATIONAL ARCHIVES OF ALLERGY AND IMMUNOLOGY, KARGER AG, CH, vol. 123, no. 2, 1 October 2000 (2000-10-01), pages 99-106, XP008014036 ISSN: 1018-2438
- HOFFMAN D R: "ALLERGENS IN HYMENOPTERA VENOM 8. ISOLATION AND PURIFICATION OF PROTEIN COMPONENTS FROM 3 SPECIES OF VESPID VENOMS" JOURNAL OF ALLERGY AND CLINICAL IMMUNOLOGY, vol. 75, no. 5, 1985, pages 599-605, XP002556201 ISSN: 0091-6749

## Description

The present invention relates to nucleic acids encoding a novel *Vespula* venom protease or fragments thereof, recombinant vectors comprising such nucleic acids, and host cells containing the recombinant vectors. The invention is further directed to expression of such nucleic acids to produce a recombinant *Vespula* venom protease, or recombinant fragments thereof, or synthetic peptides thereof. Such a protease or fragments thereof or synthetic peptides thereof are useful for diagnosis of insect venom allergy and for therapeutic treatment of insect venom allergy.

### BACKGROUND OF THE INVENTION

Immunoglobulin E (IgE) -mediated insect sting allergy to Hymenoptera is of common occurrence. Hymenoptera stings are classified into normal local reactions, large local reactions, systemic anaphylactic reactions, systemic toxic reactions, and unusual reactions. The most frequent clinical patterns are large local and systemic anaphylactic reactions. Based on the results of four studies, the prevalence of large local sting reactions ranges from 2.4% and 26.4% (for a review, see Biló et al 2005). In children the prevalence yielded by one study is 19%. Nine epidemiologic studies report a prevalence of self-reported systemic anaphylactic sting reactions between 0.3% and 7.5% (for a review, see Biló et al 2005). For most venom-allergic patients an anaphylactic reaction after a sting is a very traumatic event resulting in an altered health-related quality of life. In some instances the anaphylactic reaction may cause death, and this fatal outcome can occur as the first manifestation after a sting (Barnard 1973). Risk factors influencing the outcome of an anaphylactic reaction include the time interval between stings, the number of stings, the severity of the preceding reaction, age, cardiovascular diseases and drug intake, insect type, elevated serum tryptase, and mastocytosis.

Hymenoptera include the familiy Apidae consisting of the genera *Apis* (including the species *Apis mellifera,* honeybee) and *Bombus* (bumblebees), and the family Vespidae (vespids) consisting of the Vespinae and Polistinae subfamilies. The Vespinae subfamily includes the three genera *Vespula* (called wasps in Europe, yellow jackets in the USA), *Dolichovespula* (called hornets in the USA) and *Vespa* (called hornets in Europe and the USA). In the USA there are many species of *Vespula, Dolichovespula* and *Polistes* (called paper wasps in Europe and the USA) (King 1994). Vespine wasps are generally considered to be the most common source of stings, due to their abundance, aggressiveness and relatively large colony size (up to several thousand workers) of some species. In areas with mild winters, including Florida, California and Hawaii multiple queens may inhabit the same nest and produce enormous perennial colonies with a high potential for mass stinging (Greene and Breitsch 2005). Polistine wasps have small colonies of usually no more than a few dozen workers, but their nests can be extremely abundant around human habitations. The most pestiferous species is the recently arrived *Polistis dominulus,* an invasive European wasp (Greene and Breitsch 2005). Although honeybees are often invoked as a sting hazard, accidental disturbance of feral colonies in the US was rare until recently, because nests were typically located within tree cavities or structural voids. The arrival of Africanized bees *(Apis mellifera scutellata)* in 1990 markedly increased the stinging risk in several southwestern states, as this subspecies has an extremely low defensive response threshold and is capable of some of the most severe attacks known for any social insect (Greene and Breitsch 2005).

In Europe *Vespula* species *V. vulgaris and V. germanica* are dominating. In the genus *Dolichovespula* the most common species in Europe are *D. media, D. saxonia and D. sylvestris* and in the genus *Vespa, Vespa crabro* (European hornet) is the most prevalent in Europe. Among Polistinae (called paper wasps in Europe and the USA), *Polistes gallicus, P. nimpha and P. dominulus* are widespread especially in the Mediterranean area. In central and northern Europe vespid (mainly *Vespula* spp.) and honeybee stings are the most prevalent, whereas in the Mediterranean area stings from *Polistes* and *Vespula* are more frequent than honeybee stings, bumblebee stings are rare throughout Europe and more of an occupational hazard (Biló et al 2005).

The symptoms of IgE-mediated (type I) sting allergic reactions are due to release of mediators (e.g. histamine) resulting from cross-linking of effector cell-bound IgE antibodies by venom allergens. The symptoms can be suppressed by various pharmacologic treatments, but allergen-specific immunotherapy (SIT) represents the only curative approach. A rise in allergen-blocking IgG antibodies, particularly of the IgG4 class (Wetterwald et al 1985), a reduction in the number of mast cells and eosinophils, and a decreased release of mediators (Varney et al 1993) were found to be associated with successful SIT. Based on these observations, determination of the serum levels of allergen-specific IgE and IgG4 antibodies is useful to describe the immune status of an allergic patient.

*In vitro* measurement of specific serum IgE antibodies can be performed by the radio-allergosorbent test (RAST), various enzyme-linked immunosorbent assays (ELISA) and other IgE-binding techniques such as immunoelectrophoresis, immunoblot, immunodotblotting, bead array technology and various fluid phase systems. The analytes can also be allergen-specific antibodies of the IgG4 or other IgG subclasses. As an alternative to the above listed assay systems, basophil cells derived from patients or from basophil cell lines such as the KU812 have been used for the *in vitro* measurement of allergen-specific IgE antibodies in serum by *in vitro* mediator release assays (MRA). Furthermore, *in vivo* tests for diagnosis of hymenoptera venom allergy are performed by skin prick or intradermal testing. Such tests are well known in the art (Biló et al 2005).

### Composition of vespid venoms

Knowledge of the composition of venoms and structural as well as immunological features of individual venom allergens is a prerequisite for the accurate diagnosis and treatment of insect venom allergy. Hymenoptera venoms contain a number of toxins, enzymes, and biologically active peptides. Since it was easy to obtain sufficient quantities of material, honeybee venom has been well studied. Honeybee venom contains at least 18 active substances. Many of the proteins and polypeptides in honeybee venom have been identified as hypersensitizing agents including phospholipase A2 (Api m 1), hyaluronidase (Api m 2), acid phosphatase (Api m 3), melittin (Api m 4), dipeptidylpeptidase (Api m 5; allergen C), Api m 6 polypeptides (4 isoforms), a 39 kDa protease (Api m 7), and a 70 kDa protease (Biló et al 2005; Winningham et al 2004, Blank et al 2008. Vespid venoms (venoms of the Vespidae family) are also complex mixtures of toxins, enzymes including phospholipases, hyaluronidases and proteases, and biologically active peptides. These venom components can act on the cardiovascular system, nervous system and immunological system of mammalians. Antikoagulant effects of vespid venom obtained from a hornet species (*Vespa orientalis*) was attributed to the proteolytic degradation of coagulation factors. The purified Protease migrated under reducing conditions as a double band (42 kDa and 44 kDa) on sodium dodecyl sulfate polyacrylamide gel electrophoresis (SDS-PAGE) (Haim et al 1999). Potential allergenic properties of this enzyme (or enzymes) have not been determined.

Another anticoagulant protease has been purified from the venom of a different hornet species *(Vespa magnifica)* (Han et al 2008). This protein, named magnvesin, contains serine protease-like avitivity and inhibits blood coagulation. The cDNA of magnvesin cloned from the venom sac cDNA library of *Vespa magnifica* (GenBank accession EU267370) encodes a protein precursor of 305 amino acids and a mature protease of 242 amino acids with a molecular weight of 27.4 kDa (Han et al 2008). Potential allergenic properties of this enzyme have not been determined.

Proteases have been identified also in the venom of *Polistis* species. A cDNA cloned from the venom sac cDNA library of *Polistis dominulus* (GenBank accession AY285998) encodes a protease of 244 amino acids (Winningham et al 2004). This protease contains 6 potential N-linked glycosylation sites, 4 of which appear to be glycosylated in the natural molecule. Due to its IgE binding activity the protease has been assigned the name Pol d 4 by the IUIS Allergen Nomenclature Subcommittee (Winningham et al 2004). Another protease prepared by benzamidine affinity chromatography from *Polistis exclamans,* has been reported to migrate in SDS-PAGE with the phospholipase as a protease from *Polistis gallicus* (Winningham et al 2004), but for none of these proteases a molecular weight or sequence information is provided by the authors. All three proteases are claimed to be highly cross-reactive, although the identity of the proteases from *Polistis exclamans* and *Polistis gallicus* remains to be elucidated.

An elastase-like protein was purified from the venom of the solitary spider wasp *Cyphonyx dorsalis* of the family Pompilidae which is part of the Vespoidea superfamily (Yamamoto et al 2007). The cDNA cloned from total RNA of a whole body (GenBank accession AB264172) encodes a protein of 256 amino acids with a calculated molecular weight of 25.7 kDa (Yamamoto et al 2007). The result of a homology search showed a 30.1% homology with the elastase-like serine proteinase from the red fire ant *Solenopsis invicta.* Potential allergenic properties of this enzyme have not been determined.

A protein (Vn50) isolated from the venom of the endoparasitoid wasp *Cotesia rubecula* has been shown to be homologous to serine proteinase homologs (Asgari et al 2003). The cDNA cloned from total RNA from the venom gland of *Cotesia rubecula* encodes a protein of 388 amino acids. Vn50 contains two structural domains, a carboxy-terminal serine proteinase domain and an amino-terminal "clip" domain. However, the serine proteinase domain is not functional since it lacks a serine residue at the conserved site (Asgari et al 2003). Potential allergenic properties of this enzyme analog have not been determined.

Although in Europe *Vespula* species (*V. vulgaris* and *V. germanica*) are dominating, only few *Vespula* proteins and polypeptides have been identified as allergens so far including those in the venom of *Vespula vulgaris,* Ves v 1 (phospholipase A1), Ves v 2 (Hyaluronidase) and Ves v 5 (antigen 5) (for a review, see Biló et al 2005). The presence of proteases in the venom of *Vespula* species has not been reported and according to several publications there is no evidence for the presence of significant amounts of proteases in these venoms (Winningham et al 2004; King and Spangfort 2000; Hoffman 1985). Considering the complex venom composition of honeybees, however, the venom of *Vespula* species is likely to contain more than three allergens. Therefore, there is a need in the art to for more information about allergens in the venom of *Vespula* species to optimize accurate diagnosis and treatment of venom allergy caused by these species.

### B and T cell epitopes

In order to fully address the basis for allergic response to *Vespula* venom allergens and the molecular mechanism of allergen-based immunotherapies, there is a particular need in the art to delineate B and helper T cell epitopes of *Vespula* venom allergens. Antibody responses to a protein require the collaboration of T helper and B lymphocytes and antigen presenting cells (APC). T helper cells are activated when their T cell receptor binds to complexes of antigenic peptide-MHC class II molecule on the surface of APC. On the basis of their patterns of lymphokine production, T helper cells are divided into two groups: TH1 cells producing IL-2 and IFN-γ, and TH2 cells producing IL-4 and IL-5. These lymphokines in turn influence antigen-activated B cells to differentiate and proliferate into plasma cells secreting antibodies of different isotypes. IL-4 is one lymphokine known to influence IgE synthesis (Finkelman et al 1990).

B cell epitopes of proteinaceous allergens can include native protein structures (conformational or discontinuous or topographic epitopes), linear peptides (linear epitopes) and carbohydrates. The conformational type consists of amino acid residues which are spatially adjacent but may or may not be sequentially adjacent. The vast majority of IgE epitopes has been reported to be of the conformational type (King 1990). The linear type consists of only sequentially adjacent residues. However, even linear B cell epitopes are often conformation-dependent, and antibody-antigen interactions are improved when the epitope is displayed in the context of the folded protein. It is believed that the entire accessible surface of a protein molecule can be recognized as epitopes by the antigen receptor of B cells, although all epitopes are not necessarily recognized with equal likelihood (Benjamin et al 1984). Programs have been developed for the prediction of both linear and conformational B cell epitopes (Zhang et al 2008). For example, DiscoTope is a method for discontinuous epitope prediction that uses protein 3D structural data as input. It is based on amino acid statistics, spatial information and surface accessibility for a set of discontinuous epitopes determined by X-ray crystallography of antibody-proteinaceous antigen-complexes.

T cell epitopes consist of only the linear type since they are peptides that have been processed in the lysosomes of APC by proteases. Analysis of naturally processed antigenic peptides bound to MHC class II molecules indicates that their size ranges typically from about 13 to 25 amino acid residues, but analysis of synthetic peptide-MHC class II molecule complexes for their T cell proliferative response suggests a minimal size of about 9 amino acid residues (Rudensky et al 1991, Wang et al 2008). T cell epitopes are distributed throughout the entire protein molecule, and they may function as major or minor determinants depending on the MHC haplotype of the immunized host (O'Hehir 1991). MHC proteins are highly polymorphic and each binds to a limited set of peptides. Thus, the particular combination of MHC alleles present in a host limits the range of potential epitopes that are recognized.

Because of the central role of TH2 cells in determining the isotype switch event of B cells, the T cell epitopes of several allergens have been mapped (O'Hehir 1991). However, defining epitope sequence specificity for a particular host including cleavage of the polypeptide by the proteasome, transport of peptides into the endoplasmatic reticulum by the transporter associated with processing (TAP) and binding to MHC molecules poses serious problems. Therefore, an abundance of methods has been developed for the prediction of T cell epitopes (see, e.g., Zhang et al 2008; Korber et al 2006). The MHC class II binding prediction provided by a publication in 2008 in Nucleic Acid Res. (Zhang et al) includes 4 methods, ARB, SMM_align, the method of Sturniolo which is also the basis of TEPITOPE, and a consensus approach, all of which have been identified as top performing ones.

In order to avoid undesirable systemic reactions on specific immunotherapy with natural allergens, there has been continued interest in the development of modified allergens with reduced allergenic activities for immunotherapy. In one approach T cell epitopes are used to modulate allergen-specific immune responses. It has been observed *in vivo* in mice for the allergen Fel d 1 (cat hair), Der p 1 (acarian: *Dematophagoides pterissimus*) and Bet v 1 (birch pollen) that the nasal, oral or subcutaneous administration of peptides carrying T cell epitopes of these allergens inhibits the activation of the specific T lymphocytes (Briner et al 1993; Hoyne et al 1993; Bauer et al 1997). Based on these results allergen peptide fragments capable of stimulating T lymphocytes in allergic patients were evaluated in clinical sudies. In the case of the major honeybee venom allergen Api m 1 fragments 50-69 and 83-97 have been described as being active during a study comprising a single patient (Dhillon et al. 1992). In a study comprising forty patients (Carballido et al 1993) Api m 1 fragments 45-62, 81-92 and 113-124 proved to be active. However, these three fragments proved to be T cell epitopes for only 25 to 45% of the patients, pointing to the existence of other epitopes. Nevertheless, the three peptides have been used successfully for desentization of five allergic patients whose T lymphocytes proliferated in the presence of these peptides (Muller et al 1998). No serious systemic effect was observed and the patients became tolerant to honeybee stings. This demonstrates the benefit of using peptides for desensitization. Therefore, there is a need in the field to identify peptide fragments of allergens in vespid venoms capable of stimulating T lymphocytes in allergic patients, and in particular from allergens in the venom of *Vespula* species.

In another approach, B cell epitopes of allergens are modified to decrease the risk of potential systemic reactions. The aim of such allergen modification is to decrease the allergenicity while retaining its immunogenicity. Since allergenicity depends on the interaction of a multivalent allergen with basophil- or mast cell-bound IgE antibodies, allergenicity can be reduced by decreasing the density of B cell epitopes. One approach is by partial or complete denaturation of allergens on chemical modification because the vast majority of B cell epitopes are of the discontinuous type, being dependent on the native conformation of proteins. However, there are serious limitations to the use of such molecules. While linear T cell epitopes are preserved, the surface structure is not maintained and, thereby, the capacity of such molecules to stimulate an allergen-specific non IgE antibody response is severely limited. Similar considerations apply to an approach in which the accessibility of B cell epitopes is reduced by polymerization on formaldehyde or glutaraldehyde treatment or by attachment of nonimmunogenic polymers. Usually near-complete loss of the discontinuous B cell epitopes occurs when allergens are modified with >100-fold reduction in allergenicity.

Using recombinant DNA technology well defined allergens can be produced which allow the determination of their three-dimensional structure and site-directed modifications of their surface structure. Unfortunately, however, knowledge of IgE-specific B cell epitopes is scarce and useful tools for reliabe identification of such epitopes are not available. While several IgE epitopes could be determined by mapping with synthetic overlapping peptides synthesized according to the allergen amino acid sequence, many relevant IgE epitopes could not be identified because peptides frequently fail to display conformations mimicking discontinuous epitopes. There is no doubt that naturally occurring IgE antibodies represent ideal tools for structural analyses of IgE epitopes. However, the number of monoclonal allergen-specific IgE antibodies isolated from blood lymphocytes of allergic patients so far is extremely limited. In an alternative approach, animal derived monoclonal allergen-specific antibodies can be useful to identify IgE epitopes. For example, from a panel of mouse monoclonal antibodies that effectively inhibited binding of birch pollen allergen Bet v 1 to specific IgE, several monoclonal antibodies identified a continuous epitope within an exposed surface area of Bet v 1 that could be part of a discontinuous IgE epitope (Lebecque et al 1997) Provided such antibodies bind to Bet v 1 with high affinity, they represent useful tools for further structural analyses by X-ray diffraction of crystals obtained from allergen-antibody complexes. Although the surface area recognized by animal-derived allergen-specific antibodies may not be identical with that recognized by human IgE antibodies, both areas are closely related as indicated by the inhibition experiments. Therefore, structural information obtained from the analysis of such allergen-antibody complexes provide a valuable basis for the modification of IgE epotopes by site-directed mutagenesis. One problem of this approach, however, is the need of a panel of high affinity antibodies with different epitope specificities for each allergen to allow for a detailed analysis of the total spectrum of potential IgE epitopes. Assuming that a B cell epitope takes up an area of approximately 900 A², the vast majority of allergens is likely to display more than one IgE epitope, in the case of allergens with a molecular weight of >40 kDa most likely several IgE epitopes. Therefore, there is a need in the field to develop high affinity allergen-specific antibody panels that are capable of inhibiting IgE binding.

Another serious problem associated with the design of hypoallergenic molecules for an improved immunotherapy is the lack of understanding of the immune response that guarentees a lasting protection after specific immunotherapy. The aim to decrease the allergenicity of a given allergen while retaining its immunogenicity is widely accepted, but the term immunogenicity remains to be defined. Evaluation of modified recombinant allergens with a strongly reduced IgE reactivity that display the full spectrum of linear T cell epitopes but a different surface structure as compared to the corresponding natural allergen, have demonstrated that such molecules are capable of reducing specific IgE development towards the native allergen (Niederberger et al 2004; Karamloo et al 2005). However, a long lasting protective effect after treatment with these molecules has not been demonstrated. Apparently, the capacity of recombinant allergens to stimulate a long lasting protective allergen-specific non IgE antibody response requires also a surface structure that is closely related to that of the corresponding natural allergen. Since disruption of IgE epitopes is associated with a significant alteration of the surface structure, there is a need in the field to identify those surface structures of allergens that mediate an appropriate non-IgE response for a long lasting protection. There is a particular need in the field to identify those surface structures of allergens in the venom of *Vespula* species that mediate an appropriate non-IgE response for a long lasting protection.

There is a further need to determine whether there is cross reaction of the B and T cell epitopes of vespid venom allergens with other environmental and/or homologous proteins. Thus, there is a need to determine whether vespid venom allergens share partial identity with environmental and/or homologous proteins, and more imporantly, to obtain the sequences of the regions of the partial identitiy, in particular the specific amino acid sequence of partial identity. There is a further need to determine the level of cross reactivity of vespid allergens with other proteins at the B cell and T cell level, the relevance of this cross reactivity, and whether such cross reactivity is pathological, i.e., involved in or responsible for allergy, or benficial, i.e., inhibitory of allergy.

### SUMMARY OF THE INVENTION

The invention is based, in part, on the discovery of a novel *Vespula* venom protease, which has been named Ves v 4. This protease or fragments, derivatives or analogs thereof can be used, e.g., for diagnosis of insect venom allergy and for therapeutic treatment of insect venom allergy.

The present invention provides a nucleic acid molecule encoding a novel *Vespula* venom protease, fragments thereof, or derivatives or analogs thereof. In a specific embodiment, nucleic acid molecules of the invention encode polypeptide fragments containing one or more B cell epitopes of the *Vespula* venom protease. In another specific embodiment, nucleic acid molecules of the invention encode T cell epitope-containing polypeptide fragments of the *Vespula* venom protease capable of stimulating T cells of subjects allergic to Ves v 4. In another specific embodiment, nucleic acid molecules of the invention encode polypeptide fragments containing one or more T cell epitopes and one or more B cell epitopes of the *Vespula* venom protease. In a preferred embodiment, the polypeptide fragments are between 50-150 amino acids in length.

Further described are methods for isolating nucleic acid molecules from any species of the family Vespidae which are hybridizable under moderate or high stringency conditions to a nucleic acid having the nucleotide sequence shown in SEQ ID NO: 1.

In one embodiment the invention provides a nucleic acid molecule comprising the nucleotide sequence as shown in SEQ ID NO: 1. Also described are nucleic acids hybridizable to the nucleotide sequence as shown in SEQ ID NO: 1 under moderate or high stringency conditions, and fragments, derivatives, mutants and analogs thereof.

In one embodiment the invention provides a nucleic acid molecule comprising the nucleotide sequence as shown in SEQ ID NO: 1 and nucleic acids that are at least 90% identical to the nucleotide sequence as shown in SEQ ID NO: 1 or isolated fragments of the nucleic acid molecule comprising the nucleotide sequence as shown in SEQ ID NO: 1, wherein said fragments are between 150 and 1172 nucleotides in length.

The present invention further provides polypeptides encoded by the nucleic acids of the invention wherein the polypeptides comprise an amino acid sequence that is at least 90% identical, to the amino acid sequence of SEQ ID NO:2.

In a further embodiment, the polypeptide of the invention comprises the amino acid sequence as shown in SEQ ID NO:2 and polypeptides that are at least 90% identical, more preferably more than 95% identical and most preferably more than 99% identical to the amino sequence as shown in SEQ ID NO: 2, or an isolated fragment of the amino acid sequence as shown in SEQ ID NO: 2, wherein said fragment is a partial segment of the polypeptide sequence shown in SEQ ID NO: 2 which is at least 50 amino acids long.

In a specific embodiment, the description provides polypeptide fragments of the Ves v 4 polypeptides of the invention, containing one or more B cell epitopes of the *Vespula* venom protease. In another specific embodiment, the description provides T cell epitope-containing polypeptide fragments of the *Vespula* venom protease capable of stimulating T cells of subjects allergic to Ves v 4. In another specific embodiment, the description provides polypeptide fragments containing one or more T cell epitopes and one or more B cell epitopes of the *Vespula* venom protease. In a preferred embodiment, the polypeptide fragments are at least 50 amino acids in length. In still another specific embodiment, the description provides T cell epitope-containing peptides of at least 9 amino acids corresponding to a consecutive amino acid sequence within the *Vespula* venom protease wherein the peptides are capable of stimulating T cells of subjects allergic to Ves v 4. Such peptides are preferably immunomodulatory peptides as well in that they induce T cell anergy when administered to a subject allergic to the *Vespula* venom protease, or otherwise affect the immune response of the subject.

In another embodiment, the present description provides methods for identification and modification via site-directed mutagenesis of those amino acid residues involved in the interaction of the polypeptides of this invention with human IgE and IgG antibodies, including IgG4 antibodies. In specific embodiments, the present description provides methods for decreasing the allergenicity (IgE reactivity) of the polypeptides of this description in a structure-based approach via mutagenesis of IgE epitopes with limited impairment of the residual surface structure important for IgG and IgG, including IgG4, immunological responses. In a preferred embodiment, the allergenicity of the polypeptides of this description is reduced by at least 50% while at least 50% of IgG epitopes, including IgG4 epitopes, are maintained. In a more preferred embodiment, the allergenicity of the polypeptides of this description is reduced by at least 70% while at least 50% of IgG epitopes, including IgG4 epitopes, are maintained. In a most preferred embodiment allergenicity is reduced by at least 90% while at least 50% of IgG epitopes, including IgG4 epitopes, are maintained.

In another embodiment, the present description provides methods for modification of N-glycosylation of the polypeptides of this description. In a specific embodiment, the present description provides polypeptides comprising N-linked glycosides without detectable core α(1,3)-fucosylation. In another specific embodiment, the present description provides polypeptides comprising mutated N-glycosylation sites instead of N-glycosylation sites with Asn-Xaa-Ser/Thr sequences. In still another specific embodiment, the present description provides polypeptides lacking N-glycosylation.

The present description further provides expression vectors comprising the nucleic acids of the invention operationally associated with a promoter. The present description also provides methods for producing the polypeptides encoded by the nucleic acids of the invention. In particular, the present description provides for culturing cells transformed with an expression vector of the description so that the *Vespula* venom protease, fragments thereof, or derivatives or analogs thereof are expressed by the cells, and recovering these polypeptides so expressed from the culture.

In a further embodiment, the present description features a pharmaceutical composition that includes the Ves v 4 polypeptides of the description and a pharmaceutically acceptable carrier. In some embodiments, the pharmaceutical composition includes an additional polypeptide, e.g., a second, third, fourth, or more *Vespula* venom polypeptide or polypeptides. The additional *Vespula* venom polypeptides can include, e.g., the Ves v 1 polypeptide (phospholipase A1), the Ves v 2a polypeptide (hyaluronidase), the Ves v 2b polypeptide, the Ves v 3 polypeptide (dipeptidylpeptidase), the Ves v 5 polypeptide, glycosylated IgE-binding proteins, or analogs or derivatives thereof.

In a further embodiment, the present description features a pharmaceutical composition comprising Ves v 4 polypeptide fragments of the invention, preferably between 20-150 amino acids in length, wherein each fragment contains one or more B cell epitopes and one or more T cell epitopes, and a pharmaceutically acceptable carrier. In a preferred embodiment, the composition comprises a set of polypeptide fragments that map the total length of the Ves v 4 polypeptide. In some embodiments, the pharmaceutical composition includes polypeptide fragments derived from an additional polypeptide, e.g., a second, third, fourth, or more *Vespula* venom polypeptide or polypeptides. The additional *Vespula* venom polypeptides can include, e.g., the Ves v 1 polypeptide (phospholipase A1), the Ves v 2a polypeptide (hyaluronidase), the Ves v 2b polypeptide, the Ves v 3 polypeptide (dipeptidylpeptidase), the Ves v 5 polypeptide, glycosylated IgE-binding proteins, or analogs or derivatives thereof.

In a further embodiment, the present description features a pharmaceutical composition comprising T cell epitope containing peptides of at least 9 amino acids corresponding to a consecutive amino acid sequence within the *Vespula* venom protease (Ves v 4) wherein the peptides are capable of stimulating T cells of subjects allergic to Ves v 4. In a preferred embodiment, the composition comprises a set of T cell epitope-containing peptides capable of stimulating T cells of the great majority of subjects allergic to Ves v 4. In some embodiments, the pharmaceutical composition includes T cell epitope-containing peptides of at least 9 amino acids corresponding to a consecutive amino acid sequence within an additional polypeptide, e.g., a second, third, fourth, or more *Vespula* venom polypeptide or polypeptides. The additional *Vespula* venom polypeptides can include, e.g., the Ves v 1 polypeptide (phospholipase A1), the Ves v 2a polypeptide (hyaluronidase), the Ves v 2b polypeptide, the Ves v 3 polypeptide (dipeptidylpeptidase), the Ves v 5 polypeptide, glycosylated IgE-binding proteins, or analogs or derivatives thereof.

In another aspect, the present description features a method of modulating an immune response. The method includes administering a Ves v 4 polypeptide of the description or a set of polypeptide fragments thereof wherein each of the polypeptide fragments contains one or more T cell epitopes and one or more B cell epitopes of the *Vespula* venom protease, to a subject in need thereof in an amount sufficient to inhibit an immune reaction by the subject against the Ves v 4 polypeptide. If desired, one or more additional *Vespula* venom polypeptides, or sets of fragments thereof, may also be administered to the subject. The additional *Vespula* venom polypeptides can include, e.g., the Ves v 1 polypeptide (phospholipase A1), the Ves v 2a polypeptide (hyaluronidase), the Ves v 2b polypeptide, the Ves v 3 polypeptide (dipeptidylpeptidase), the Ves v 5 polypeptide, glycosylated IgE-binding proteins, or analogs or derivatives thereof.

In another aspect, the present description features a method of modulating an immune response by administering T cell epitope-containing peptides of at least 9 amino acids corresponding to a consecutive amino acid sequence within the *Vespula* venom protease (Ves v 4), to a

subject in need thereof in an amount sufficient to inhibit an immune reaction by the subject against the Ves v 4 polypeptide. If desired, T cell epitope-containing peptides of at least 9 amino acids corresponding to a consecutive amino acid sequence within one or more additional *Vespula* venom polypeptides may also be administered to the subject. The additional *Vespula* venom polypeptides can include, e.g., the Ves v 1 polypeptide (phospholipase A1), the Ves v 2a polypeptide (hyaluronidase), the Ves v 2b polypeptide, the Ves v 3 polypeptide (dipeptidylpeptidase), the Ves v 5 polypeptide, glycosylated IgE-binding proteins, or analogs or derivatives thereof.

In a further aspect, the present description provides methods for identifying an individual at risk for *Vespula* venom hypersensitivity. One method includes administering to the individual the Ves v 4 polypeptide, or a set of polypeptide fragments thereof, and measuring an immune response raised against the polypeptide or fragments thereof. A detectable immune response indicates that the individual is at risk for *Vespula* venom hypersensitivity. In preferred embodiments, the Ves v 4 polypeptide, or a set of polypeptide fragments thereof, is administered intradermally. Another method includes *in vitro* measurement of Ves v 4-specific serum IgE antibodies by a variety of techniques, e.g., radio-allergosorbent test (RAST), enzyme-linked immunosorbent assays (ELISA), immunoelectrophoresis, immunoblot, immunodotblotting, bead array technology, fluid phase systems, and by *in vitro* mediator release assays (MRA).

In a further aspect, the present description provides methods for evaluating the success of immunotherapeutical treatment of a subject allergic to Ves v 4 by *in vitro* measurement of the ratio of Ves v 4-specific serum IgE and IgG4 antibodies by a variety of techniques, e.g., radio-allergosorbent test (RAST), enzyme-linked immunosorbent assays (ELISA), immunoelectrophoresis, immunoblot, immunodotblotting, bead array technology, fluid phase systems, and by *in vitro* mediator release assays (MRA).

Also provided by the description is a kit that includes, in one or more containers, a Ves v 4 polypeptide of the invention, or polypeptide fragments thereof, one or more anti-Ves v 4 antibodies (e.g., monoclonal or polyclonal), or a combination of these polypeptides, polypeptide fragments, and antibodies.

Unless otherwise defined, all technical and scientific terms used herein have the same meaning as commonly understood by one of ordinary skill in the art to which this invention belongs. Although methods and materials similar or equivalent to those described herein can be used in practicing or testing the present invention, suitable methods and materials are described below.

Other features and advantages of the invention will be apparent from the following detailed description and claims.

### Brief Description of the Figures and Tables

Figure 1 shows the cloning and expression of Ves v 4, as explained in Example 1 (Figures 1A and 1B) and in Examples 2 and 3 (Figure 1C).
Figure 2 shows a multiple alignment of the protease sequences of Magnvesin, Pol d 4, Api m 7 and Bom p 4 with Ves v 4, as explained in Example 5.
Figure 3 shows the amino acid sequence of Ves v 4 with highlighted secondary structures as predicted in Example 6.
Figure 4 shows the detection of Ves v 4-MBP produced in E.coli with IgE from several sera of yellow jacket venom-allergic individuals, as explained in Example 10.
Figure 5 is a graphical overview of peptide fragments F1-F5 of Ves v 4 as explained in Example 7. The selection of fragments and position in the Ves v 4 molecule with the respective fragment length is given in Table 1.
Figure 6 is a graphical overview of peptide fragments F6-F12 of Ves v 4 as explained in Example 7. The selection of fragments and position in the Ves v 4 molecule with the respective fragment length is given in Table 1.
Figure 7 is a graphical overview of peptide fragments F13-F25 of Ves v 4 as explained in Example 7. The selection of fragments and position in the Ves v 4 molecule with the respective fragment length is given in Table 1.
Figure 8 shows a Southern Blot of the Ves v 4 fragment as a probe with the genomic DNA from *Vespula germanica,* as explained in Example 11.

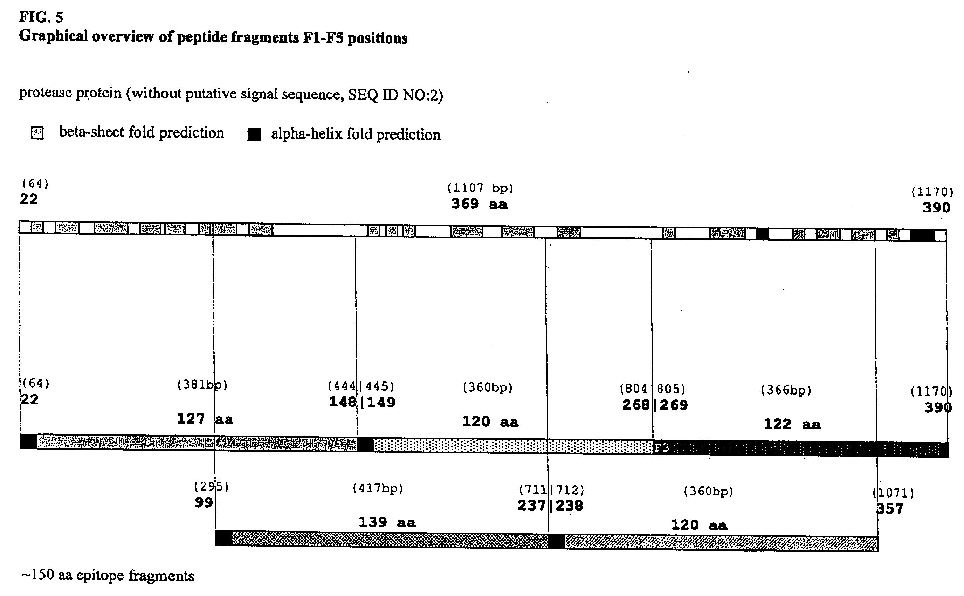

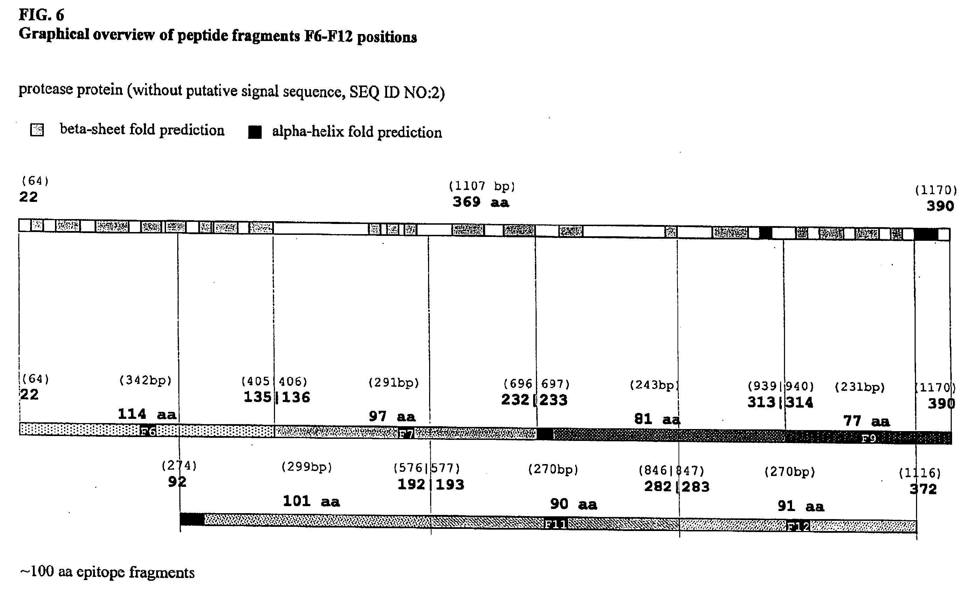

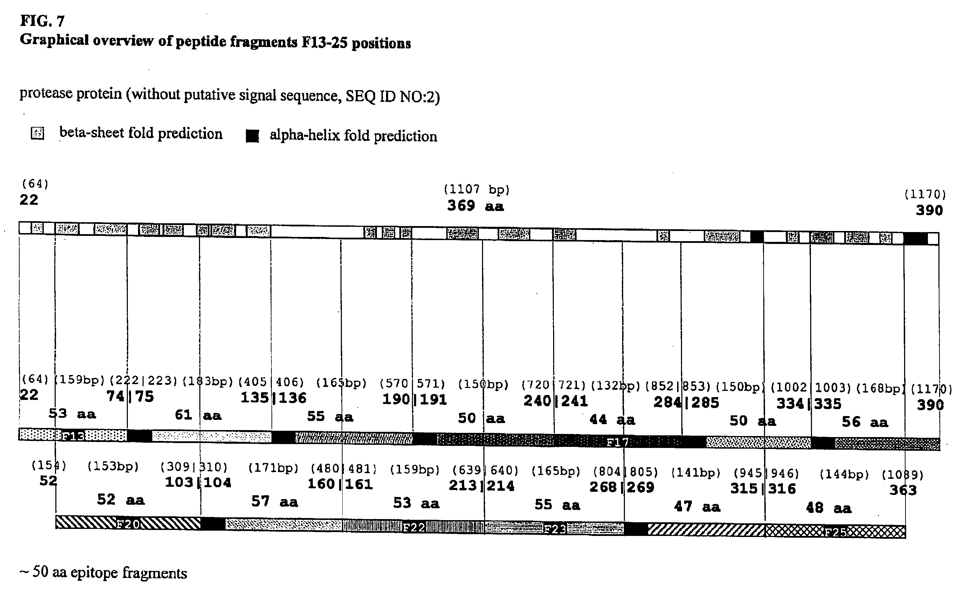

Table 1 shows the position of peptide fragments F1-F25 on the Ves v 4 poylpeptide and on the encoding Ves v 4 nucleic acid, as explained in Example 7 and as shown in Figures 5, 6 and 7.
Tables 2 and 3 show predicted T cell epitopes on the Ves v 4 poylpeptide and on the encoding Ves v 4 nucleic acid, as explained in Example 8.
Table 4 shows a calculation of putative surface IgE epitopes for a number of antigens per protein size ratio, as explained in section VII.
Table 5 shows a calculation of the average number of IgE epitopes on a number of allergens, as explained in section VII.
Table 6 shows a prediction of linear B cell epitopes on the Ves v 4 polypeptide, as explained in Example 9.

### Definitions

A "nucleic acid molecule" refers to the phosphate ester polymeric form of ribonucleosides (RNA molecules) or deoxy ribonucleosides (DNA molecules) in either single stranded form, or double stranded form. Double strande DNA-DNA, DNA-RNA, and RNA-RNA helices are possible. The term nucleic acid molecule refers to the primary and secondary structure of the molecule, but does not limit it to any particular tertiary forms. Thus, this term includes double stranded DNA found in linear or circular DNA molecules (e.g., restriction fragments), viruses, plasmids, and chromosomes. DNA sequences may be described herein according to the normal convention of giving only the sequence in the 5' to 3' direction along the nontranscribed strand of DNA (i.e., the strand having a sequence homologous to the mRNA). A recombinant DNA molecule is a DNA molecule that has undergone a molecular biological manipulation.

A DNA "coding sequence" is a double-stranded DNA sequence which is transcribed and translated into a polypeptide in vivo when placed under the control of appropriate regulatory sequences. The boundaries of the encoding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (e.g., mammalian) DNA, and even synthetic DNA sequences. If the coding sequence is intended for expression in a eukaryotic cell, a polyadenylation signal and transcription termination sequence will usually be located 3' to the coding sequence. Transcriptional and translational control sequences are DNA regulatory sequences, such as promoters, enhancers, terminators, and the like, that provide for the expression of a coding sequence in a host cell. A promotor sequence is a DNA regulatory region capable of binding RNA polymerase in a cell and initiating transcription of a downstream (3' direction) coding sequence. For purposes of the present invention, the promoter sequence is bounded at its 3' terminus by the transcription initiation site and extends upstream (5' direction) to include the minimum number of bases or elements to initiate transcription at levels detectable above background. Within the promoter sequence will be found a transcription initiation site, as well as protein binding domains (consensus sequences) responsible for the binding of RNA polymerase. Eukaryotic promoters will often, but not always, contain "TATA" boxes and "CAT" boxes. A coding sequence is under the control of transcriptional and translational control sequences in a cell when RNA polymerase transcribes the coding sequence into mRNA, which is then translated into the protein encoded by the coding sequence. A signal sequence can be included before the coding sequence. This sequence encodes a signal peptide, N-terminal to the polypeptide, that directs the host cell to transport the polypeptide to the cell surface or secrete the polypeptide into the media, and this signal peptide is usually selectively degraded by the cell upon exportation. Signal sequences can be found associated with a variety of proteins native to prokaryotes and eukaryotes. In accordance with the present invention there may be employed conventional molecular biology, microbiology, and recombinant DNA techniques within the skill of the art. Such techniques are explained fully in the literature.

As used herein, the term "coding region" refers to the nucleotide sequences that encode the amino acid sequences found in the nascent polypeptide as a result of translation of an mRNA molecule. The coding region is bounded in eukaryotes, on the 5' side by the nucleotide triplet "ATG" that encodes the initiator methionine and on the 3' side by one of the three triplets which specify stop codons *(i.e.,* TAA, TAG, and TGA). The term "encoding" or "encodes" refers to said "coding sequence" and said "coding region".

As used herein, the term "amino acid sequence" is recited herein to refer to an amino acid sequence of a protein molecule. The term "amino acid sequence" and like terms such as "polypeptide" or "protein" are not meant to limit the amino acid sequence to the complete, native amino acid sequence associated with the recited protein molecule. Rather the terms "amino acid sequence" and "protein" encompass partial sequences, fragments of the protein or polypeptide and modified sequences.

The term "wild type" refers to a nucleic acid molecule, a gene or gene product that has the characteristics of that nucleic acid molecule, gene or gene product when isolated from a naturally occurring source. A wild type gene is that which is most frequently observed in a population and is thus arbitrarily designated the "normal" or "wild-type" form of the gene.

In contrast, the terms "modified," "mutant", and "comprising a mutation" refer to a nucleic acid molecule, gene or gene product that displays modifications in sequence and or functional properties (*i.e*., altered characteristics) when compared to the wild-type nucleic acid molecule, gene or gene product. In some embodiments, the modification comprises at least one insertion, deletion, or substitution of one or more nucleotides or amino acids, respectively. A "mutation" in a nucleic acid sequence or gene can lead to an amino acid substitution in the corresponding amino acid sequence, wherein the amino acid substitution can be "conservative" or "non-conservative" or "random".

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophane), beta-branched side chains (e.g., threonine, valine, isoleucine), and aromatic side chains (e.g., tyrosine, phenylalanine, tryptophane, histidine). Thus, a predicted non-essential amino acid residue in a polypeptide according to the invention, preferably the Ves v 4 polypeptide, fragments, and analogs thereof is preferably replaced with another amino acid residue from the same side chain family.

In the context of the present invention, the term "nucleic acid homology" is equivalent to "nucleic acid identity". In the context of the present invention, the term "amino acid homology" is equivalent to "amino acid identity". The percent homology between two sequences is a function of the number of identical positions shared by the sequences (i.e., percent homology equals the number of identical positions divided by the total number of positions times 100). For comparison purposes the sequences are aligned, wherein gaps can be introduced in the sequence of a first amino acid sequence for optimal alignment with a second amino acid sequence.

As used herein, the term "vespid" is used according to the practice of those in the field of allergy, and refers to insects belonging to the worldwide familiy of Vespidae consisting of the Vespinae and Polistinae subfamilies. The Vespinae subfamily includes the three genera *Vespula* (called wasps in Europe, yellow jackets in the USA), *Dolichovespula* (called hornets in the USA) and *Vespa* (called hornets in Europe and the USA). Species in the genus *Polistes* (called paper wasps in Europe and the USA) include but are not limited to *P. annularis* (Linnaeus), *P*. *exclamans* (Viereck), *P. metricus* (Say), *P. fuscatus* (Fabricius), and *P. apachus* (Saussure). Species in the genus *Vespa* include but are not limited to *V. crabro* (L.) and *V. orientalis* (Linnaeus). Species in the genus *Dolichovespula* include but are not limited to *D. maculata* (L.) and *D. arenaria* (Fab.). Species in the genus *Vespula* include but are not limited to *V. germanica* (Fab.), *V. squamosa* (Drury), *V. maculifrons* (Buysson), *V. fiavopilosa* (Jacobson), *V*. *vulgaris* (L.), and *V. pensylvanica* (Saussure).

In the context of the present invention, the term "amino acid homology" is equivalent to "amino acid identity". The percent homology between two sequences is a function of the number of identical positions shared by the sequences (i.e., percent homology equals the number of identical positions divided by the total number of positions times 100). For comparison purposes the sequences are aligned, wherein gaps can be introduced in the sequence of a first amino acid sequence for optimal alignment with a second amino acid sequence.

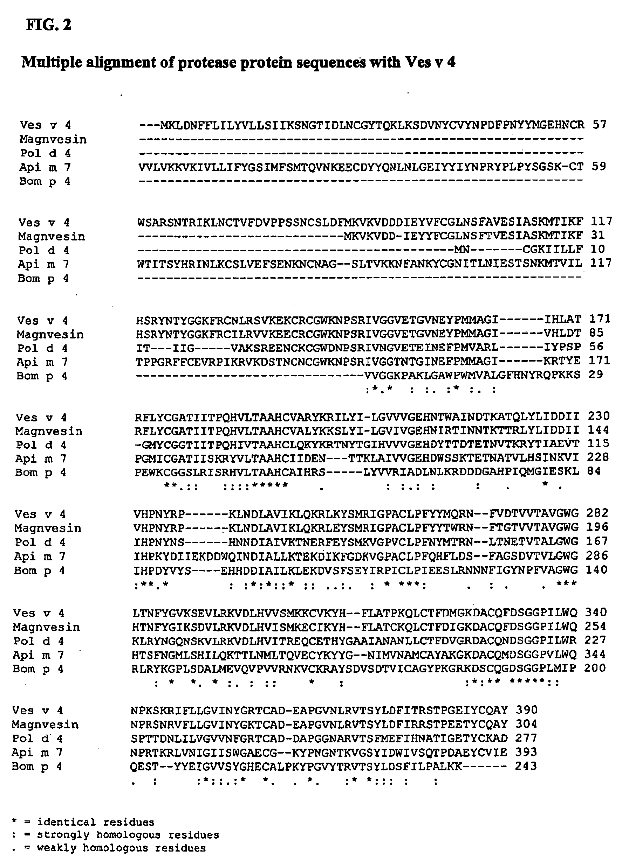

The term "homology" refers to a degree of complementarity. There may be partial homology or complete homology. One example of analyzing such homology is shown in Example 5 and in Figure 2.

A nucleic acid molecule is "hybridizable" to another acid molecule, such as cDNA, genomic DNA, or RNA, when a single stranded form of the nucleic acid molecule can anneal to the other nucleic acid molecule under the appropriate conditions of temperature and solution ionic strength. The conditions of temperature and solution ionic strength determine the "stringency" of the hybridization. Hybridization requires that the two nucleic acid molecules contain complementary sequences, although dependending on the stringency of the hybridization, mismatches between bases are possible. The appropriate stringency for hybridizing nucleic acids depends on the length of the nucleic acids and the degree of complementarity, variables well known in the art.

As used herein, the term "hybridization" is used in reference to the pairing of complementary nucleic acids. Hybridization and the strength of hybridization (*i*.*e*., the strength of the association between the nucleic acids) is impacted by such factors as the degree of complementarity between the nucleic acids, stringency of the conditions involved, the Tₘ ("melting temperature") of the formed hybrid, and the G:C ratio within the nucleic acids.

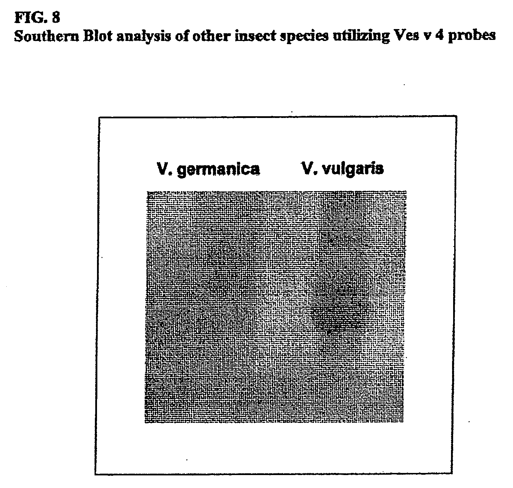

As used herein the term "stringency" is used in reference to the conditions of temperature, ionic strength, and the presence of other compounds such as organic solvents, under which nucleic acid hybridizations are conducted. The term "stringency" can refer to e.g. conditions in PCR (such as in e.g. Example 1) or to conditions in Southern Blotting (such as in e.g. Example 11 and in e.g. Figure 8). Those skilled in the art will recognize that "stringency" conditions may be altered by varying the parameters just described either individually or in concert.

With "high stringency" conditions, nucleic acid base pairing will occur only between nucleic acid fragments that have a high frequency of complementary base sequences *(e.g*., hybridization under "high stringency" conditions may occur between homologs with 85-100% identity, preferably 70-100% identity).

With moderate stringency conditions, nucleic acid base pairing will occur between nucleic acids with an intermediate frequency of complementary base sequences *(e.g*., hybridization under "moderate stringency" conditions may occur between homologs with 50-70% identity).

Thus, conditions of "weak" or "low" stringency are often required with nucleic acids that are derived from organisms that are genetically diverse, as the frequency of complementary sequences is usually less.

Standard stringency conditions in general PCR can not be defined because of the variety of different polymerase molecules that can be employed for the reaction, having different optimal temperature conditions and buffer compositions.

Standard reaction mix conditions for *Taq* (*Thermus aquaticus*) polymerase consist of: 10 mM Tris-HCl (pH 8.8 at 25 degree Celcius), 50 mM KCI, 0.08% Nonidet P40, 100 pM each primer, 200 µM each dNTP, 1.5 mM MgCl₂, 1.25 U polymerase and 100 pg template DNA.

Standard cycle conditions are: Denaturating step at 95 degree Celsius for 0.5-2 minutes. The temperature of the following annealing step is specified by the primers. The annealing time will be between 0.5-2 minutes. Each specific amplification primer exhibits an individual optimal hybridisation temperature based on oligonucleotide length and base sequence. Annealing temperature should be 5 degree Celsius lower than the calculated melting temperature (TM) of the primer with the lowest Tm. The extending step is performed at 72 degree Celsius temperature for 1 min each 1000 base pairs. The number of cycles will be between 25-35. Usually, a final extension step at 72 degree Celsius for 5-15 minutes is performed.

In general, stringency in PCR is related to the annealing temperature in relation to the theoretical Tm of the used primers and magnesium concentration in the buffer. The optimal stringency must be empirically determined, e.g. by temperature gradient experiments. Using shorter cycle times will result in the preferred amplification of smaller sequence fragments. Low stringency conditions can be defined by using standard buffer conditions and annealing temperatures below Tm-minus-5 degree Celsius. Standard stringency conditions will use standard buffer conditions and annealing temperatures between the theoretical Tm and Tm-minus-5 degree Celsius (Fermentas GmbH, St. Leon-Rot, Product Manual). High stringency conditions will use standard buffer conditions and annealing temperatures above the theoretical Tm, preferably 3 degree Celsius above Tm. If several calculation models for Tm exist, the accepted model with the highest resulting Tm should be used (Panjkovich and Melo 2005).

"High stringency conditions" when used in reference to nucleic acid hybridization in Southern Blotting comprise conditions equivalent to binding or hybridization at 42°C in a solution comprising 5X SSPE (43,8 g/l NaCl, 6,9 g/l NaH₂PO₄ H₂O and 1,85 g/l EDTA, pH adjusted to 7,4 with NaOH), 0.5% SDS, 5X Denhardt's reagent and 100 µg/ml denatured salmon sperm DNA followed by washing in a solution comprising 0,1X SSPE, 1,0% SDS at 42°C when a probe of about 100 to about 1000 nucleotides in length is employed.

"Moderate stringency conditions" when used in reference to nucleic acid hybridization in Southern Blotting comprise conditions equivalent to binding or hybridization at 42°C in a solution comprising 5X SSPE (43,8 g/l NaCl, 6,9 g/l NaH₂PO₄ H₂O and 1,85 g/l EDTA, pH adjusted to 7,4 with NaOH), 0,5% SDS, 5X Denhardt's reagent and 100 µg/ml denatured salmon sperm DNA followed by washing in a solution comprising 1,0X SSPE, 1,0% SDS at 42°C when a probe of about 100 to about 1000 nucleotides in length is employed.

"Low stringency conditions" comprise Southern Blotting conditions equivalent to binding or hybridization at 42°C in a solution comprising 5X SSPE (43,8 g/l NaCl, 6,9 g/l NaH₂PO₄ H₂O and 1,85 g/l EDTA, pH adjusted to 7,4 with NaOH), 0,1 % SDS, 5X Denhardt's reagent [50X Denhardt's contains per 500 ml: 5 g Ficoll (Type 400, Pharmacia), 5 g BSA (Fraction V; Sigma)] and 100 µg/ml denatured salmon sperm DNA followed by washing in a solution comprising 5X SSPE, 0,1% SDS at 42°C when a probe of about 100 to about 1000 nucleotides in length is employed.

Standard hybridization as done in e.g. Example 11 and in e.g. Figure 8 was carried out in SSC buffer (SSC = Saline Sodium Chloride). The standard hybridization solution was 5xSSC, 0,1% N-Lauroylsarcosine w/v (liquid), 0,02% SDS, wherein the addition of 50% formamide allows hybridization at 42-45 degree Celsius. A 2x SSC solution was 300mM NaCl, 30 mM Sodium Citrate, adjusted with HCl to pH 7,0. A standard wash was done with 2x SSC, 0,1% SDS.

For "low stringency conditions" the wash was done in 2x SSC, 0,1% SDS ("Low stringency wash").

For "moderate stringency conditions" the wash was done in 0,5x SSC, 0,1% SDS ("Moderate stringency wash").

For "increased stringency conditions" the wash was done in 0,1x SSC, 0,1% SDS ("Increased stringency wash").

For "high stringency conditions" the wash was done in 0,01x SSC, 0,1% SDS ("High stringency wash").

The term "Southern blot," refers to the analysis of DNA on agarose or acrylamide gels to fractionate the DNA according to size followed by transfer of the DNA from the gel to a solid support, such as nitrocellulose or a nylon membrane. The immobilized DNA is then probed with a labeled probe to detect DNA species complementary to the probe used. The DNA may be cleaved with restriction enzymes prior to electrophoresis. Following electrophoresis, the DNA may be partially depurinated and denatured prior to or during transfer to the solid support. Southern blots are a standard tool of molecular biologists (Sambrook *et al 1989*).

The term "recombinant DNA molecule" as used herein refers to a DNA molecule that is comprised of segments of DNA joined together by means of molecular biological techniques. Similarly, the

term "recombinant protein" or "recombinant polypeptide" or "recombinant antibody" as used herein refers to a protein molecule that is expressed from a recombinant DNA molecule.

The term "native protein" as used herein refers to a protein that does not contain amino acid residues encoded by vector sequences. That is the native protein contains only those amino acids found in the protein as it occurs in nature. A native protein may be produced by recombinant means or may be isolated from a naturally occurring source.

The term "fragment" when used in reference to a nucleotide sequence (as in "fragment of a given nucleotide sequence") refers to partial segments of that sequence. The fragments may range in size from 4 nucleotides to the entire nucleotide sequence minus one nucleotide (10 nucleotides, 20, 30, 40, 50, 100, 200, etc.).

Similarly, the term "fragment" when used in reference to a polypeptide sequence refers to partial segments of that sequence. In some embodiments, the fragment has an amino-terminal and/or carboxy-terminal deletion as compared to the native protein, but the remaining amino acid sequence is identical to the corresponding positions in the amino acid sequence deduced from a full-length cDNA sequence. Fragments are preferably at least 4 amino acids long, more preferably at least 15 amino acids long, more preferably at least 20 amino acids long and most preferably at least 50 amino acids long or longer (the entire amino acid sequence minus on amino acid).

In one embodiment, the invention provides polypeptide fragments of the Ves v 4 polypeptides of the invention as set forth in the claims containing one or more B cell epitopes of the *Vespula* venom protease. In another embodiment, the invention provides T cell epitope-containing polypeptide fragments of the *Vespula* venom protease as set forth in the claims capable of stimulating T cells of subjects allergic to Ves v 4. In a preferred embodiment, the invention provides polypeptide fragments as set forth in the claims containing one or more T cell epitopes and one or more B cell epitopes of the *Vespula* venom protease.

Fragments provide important advantages for the development of therapeutic approaches for the treatment of individuals allergic to Ves v 4. For example, recombinant fragments are easier to modify by site-directed mutagenesis than the full-length polypeptide, a fact that allows fast analysis of amino acid residues involved in the formation of B cell epitopes.

In a preferred embodiment, Ves v 4 polyptide fragments are selected on the basis of their immunological features and structural characteristics. The evaluation of immunological features includes, but is not limited to, analysis of the reactivity of IgE and IgG antibodies obtained from subjects allergic to venom of an insect from the familiy of Vespidae, with the polypeptide fragment, a predictive analysis of B cell epitopes, and a predictive analysis of T cell epitopes.

As used herein, the term "derivative or analog thereof" refers to any nucleic acid related to the nucleic acid shown in SEQ ID NO: 1 and to any polyeptide related to the polypeptide shown in SEQ ID NO: 2. In a derivative or analog according to the invention, the structure of the polypeptide can be modified to increase resistance of, for example, the Ves v 4 polypeptide and fragments thereof to proteolytic degradation in vivo. For this purpose, amino acid residues of a polypeptide of the invention, preferably the Ves v 4 polypeptide and fragments thereof, can be substituted by D-amino acids, non-natural amino acids or non-amino acid analogs, or such non-natural amino acids and analogs can be added to produce a modified peptide within the scope of the invention.

A "derivative" according to the invention can be selected from the group consisting of chimeric or fusion protein, a mutant comprising one or more amino acid substitutions, a mutant comprising one or more amino acid substitutions that increase resistance of the polypeptide to proteolytic degradation and a mutant comprising one or more amino acid substitutions that introduce one or more canonical protease sensitive sites, and wherein the chimeric or fusion protein preferably comprises a non-Ves v 4 polypeptide selected from the group consisting of poly-Histidine tag (His tag), glutathione-S-transferase (GST), maltose binding protein (MBP), a chitin binding domain (CBD), β-galactosidase, an IgG-Fc, a therapeutic polypeptide, preferably a cytokine, and other vespid venom proteins or fragments thereof.

The term "epitope" and "antigenic determinant" as used herein refer to that portion of an antigen that makes contact with a particular antibody and/or T cell receptor. The terms "epitope" and "antigenic determinant" can be used interchangeably. When a protein or fragment of a protein is used to immunize a host animal, numerous regions of the protein may induce the production of antibodies that bind specifically to a given region or three-dimensional structure on the protein; these regions or structures are referred to as "epitopes" or "antigenic determinants". Antigens, which are generally very large and complex, are not recognized in their entirety by lymphocytes. Instead, both B and T lymphocytes recognize discrete sites on the antigen called "epitopes" or antigenic determinants" via their B-cell receptors ("antibodies") and T-cell receptors, respectively. An epitope or antigenic determinant may compete with the intact antigen *(i.e*., the "immunogen" used to elicit the immune response) for binding to an antibody.

The term "B cell epitope" as used herein refers to an antigenic determinant (protein or carbohydrate) to which a single antibody molecule binds. B cell epitopes may comprise linear epitopes (amino acids adjacent to each other in the primary sequence) or conformational epitopes (moieties distant from each other in the primary sequence, but brought into close proximity of one another during folding of the antigen) of at least four amino acid residues.

The term "T cell epitope" as used herein refers to an antigenic determinant presented by a MHC class I or a MHC class II molecule, for binding by a single T cell receptor. T cell epitopes are linear epitopes comprising at least seven amino acid residues. In some embodiments of the present invention, the term T cell epitope comprises a T helper cell epitope, an antigen fragment presented by an MHC class II molecule for binding to T cell receptor on the surface of a helper T cell (*e.g*., generally CD4⁺).

In the context of the invention, the term "epitope recognized by human IgE (IgE epitope) or human IgG (IgG epitope), including human IgG4 (IgG4 epitope)", relates to the surface area of an allergen that is in contact to these antibodies upon binding to the allergen. It also relates to the surface area of the allergen that is in contact with an antibody construct comprised in the composition of the invention, that overlaps with the first-mentioned IgE epitope or IgG epitope, including IgG4 epitope", so binding of the antibody construct can inhibit binding of the human IgE or IgG, including IgG4; from the sera of patients allergic to the allergen (IgE related epitopes, IgG-related epitopes, IgG4 related epitopes). Preferably, the epitopes overlap by 20% or more, 50% or more, 60% or more, 70% or more, or 80% or more. Most preferably, the epitopes overlap by 90 or 95% or more or are identical. With reference to the number of epitopes of an allergen, the first-mentioned epitopes and the related epitopes are considered to represent the same epitopes.

The terms "antigen" and "antigenic" refer to any substance that is capable of inducing a specific humoral and/or cell-mediated immune response. An antigen generally contains at least one epitope. Antigens are exemplified by, but not restricted to molecules that contain a peptide, polysaccharide, nucleic acid sequence, and/or lipid. Complexes of peptides with lipids, polysaccharides, or with nucleic acid sequences are also contemplated, including (without limitation) glycopeptide, lipopeptide, glycolipid, *etc.* An "antigen" can be or can be present on an "allergen". "Antigens" can be the polypeptides of the invention, such as the Ves v 4 polypeptide, or fragments derivatives and analogs thereof.

As used herein, the term "allergy" refers to an inappropriate immune reaction in response to an antigen, wherein the terms "allergy" and "hypersensitivity" can be used interchangeably. There are four types of hypersensitive responses (Type I, II, III and IV), wherein IgE-mediated allergy or hypersensitivity is termed "Type I hypersensitivity". Immunoglobulin E (IgE) -mediated insect sting allergy to Hymenoptera is of common occurrence. The symptoms of IgE-mediated (type I) sting allergic reactions are due to release of mediators (e.g. histamine) resulting from cross-linking of effector cell-bound IgE antibodies by venom allergens. The symptoms can be suppressed by various pharmacologic treatments, but allergen-specific immunotherapy (SIT) represents the only curative approach. A rise in allergen-blocking IgG antibodies, particularly of the IgG4 class, a reduction in the number of mast cells and eosinophils, and a decreased release of mediators are known to be associated with successful SIT. Based on these observations, determination of the serum levels of allergen-specific IgE and IgG4 antibodies is useful to describe the immune status of an allergic patient.

As used herein, the term "allergen" describes an antigen that is capable of eliciting an allergic or hypersensitive reaction in a patient. Such an allergen can induce a humoral antibody response resulting in the generation of antibody-secreting plasma cells and memory cell, wherein the plasma cells secrete IgE. For example, an allergen can be present in the venom of the family Vespidae (vespids), such as in the venom of *Vespula vulgaris.* One example of such an allergen is the Ves v 4 polypetide. Other examples of allergens are shown in Tables 4 and 5.

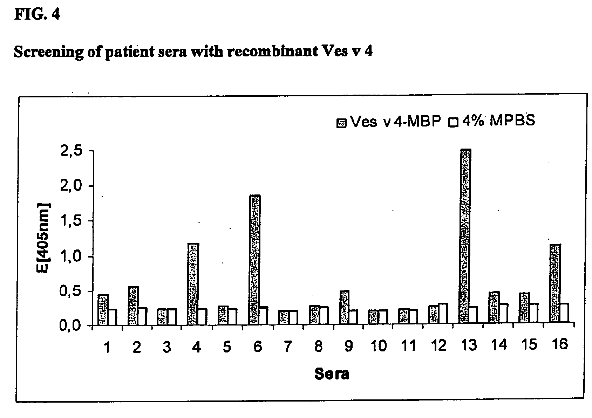

As used herein, the term "allergenicity" can be used interchangeably with "IgE reactivity", which can be described as the capability of a given antigen or allergen to bind to serum IgE in a patient. In specific embodiments, the present invention provides methods for decreasing the allergenicity (IgE reactivity) of the polypeptides of the invention in a structure-based approach via mutagenesis of IgE epitopes present on the polypeptide with limited impairment of the residual surface structure important for IgG immunological responses. In a preferred embodiment, the allergenicity of the polypeptides of the invention is reduced by at least 50% while at least 50% of IgG epitopes are maintained. In a more preferred embodiment, the allergenicity of the polypeptides of the invention is reduced by at least 70% while at least 50% of IgG epitopes are maintained. In a most preferred embodiment allergenicity is reduced by at least 90% while at least 50% of IgG epitopes are maintained. One Example of detecting or measuring "allergenicity" or "IgE reactivity", respectively is shown in Example 10 and in Figure 4. The "allergenicity" or "IgE reactivity" of a given allergen, such as an allergen from the venom of *Vespula vulgaris,* and such as the polypeptides of the invention, can be measured by standard ELISA techniques, e.g. as shown in Example 10 and in Figure 4.

The term "chimeric protein" or "fusion protein" as used herein refers to a protein formed by expression of a hybrid gene made by combining two gene sequences. Typically this is accomplished by cloning a cDNA into an expression vector in frame with an existing gene. The fusion partner may act as a reporter *(e.g.*, β-gal, β-galactosidase) or may provide a tool for isolation purposes *(e.g*., GST). The chimeric or fusion protein preferably comprises a non-Ves v 4 polypeptide selected from the group consisting of poly-Histidine tag (His tag), glutathione-S-transferase (GST), maltose binding protein (MBP), a chitin binding domain (CBD), β-galactosidase, an IgG-Fc, a therapeutic polypeptide, preferably a cytokine, and other vespid venom proteins or fragments thereof.

Suitable systems for production of recombinant proteins include but are not limited to prokaryotic *(e.g*., *Escherichia coli*), yeast *(e.g*., *Saccaromyces cerevisiae*), insect *(e.g*., baculovirus), mammalian *(e.g*., Chinese hamster ovary), plant *(e.g*., safflower), and cell-free systems *(e.g*., rabbit reticulocyte lysate).

As used herein, the term "T cell anergy" refers to a state of nonresponsiveness of a given T cell clone. T_{H}-cell recognition of an antigenic peptide-MHC complex can result in such state of nonresponsiveness, which is marked by the inability of the T cell to proliferate in response to the peptide-MHC complex. Such antigenic peptide capable of inducing T cell anergy can be an "immunomodulatory" peptide of the invention. The polypeptides or peptides of the invention are preferably "immunomodulatory" peptides in that they induce T-cell anergy when administered to a subject allergic to the Vespula venom protease Ves v 4, or otherwise affect the immune response of the subject.

As used herein, the term "vector" is used in reference to nucleic acid molecules that 15 transfer DNA segment(s) from one cell to another. The term "vehicle" is sometimes used interchangeably with "vector."

The term "expression vector" as used herein refers to a recombinant DNA molecule containing a desired coding sequence and appropriate nucleic acid sequences necessary for the expression of the operably or operationally linked coding sequence in a particular host organism. Nucleic acid sequences necessary for expression in prokaryotes usually include a promoter, an operator (optional), and a ribosome-binding site, often along with other sequences. Eukaryotic cells are known to utilize promoters, enhancers, and termination and polyadenylation signals.

As used herein, the term "host cell" refers to any eukaryotic or prokaryotic cell *(e.g*., bacterial cells such as *E. coli,* yeast cells, mammalian cells, avian cells, amphibian cells, plant cells, fish cells, and insect cells), whether located *in vitro* or *in vivo.* For example, host cells may be located in a cell culture or in a transgenic animal. The invention can make use of eukaryotic expression systems including yeast, mammalian, plant, and insect cells for the production of the polypeptides of the present invention. Mammalian cells are also suitable for the production of the polypeptides of the present invention, but do not produce high yields. In addition, both intact plants and cell lines are suitable for the production of the polypeptides of the present invention. The recombinant polypeptides can be expressed by either transgenic technology or by infection with chimeric virus. The standard method to introduce foreign genes into plants is the well characterized single-stranded RNA virus, tobacco mosaic virus. "Host cells" can be insect cells which are utilized for the production of large quantities of the polypeptides of the present invention. In a more preferred embodiment, the baculovirus system which provides all the advantages of higher eukaryotic organisms, is utilized. The host cells include, but are not limited to *Spodoptera frugiperda* ovarian cell lines SF9 and SF21 and the *Trichoplusia ni* egg-derived cell line High Five.

The term "transformation" as used herein refers to the introduction of foreign DNA into prokaryotic or eukaryotic cells. Transformation maybe accomplished by a variety of means known to the art including calcium phosphate-DNA co-precipitation, DEAE-dextran-mediated transfection, polybrene-mediated transfection, electroporation, microinjection, liposome fusion, lipofection, protoplast fusion, retroviral infection, and biolistics.

The term "antibody" refers to polyclonal and monoclonal antibodies. Polyclonal antibodies which are formed in the animal as the result of an immunological reaction against a protein of interest or a fragment thereof, can then be readily isolated from the blood using well-known methods and purified by column chromatography, for example. Monoclonal antibodies can also be prepared using known methods (*See*, Winter and Milstein 1991). As used herein, the term "antibody" encompasses recombinantly prepared, and modified antibodies and antigen-binding fragments thereof, such as chimeric antibodies, humanized antibodies, multifunctional antibodies, bispecific or oligo-specific antibodies, single-stranded antibodies and F(ab) or F(ab)₂ fragments.

The term "reactive" when used in reference to an antibody indicates that the antibody is capable of binding an antigen of interest. For example, a Ves v 4-reactive antibody may bind to one or more epitopes of the polypeptides of the invention, wherein such antibody can be a human IgE or human IgG antibody or a recombinant antibody.

The terms "peptide," "peptide sequence," "amino acid sequence," "polypeptide," and "polypeptide sequence" are used interchangeably herein to refer to at least two amino acids or amino acid analogs, which are covalently linked by a peptide bond or an analog of a peptide bond. The term "peptide" includes oligomers ("oligopeptides") and polymers ("polypeptides") of amino acids or amino acid analogs. The term "peptide" also includes molecules commonly referred to as peptides, which generally contain from about two (2) to about twenty (20) amino acids. The term "peptide" also includes molecules commonly referred to as polypeptides, which generally contain from about twenty (20) to about fifty amino acids (50). The term "peptide" also includes molecules commonly referred to as proteins, which generally contain from about fifty (50) to about three thousand (3000) amino acids. One example of a "polypeptide" or "protein" is the Ves v 4 polypetide, which is of a length of 390 amino acids, as can be seen in Figures 2 to 4 and 6 to 8 and in SEQ ID NO. 2. The amino acids of the peptide may be L-amino acids or D-amino acids. A peptide, polypeptide or protein may be synthetic, recombinant or naturally occurring. A synthetic peptide is a peptide produced by artificial means *in vitro.* A "polypeptide" according to the invention can be the Ves v 4 polypetide and fragments, derivatives and analogs thereof..

The terms "carrier" and "pharmaceutically acceptable carrier" as used herein refer to usually inactive accessory substances into which a pharmaceutical substance (e.g., a polypeptide of the invention, such as the Ves v 4 polypetide and fragments, derivatives and analogs thereof) is suspended. Exemplary carriers include liquid carriers (such as water, saline, culture, medium, saline, aqueous dextrose, and glycols) and solid carriers (such as carbohydrates exemplified by starch, glucose, lactose, sucrose, and dextrans, anti-oxidants exemplified by ascorbic acid and glutathione, and hydrolyzed proteins. As used herein, a "pharmaceutically acceptable carrier" is intended to include any and all solvents, dispersion media, coatings, antibacterial and antifungal agents, isotonic and adsorption delaying systems, and the like, compatible with the active compound and pharmaceutical administration. The use of such media and agents for pharmaceutically active substances is well known in the art. Supplementary active compounds can also be incorporated into the composition.

### DETAILED DESCRIPTION OF THE INVENTION

The invention provides a novel *Vespula* venom protease, termed Ves v 4 that has been identified as *Vespula* venom allergen based on its reactivity to IgE antibodies in sera of individuals who are allergic to *Vespula* venom.

### I. Nucleic acids encoding the Vespula venom protease of the present invention, and fragments thereof, derivatives and analogs thereof

In one embodiment, the present invention provides a nucleic acid molecule encoding a novel *Vespula* venom protease, and fragments, derivatives and analogs thereof, all of which are useful in the diagnosis and therapy of vespid venom-specific allergy. The sequence of the nucleic acid encoding the *Vespula* venom protease including 1173 nucleotides is shown in SEQ ID NO:1.

In a specific embodiment, to obtain a nucleic acid encoding the *Vespula* venom protease of the present invention, polymerase chain reaction (PCR) is combined with the rapid amplification of cDNA ends (RACE) techniques well known in the art. Preferably, the primers are based on the nucleic acid sequence for the *Vespula* venom protease of the present invention. Generally, such primers are prepared synthetically. Alternatives to isolating the genomic DNA or cDNA encoding the *Vespula* venom protease of the present invention include, but are not limited to, chemically synthesizing the gene sequence itself from the sequence provided herein. One example of obtaining a nucleic acid encoding the *Vespula* venom protease Ves v 4 is shown in Example 1.

In one embodiment the invention provides a nucleic acid molecule comprising the nucleotide sequence as shown in SEQ ID NO: 1. Further described are nucleic acids hybridizable to the nucleotide sequence as shown in SEQ ID NO: 1 under moderate or high stringency conditions, and fragments, derivatives, mutants and analogs thereof.

In one embodiment the invention provides a nucleic acid molecule comprising the nucleotide sequence as shown in SEQ ID NO: 1 and nucleic acids that are more than 90% identical to the nucleotide sequence as shown in SEQ ID NO: 1, or isolated fragments of the nucleic acid molecule comprising the nucleotide sequence as shown in SEQ ID NO: 1, wherein said fragments are between 150 and 1172 nucleotides in length.

In one embodiment the invention provides a nucleic acid molecule consisting of the nucleotide sequence as shown in SEQ ID NO: 1.

In one embodiment, the nucleotide sequence encodes the *Vespula* venom protease Ves v 4 or fragments, derivatives and analogs thereof.
In a further embodiment, the nucleotide sequence encodes polypeptide fragments as set forth in the claims comprising one or more B cell eptiopes of the *Vespula* venom protease Ves v 4, one or more T cell eptiopes of the *Vespula* venom protease Ves v 4 or one or more B cell eptiopes and one or more T cell eptiopes of the *Vespula* venom protease Ves v 4.

The description also provides a nucleic acid, which is a fragment having a length of more than 150 nucleotides of a nucleic acid encoding a polypeptide having a homology of at least 90%, to the amino acid sequence of SEQ ID NO:2, wherein the fragment encodes a polypeptide capable of binding to IgE or IgG from subjects allergic to venom of an insect from the familiy of Vespidae. Preferably, the nucleic acid is a fragment having a length of more than 150 nucleotides of a nucleic acid encoding a polypeptide having the amino acid sequence of SEQ ID NO: 2, wherein the polynucleotide is selected from the group consisting of nucleotides 64 to 222, 223 to 405, 406 to 570, 571 to 720, 721 to 852, 853 to 1002, and 1003 to 1170 of said nucleic acid, wherein the numbering corresponds to the region encoding said polypeptide. Specifically, said nucleic acid has the nucleotide sequence shown in SEQ ID NO: 1. More preferably, the nucleic acid is a fragment having a length of more than 228 nucleotides of a nucleic acid encoding a polypeptide having the amino acid sequence of SEQ ID NO: 2, wherein the polynucleotide is selected from the group consisting of nucleotides 64 to 405, 406 to 696, 697 to 939, and 940 to 1170 of said nucleic acid. Most preferably, the nucleic acid is a fragment having a length of more than 357 nucleotides of a nucleic acid encoding a polypeptide having the amino acid sequence of SEQ ID NO: 2, wherein the polynucleotide is selected from the group consisting of nucleotides 64 to 444, 445 to 804, and 805 to 1170 of said nucleic acid.

In another embodiment, the description provides additional fragments of the above mentioned nucleic acid, having a length of more than 150 nucleotides and encoding a polypeptide having a homology of more than 90% to the amino acid sequence of SEQ ID NO:2, wherein the fragment encodes a polypeptide capable of binding to IgE from subjects allergic to venom of an insect from the familiy of Vespidae, overlapping with fragments from the preceding embodiment. Preferably, the nucleic acid is a fragment having a length of more than 150 nucleotides of a nucleic acid encoding a polypeptide having the amino acid sequence of SEQ ID NO: 2, wherein the polynucleotide is selected from the group consisting of nucleotides 154 to 309, 310 to 480, 481 to 639, 640 to 804, 805 to 945, and 946 to 1089 of said nucleic acid, wherein the numbering corresponds to the region encoding said polypeptide. Specifically, said nucleic acid has the nucleotide sequence shown in SEQ ID NO: 1. More preferably, the nucleic acid is a fragment having a length of more than 267 nucleotides of a nucleic acid encoding a polypeptide having the amino acid sequence of SEQ ID NO: 2, wherein the polynucleotide is selected from the group consisting of nucleotides 274 to 576, 577 to 846, and 847 to 1116 of said nucleic acid. Most preferably, the nucleic acid is a fragment having a length of more than 357 nucleotides of a nucleic acid encoding a polypeptide having the amino acid sequence of SEQ ID NO: 2, wherein the polynucleotide is selected from the group consisting of nucleotides 295 to 711, and 712 to 1071 of said nucleic acid.

Furthermore a nucleic acid fragment (oligonucleotide) is described that comprises at least 39 contiguous nucleotides of the nucleic acid encoding an oligopeptide having a homology of more than 70% to the amino acid sequence of SEQ ID NO: 2, wherein the fragment encodes an oligopeptide capable of stimulating T cells of subjects allergic to Ves v 4. In a preferred embodiment, the fragment is an oligopeptide capable of stimulating T cells of the great majority of subjects allergic to Ves v 4. Preferably, the nucleic acid fragment encodes an oligopeptide having the amino acid sequence of SEQ ID NO: 2, wherein the oligonucleotide is selected from the group consisting of 45 contiguous nucleotides as defined in Tables 2 and 3 of said nucleic acid, wherein the numbering corresponds to the region encoding said polypeptide. Specifically, said nucleic acid has the nucleotide sequence shown in SEQ ID NO: 1.

In another embodiment, the nucleic acid can be modified by site-directed mutagenesis, wherein the modification preferably affects one or more of the native N-glycosylation sites. The description also provides a nucleic acid encoding the *Vespula* venom protease, or fragments thereof, which is modified by site-directed mutagenesis. In one specific embodiment, the present description provides a nucleic acid encoding a Ves v 4 polypeptide of the invention comprising at least one, preferably 2, or 3 mutated glycosylation sites instead of native N-glycosylation sites with Asn-Xaa-Ser/Thr sequences. Most preferably, all 4 putative N-glycosylation sites comprising nucleotides 205 to 213 of SEQ ID NO: 1 encoding the peptide sequence Asn-Cys-Thr, nucleotides 238 to 246 of SEQ ID NO:1 encoding the peptide sequence Asn-Cys-Ser, nucleotides 433 to 441 of SEQ ID NO:1 encoding the peptide sequence Asn-Pro-Ser, and nucleotides 646 to 654 of SEQ ID NO:1 encoding the peptide sequence Asn-Asp-Thr, are mutated. In one embodiment, the nucleotide sequence encoding serine residues of the N-glycosylation sites is exchanged by site-directed mutagenesis to a nucleotide sequence encoding alanine residues. In another embodiment, the nucleotide sequence encoding threonine residues of the N-glycosylation sites is exchanged by site-directed mutagenesis to a nucleotide sequence encoding valine residues. In a preferable embodiment, the nucleotide sequence encoding asparagine residues of the N-glycosylation sites is exchanged by site-directed mutagenesis to a nucleotide sequence encoding glutamine residues.

The nucleic acids of the description are for use as a medicament.

Described is a nucleic acid comprising or consisting of SEQ ID NO: 1 and fragments, derivatives, mutants and analogs thereof are for use as a medicament. In a more specific embodiment, the nucleic acids of the description are for use in the treatment of allergy against *Vespula* venom, preferably of allergy against the *Vespula* venom Ves v 4 protease.

Described is a nucleic acid comprising or consisting of SEQ ID NO: 1 and fragments, derivatives, mutants and analogs thereof that are for use in the treatment of allergy against *Vespula* venom, preferably of allergy against the *Vespula* venom Ves v 4 protease.

In another embodiment, the present invention provides a nucleic acid encoding a chimeric or fusion protein, as set forth in the claims wherein said chimeric or fusion protein preferably comprises one or more polypeptide(s) of the invention. In one embodiment, the chimeric or fusion protein is a Ves v 4 chimeric or fusion protein. As used herein, a Ves v 4 'chimeric protein' or 'fusion protein' comprises a Ves v 4 polypeptide of the invention operatively linked to a non-Ves v 4 polypeptide. A Ves v 4 polypeptide refers to a polypeptide having an amino acid sequence corresponding to Ves v 4 (*Vespula* venom protease) or fragments thereof, whereas a 'non-Ves v 4 polypeptide' refers to an oligopeptide or polypeptide having an amino acid sequence which is not substantially homologous to the Ves v 4 polypeptides of the invention, e.g. a polypeptide which is different from the Ves v 4 polypeptides and which is derived from the same or different organism. Within the fusion protein, the term 'operatively linked' is intended to indicate that the Ves v 4 polypeptide and the non-Ves v 4 polypeptide are fused in-frame to each other. The non-Ves v 4 polypeptide can be fused to the N-terminus or C-terminus of the Ves v 4 polypeptide. The non-Ves v 4 polypeptide can be selected from the group including, but not limited to a poly-Histidine tag (His tag), glutathione-S-transferase (GST), maltose binding protein (MBP), a chitin binding domain (CBD), β-galactosidase, and an IgG-Fc. In a preferred embodiment, non-Ves v 4 polypeptides that improve solubility of the poypeptides of the invention, e.g., GST or MBP, or simplify purification of the recombinant protein, e.g., a His tag, GST, MBP, CBD or IgG-Fc are employed. For such fusion protein a corresponding affinity column can be used for purification, e.g., a Ni²⁺ glutathione, maltose, or chitin affinity column. For purification of an IgG fusion protein, a protein A or protein G column is suitable. After purification of the protein the non-Ves v 4 polypeptide may be cleaved off chemically or by specific proteases.

Alternatively, it can be beneficial for therapeutic applications to express the polypeptide of the invention linked to a therapeutic polypeptide, e.g. a cytokine. For example, a fusion protein with a cytokine enhancing T_{H}1 and downregulating T_{H}2 responses or inducing class switch to IgG, such as IFN-γ, IL-10, IL-12 or TGF-β, can improve efficiency of desensitisation. If the expression vector is used for gene therapy, it is envisaged to use sequences rich in CpG (unmethylated cytosine guanidine dinucleotides), which promote T_{H}1 responses. Additionally or alternatively, the polypeptide of the invention can be linked to another polypeptide or protein, such as in the form of a fusion protein or as separate proteins expressed by the same vector. Preferably, the further polypeptides or proteins are other vespid venom proteins or fragments thereof.

### II. Identification of nucleic acid molecules from the family Vespidae hybridizable to the nucleic acid encoding the Vespula venom protease

In another embodiment, the present description provides methods for isolating nucleic acid molecules from any species of the family Vespidae which are hybridizable under moderate or high stringency conditions to a nucleic acid having the nucleotide sequence shown in SEQ ID NO:1.

For isolating a nucleic acid homologue of the the nucleic acid encoding the *Vespula* venom protease of the present description, one can choose to synthesize several different degenerate primers for use, e.g., in PCR reactions. It is also possible to vary the stringency of hybridization conditions used in priming PCR reactions, to allow for greater or lesser degrees of nucleotide sequence similarity between a homologue of a vespid venom enzyme and the *Vespula* venom protease of the present invention. After successful amplification of a segment of a homologue, that segment may be cloned and sequenced, and utilized as probe to isolate the complete cDNA or genomic clone. This, in turn, will permit the determination of the complete nucleotide sequence, the analysis of its expression, and the production of its protein product for functional analysis, as described herein. In this fashion, additional genes encoding vespid venom enzymes homologous to the *Vespula* venom protease of the present invention, may be identified and expressed.

In another embodiment, genes encoding vespid venom enzymes homologous to the *Vespula* venom protease of the present description can be isolated from a suitable library by screening with a probe. Useful probes for isolating such genes can be generated from the sequence information provided herein. A suitable library can be constructed by methods known in the art. Preferably, a cDNA library is prepared from cells or tissues that express vespid venom proteins, i.e, cells from the poison gland located near the venom sac. Sometimes, the poison gland is referred to as the acid gland. For example, mRNA or total RNA can be isolated, cDNA is prepared and ligated into an expression vector (e.g., a plasmid or bacteriophage derivative) such that it is capable of being expressed by the host cell into which it is then introduced. Various screening assays can be used then to select for the positive clones. For example, PCR with appropriate primers which can be synthesized based on the nucleic acid sequences provided herein, can be used. PCR is preferred as the amplified sequences can be detected easily, e.g., by ethidium bromide staining. Alternatively, labelled probes derived from the nucleic acid sequences provided herein can be used to screen the colonies. Alternatively, genes encoding vespid venom enzymes homologous to the *Vespula* venom protease of the present invention may be detected by assays based on the physical, chemical, or immunological properties of its expressed product. For example, cDNA clones, or DNA clones which hybrid-select the proper mRNAs, can be selected which produce a protein that, e.g., has similar or identical electrophoretic migration, isoelectric focussing behavior, proteolytic digestion maps, or antigenic properties as known for the *Vespula* venom protease of the present invention. In cases where the recombinant proteins are immunoreactive, e.g., reactive with IgE antibodies from sera of individuals allergic to vespid venom proteins, it is possible to select for positive clones by immunoblot. In another embodiment, the specific catalytic activity of proteases can be used for selection, although bacterially expressed eukaryotic proteins may not fold in an active conformation. Generally, according to the present description any method of screening for positive clones can be used.

One embodiment of isolating nucleic acid molecules from any species of the family Vespidae which are hybridizable under moderate or high stringency conditions to a nucleic acid having the nucleotide sequence shown in SEQ ID NO:1 is shown in Example 11 and in Figure 8.

Southern Blotting of a nucleic acid molecule of the invention, such as a Ves v 4 encoding nucleic acid fragment as a probe, with genomic DNA of another species of the family Vespidae as shown in Example 11 and in Figure 8 can be used to identify nucleic acid molecules from said family that are hybridisable to said nucleic acid molecule of the invention, such as the Ves v 4 encoding nucleic acid fragment.

### III. Expression of the Vespula venom protease, fragments thereof, or derivatives thereof

A large number of vector-host systems known in the art may be used. Possible vectors include, but are not limited to, plasmids or modified viruses, but the vector system must be compatible with the host cell used. Potential host-vector sytems include, but are not limited to mammalian cell systems infected with virus (e.g., vaccinia virus, adenovirus, etc.), insect cell systems infected with virus (e.g.; baculovirus), microorganisms such as yeast containing yeast vectors, or bacteria transformed with bacteriophages (e.g., lambda derivatives), DNA, plasmid DNA (e.g., various pBR322 derivatives), or cosmid DNA. The vector can be an expression vector. The expression elements of vectors vary in their strenghts and specificities. Depending on the host-vector system utilized, any one of a number of suitable transcription and translation elements may be used. In one embodiment, the vector comprises one or more nucleic acid molecule(s) of the invention, wherein the one or more nucleic acid molecule(s) are operationally associated with a promoter.

In an alternative embodiment, a recombinant *Vespula* venom protease of the present invention, or a fragment, derivative or analogue thereof is expressed chromosomally, after integration after the coding sequence by recombination. In this regard, any of a number of amplification systems may be used to achieve high levels of stable gene expression.

The present invention further relates to a host cell comprising said expression vector. This host cell includes, but is not limited to a bacterial cell, a yeast cell, a mammalian cell, an insect cell, or a plant cell. In one embodiment, the host cell comprises a vector comprising one or more nucleic acid molecule(s) of the invention.

Alternatively, a cell-free expression system may be used. The development and optimization of specific methods as well as approaches to overcome a variety of problems associated with any of these expression systems have been published in many articles (for a review, see Schmidt and Hoffman 2002).

In a one embodiment, the description provides a method for producing the polypeptides of the invention comprising the steps of a) transforming cells with a vector comprising one or more polypeptide(s) of the invention or fragment, derivative and analog thereof to give a transformed host cell, b) culturing said transformed host cells so that the polypeptide(s), fragment, derivative or analog thereof is expressed, and c) recovering the expressed polypeptide(s), fragment, derivative and analog from the culture.

In one embodiment, bacterial expression systems (e.g *E. coli*) are utilized for the production of large quantities of the polypeptides of the present invention. To avoid potential misfolding and segregation of said polypeptides into insoluble aggregates as inclusion bodies upon overproduction, an expression system that secretes the polypeptides into the periplasmatic space can be used to improve solubility of said polypeptides. The periplasmatic space is an oxidizing environment that contains enzymes catalyzing disulfide bonds. Co-overproduction of protein disulfide isomerases may be applied to enhance disulfide bond formation. Other proper folding factors such as peptidyl-prolyl-*cis*/*trans* isomerise also have been demonstrated to be helpful in improving solubility. To avoid potential problems in translation, arginine codons AGA and AGG which are rare and inefficiently translated in prokaryotes, can be replaced by site-directed mutagenesis. Alternatively, special strains of E. coli with extra copies of the necessary tRNA genes suitable to circumvent this problem can be selected.

In another embodiment, eukaryotic expression systems including yeast, mammalian, plant, and insect cells are for the production of the polypeptides of the present invention. Eukaryotic cells offer the advantage of being able to perform posttranslational modifications including processing signal sequences, folding, disulfide bond formation, and glycosylation. Among the many types of yeast the methylotrophic *Pichia pastoris* is capable to produce recombinant proteins in high yield and to add both O- and N-linked carbohydrates. However, as a lower eukaryote it adds only mannose residues, but hyperglycosylation as observed in *Saccharomyces cerevisiae* occurs less frequently. Mammalian cells are also suitable for the production of the polypeptides of the present invention, but do not produce high yields. In addition, both intact plants and cell lines are suitable for the production of the polypeptides of the present invention. The recombinant polypeptides can be expressed by either transgenic technology or by infection with chimeric virus. The standard method to introduce foreign genes into plants is the well characterized single-stranded RNA virus, tobacco mosaic virus. In a preferred embodiment, insect cells are utilized for the production of large quantities of the polypeptides of the present invention. In a more preferred embodiment, the baculovirus system which provides all the advantages of higher eukaryotic organisms, is utilized. The host cells include, but are not limited to *Spodoptera frugiperda* ovarian cell lines SF9 and SF21 and the *Trichoplusia ni* egg-derived cell line High Five.

### IV. Ves v 4 polypeptide and fragments thereof

In one embodiment, the invention provides a polypeptide encoded by a nucleic acid comprising the sequence shown in SEQ ID NO: 1 and fragments, derivatives and analogs thereof as set forth in the claims.

In a further embodiment, the invention provides a polypeptide encoded by a nucleic acid consisting of the sequence shown in SEQ ID NO: 1. In the context of the present invention, the terms 'polypeptide' and 'protein' are used interchangeably, without any limitation as to the number of amino acids linked. The polypeptides may also comprise non-naturally occuring amino acids.

In a preferred embodiment, the polypeptide is a full length protease from the venom of an insect from the family Vespidae.

In one embodiment, the polypeptide consists of the amino acid sequence shown in SEQ ID NO:2.

In a further embodiment, the polypeptide of the invention comprises the amino acid sequence as shown in SEQ ID NO:2 and polypeptides that are at least 90% identical, more preferably more than 95% identical and most preferably more than 99% identical to the amino sequence as shown in SEQ ID NO: 2, and fragments, derivatives and analogs thereof as set forth in the claims.

In one embodiment, the polypeptide has an homology of more than 90%, more than 95% or more than 99% to the amino acid sequence of SEQ ID NO: 2. Most preferred is a polypeptide having the amino acid sequence of SEQ ID NO: 2. The Ves v 4 polypeptide having the amino acid sequence of SEQ ID NO: 2 has a predicted molecular weight of 44.4 kDa.

Homology analyses revealed that the amino acid sequence of the Ves v 4 polypeptide differs significantly from amino acid sequences of other insect venom proteases. In the family Apidae consisting of the genera *Apis* (including the species *Apis mellifera,* honeybee) and *Bombus* (bumblebees), the venom serine protease Bom p 4 of *Bombus pennsylvanicus* with a molecular weight of 27 kDa (Hoffman and Jacobson 1996) shares an amino acid homology of only 18% with Ves v 4, and the venom protease Api m 7 of *Apis mellifera* with a molecular weight of 39 kDa (Winningham et al 2004) shares an amino acid homology of 35% with Ves v 4. In the subfamily Polistinae of the family Vespidae (vespids), the venom serine protease Pol p 4 of *Polistes dominulus* with a molecular weight of 27 kDa (Winningham et al 2004) shares an amino acid homology of 35% with Ves v 4. In the subfamily Vespinae of the family Vespidae including the three genera *Vespula, Dolichovespula,* and *Vespa,* the venom serine protease magnvesin of *Vespa magnifica* with a molecular weight of 27.4 kDa (Han et al 2008) shares an amino acid homology of 69% with Ves v 4. From all known insect venom proteases magnevesin shares the highest amino acid homology with Ves v 4. However, both venom proteases exhibit significant differences. The cDNA of magnvesin cloned from the venom sac cDNA library of *Vespa magnifica* encodes a protein precursor of 305 amino acids and a mature protease of 242 amino acids, whereas the Ves v 4 polypeptide comprises 369 amino acid residues due to an additional N-terminal CUB domain. This domain mediates protein-protein interactions during development and in interacting protein cascades like the classical complement pathway (Bork and Beckmann 1993).

In one embodiment, the invention provides polypeptide fragments of the Ves v 4 polypeptides of the invention containing one or more B cell epitopes of the *Vespula* venom protease Ves v 4. In another embodiment, the invention provides T cell epitope-containing polypeptide fragments of the *Vespula* venom protease Ves v 4 capable of stimulating T cells of subjects allergic to Ves v 4. In a preferred embodiment, the invention provides polypeptide fragments containing one or more T cell epitopes and one or more B cell epitopes of the *Vespula* venom protease Ves v 4.

In one embodiment, the polypeptide according to the invention is the *Vespula* venom protease Ves v 4 and fragments, derivatives and analogs thereof, wherein the polypeptide preferably is capable of binding to IgE or IgG from subjects allergic to venom of an insect from the family of Vespidae.

In one embodiment, the polypeptide according to the description comprises one or more B cell eptiopes of the *Vespula* venom protease Ves v 4, one or more T cell eptiopes of the *Vespula* venom protease Ves v 4 or one or more B cell eptiopes and one or more T cell eptiopes of the *Vespula* venom protease Ves v 4.

Said T cell epitopes can be selected from the group of oligopeptides of 15 contiguous amino acids in length listed in Tables 2 and 3, wherein said T cell epitope is preferably encoded by the group of oligonucleotides listed in Tables 2 and 3, wherein the numbering corresponds to the numbering as defined in SEQ ID NO: 1 and SEQ ID NO: 2, respectively.

Said B cell epitopes can be selected from the group of amino acids and oligopeptides listed in Table 6, wherein the numbering corresponds to the numbering as defined in SEQ ID NO: 2. Such B cell epitopes can be determined as shown in Example 9.

In a further embodiment, the polypeptide preferably is capable of stimulating T cells of subjects allergic to Ves v 4 or of the great majority of subjects allergic to Ves v 4.

Fragments provide important advantages for the development of therapeutic approaches for the treatment of individuals allergic to Ves v 4. For example, recombinant fragments are easier to modify by site-directed mutagenesis than the full-length polypeptide, a fact that allows fast analysis of amino acid residues involved in the formation of B cell epitopes. In particular knowledge of conformational (discontinuous or topographic) epitopes recognized by IgE antibodies is important for the design of novel recombinant Ves v 4 molecules capable of mediating protection in specific immunotherapy with little or no risk of anaphylactic reactions.

In a preferred embodiment, the polypeptide fragments according to the description, preferably Ves v 4 polyptide fragments, are selected on the basis of their immunological features and structural characteristics. The evaluation of immunological features includes, but is not limited to, analysis of the reactivity of IgE and IgG antibodies obtained from subjects allergic to venom of an insect from the familiy of Vespidae, with the polypeptide fragment, a predictive analysis of B cell epitopes, and a predictive analysis of T cell epitopes. For the prediction of both linear and conformational B cell epitopes several programs are available (see, e.g.,

Zhang et al 2008). For the prediction of T cell epitopes an abundance of methods is available (see, e.g., Zhang et al., 2008; Korber et al 2006). In particular four methods including ARB, SMM_align, the method of Sturniolo which is also the basis of TEPITOPE, and a consensus approach have been identified as top performing ones (Zhang et al 2008). The evaluation of structural characteristics include, but is not limited to, analysis of secondary structures (see, e.g., Chou and Fasman 1974) and hydrophilicity-hydrophobicity profiles (see, e.g., Hopp and Woods 1981). Other methods of structural analysis including, but not limited to, X-ray crystallography, mass spectroscopy, and computer modelling may also be used.

In one embodiment of the invention, the polypeptide fragments are 50-150 amino acids, in length.In a specific embodiment, the invention provides a polypeptide fragment having a length of more than 50 amino acid residues of a polypeptide having the amino acid sequence as shown in SEQ ID NO: 2, wherein the polypeptide fragment is capable of binding to IgE or IgG from subjects allergic to Ves v 4, and capable of stimulating T cells of subjects allergic to Ves v 4.

Preferably, the polypeptide fragment has a length of more than 50 amino acid residues of a polypeptide having the amino acid sequence of SEQ ID NO: 2, wherein the polypeptide is selected from the group consisting of amino acid residues 22 to 74, 75 to 135, 136 to 190, 191 to 240, 241 to 284, 285 to 334, and 335 to 390 of said polypeptide, wherein the numbering corresponds to the region of said polypeptide having the sequence shown in SEQ ID NO: 2. More preferably, the polypeptide fragment has a length of more than 76 amino acid residues of a polypeptide having the amino acid sequence of SEQ ID NO: 2, wherein the polypeptide is selected from the group consisting of amino acid residues 22 to 135, 136 to 232,233 to 313, and 314 to 390 of said polypeptide. Most preferably, the polypeptide fragment has a length of more than 119 amino acid residues of a polypeptide having the amino acid sequence of SEQ ID NO: 2, wherein the polypeptide is selected from the group consisting of amino acid residues 22 to 148, 149 to 268, and 269 to 390 of said polypeptide.

Further described is a polypeptide fragment selected from the group of polypeptide fragments listed in Table 1, wherein the amino acid numbering is according to the amino acid numbering as shown in SEQ ID NO: 2. These polyeptide fragments are referred to as "epitope fragments".

Further described is a polypeptide fragment that is encoded by one of the nucleic acid fragments listed in Table 1, wherein the nucleic acid numbering is according to the nucleic acid numbering as shown in SEQ ID NO: 1. These polyeptide fragments are referred to as "epitope fragments".

In another embodiment, the invention provides additional polypeptide fragments overlapping with polypeptide fragments from the preceding embodiment, having a length of more than 50 amino acid residues of a polypeptide having the amino acid sequence as shown in SEQ ID NO: 2, wherein the polypeptide fragment is capable of binding to IgE or IgG from subjects allergic to Ves v 4, and capable of stimulating T cells of subjects allergic to Ves v 4.

Preferably, the polypeptide fragment described here has a length of more than 50 amino acid residues of a polypeptide having the amino acid sequence as shown in SEQ ID NO: 2, wherein the polypeptide is selected from the group consisting of amino acid residues 52 to 103, 104 to 160, 161 to 213, 214 to 268, 269 to 315, and 316 to 363 of said polypeptide, wherein the numbering corresponds to the region of said polypeptide having the sequence shown in SEQ ID NO: 2. More preferably, the polypeptide fragment has a length of more than 89 amino acid residues of a polypeptide having the amino acid sequence of SEQ ID NO: 2, wherein the polypeptide is selected from the group consisting of amino acid residues 92 to 192, 193 to 282, and 283 to 372 of said polypeptide. Most preferably, the polypeptide fragment has a length of more than 119 amino acid residues of a polypeptide having the amino acid sequence of SEQ ID NO: 2, wherein the polypeptide is selected from the group consisting of amino acid residues 99 to 237, and 238 to 357 of said polypeptide.

Described is a polypeptide domain of a polypeptide having a homology of at least 90%, to the amino acid sequence as shown in SEQ ID NO: 2, wherein the polypeptide fragment is capable of binding to IgE or IgG from subjects allergic to Ves v 4, and capable of stimulating T cells of subjects allergic to Ves v 4. Preferably, the polypeptide domain has a length of more than 110 amino acid residues of a polypeptide having the amino acid sequence of SEQ ID NO: 2, wherein the polypeptide is selected from the group consisting of amino acid residues 26 to 136 (CUB domain), and 146 to 379 (trypsin-like domain) of said polypeptide, wherein the numbering corresponds to the region of said polypeptide having the sequence shown in SEQ ID NO: 2.

Also described is a T-cell epitope-containing oligopeptides of at least 9 amino acids corresponding to a consecutive amino acid sequence within the *Vespula* venom protease Ves v 4, wherein the peptides are capable of stimulating T-cells of subjects allergic to Ves v 4. Such peptides are preferably immunomodulatory peptides as well in that they induce T-cell anergy when administered to a subject allergic to the *Vespula* venom protease Ves v 4, or otherwise affect the immune response of the subject. Preferably, the amino acid sequence of the T-cell epitope-containing oligopeptide corresponds to a consecutive amino acid sequence of a polypeptide comprising or consisting of the amino acid sequence as shown in SEQ ID NO: 2, wherein the T-cell epitope-containing oligopeptide is selected from the group consisting of 15 contiguous amino acid residues as defined in Tables 2 and 3 of said polypeptide, wherein the numbering corresponds to the region of said polypeptide.

In a further embodiment, the description provides a T cell epitope selected from the group of oligopeptides of 15 contiguous amino acids in length listed in Tables 2 and 3, wherein said T cell epitope is preferably encoded by the group of oligonucleotides listed in Tables 2 and 3, wherein the numbering corresponds to the numbering as defined in SEQ ID NO: 1 and SEQ ID NO: 2, respectively.

T-cell stimulating activity can be tested by culturing T-cells obtained from an individual sensitive to the Ves v 4 polypeptide, fragments, and analogs thereof described herein, with the Ves v 4 polypeptide, fragments, and analogs thereof, and determining the presence or absence of proliferation by the T-cells in response to the peptide as measured by, for example, uptake of tritriated thymidine. Stimulation indicies for responses by T-cells to peptides useful in methods of the invention can be calculated as the maximum counts per minute (cpm) taken up in response to the peptide divided by the cpm of the control medium. For example, a peptide derived from a protein allergen may have a stimulation index of about 2.0. As a result, a stimulation index of at least 2.0 is generally considered positive for purposes of defining peptides useful as immunotherapeutic agents. Preferred peptides have a stimulation index of at least 2.5, more preferably at least 3.5 and most preferably at least 5.0.

The description provides a B cell epitope selected from the group of amino acids and oligopeptides listed in Table 6, wherein the numbering corresponds to the numbering as defined in SEQ ID NO: 2. Such B cell epitopes can be determined as shown in Example 9.

### V. Modification of the N-glycosylation of the Ves v 4 polypeptide, fragments, and analogs thereof

Sequence analysis of Ves v 4 shows that the protein comprises 4 putative N-glycosylation sites of the sequence Asn-Xaa-Ser/Thr. Depending on the host cell producing the recombinant Ves v 4 polypeptide, the protein is glycosylated (e.g., after expression in insect, yeast, plant or mammalian cells) or non-glycosylated (after expression in bacterial cells). Glycosylation can have profound effects on the binding of specific antibodies. It was shown that carbohydrates can represent IgE epitopes and contribute to observed non-specific cross-reactivity of allergens, e.g., between bee and wasp proteins, due to similar features of the carbohydrate chains. About 30 to 40% of patients with insect venom allergy have IgE antibodies reacting with both honeybee and *Vespula* venom. Apart from true double sensitization, IgE against cross-reactive carbohydrate determinants (CCD) is the most frequent and often only cause for the multiple reactivity and, thereby, for false positive results in assays for the diagnosis of insect venom allergy (Petersen and Mundt 2001; Hancock et al. 2008). Recently, alpha(1,3)-fucosyl residues attached to the innermost GlcNAc of N-glycans of allergens have been identified as prime cross-reactive carbohydrate determinants for insect venom allergens (Hemmer et al. 2004).

In eukaryotes the glycosylation pattern can vary depending on the host cell used (for reviews, see Schmidt and Hoffman 2002) and can thus differ from the glycosylation pattern of natural Ves v 4 isolated from *Vespula* venom. Furthermore, eukaryotic expression systems exhibit drastic differences in their capability of core alpha(1,3)-fucosylation of N-glycan structures (for reviews, see Tomiya et al. 2004). For example, High Five insect cells are capable of modifying N-glycans by core alpha(1,3)-fucosylation. This core fucosylation involves an alpha(1,3)-fucose attachment to the innermost GlcNAc of an N-glycan that may or may not also include an alpha(1,6)-fucose linkage. In contrast, SF9 insect cells do not possess detectable alpha(1,3)-fucosyltransferase activity. N-glycans of recombinant proteins expressed in SF9 cells exhibit only alpha(1,6)-fucose linkages. The absence of core alpha(1,3)-fucosylated N-glycans has also been reported for Ea4 insect cells and the gypsy moth cell line Ld652Y. An alpha(1,3)-fucosyltransferase gene has been identified in the genome of *Drosophila melanogaster* Schneider 2 (S2) insect cells, but it is apparently not active in the S2 cell line. Core alpha(1,3)-fucosylation is also not present in mammalian cells.

In one embodiment, an insect cell expression system is selected which is capable of attaching N-glycans to the recombinant Ves v 4 polypeptide that are identical to the N-glycans of natural Ves v 4 isolated from *Vespula* venom.

In one embodiment, the N-glycosylation of the polypeptide has been modified, wherein the modified N-glycosylation preferably is selected from the group consisting of absence of detectable core alpha(1,3)-fucosylation, mutated N-glycosylation sites, and absence of N-glycosylation.

In a preferred embodiment, insect cell lines lacking detectable alpha(1,3)-fucosyltransferase activity are utilized for the production of the polypeptides that display absence of detectable core alpha(1,3)-fucosylation in order to minimize non-specific cross-reactivity in IgE antibodies in assays for the diagnosis of insect venom allergy.

Alternatively, insect cells with detectable alpha(1,3)-fucosyltransferase activity are utilized as expression system after inhibition of the alpha(1,3)-fucosyltransferase gene by inhibitory RNA or after introduction of a gene encoding an alpha(1,3)-fucosidase (for reviews, see Schmidt and Hoffman 2002). In vitro tests using alpha(1,3)-fucosylated plant glycoproteins (e.g., bromelain) are helpful in identifying sera containing CCD-specific IgE, although a positive result (occurring in 70 to 80% of all double-positive sera) does not reliably exclude true double-sensitization. Reciprocal in vitro inhibition including non-venom inhibitor protein rich in CCDs is the method of choice currently to discriminate between double-sensitization and cross-reactivity. In vitro diagnosis can be markedly improved when recombinant Ves v 4 lacking CCDs are used for testing. The present invention provides polypeptides, preferablyVes v 4 polypeptides, fragments, derivatives and analogs thereof, that lack alpha(1,3)-fucosyl residues and, therefore, are suitable to discriminate between double-sensitization and cross-reactivity.

In another preferred embodiment, the polypeptides of the description, preferably Ves v 4 polypeptides o and fragments thereof are expressed in bacterial cells. Such polypeptides and fragments thereof lack glycosylation. Utilization of the non-glycosylated Ves v 4 polypeptide, fragments, and analogs thereof for diagnostic procedures eliminates false positive results due to cross-reactive carbohydrate determinants (CCD) and can therefore be used to advantage in diagnostic applications.

In still another preferred embodiment, the polypeptides of the description comprise mutated N-glycosylation sites instead of native N-glycosylation sites. In one specific embodiment, the present invention provides a polypeptide, preferably a Ves v 4 polypeptide of the description, comprising at least one, preferably 2, or 3 mutated glycosylation sites instead of native N-glycosylation sites with Asn-Xaa-Ser/Thr sequences. Most preferably, all 4 putative N-glycosylation sites comprising amino acid residues 69-71 of SEQ ID NO: 2, amino acid residues 80-82 of SEQ ID NO: 2, amino acid residues 145-147 of SEQ ID NO: 2, and amino acid residues 216-218 of SEQ ID NO: 2, are mutated. In one embodiment, the serine (Ser) residue of the N-glycosylation site is exchanged by an alanine (Ala) residue. In another embodiment, the threonine (Thr) residue of the N-glycosylation sites is exchanged by a valine (Val) residue. In a preferred embodiment, the asparagine (Asn) residue of the N-glycosylation sites is exchanged by a glutamine (Gln) residue (Elbein 1991).

In a further embodiment, the description provides a method for modification of N-glycosylation of a Ves v 4 polypeptide comprising the steps of a) providing a nucleic acid molecule of the invention or a nucleic acid molecule encoding a polypeptide of the description, preferably a Ves v 4 polypeptide, or fragments, derivatives and analogs thereof, b) applying site-directed mutagenesis to one or more of the native glycosylation site(s) of said nucleic acid molecule to give a modified nucleic acid molecule encoding a polypeptide having modified N-glycosylation.

Said modified N-glycosylation can be selected from the group consisting of absence of detectable core alpha(1,3)-fucosylation, mutated N-glycosylation sites, and absence of N-glycosylation.

### VI. Derivatives of the Ves v 4 polypeptide, fragments, and analogs thereof

Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophane), beta-branched side chains (e.g., threonine, valine, isoleucine), and aromatic side chains (e.g., tyrosine, phenylalanine, tryptophane, histidine). Thus, a predicted non-essential amino acid residue in a polypeptide of the invention, preferably in the Ves v 4 polypeptide, fragments, and analogs thereof, is preferably replaced with another amino acid residue from the same side chain family. Alternatively, in another embodiment, mutations can be introduced randomly along all or part of a Ves v 4-coding sequence, to identify mutants that retain activity, including IgE binding capability.

To facilitate purification and potentially increase solubility of the Ves v 4 polypeptide, fragments, and analogs thereof, it is possible to add a suitable non-Ves v 4 polypeptide to the peptide backbone. A "non-Ves v 4 polypeptide" refers to an oligopeptide or polypeptide having an amino acid sequence which is not substantially homologous to the Ves v 4 polypeptides of the invention, e.g. a polypeptide which is different from the Ves v 4 polypeptides and which is derived from the same or different organism. The non-Ves v 4 polypeptide can be fused to the N-terminus or C-terminus of the Ves v 4 polypeptide. The non-Ves v 4 polypeptide can be selected from the group including, but not limited to a poly-Histidine tag (His tag), glutathione-S-transferase (GST), maltose binding protein (MBP), a chitin binding domain (CBD), P-galactosidase, and an IgG-Fc. In a preferred embodiment, non-Ves v 4 polypeptides that improve solubility of the polypeptides of the invention, e.g., GST or MBP, or simplify purification of the recombinant protein, e.g., a His tag, GST, MBP, CBD or IgG-Fc are employed. For such fusion protein a corresponding affinity column can be used for purification, e.g., a Ni²⁺ glutathione, maltose, or chitin affinity column. For purification of an IgG fusion protein, a protein A or protein G column is suitable. After purification of the protein the non-Ves v 4 polypeptide may be cleaved off chemically or by specific proteases. Specific endoprotease cleavage sites can be introduced, if desired, between the Ves v 4 polypeptide and the non-Ves v 4 polypeptide to facilitate isolation of Ves v 4 polypeptides free of irrelevant sequences. In addition, it may be necessary to increase the solubility of the Ves v 4 fragments and analogs thereof by not including hydrophobic regions. Furthermore, it is possible to increase the solubility of the Ves v 4 fragments and analogs thereof by modification with polyethylene glycol (PEG). Substitution of cysteine residues in Ves v 4 fragments and analogs thereof by alanine, or glutamic acid, or alternatively with serine or threonine is another example for increasing the solubility due to inhibition of potential oligomerization.

In one embodiment, the structure of is modified to increase resistance of Ves v 4 polypeptide, fragments and analogs thereof to proteolytic degradation in vivo. For this purpose, amino acid residues of a polypeptide of the invention, preferably the Ves v 4 polypeptide, fragments and analogs thereof, can be substituted by D-amino acids, non-natural amino acids or non-amino acid analogs, or such non-natural amino acids and analogs can be added to produce a modified peptide within the scope of this invention.

To potentially aid proper antigen processing of T-cell epitopes within polypeptides of the invention, preferably the Ves v 4 polypeptide, fragments and analogs thereof, canonical protease sensitive sites can be recombinantly or synthetically engineered between regions, each comprising at least one T-cell epitope. For example, charged amino acid pairs, such as KK or RR, can be introduced between T-cell epitope comprising regions.

Alternatively, it can be beneficial for therapeutic applications to express the polypeptide according to the invention, preferably the Ves v 4 polypeptide, fragments and analogs thereof, linked to a therapeutic polypeptide, e.g. a cytokine. For example, a fusion protein with a cytokine enhancing T_{H}1 and downregulating T_{H}2 responses or inducing class switch to IgG, such as IFNγ, IL-10, IL-12 or TGF-β can improve efficiency of desensitisation. If the expression vector is used for gene therapy, it is envisaged to use sequences rich in CpG (unmethylated cytosine guanidine dinucleotides), which promote T_{H}1 responses. Additionally or alternatively, the polypeptide of the invention, preferably the Ves v 4 polypeptide, fragments and analogs thereof, can be linked to another polypeptide or protein, such as in the form of a fusion protein or as separate proteins expressed by the same vector. Preferably, the further polypeptides or proteins are other vespid venom proteins or fragments thereof.

In a further embodiment of the description, the polypeptide, preferably the Ves v 4 polypeptide is a derivative selected from the group consisting of chimeric or fusion protein, a mutant comprising one or more amino acid substitutions, a mutant comprising one or more amino acid substitutions that increase resistance of the polypeptide to proteolytic degradation and a mutant comprising one or more amino acid substitutions that introduce one or more canonical protease sensitive sites.

In a more specific embodiment, the chimeric or fusion protein comprises a non-Ves v 4 polypeptide selected from the group consisting of poly-Histidine tag (His tag), glutathione-S-transferase (GST), maltose binding protein (MBP), a chitin binding domain (CBD), β-galactosidase, an IgG-Fc, a therapeutic polypeptide, preferably a cytokine, and other vespid venom proteins or fragments thereof.

### VII. Hypoallergenic derivatives of the Ves v 4 polypeptide, fragments, and analogs thereof

In one embodiment, the present description provides methods for identification and modification via site-directed mutagenesis of those amino acid residues involved in the interaction of the polypeptides of this invention with human IgE or IgG antibodies. In particular, the present description provides compositions comprising recombinant antibodies wherein each composition is capable of binding to all epitopes recognized by either human IgE or IgG antibodies, including IgG4 antibodies, a method of obtaining such a composition and the use of individual antibodies of such a composition as tools for the design of hypoallergenic molecules, preferably Ves v 4 molecules, for specific immunotherapy (SIT).

In a specific embodiment, antibody compositions capable of binding to all epitopes of the polypeptide of the description, preferably of the Ves v 4 polypeptide, fragments and analogs thereof that are recognized by human IgE antibodies, are utilized to identify and modify by site-directed mutagenesis those amino acid residues involved in the interaction with allergen-specific human IgE antibodies, thereby eliminating or decreasing the allergenicity of the Ves v 4 polypeptide, fragments and analogs thereof in a structure-based approach. By site-directed mutagenesis of amino acid residues essential for the allergen-IgE antibody interaction, IgE epitopes are eliminated with minimal impairment of the residual surface structure important for a non-IgE immunological response.

In another specific embodiment, antibody compositions capable of binding to all epitopes of the polypeptide of the invention, preferably of the Ves v 4 polypeptide that are recognized by human IgG antibodies, including IgG4 antibodies, are utilized to maintain those structures that mediate an appropriate non IgE response for a long lasting protection after specific immunotherapy (SIT). This rational is based on the recent observation that immune deviation towards T regulatory (Treg) cells is an essential step in successful SIT (for a review, see Jutel et al 2006). Treg cells are defined by their ability to produce high levels of IL- 10 and TGF-β and to suppress naive and memory T helper type 1 and 2 responses. There is now clear evidence that IL-10- and/or TGF-β-producing type 1 T regulatory cells are generated in humans during the early course of SIT. Since Treg cells have been shown to differentiate from naive T cells in the periphery upon encountering antigens present at high concentrations, it can be assumed that Treg cells are also induced by high and increasing doses of allergens. Most important is the fact that IL-10 and TGF-β suppress directly or indirectly effector cells of allergic inflammation such as basophils and mast cells, and induce IgG4 from B cells as non-inflammatory immunoglobulin isotypes and suppress IgE production. Based on these observations, antibody compositions capable of binding to all epitopes of the Ves v 4 polypeptide, fragments and analogs thereof that are recognized by human IgG and particularly by human IgG4 antibodies, are utilized to identify and maintain those amino acid residues involved in the interaction with allergen-specific human IgG antibodies.

In the context of the invention, the term "epitope recognized by human IgE (IgE epitope) or human IgG (IgG epitope), including human IgG4 (IgG4 epitope)", relates to the surface area of an allergen that is in contact to these antibodies upon binding to the allergen. It also relates to the surface area of the allergen that is in contact with an antibody construct comprised in the composition of the description, that overlaps with the first-mentioned IgE epitope or IgG epitope, including IgG4 epitope", so binding of the antibody construct can inhibit binding of the human IgE or IgG, including IgG4; from the sera of patients allergic to the allergen (IgE related epitopes, IgG-related epitopes, IgG4 related epitopes).

In one embodiment, the epitopes overlap by 20% or more, 50% or more, 60% or more, 70% or more, or 80% or more

In a further embodiment, the epitopes overlap by 90 or 95% or more or are identical. With reference to the number of epitopes of an allergen, the first-mentioned epitopes and the related epitopes are considered to represent the same epitopes.

For an estimation of the number of antibodies sufficient for binding to all epitopes recognized by human IgE or IgG antibodies, including IgG4 antibodies, on the Ves v 4 polypeptide, it is important to know the approximate number of possible B cell epitopes per allergen. Therefore, methods for estimating the number of B cell epitopes per allergen have been developed. These methods are based on the following parameters:
a) Calculation of the surface of structurally characterized allergens in A²: The solvent accessible surfaces of proteins can be calculated with the aid of POPS (parameter optimized surfaces) according to Fraternali and Cavallo (2002).
b) Surface area of B-cell epitopes in A²: At the moment, one co-crystallization of allergen and antibody is available only, namely for the allergen Bet v 1 and a murine allergen-specific Fab-fragment. The surface area of this discontinuous epitope is 931 A² (Mirza et al 2000). This correlates well with the area of other B cell epitopes (circa 2x3 nm).

In Table 4, the surface of allergens for which structural data is available in the protein data bank (PDB) was calculated with the aid of a molecule of water. Under the assumption that a B cell epitope takes up an area of 950 A², the maximal possible number of B cell epitopes for an allergen (without differentiation for IgE or IgG epitopes) was determined. The number calculated in this way is much too high, but can be considered to provide an upper limit for the number of necessary antibody constructs for an allergen. On the basis of this data, an approximate relation between molecular weight and potential B cell epitopes was calculated. The mean value of the upper limit for potential B cell epitopes is approx. 0.5 B cell epitopes per 1 kDa. The polypeptides of the invention, including Ves v4 polypeptides, fragments, derivatives and analogs thereof, including altered polypeptides or modified allergens, respectively, can comprise or can be crossreactive with the B cell epitopes shown in Table 4.

Table 5 summarizes allergens that have been examined for IgE binding structures with overlapping oligopeptides. Utilizing overlapping oligopeptides (e.g., decapeptides), more potential IgE epitopes are identified than exist in reality, as the majority of IgE epitopes are discontinuous epitopes composed of at least two different areas of the molecule brought together by folding. Different relevant allergens, such as Phospholipase A2 and the birch pollen allergens Bet v1, Bet v3 and Bet v4 exclusively have discontinuous epitopes (Valenta et al 1998). Since the identified linear epitopes probably only form part of these discontinuous epitopes, for estimation of the number of epitopes it is supposed that at least three linear IgE binding epitopes are, as partial structures, involved in forming a discontinuous IgE epitope. Therefore, the number of identified IgE binding peptides has been divided by three and related to the molecular weight of the allergen. A number of 0,06 to 0,19 epitopes per 1 kDa calculated on the basis of linear IgE binding peptides is preferred. The best estimation is possible on the basis of the number of 0,12 IgE epitopes per 1 kDa, which is possibly still too high but could be considered realistic. The preferred compositions correlate well with known data for Bet v 2 (17.4 kDa), which can be bound by three different monoclonal Fab fragments (without differentiating between IgG and IgE epitopes, Valenta et al 1998). Bet v 2 has at least two IgE epitopes, since it can induce *in vivo* cross-linking of surface bound IgE antibodies. The polypeptides of the invention, including Ves v 4 polypeptides, fragments, derivatives and analogs thereof, including altered polypeptides or modified allergens, respectively, can comprise or can be crossreactive with the IgE epitopes shown in Table 5.

The plurality of Ves v 4-specific monoclonal antibodies can be generated from different sources. Naturally occurring IgE antibodies represent ideal tools for structural analyses of IgE epitopes, but their availability is limited. Cloning and selecting allergen-specific IgE antibodies from the immune repertoire of peripheral blood mononuclear cells of allergic donors is extremely difficult. The low number of IgE-secreting B cells in the peripheral blood of allergic patients (MacKenzie and Dosch 1989) seriously hampers this approach for generating monoclonal IgE antibodies.

Cloning and selecting Ves v 4-specific IgG antibodies, including IgG4 antibodies, from the immune repertoire of peripheral mononuclear cells of allergic donors may be less difficult due to the significantly higher number of IgG-secreting B cells in the peripheral blood of allergic patients as compared to IgE-secreting B cells. Currently, however, the availability of human monoclonal allergen-specific IgG4 antibodies is limited.

Described is a composition comprising one or more antibodies capable of binding to one or more epitope(s) of the polypeptides of the invention, preferably to one or more epitope(s) of the Ves v 4 poylpeptides, fragments, analogs and derivatives thereof, wherein the one or more epitope(s) are recognized by human IgE or human IgG antibodies.
Semisynthetic or synthetic immunolibraries (e.g., scFv or Fab format) provide a high degree of variability and, therey, a valuable alternative for generating the required plurality of Ves v 4-specific monoclonal antibody fragments. However, newly generated immunolibraries derived from animals (mammalian species as well as avian species) after immunization with the Ves v 4 polypeptide or fragments thereof provide a significantly higher number of Ves v 4-specific variable antibody domains and, thereby, an increased probability for the selection of the required plurality of Ves v 4-specific high affinity monoclonal antibody fragments. In a preferred embodiment a combination of immunolibraries derived from avian and mammalian species after immunization with the Ves v 4 polypeptide or fragments thereof are used. The phylogenetic difference between avian and mammalian species provides access to a different antibody repertoire than the traditional mammalian antibodies. IgY antibodies recognize other epitopes than mammalian antibodies. Therefore, a combination of immunolibraries from avian and mammalian species provides a significant advantage for generating a plurality of Ves v 4-specific high affinity monoclonal antibodies. If it should - unexpectedly - be found that the combination of all antibodies capable of binding to the Ves v 4 polypeptide is not sufficient to effect essentially complete inhibition of binding of Ves v 4 to antibodies in a pool serum of patients allergic to said allergen or obtained from said sera, it is recommended to additionally use further antibodies from a different library. Methods for generating immunolibraries are known in the art (e.g., Steinberger et al 1996; Edwards et al 2002; Powers et al 2001; Boel et al 2000) The description provides a composition comprising one or more recombinant antibodies capable of binding to one or more epitope(s) of the polypeptides of the invention, preferably to one or more epitope(s) of the Ves v 4 poylpeptides, fragments, analogs and derivatives thereof, wherein the one or more epitope(s) are recognized by human IgE or human IgG antibodies.

Each antibody composition is obtainable by a method for generating a composition comprising recombinant antibodies, comprising steps of
a) generating a plurality of allergen-specific antibodies capable of binding to the Ves v 4 polypeptide,
b) combining all generated antibodies and testing whether essentially complete inhibition of binding of the Ves v 4 polypeptide to IgE and IgG antibodies, including IgG4 antibodies; in a pool serum of patients allergic to said allergen or obtained from said serum is achieved,
c) in case essentially complete inhibition is not achieved in step b), steps a) and b) are repeated;
d) in case essentially complete inhibition is achieved, the number of antibodies is reduced to the minimal number of antibodies sufficient for essentially complete inhibition, preferably by a method wherein
   i) groups of the antibodies obtained in step a) are generated, comprising different numbers and combinations of antibodies;
   ii) said groups are tested for essentially complete inhibition of binding of the Ves v 4 polypeptide to IgE and IgG antibodies, including IgG4 antibodies, in a pool serum of patients allergic to said allergen or obtained from said sera;
   iii) wherein, in case one or more group effects essentially complete inhibition in step ii), steps i) and ii) are repeated with subcombinations of the antibodies from said group or groups until the minimal number of antibodies in said group or groups is identified which effects essentially complete inhibition;
   iv) wherein, in case essentially complete inhibition is not achieved in step ii) or iii), steps i), ii) and iii) are repeated with different groups of antibodies;
and wherein the group identified in step d), iii) is said composition. It is preferred that the composition comprises the minimal number of antibodies necessary and sufficient for binding to all epitopes recognized by human IgE and IgG antibodies, including IgG4 antibodies; on the Ves v 4 polypeptide Additional antibodies may, however, be added.

In the method of the description, inhibition of binding of the the Ves v 4 polypeptide to IgE can be determined by incubating IgE antibodies in a pool serum of patients allergic to the the Ves v 4 polypeptide or antibodies obtained from said serum with human basophils after stripping of said basophils, and with or without preincubation of the Ves v 4 polypeptide with the recombinant antibodies or recombinant antibody fragments or, for comparison, antibodies in a pool serum of patients allergic to said allergen or obtained from said serum, contacting said basophils with said allergen, and detecting release of histamine.

Alternatively, inhibition can be determined by contacting anti IgE antibodies immobilized on a carrier with antibodies in a pool serum of patients allergic to the Ves v 4 polypeptide or obtained from said serum, and, with or without preincubation of labelled Ves v 4 polypeptide with the recombinant antibodies or recombinant antibody fragments or, for comparison, antibodies in a pool serum of patients allergic to said allergen or obtained from said serum, contacting the carrier with said allergen and detecting binding of the labelled Ves v 4 polypeptide to the carrier. For this purpose, the Ves v 4 polypeptide can be labelled with an enzyme, a radioisotope, biotin or a fluorescent marker.

In the method of the description, inhibition of binding of the Ves v 4 polypeptide to IgG can be determined by contacting anti IgG antibodies immobilized on a carrier with antibodies in a pool serum of patients allergic to the Ves v 4 polypeptide or obtained from said serum, and, with or without preincubation of labelled Ves v 4 polypeptide with the recombinant antibodies or recombinant antibody fragments or, for comparison, antibodies in a pool serum of patients allergic to said allergen or obtained from said serum, contacting the carrier with said allergen and detecting binding of the labelled Ves v 4 polypeptide to the carrier. For this purpose, the Ves v 4 polypeptide can be labelled with an enzyme, a radioisotope, biotin or a fluorescent marker.

In the method of the description, inhibition of binding of the Ves v 4 polypeptide to IgG4 can be determined by contacting anti IgG4 antibodies immobilized on a carrier with antibodies in a pool serum of patients allergic to the Ves v 4 polypeptide or obtained from said serum, and, with or without preincubation of labelled Ves v 4 polypeptide with the recombinant antibodies or recombinant antibody fragments or, for comparison, antibodies in a pool serum of patients allergic to said allergen or obtained from said serum, contacting the carrier with said allergen and detecting binding of the labelled Ves v 4 polypeptide to the carrier. For this purpose, the Ves v 4 polypeptide can be labelled with an enzyme, a radioisotope, biotin or a fluorescent marker.

The inhibition by recombinant antibodies or recombinant antibody fragments is considered essentially complete if it is comparable to the inhibition by IgE, IgG or IgG4 antibodies in a pool serum of patients allergic to the Ves v 4 polypeptide or obtained from said serum, i.e. if it varies from that inhibition by 20% or less, preferably by 10% or less, or most preferably, by 5% or less.

The pool serum used in the present description comprises serum from several patients allergic to the Ves v 4 polypeptide. Preferably, said pool serum comprises the antibodies from the sera of at least 5 patients, at least 10 patients or at least 15 patients allergic to said allergen. For IgE inhibition experiments, patients are preferred that are highly sensitized to the allergen. For IgG and IgG4 inhibition experiments, patients after successful SIT are preferred.

IgE antibodies can be obtained from the pool serum, e.g., by affinity chromatography using anti-human IgE antibodies. Preferably, IgG antibodies are removed from the pool serum, e.g. by pre-treatment with a protein A matrix, such as a protein A column. This step, however, is not essential, as sera of allergic patients in all probability only contain relatively low amounts of allergen-specific IgG antibodies, even though the serum level of IgG is about 10.000 times higher than the serum level of IgE. For example, serum obtained fom a birch pollen allergic patients which was purified by affinity chromatography on immobilized Bet v 1, did not contain significant quantities of allergen specific IgG antibodies (Ganglberger et al 2000). IgG4 antibodies can also be obtained from the pool serum by affinity chromatography using anti-human IgG4 antibodies.

The individual antibodies of a generated composition are used for structural analyses of IgE and IgG epitopes, including IgG4 epitopes. Since each composition effects essentially complete inhibition of binding of the Ves v 4-polypeptide to patient-derived IgE and IgG antibodies, including IgG4 antibodies, the individual antibodies of each generated composition are capable of identifying all epitopes on the Ves v 4 polypeptide that are accessible for patient-derived IgE and IgG antibodies, including IgG4 antibodies.

According to the present description the most potent Ves v 4-related hypoallergenic molecule for specificic immunotherapy is an allergen that does not exhibit allergenicity, contains an array of T cell epitopes that is comparable to that of the corresponding natural allergen, and displays a surface structure that is recognized by human IgG and particularly by human IgG4 antibodies with specificity for the corresponding natural allergen. For the design of such a molecule, the individual antibodies of the different antibody compositions are essential to maintain IgG epitopes upon modification of the IgE epitopes by a structure-based approach.

In a preferred embodiment, the description provides a polypeptide, wherein one or more IgE epitope(s) present on said polypeptide are altered to reduce the allergenicity of said polypeptide to give an altered polypeptide or modified allergen, respectively.

In a further embodiment, said one or more IgE epitope(s) present on said polypeptide are selected from the group of IgE epitopes listed in Table 4 and Table 5. The immunoreactivity, IgE-reactivity and allergenicity, respectively, of such altered polypeptide or modified allergen can be determined as shown in Figure 4 and Example 10.In specific embodiments, the present description provides methods for decreasing the allergenicity (IgE reactivity) of the polypeptides of this invention in a structure-based approach via mutagenesis of IgE epitopes with limited impairment of the residual surface structure important for IgG immunological responses. In a preferred embodiment, the allergenicity of the polypeptides of this invention is reduced by at least 50% while at least 50% of IgG epitopes are maintained. In a more preferred embodiment, the allergenicity of the polypeptides of this description is reduced by at least 70% while at least 50% of IgG epitopes are maintained. In a most preferred embodiment allergenicity is reduced by at least 90% while at least 50% of IgG epitopes are maintained.

In a further embodiment, the description provides a method for decreasing the allergenicity of a polypeptide of the invention, preferably of a Ves v 4 polypeptide, or fragments, derivatives and analogs thereof, comprising the steps of a) providing a nucleic acid molecule of the invention or a nucleic acid molecule encoding a polypeptide of the invention or fragments, derivatives and analogs thereof, b) applying site-directed mutagenesis to said nucleic acid molecule so that one or more IgE epitope(s) on the encoded polypeptide is altered to give a modified allergen, and c) determining the reduction in allergenicity of said modified allergen.

For example, IgE epitope(s) present on a given polypeptide or allergen can be determined as shown in Example 10 and in Figure 4. Such IgE epitope(s) can be selected from the group of IgE epitopes listed in Table 4 and Table 5.

The immunoreactivity, IgE-reactivity and allergenicity, respectively, of such altered polypeptide or modified allergen can be determined as shown in Figure 4 and Example 10.

In another embodiment, such altered peptides or modified allergens, respectively, can be used in the treatment of allergy or hypersensitivity, respectively, against the *Vespula* venom, preferably of allergy or hypersensitivity against the *Vespula* venom protease Ves v 4, wherein said altered peptides or modified allergens of the invention preferably cause less side effects as compared to the unaltered, wild-type polypeptide or allergen.

### VIII. Diagnostic use of the Ves v 4 polypeptide, fragments, derivatives and analogs thereof

As used herein, the term diagnostic includes *in vitro* and *in vivo* diagnostic assays. Generally, such assays are designed to measure the level of IgE antibodies specific for a given allergen. However, determining the level of allergen-specific antibodies of different isotypes including, but not limited to, antibodies of the IgG and IgA class can also be useful to evaluate the immune status of an allergic patient. For example, a rise in allergen-blocking IgG antibodies, particularly of the IgG4 class (Reid et al 1986), a reduction in the number of mast cells and eosinophils, and a decreased release of mediators (Varney et al 1993) were found to be associated with successful SIT. Therefore, determination of the serum levels of allergen-specific IgE and IgG4 antibodies is useful to analyse the immune status of an allergic patient.

In one embodiment, the description provides a method for detecting serum IgE or IgG antibodies specific for the polypeptides of the invention, preferably specific for the Ves v 4 polypeptide, or fragments, derivatives and analogs thereof, comprising the step of a) providing serum from an allergic patient, and b) detecting said IgE or IgG antibodies in said serum by a detection method, wherein said detection method is selected from the group consisiting of radio-allergosorbent test (RAST), enzyme-linked immunosorbent assays (ELISA), immunoelectrophoresis, immunoblot, immunodotblotting, bead array technology, fluid phase systems, and *in vitro* mediator release assays (MRA), and wherein the IgG antibodies preferably are IgG4 antibodies.

In a more specific embodiment, said detecting comprises *in vitro* measuring the ratio of serum IgE and IgG4 antibodies specific for the polypeptides of the invention, preferably specific for the Ves v 4 polypeptide, or fragments, derivatives and analogs thereof, and wherein said method preferably further comprises the step of c) evaluating the success of immunotherapeutical treatment.

Said immunotherapeutical treatment can be the use of the polypeptides of the invention, prefeably the Ves v 4 polypetides and fragments thereof as set forth in the claims for the generation of a medicament for the treatment of allergy against *Vespula* venom, preferably against the *Vespula* venom protease Ves v 4.

In vitro assays for the measurement of allergen-specific serum IgE antibodies or other isotypes such as IgG, and in particular IgG4, include, but are not limited to the radio-allergosorbent test (RAST), various enzyme-linked immunosorbent assays (ELISA) and other IgE-binding techniques such as immunoelectrophoresis, immunoblot, immunodotblotting, bead array technology and various fluid phase systems, and the like. As an alternative to the above listed assay systems, basophil cells derived from patients or from basophil cell lines such as the KU812 can be used for *the in vitro* measurement of allergen-specific IgE antibodies in serum by *in vitro* mediator release assays (MRA). The sensitivity and specificity of such assays depend on the availability of pure allergen. In one embodiment, the present description provides pure Ves v 4 polypeptide, fragments, derivatives and analogs thereof, that are suitable for determining the serum levels of allergen-specific IgE antibodies and other isotypes. The availability of pure Ves v 4 polypeptide, fragments, derivatives and analogs thereof, allows the measurement of serum IgE antibodies or other isotypes that bind specifically to Ves v 4 epitopes. Utilizing such a component resolved allergy test, assessment of sensitization of vespid venom allergic individuals towards Ves v 4 is possible, which in turn will have an impact on the potential immunotherapeutic treatment of these individuals.

In another embodiment, the present description provides in vitro diagnostic assays on peripheral blood lymphocytes useful for obtaining information on Ves v 4-specific T cell responses, the phenotype of the T cell response, and preferably the T cell epitope(s) of Ves v 4 involved in T cell responses. The immunodominant epitope(s) and the epitope(s) involved in IgE isotype class switch events can be detected, if they are not identical. In particular, the T cell epiotope(s) of Ves v 4 that stimulate proliferation and/or lymphokine secretion of T cells of a phenotype associated with IgE isotype class switching events can be identified for a specific individual, or for a class of individuals who share MHC haplotype or a predominant T cell receptor variable region expression, or both.

In vivo assays for vespid venom allergy generally consist of skin prick sensitivity assays, in which serially diluted amounts of allergen are administered either subcutaneously or intradermally into a patient's skin, and wheel and erythema reactions are detected. Such tests are well known in the art (Biló et al 2005). As with in vitro assays, the availability of pure Ves v 4 allergen greatly increases the value of the results of the in vivo diagnostic assays since cross-reactivity with impurities in extracts prepared from vespid venom sacs can be avoided.

In one embodiment, the description provides a method for identifying an individual at risk for *Vespula* venom hypersensitivity or allergy, respectively, comprising the step of detecting an immune response raised against one or more of the polypeptides of the invention, preferably against the Ves v 4 polypeptide or fragments, derivatives and analogs thereof as set forth in the claims.

In a further embodiment said method can be performed as a skin prick sensitivity assay, in which preferably serially diluted amounts of one or more polypeptide(s) of the invention (i.e. the "allergen") is administered preferably either subcutaneously or intradermally into a patient's skin, and preferably wheel and erythema reactions are detected.

In a preferred embodiment, said detection of said immune response is indicative for a risk for *Vespula* venom hypersensitivity. In another preferred embodiment, said polypeptides are for intradermal administration. In another preferred embodiment, said polypeptides are for subcutaneous administration.

In another preferred embodiment, said detection of an immune response is indicative for a risk for hypersensitivity or allergy, respectively, against the polypeptides of the invention, preferably against the Ves v 4 poylpeptide, or fragments, derivatives and analogs thereof.

In a further embodiment, the description provides a kit comprising the following components in one or more containers: a) one or more polypeptide(s) of the invention or fragments, derivatives and analogs thereof, and b) one or more antibodies against said polypeptides, fragments, derivatives and analogs thereof.

### IX. Ves v 4-based therapeutic methods

The polypeptides of the invention, and fragments derivatives and analogs thereof as set forth in the claims are for use as a medicament. In a specific embodiment, the Ves v 4 polypeptide, fragments, derivatives and analogs thereof as well as altered peptides and modified allergens are for use as a medicament.

In one embodiment, the Ves v 4 polypeptide, fragments, derivatives and analogs thereof of the invention are used in specific immunotherapy (SIT) of vespid venom allergy, also referred to as hyposensitization therapy of vespid venom allergy. SIT has proven effective in allergic diseases, particular insect venom allergy. Such therapy may be particularly effective when the allergen or allergens to which the patient is sensitive have been specifically identified and the therapy is targeted to that allergen or those allergens. Therefore, the availability of pure allergen in large quantities is important for specific immunotherapy of allergy.

In one embodiment, the present description features a method of modulating an immune response by administering the Ves v 4 polypeptide, a fragment, a derivative or an analog thereof to a mammal (such as a human) sensitive to Ves v 4 in a form which results in a decrease in the T cell response of the mammal upon subsequent exposure to the protein allergen. If desired, one or more additional *Vespula* venom polypeptides, or sets of fragments thereof, may also be administered to the subject. The additional *Vespula* venom polypeptides can include, e.g., the Ves v 1 polypeptide (phospholipase A1), the Ves v 2a polypeptide (hyaluronidase), the Ves v 2b polypeptide, the Ves v 3 polypeptide (dipeptidylpeptidase), the Ves v 5 polypeptide, glycosylated IgE-binding proteins, or analogs or derivatives thereof.

In a specific embodiment, the present description features a method of modulating an immune response by administering T cell epitope-containing peptides of at least 9 amino acids corresponding to a consecutive amino acid sequence within the *Vespula* venom protease (Ves v 4), to a subject in need thereof in an amount sufficient to inhibit an immune reaction by the subject against the Ves v 4 polypeptide. If desired, T cell epitope-containing peptides of at least 9 amino acids corresponding to a consecutive amino acid sequence within one or more additional *Vespula* venom polypeptides may also be administered to the subject. The additional *Vespula* venom polypeptides can include, e.g., the Ves v 1 polypeptide (phospholipase A1), the Ves v 2a polypeptide (hyaluronidase), the Ves v 2b polypeptide, the Ves v 3 polypeptide (dipeptidylpeptidase), the Ves v 5 polypeptide, glycosylated IgE-binding proteins, or analogs or derivatives thereof.

As used herein, a decrease or modification of the T cell response of a mammal sensitive to a protein allergen is defined as non-responsiveness or diminution in symptoms to the protein allergen in the mammal, as determined by standard clinical procedures (see e.g., Varney et al 1991). As referred to herein, a diminution in symptoms to an allergen includes any reduction in the allergic response of a mammal (such as a human) to the allergen following a treatment regimen with a polypeptide as described herein. This diminution in symptoms may be determined subjectively in humans (e.g., the patient fells more comfortable upon exposure to the allergen), or clinically, such as with a standard skin test.

In one embodiment, the polypeptides of the invention and fragments, derivatives and analogs thereof as well as as altered peptides and modified allergens as set forth in the claims are for use in the treatment of allergy against *Vespula* venom.

In another embodiment, the polypeptides of the invention and fragments, derivatives and analogs thereof as well as as altered peptides and modified allergens are for use in the treatment of allergy against the *Vespula* venom protease Ves v 4.

In a further embodiment, the polypeptides, or fragments, derivatives and analogs thereof are administered in an amount sufficient to reduce or inhibit an immune reaction against the Ves v 4 polypeptide.

In a further embodiment, the polypeptides or fragments, derivatives and analogs thereof are administered over a period of time in gradually increasing doses.

In a further embodiment, the invention provides a use of the polypeptides of the invention or fragments, derivatives and analogs thereof as well as as of altered peptides and modified allergens for the generation of a medicament for the treatment of allergy against the *Vespula* venom, preferably of allergy against the *Vespula* venom protease Ves v 4.

In another embodiment, when altered peptides or modified allergens, respectively, are used in the treatment of allergy or hypersensitivity, respectively, against the *Vespula* venom, preferably of allergy or hypersensitivity against the *Vespula* venom protease Ves v 4, said altered peptides or modified allergens of the invention preferably cause less side effects as compared to the unaltered, wild-type polypeptide or allergen.

The Ves v 4 polypeptide, a fragment, a derivative and an analog thereof can be administered over a period of time in gradually increasing doses effective to reduce the allergic response of the individual to the protein allergen. Examples of routes of administration include parenteral (e.g., intraveneous), intradermal, subcutaneous, oral (e.g., sublingual or via inhalation), transdermal (topical), and rectal administrations. The effective amount of the Ves v 4 polypeptide, a fragment, a derivative and an analog thereof will vary according to factors such as the degree of sensitivity of the individual to Ves v 4, the age, sex, and weight of the individual, and the ability of the fragment, derivative, or analog thereof to elicit an antigenic response in the individual. In one embodiment, the amount of Ves v 4 polypeptide administered to an individual corresponds to the amount of Ves v 4 in the venom of vespids that is injected into an individual by a sting. Dosage regimens may be adjusted to provide the optimum therapeutic response. For example, several divided doses may be administered daily.

### X. Ves v 4-based pharmaceutical compositions

In one embodiment, the poylpeptides of the invention, preferably the Ves v 4 polypeptide, fragments, derivatives and analogs thereof, as set forth in the claims are incorporated into pharmaceutical compositions suitable for administration. Such compositions typically comprise the Ves v 4 polypeptide, fragments, derivatives or analogs thereof, and a pharmaceutically acceptable carrier. As used herein, a "pharmaceutically acceptable carrier" is intended to include any and all solvents, dispersion media, coatings, antibacterial and antifungal agents, isotonic and adsorption delaying systems, and the like, compatible with the active compound and pharmaceutical administration. The use of such media and agents for pharmaceutically active substances is well known in the art. Supplementary active compounds can also be incorporated into the composition.

In one embodiment, the description provides a pharmaceutical composition comprising one or more of the polypeptide(s) of the invention and fragments, derivatives and analogs thereof and a pharmaceutically acceptable carrier.

In a further embodiment, the description provides a pharmaceutical composition comprising one or more of the polypeptide(s) of the invention and fragments, derivatives and analogs thereof and a pharmaceutically acceptable carrier, wherein the one or more polypeptide(s) map the total length of the Ves v 4 polypeptide.

In a further embodiment, the description provides a pharmaceutical composition comprising one or more of the polypeptide(s) of the invention and fragments, derivatives and analogs thereof and a pharmaceutically acceptable carrier, further comprising one or more additional polypeptide(s) or fragments thereof. Said one or more additional polypeptide(s) or fragments thereof preferably are selected from the group consisting of Ves v 1 polypeptide (phospolipase A1), Ves v 2a polypeptide (hyaluronidase), Ves v 2b polypeptide, Ves v 3 polypeptide (dipeptidylpeptidase), Ves v 5 polypeptide, glycosylated IgE-binding proteins and analogs or derivatives thereof.

In a further embodiment, the description provides a pharmaceutical composition comprising one or more of the polypeptide(s) of the invention and fragments, derivatives and analogs thereof and a pharmaceutically acceptable carrier, wherein the one or more polypeptide(s) comprises one or more T cell epitope-containing peptides of at least 9 amino acids corresponding to a consecutive amino acid sequence within the *Vespula* venom protease Ves v 4.

The pharmaceutical compositions of the invention, comprising one or more polypeptide(s) of the invention and fragments derivatives and analogs thereof as set forth in the claims are for use as a medicament. In a specific embodiment, the pharmaceutical compositions of the invention comprising the Ves v 4 polypeptide, fragments, derivatives and analogs thereof are for use as a medicament.

In one embodiment, the pharmaceutical compositions of the invention comprising one or more polypeptide(s) of the invention and fragments, derivatives and analogs thereof are for use in the treatment of allergy against *Vespula* venom, preferably of allergy against the *Vespula* venom protease Ves v 4.

In a further embodiment, the pharmaceutical composition of the invention comprising one or more polypeptide(s) of the invention and fragments, derivatives and analogs thereof are administered in an amount sufficient to reduce or inhibit an immune reaction against the Ves v 4 polypeptide.

In a further embodiment, the pharmaceutical compositions of the invention comprising one or more polypeptide(s) of the invention and fragments, derivatives and analogs thereof are administered over a period of time in gradually increasing doses.

In a further embodiment, the invention provides a use of the pharmaceutical compositions of the invention comprising one or more polypeptide(s) of the invention or fragments, derivatives and analogs thereof for the generation of a medicament for the treatment of allergy against the *Vespula* venom, preferably of allergy against the *Vespula* venom protease Ves v 4.

In another embodiment, the pharmaceutical composition includes an additional polypeptide, e.g., a second, third, fourth, or more *Vespula* venom polypeptide or polypeptides. The additional *Vespula* venom polypeptides can include, e.g., the Ves v 1 polypeptide (phospholipase A1), the Ves v 2a polypeptide (hyaluronidase), the Ves v 2b polypeptide, the Ves v 3 polypeptide (dipeptidylpeptidase), the Ves v 5 polypeptide, glycosylated IgE-binding proteins, or analogs or derivatives thereof.

In another embodiment, the present description features a pharmaceutical composition comprising Ves v 4 polypeptide fragments of the invention, preferably between 20-150 amino acids in length, wherein each fragment contains one or more B cell epitopes and one or more T cell epitopes, and a pharmaceutically acceptable carrier. In a preferred embodiment, the composition comprises a set of polypeptide fragments that map the total length of the Ves v 4 polypeptide.

In another embodiment, the pharmaceutical composition includes polypeptide fragments derived from an additional polypeptide, e.g., a second, third, fourth, or more *Vespula* venom polypeptide or polypeptides. The additional *Vespula* venom polypeptides can include, e.g., the Ves v 1 polypeptide (phospholipase A1), the Ves v 2a polypeptide (hyaluronidase), the Ves v 2b polypeptide, the Ves v 3 polypeptide (dipeptidylpeptidase), the Ves v 5 polypeptide, glycosylated IgE-binding proteins, or analogs or derivatives thereof.

In another embodiment, the present description features a pharmaceutical composition comprising T cell epitope containing peptides of at least 9 amino acids corresponding to a consecutive amino acid sequence within the *Vespula* venom protease (Ves v 4) wherein the peptides are capable of stimulating T cells of subjects allergic to Ves v 4. In a preferred embodiment, the composition comprises a set of T cell epitope-containing peptides capable of stimulating T cells of the great majority of subjects allergic to Ves v 4.

In another embodiment, the pharmaceutical composition includes T cell epitope-containing peptides of at least 9 amino acids corresponding to a consecutive amino acid sequence within an additional polypeptide, e.g., a second, third, fourth, or more *Vespula* venom polypeptide or polypeptides. The additional *Vespula* venom polypeptides can include, e.g., the Ves v 1 polypeptide (phospholipase A1), the Ves v 2a polypeptide (hyaluronidase), the Ves v 2b polypeptide, the Ves v 3 polypeptide (dipeptidylpeptidase), the Ves v 5 polypeptide, glycosylated IgE-binding proteins, or analogs or derivatives thereof.

Solutions or suspensions used for parenteral, intradermal, or subcutaneous application can include the following components: a sterile diluent such as water for injection, saline solution, fixed oils, polyethylene glycols, glycerine, propylene glycol or other synthetic solvents, antioxidants such as ascorbic acid or sodium bisulfite, chelating agents such as ethylenediaminetetraacetic acid, buffers such as acetates, citrates or phosphates and agents for the adjustment of toxicity such as sodium chloride or dextrose. The pH can be adjusted with acids or bases, such as hydrochloric acid or sodium hydroxide. In many cases, it will be preferable to include isotonic agents, for example, sugars, polyalcohols such as mannitol, sorbitol, sodium chloride in the composition. The composition should be fluid to the extent that easy syringability exists. The proper fluidity can be maintained, for example, by the use of a coating such as lecithin, by the maintenance of the required particle size in the case dispersion and by use of surfactants. The composition must be stable under the conditions of manufacture and storage and must be preserved against the contaminating action of microorganisms such as bacteria and fungi. Prevention of the action of microorganisms can be achieved by various antibacterial and antifungal agents such as parabens, chlorobutanol, phenol, ascorbic acid, thimoseral, and the like. Delayed absorption of the injectable compositions can be achieved by including in the composition an agent such as aluminium monostearate and gelatin. In all cases, the composition must be sterile. Sterile injectable solutions can be prepared by filtered sterilization. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof. The parenteral preparation can be enclosed in ampoules, disposable syringes or multiple dose vials made of glass or plastic.

Oral compositions generally include an inert diluent or an edible carrier. They can be enclosed in gelatine capsules or compressed into tablets. For the purpose of oral therapeutic administration, the active compound can be incorporated with excipients and used in the form of tablets, troches, or capsules. Oral compositions can also be prepared using a fluid carrier for use as mouthwash, wherein the active compound in the fluid carrier is swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth, or gelatine; an excipient such as starch or lactose; a disintegrating agent such as alginic acid, Primogel, or corn starch; a lubricant such as magnesium stearate or Sterotes; a glidant such as colloidal silicon dioxide; a sweetening agent such as sucrose or saccharin; or a flavouring agent such as peppermint, methyl salicylate, or orange flavouring.
For administration by inhalation, the active compounds are delivered in the form of an aerosol spray from a pressured container or dispenser which contains a suitable propellant, e.g. a gas such as carbon dioxide, or a nebulizer.

For transmucosal or transdermal administration, penetrants appropriate to the barrier to be permeated are used in the formulation. Such penetrants are generally known in the art, and include for transmucosal administration, for example; detergents, bile salts, and fusidic acid derivatives. Transmucosal administration can be accomplished through the use of nasal sprays or suppositories. Suppositories can be prepared using conventional suppository base such as cocoa butter or other glycerides. For transdermal administration, the active compounds are formulated into ointments, salves, gels, or creams as generally known in the art. For rectal delivery the compounds can also be prepared in the form of retention enemas.

In a further embodiment, the active compounds are prepared with carriers that will protect the active compounds against rapid elimination from the body, such as controlled release formulation, including implants and microencapsulated delivery systems. Biodegradable, biocompatible polymers can be used, such as ethylene vinyl acetate, polyanhydrides, polyglycolic acid, collagen, polyorthoesters, and polyacetic acid. Methods for preparation of such formulations will be apparent to those skilled in the art. The materials can also be obtained commercially. Liposomal suspensions can also be used as pharmaceutically acceptable carriers. These can be prepared according to methods known to those skilled in the art.

For oral and parenteral applications it is advantageous to formulate the compositions in dosage unit form for ease of administration and uniformity of dosage. Dosage unit form as used herein refers to physically discrete units suited as unitary dosages for the subject to be treated. Each unit contains a predetermined quantity of the active compound calculated to produce the desired therapeutic effect in association with the included pharmaceutical carrier. The specification for the dosage unit forms of the invention are dependent on the unique characteristics of the active compound and the particular therapeutic effect to be achieved, and the limitations inherent in the art of compounding such an active compound for the treatment of individuals. The pharmaceutical compositions can be included in a container, pack, or dispenser together with instructions for administration.

### EXAMPLES

### EXAMPLE 1: Cloning of Ves v 4

### Materials and Methods

### cDNA synthesis

Total RNA was isolated from the separated stinger with attached venom sack and additional glands of yellow jacket *(Vespula vulgaris)* using peqGold TriFast™ (Peqlab Biotechnologie, Erlangen, Germany). SuperScript III Reverse Transcriptase (Invitrogen, Karlsruhe, Germany) was used to synthesize cDNA from the isolated total RNA. RNaseOut™ recombinant ribonuclease inhibitor (0.1 µl) (Invitrogen, Karlsruhe, Germany) was added to the standard 20 µl reaction mix containing 5 µl venom gland RNA. Reverse transcription was performed at 50 °C for 60 minutes. First strand cDNA was used as template for PCR amplification of Api m 5 and Ves v 3 DNA sequences.

### Isolation of Ves v 4 fragment

A fragment of the Ves v 4 ORF was amplified from *Vespula vulgaris* venom gland cDNA with *Pfu* DNA polymerase (Fermentas, St. Leon-Rot, Germany) using the degenerated primer 5'-NAC DGC KGC YCA YTG-3'. DNA from the PCR reaction was isolated from 1% agarose gels (peqGOLD universal agarose, Peqlab, Erlangen, Germany) using the peqGOLD Gel Extraction Kit (Peqlab). Subcloning for sequencing was done using Zero Blunt TOPO PCR Cloning Kit (Invitrogen) with pCR-Blunt II-TOPO vector. The ligated DNA was transformed into *Escherichia coli* of the strain XL1Blue by electroporation (2 mm cuvettes, EasyJect+; Eurogentec, Seraing, Belgium) and selected on ampicillin agar plates.

### Generation of full-length Ves v 4

After sequencing of selected subcloned cDNA clones and verification of the sequence, the primer 5'-GGA TGT ACG ATT ATA TCA TCA ATA AG-3' was deduced from the sequence and used for cDNA synthesis as already described. The cDNA was used for 5'RACE employing the 5'/3'RACE Kit, Second Generation (Roche, Grenzach, Germany) according to the recommendations of the manufacturer. Subsequent nested PCR was performed using the primers 5'-CCT TTG TAT CGT TAA TTG GCC ATG-3' and 5'- GTT CTC CAA CAA CAA CTC CTA - 3'.

The obtained cDNA fragments were used as basis for further sequence determination. Full length cDNA was then amplified using the forward primer 5'- ATG AAA TTA GAT AAT TTT TTT TTA ATA CTT TAT G-3' and the reverse primer 5'- TTA ATA CGC CTG ACA ATA TAT TTC-3'.

After sequencing of selected full length cDNA clones and verification of the sequence, the clone was used for secondary amplification of the coding region of the mature chain with *Pfu* DNA polymerase using primers incorporating 5' BamHI and 3' NotI restriction sites as well as a 3' V5 epitope and a 10 fold His-tag. The PCR product was subcloned into the BamHI and NotI digested baculovirus transfer vector pAcGP67-B after restriction digest with the respective enzymes.

### Results

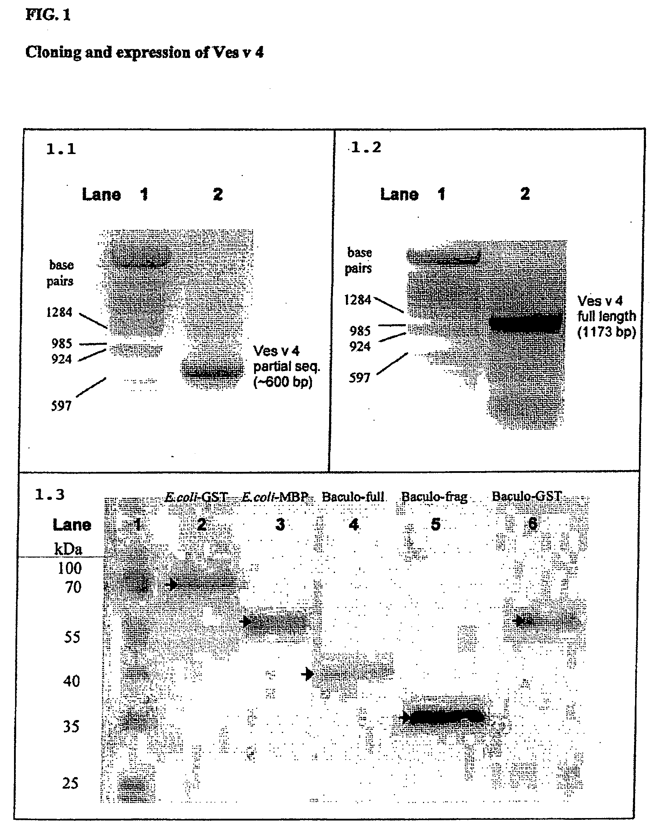

Using degenerated primers, targeting conserved serine protease motifs, a fragment of the Ves v 4 gene was amplified from cDNA as shown in figure 1 box A. Depicted is the electrophoretic analysis in 1% agarose gel of the gene fragment derived from PCR with degenerated primers. An approximately 600 base pair fragment (lane 2) was generated, as estimated from comparison with the molecular size marker (Lambda DNA/Eco47I, Fermentas GmbH, St Leon-Rot) in lane 1. The fragment was subsequently used to perform 5'-RACE methods to generate and select a full length clone. The full length clone consists of a 1173 base pair DNA as shown in lane 2 of the electrophoretic analysis in 1% agarose gel in Figure 1 box B, in comparison to the molecular size standard in lane 1.
The nucleic acid sequence was analysed and is given in SEQ ID NO: 1.

### Discussion

The full-length cDNA of Ves v 4 of 1173 base pairs (including the stop codon) has been successfully isolated from the stinging apparatus of *Vespula vulgaris.* Sequencing verified the isolation of a new serine protease-like protein containing a N-terminal CUB domain and putative signal peptide sequence, coding for a 390 amino acid long protein (see SEQ ID NO:1 and SEQ ID NO:2).

### EXAMPLE 2: Recombinant bacterial expression and purification of Ves v 4

### Materials and Methods

For expression of Ves v 4 in *E. coli,* the cDNA was cloned into the prokaryotic expression vector pMAL-c2x (NEB, Frankfurt, Germany). Expression in XL1 Blue *E. coli* cells and purification of the fusion protein was performed according to the recommendations of the manufacturers.

### Results

The gene sequence is used to construct several expression vectors to produce recombinant protein. As an example, Figure 1 box C shows the analysis of Western blotting and detection of recombinant proteins with anti-V5 antibodies derived from expression in *E. coli* as fusion to the glutathione-S-transferase (GST) domain (lane 2), fusion to the maltose-binding-protein (MBP) domain (lane 3).

### Discussion

The full length sequence of Ves v 4 was cloned, sequenced and used for successful expression of the recombinant proteins. The recombinant expression in prokaryotic systems revealed difficulties in obtaining suitable amounts of soluble protein which was subsequently improved by expression as fusion proteins with GST- or MBP-moieties. Purification was simplified by introduction of a His-Tag and metal affinity chromatography.

### EXAMPLE 3: Production of recombinant Ves v 4 in Sf9 cells and purification

### Materials and Methods

### Production of recombinant Baculovirus

*Spodoptera frugiperda* cells (Sf9, Invitrogen, Karlsruhe, Germany) were grown at 27°C in serum-free medium (Express Five SFM, containing 16.5 mM glutamine and 10 µg/ml gentamycin; Invitrogen, Karlsruhe, Germany). Cell density was determined by haemocytometer counts, cell viability was evaluated by staining with Trypan Blue. Recombinant baculovirus was generated by cotransfection of Sf9 cells with BaculoGold bright DNA (BD Pharmingen, Heidelberg, Germany) and the baculovirus transfer vector pAcGP67-B Ves v 4 according to recommendations of the manufacturer. High titer stocks were produced by three rounds of virus amplification and optimal MOI for recombinant protein expression was determined empirically by infection of Sf9 cells in 100 ml suspension flask (1.5-2x10⁶ cells/ml in 20 ml suspension culture) with serial dilutions of high titer virus stock.

### Expression in baculovirus-infected Sf9 cells

High titer stocks of recombinant baculovirus containing the Ves v 4-coding DNA were used to infect SF9 cells (1.5-2.0x10⁶ cells per ml) in a 2000 ml suspension flask (400 ml suspension culture). For protein production the cells were incubated at 27 °C and 110 rpm for 72 h.

### Protein purification

The supernatant of baculovirus-infected cells was collected, adjusted to pH 8 and centrifuged at 4000 x g for 5 minutes. Supernatants were applied to a nickel-chelating affinity matrix (NTA-agarose, Qiagen, Hilden, Germany). The column was washed with NTA-binding buffer (50 mM sodium phosphate, pH 7.6, 500 mM NaCl) and pre-eluted with NTA-binding buffer containing 20 mM imidazole. The recombinant protein was eluted from the matrix with NTA-binding buffer containing 300 mM imidazole. Purification was confirmed by SDS-PAGE.

### Results

The gene sequence is used to construct expression vectors to produce recombinant protein in eukaryotic cells. As an example, Figure 1 box C shows the analysis of Western blotting and detection of recombinant proteins with anti-V5 antibodies derived from expression in insect cells by the Baculovirus system (lanes 4-6) after separation by 7.5% poly-acryl amide gel electrophoresis (PAGE). For estimation of the molecular size of the proteins a standard marker in lane 1 is used. In figure 1 box C lane 4 the recombinant full-length protein with additional V5 and 10xHis-Tag is shown. In lane 5 a truncated form without CUB domain is shown. In lane 6 the PAGE analysis of full-length protein with additional GST-domain is shown.

### Discussion

The full length sequence of Ves v 4 was cloned, sequenced and used for successful expression of the recombinant proteins. The protein is successfully expressed in eukaryotic expression system, like the Baculovirus system utilizing an insect cell culture. Purification was simplified by introduction of a His-Tag and metal affinity chromatography.

### EXAMPLE 4: Production of recombinant Ves v 4 in HighFive cells and purification

### Materials and Methods

### Production of recombinant Baculovirus

*Trichoplusia ni* cells (HighFive, Invitrogen, Karlsruhe, Germany) were grown at 27°C in serum-free medium (Express Five SFM, containing 16.5 mM glutamine and 10 µg/ml gentamycin; Invitrogen, Karlsruhe, Germany). Cell density was determined by haemocytometer counts, cell viability was evaluated by staining with Trypan Blue. Recombinant baculovirus was generated by cotransfection of HighFive cells with BaculoGold bright DNA (BD Pharmingen, Heidelberg, Germany) and the baculovirus transfer vector pAcGP67-B Ves v 4 according to recommendations of the manufacturer. High titer stocks were produced by three rounds of virus amplification and optimal MOI for recombinant protein expression was determined empirically by infection of HighFive cells in 100 ml suspension flask (1.5-2x10⁶ cells/ml in 20 ml) suspension culture) with serial dilutions of high titer virus stock.

### Expression in baculovirus-infected HighFive cells

High titer stocks of recombinant baculovirus containing the Ves v 4-coding DNA were used to infect HighFive cells (1.5-2.0x10⁶ cells per ml) in a 2000 ml suspension flask (400 ml suspension culture). For protein production the cells were incubated at 27 °C and 110 rpm for 72 h.

### Protein purification

The supernatant of baculovirus-infected cells was collected, adjusted to pH 8 and centrifuged at 4000 x g for 5 minutes. Supernatants were applied to a nickel-chelating affinity matrix (NTA-agarose, Qiagen, Hilden, Germany). The column was washed with NTA-binding buffer (50 mM sodium phosphate, pH 7.6, 500 mM NaCl) and pre-eluted with NTA-binding buffer containing 20 mM imidazole. The recombinant protein was eluted from the matrix with NTA-binding buffer containing 300 mM imidazole. Purification was confirmed by SDS-PAGE.

### Results

The gene sequence is used to construct expression vectors to produce recombinant protein in eukaryotic cells, e.g. insect derived (*T*. *ni*) HighFive cells.

### Discussion

The full length sequence of Ves v 4 was cloned, sequenced and used for successful expression of the recombinant proteins in HighFive cells. In comparison with the expression in SF9 cells a different expression yield and glycosylation pattern regarding α(1,3)-fucosylation was found.

### EXAMPLE 5: Analysis of homologies

### Materials and Methods

The nucleic acid sequence of Ves v 4 was used for comparison with other known sequences. An online BLAST search (Altschul et al 1990; Morgulis et al 2008) was performed and sequences suitable for further analysis and alignment identified. The sequences were pairwise aligned using Align-X from the NTI Vector software package (Version 10, Invitrogen Inc) and the degree of sequence identity calculated.

The ClustalW2 multiple sequence alignment programme (Higgins et al 1994; Lopez et al 1997; Larkin et al 2007), available from the EMBL-EBI web site, was used with standard settings to construct a multiple alignment of selected related sequences with Ves v 4.

### Results

Four proteins have been selected by identification of sequence similarity to Ves v 4. The nucleic acid and protein sequences of Ves v 4 have been aligned with Magnvesin from *Vespa magnifica* (Han et al 2008), Pol d 4 from *Polistes dominulus* (Winningham et al 2004), Api m 7 from *Apis mellifera* (Winningham et al, 2004) and Bom p 4 from *Bombus pennsylvanicus* (Hoffman and Jacobson 1996). The sequences have been retrieved from public domain sequence databases.

Magnvesin from the venom of the hornet *Vespa magnifica* is a 28 kDa serine protease. A 1250 base pair full-length clone has been identified, coding a proprotein of 305 amino acids. The mature protein length is 242 amino acids. The sequence analysis revealed a N-terminal 48 amino acid domain with high homology to the CUB domain of Ves V 4, not seen in other venom proteases. The pairwise alignment reveals that the homology to Ves v 4 on DNA level, according to the given definition, is 72%, on protein level it is 69%. The pairwise alignment of Ves v 4 with Magnvesin is depicted in figure 2.

Pol d 4 is a serine protease from the venom of the European paper wasp. The theoretical molecular weight is 27 kDa. Higher homology in pairwise alignment to Ves v 4 can be found in the C-terminal protease domain and is 46% on DNA level and 35% on protein level for the whole sequence length.

Api m 7 is a protease from the honeybee venom. The theoretical molecular weight is 39 kDa. The sequence shows N-terminal CUB and C-terminal protease domains. The most homology on protein level to Ves v 4 is seen in the protease domain, the difference is less on DNA level. The pairwise homology analysis with Ves v 4 shows 53% DNA level, and 35% on protein level.

Bom p 4 is a serine protease from bumblebee venom. The protein has a theoretical molecular weight of 27 kDa and pairwise sequence alignment analysis shows limited homology to the protease domain. Homology to Ves v 4 on DNA level not known, on protein level it is 18%.

The multiple alignments of all above sequences on protein level are depicted in Figure 2.

### Discussion

Alignment of Ves v 4 sequences to known sequences showed the expected homology to serine proteases from other closely related species. The recently published sequence of Magnvesin from the closely related hymenoptera species *Vespa magnifica* exhibits the highest sequence homology to Ves v 4 of all identified genes.

### EXAMPLE 6: Analysis of secondary structural domains of Ves v 4

### Materials and Methods

The online bioinformatics tool SWISS-Model (Arnold et al, 2006) was used to predict the secondary structure of Ves v 4. The protein sequence was used as input with standard settings. In addition, the domain structure was analysed using the online bioinformatics tool InterProScan (Quevillon et al, 2005). As with SWISS-Model the protein sequence was used as input with standard settings.

### Results

The result of the SWISS-Model analysis is depicted in figure 4. Larger stretches of beta-sheet structures are predicted throughout the protein. In addition, two smaller C-terminal patches of alpha-helical structure were predicted.

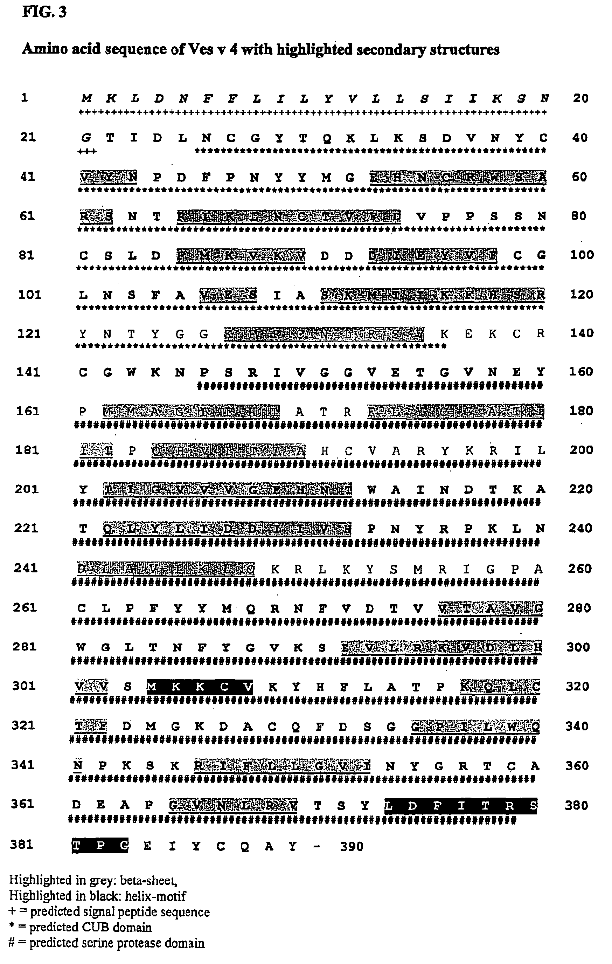

The analysis with the InterScanPro tool shows 3 motifs in the Ves v 4 sequence. A putative signal peptide sequence was identified within residues 1-21. Using the SignalP 3.0 server of the Technical University of Denmark (Emanuelsson et al, 2007) this signal peptide prediction has been verified. The second predicted motif is a CUB domain spanning residues 26-136. The third motif represents a serine protease-like domain. This domain includes the residues 146-379. The depiction of the position of predicted domain structures can be found in Figure 3.

### Discussion

The analysis of the secondary structure of Ves v 4 reveals the expected domain of serine proteases, in alignment with the previous sequence homology analysis. In addition a CUB domain was identified, of which the function is unknown.

### EXAMPLE 7: Selection of Ves v 4 fragments

### Materials and Methods

Based on the analysis of the secondary structure of Ves v 4, fragments have been selected to preserve structural motifs, representing possible conformational B cell epitopes and including linear T cell epitopes and covering the range of reactivities. The length of the fragments was selected to enable simplified cloning and expression (approximately 150 and 100 amino acids) or even chemical synthesis of the peptides (approximately 50 amino acid length) and usability for, e.g. different pharmaceutical applications.

### Results

The selection of peptide fragments and position in Ves v 4 molecule with the different targeted length is given in Table 1. The positioning of the differently sized fragments is shown in Figures 5, 6 and 7.

### Discussion

The secondary structure of Ves v 4 provided the basis for the selection of peptide fragments to provide the optimal presentation of B- and T-cell epitopes for diagnosis and therapeutic approaches in insect venom allergy.

### EXAMPLE 8: Analysis of T cell epitopes of Ves v 4

### Materials and Methods

The analysis of linear T cell epitopes was done using specific weight matrices with the online prediction tool NetMHCII (Morten et al 2007), provided by the Center for Biological Sequence Analysis (CBS) of the Technical University of Denmark with standard settings for all 14 HLA-DR alleles covering the 9 HLA-DR supertypes.

The prediction tool NetMHCIIPAN (Nielsen et al 2008) on the CBS web site was used to predict T cell epitopes in a range of similar HLA-DR alleles using artificial neural networks. For the prediction 15 alleles from DRB1*01xx pool of alleles were used.

### Results

The protein sequence of Ves v 4 (SEQ ID NO:2) without signal peptide was used as input for the prediction programme. According to the predicted affinity, putative 15mer T cell peptides were listed and are shown in Tables 2 and 3. Table 2 shows the results from the analysis in regard to the HLA-DR supertypes. Table 3 shows the more detailed results of the analysis within one HLR-DR supertype. Results were limited to peptides with predicted higher affinity to the corresponding MHC I molecules.

### Discussion

The results from the prediction of T cell epitopes shows the restriction regarding to human MHC alleles and predicted affinity. The distribution of peptides spans nearly the whole length of the Ves v 4 molecule.

### EXAMPLE 9: Analysis of linear B cell epitopes of Ves v 4

### Materials and Methods

The protein sequence of Ves v 4 was used for prediction of linear B cell epitopes employing the online server tool BepiPred 1.0b (Larsen et al 2006). The protein sequence was used as input with standard settings.

### Results

13 peptids were predicted by BepiPred to represent linear B cell epitopes. The results are shown in Table 6. The length of the peptides varies from 1-16 residues. The position of the epitopes is given in accordance with the numbering in SEQ ID NO: 2.

### Discussion

13 Potential linear B cell epitopes of Ves v 4 have been predicted using BepiPred 1.0b.

### EXAMPLE 10: Immunoreactivity of Ves v 4

### Materials and Methods

For assessment of specific IgE immunoreactivity of human sera with purified recombinant Api m 5 and Ves v 3 in ELISA, 384 well microtiter plates (Greiner, Frickenhausen, Germany) were coated with 20 µl of purified recombinant Ves v 4-MBP (20 µg/ml) at 4 °C overnight and blocked with 40 mg/ml MPBS at room temperature. Afterwards human sera were diluted 1:1 with 5 mg/ml BSA in PBS and incubated in a final volume of 20 µl for 4 hours at room temperature. Wells were washed 4 times with PBS before bound IgE was detected with a monoclonal alkaline phosphatase-conjugated mouse anti-human IgE antibody (BD Pharmingen, Heidelberg, Germany) diluted 1:1000. Wells were again washed 4 times with PBS and 50 µl of substrate solution (5 mg/ml 4-nitrophenylphosphate, AppliChem, Darmstadt, Germany) per well were added. After 30 minutes the plates were read at 405 nm. The cutoff was calculated as the mean of the negative controls plus 2 SDs.

### Results

The results of the ELISA experiment are shown in Figure 4. Detection of Ves v 4-MBP produced in *E. coli* with IgE from several sera of yellow jacket venom-allergic individuals.

### Discussion

16 patients allergic to wasp venom have been tested for reactivity to Ves v 4. Of this panel 4 patients showed strong reaction towards the MBP-fusion protein which was correctly folded, other 5 patients showed weaker reactivity. In total 56% of the tested patients showed reactivity for Ves v 4-MBP.

### EXAMPLE 11: Identification of nucleic acid molecules from the family Vespidae hybridizable to the nucleic acid encoding Ves v 4

### Materials and Methods

Fragments of the Ves v 4 ORF were amplified from venom gland cDNA from *Vespula vulgaris* and *Vespula germanica.* 1 µg of the isolated DNA was separated on a 1% agarose gel and blotted to a Hybond-NX nylon membrane (GE Healthcare) by alkaline transfer. After UV-crosslinking and blocking of the membrane with 1% blocking reagent (Roche) the membrane was incubated with the probe at 42°C overnight.

The probe was genereated from DNA from the PCR reaction using *Vespula vulgaris* cDNA was isolated from 1 % agarose gels (peqGOLD universal agarose, Peqlab, Erlangen, Germany) using the peqGOLD Gel Extraction Kit (Peqlab). 1 µg of DNA was used for generation of a DIG labelled probe using the DIG DNA Labelling Kit (Roche) according to recommendations of the manufacturer. After washing with 2x SSC buffer, the membrane was blocked again and then incubated with anti-DIG-AP Fab fragments, diluted 1:10000 in 1 % blocking reagent for 30 minutes. Visualization of bound Fab fragments was performed with nitrotetrazolium blue and chloride/5-bromo-4-chloro-3-indoyl phosphate according to standard protocols.

### Results

The results of the hybridisation experiment under low stringency conditions are shown in Figure 8. Southern Blot of Ves v 4 fragment with genomic DNA from *Vespula germanica.*

### Discussion

As shown in Figure 8 the probe derived from *Vespula vulgaris* also hybridised to the gene fragment derived from the species *Vespula germanica,* and could be detected. The strong signal in the Southern blot experiment shows that both sequences exhibit a significant degree of sequence identity.

### EXAMPLE 12: Recombinant bacterial expression and purification of Ves v 4 as a non-fusion protein

### Materials and Methods

For expression of Ves v 4 as a non-fusion protein in *E. coli,* the cDNA was cloned into the prokaryotic expression vector pET22-b(+) (Novagen, Germany) by concomitant deletion f the *pelB* leader sequence and introduction of a C-terminal 10 fold His-Tag and a V5 epitope. For Expression BL21DE3 *E. coli* cells were used and the insoluble protein was purified under denaturing conditions according to the recommendations of the manufacturers.

### Results

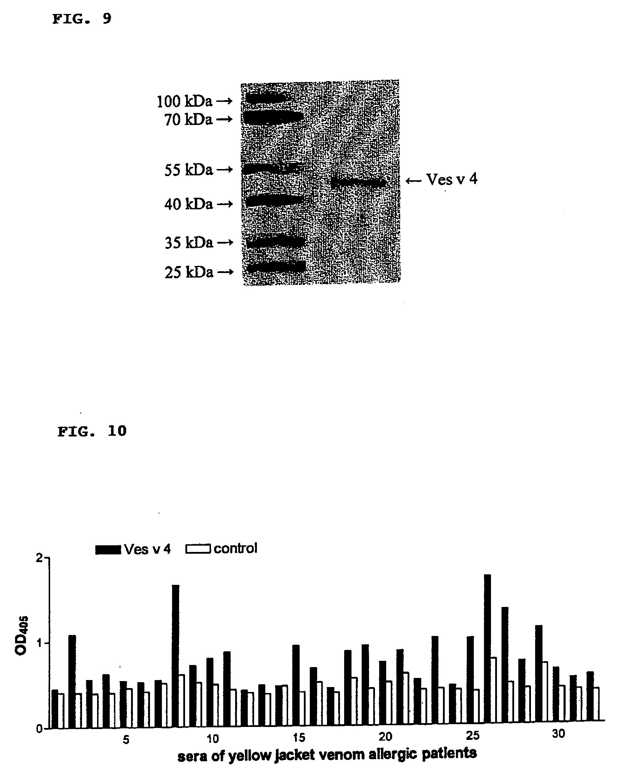

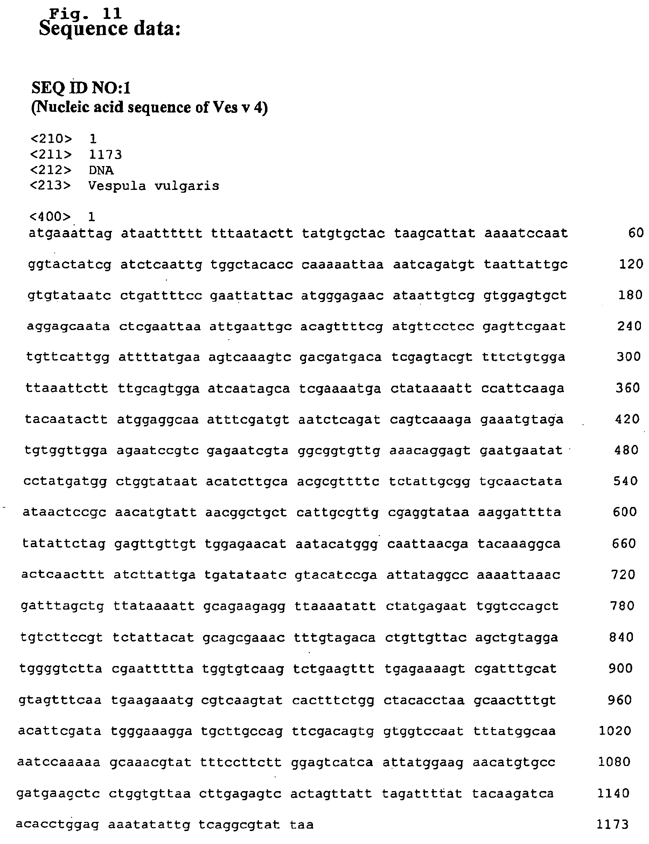

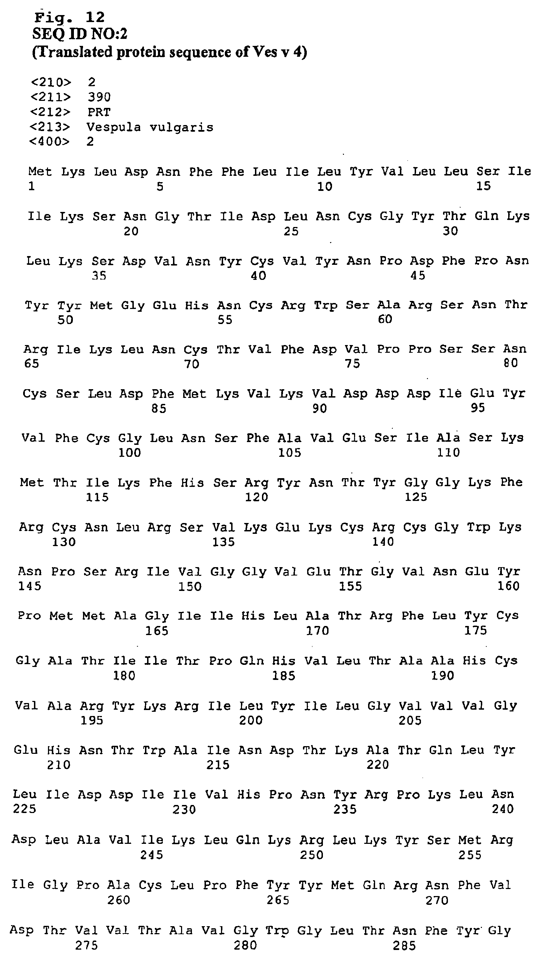

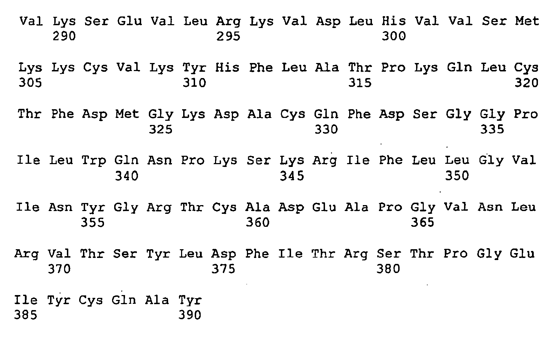

The non-fusion protein of Ves v 4 could be obtained in insoluble form from expression in E. coli. Purification under denaturating conditions using IMAC resulted in purified protein of the expected molecular weight as proven by Western blotting with an anti-V5 antibody (figure 9).

### Discussion

The full length sequence of Ves v 4 was cloned, sequenced and used for successful expression of the recombinant non-fusion protein. The recombinant production of the non-fusion protein resulted in insoluble protein and no soluble protein could be observed. Using denaturating conditions, the recombinant protein could be purified via IMAC whereas the protein yield was low.

### EXAMPLE 13: Immunoreactivity of the recombinant Ves v 4 non-fusion protein

### Materials and Methods

For assessment of specific IgE immunoreactivity of human sera with the purified recombinant Ves v 4 non-fusion protein the ELISA procedure described in Example 10 was used.

### Results

From the 32 human sera analysed in the ELISA system, 7 showed specific IgE immunoreactivity with the recombinant Ves v 4 non-fusion protein as shown in figure 10.

### Discussion

The percentage of specific IgE immunoreactivity with the recombinant Ves v 4 non-fusion protein of 22 % is in accordance with the specific IgE immunoreactivity observed for the MBP fusion proteins of Ves v 4.

### REFERENCES

Altschul S.F., Gish W., Miller W., Myers E.W. and Lipman D.J. Basic local alignment search tool. J. Mol. Biol. 1990;215:403-410.
Arnold K. et al. The SWISS-MODEL Workspace: A web-based environment for protein structure homology modelling. [online, last accessed 23rd January 2009] http://swissmodel.expasy.org/SWISS-MODEL.html Bioinformatics 2006; 22:195-201.
Asgari S., Zhang G., Zareie R., and Schmidt O. A serine proteinase homolog venom protein from endoparasitoid wasp inhibits melanization of the host hemolymph. Insect Biochem. Mol. Biol. 2003; 33:1017-1024.
Barnard, J.H. Studies of 400 Hymenoptera sting deaths in United States. J. Allergy Clin Immunol 1973;52:259-264.
Bauer L. et al. Modulation of the allergenic immune response in BALB/c mice by subcutaneous injection of high doses of the dominant T cell epitope from the major birch pollen allergen Bet v1. Clin. Exp. Immunol. 1997; 107:536-541.
Benjamin D.C. et al.. The Antigenic Structure of Proteins : A Reappraisal. Ann. Rev. Immunol. 1984; 2: 67-101.
Bilo, B.M., Rueff, F., Mosbech, H., Bonifazi, F., Oude-Elberink, J.N.G., and the EAACI Interest Group on Insect Venom Hypersensitivity. Diagnosis of Hymenoptera venom allergy. Allergy 2005; 60:1339-1349.
Blank S. et al. Identification and recombinant expression of a novel IgE-reactive 70 kDa carboxylesterase from Apis mellifera venom. Submitted, Uniprot entry B2D0J5.
Boel E. et al. Functional human monoclonal antibodies of all isotypes constructed from phage display library-derived single-chain Fv antibody fragments J. Immunol. Meth. 2000; 26: 153-166.
Bork P. and Beckmann, G. The CUB domain : a widespread module in developmentally regulated proteins. J. Mol. Biol. 1993; 231:539-545.
Briner et al. Peripheral T-cell tolerance induced in naive and primed mice by subcutaneous injection of peptides from the major cat allergen Fel d I. Proc. Natl. Acad. Sci. USA 1993; 90:7608-7612.
Carballido J.M. et al. T Cell Epitope Specificity in Human Allergic and Nonallergic Subjects to Bee Venom Phospholipase A2. J. Immunol. 1993; 150:3582-3591
Chou P.Y. and Fasman G.D. Prediction of protein conformation Biochemistry 1974; 13:222-245.
Dhillon M. et al. Mapping human T cell epitopes on phospholipase A2 : The major bee-venom allergen. J. Allergy Clin. Immunol. 1992; 90:42-51.
Edwards M.R. et al. Analysis of IgE Antibodies from a Patient with Atopic Dermatitis : Biased V Gene Usage and Evidence for Polyreactive IgE Heavy Chain Complementary-Determining Region 3. J Immunol 2002; 168: 6305-6313.
Elbein A.D. The Role of N-linked Oligosaccharides in Glycoprotein function. Trends in Biotech 1991; 9:346-352.
Emanuelsson O. et al.. Locating proteins in the cell using TargetP, SignalP, and related tools. [online, last accessed 23rd January 2009] http://www.cbs.dtu.dk/services/SignalP/ Nature Protocols 2007; 2:953-971.
Finkelman F.D. et al. Lymphokine Control of in vivo Immunoglobulin Isotype Selection Ann. Rev. Immunol. 1990; 8:303-333.
Fraternali F.and Cavallo L. Parameter optimized surfaces (POPS) : analysis of interactions and conformational changes in the ribosome. Nucleic Acids Res. 2002; 30:2950-2960.
Ganglberger E. et al. Allergen mimotopes for 3-dimensional epitope search and induction of antibodies inhibiting human IgE. FASEB J. 2000; 14:2177-2184.
Greene A. and Breisch N.L. Avoidance of bee and wasp stings: an entomological perspective. Curr. Opin. Allergy Clin. Immunol.2005; 5:337-341.
Haim B. et al. Characterization and anticoagulant activity of a proteolytic enzyme from Vespa orientalis venom. Toxicon 1999; 37:825-829.
Han J. et al.. An anticoagulant serine protease from the wasp venom of Vespa magnifica. NCBI nucleic acid database entry EU267370. Toxicon 2008; 51:914-922.
Hancock K. et al. False positive reactivity of recombinant, diagnostic, glycoproteins produced in High Five insect cells: Effect of glycosylation. J. Immunol. Meth. 2008; 330:130-136.
Hemmer W. et al Identification by immunoblot of venom glycoproteins displaying immunoglobulin E-binding N-glycans as cross-reactive allergens in honeybee and yellow jacket venom. Clin. Exp. Allergy 2004; 34:460-469
Higgins D. et al.. CLUSTAL W: improving the sensitivity of progressivemultiple sequence alignment through sequence weighting,position-specific gap penalties and weight matrix choice. Nucleic Acids Research 1994; 22:4673-4680.
Hoffman, D.R. Allergens in Hymenoptera XIII: isolation and purification of protein components from three species of vespid venoms. J. Allergy Clin. Immunol. 1985; 75:599-605.
Hoffman D.R. and Jacobson R.S. Allergens in Hymenoptera venom XXVII: Bumblebee enom allergy and allergens. UniProtKB database entry Q7M4I3 and Q7M4I6. J Allergy Clin Immunol 1996; 97:812-21.
Hopp T.P and Woods K.R. Prediction of protein antigenic determinants from amino acid sequences. Proc. Natl. Acad. Sci. USA 1981; 78:3824-3828.
Hoyne G.F. et al. Inhibition of T Cell and Antibody Response to House Dust Mite Allergen by Inhalation of the Dominant T Cell Epitope in Naive and Sensitized Mice. J. Exp. Med. 1993; 178:1783-1788.
Jutel M. et al. Mechanism of allergen specific immunotherapy - T-cell tolerance and more. Allergy 2006; 61:796-807.
Karamloo F. et al. Prevention of allergy by a recombinant multi-allergen vaccine with reduced IgE binding and preserved T cell epitopes. Eur. J. Immunol. 2005; 35:3268-3276.
King T.P. Insect venom allergens. Monogr. Allergy 1990; 28: 84-100.
King T.P. (1994) US Patent 5,593,877: p.1
King T.P. and Spanfort M.D. Structure and biology of stinging insect venom allergens. Int. Arch. Allergy Immunol. 2000; 123:99-106.
Korber B. et al. Immunoinformatics comes of age. PLoS Computational Biology 2006; 2:0484-0492
Larkin M.A. et al. ClustalW and ClustalX version 2. Bioinformatics 2007; 23:2947-2948.
Larsen J.E.P. et al. Improved method for predicting linear B cell epitopes Immunome Research 2006; 2:2.
Lebecque S. et al. Immunologic characterization of monoclonal antibodies that modulate human IgE binding to the major birch pollen allergen Bet v1. J. Allergy Clin. Immunol. 1997; 99:374-384.
Lopez R.. Services Programme and Lloyd A. ClustalW WWW Service at the European Bioinformatics Institute (1997) [online, last accessed 23rd January 2009] http://www.ebi.ac.uk/Tools/clustalw2/
MacKenzie T. and Dosch H.-M. Clonal and Molecular Characterization of the Human IgE-Committed B Cell Subset. J. Exp. Med. 1989; 169: 407-430
Mirza O. et al. Dominant Epitopes and Allergic Cross-Reactivity : Complex Formation Between a Fab Fragment of a Monoclonal Murine IgG Antibody and the Major Allergen from Birch Pollen Bet v 1. J. Immunol. 2000; 165:331-338.
Morgulis A et al. Database indexing for production MegaBLAST searches. Bioinformatics 2008;15:1757-1764.
Morten N. et al. Prediction of MHC class II binding affinity using SMM-align, a novel stabilization matrix alignment method. NetMHCII 1.0 Server at CBS [online, last accessed on January 23rd 2009] http://www.cbs.dtu.dk/services/NetMHCII/. BMC Bioinformatics 2007; 8:238.
Müller U. et al. Successful immunotherapy with T-cell epitope peptides of bee venom phospholipase A2 induces specific T-cell anergy in patients allergic to bee venom. J. Allergy Clin. Immunol. 1998; 101:747-754.
Niederberger V. et al. Vaccination with genetically engineered allergens prevents progression of allergic disease. Proc. Natl. Acad. Sci. USA 2004; 101:14677-14682.
Nielsen M. et al. Quantitative predictions of peptide binding to any HLA-DR molecule of known sequence: NetMHCIIpan. NetMHCIIpan Server at CBS [online, last accessed on January 23rd 2009] http://www.cbs.dtu.dk/services/NetMHCIIpan/ PLoS Comput Biol. 2008; 4(7).
O'Hehir R.E. et al. The Specificity and Regulation of T-Cell Responsiveness to Allergens. Ann. Rev. Immunol. 1991; 6:67-95.
Panjkovich A. and Melo F. Comparison of different melting temperature calculation methods for short DNA sequences. Bioinformatics 2005; 21:711-722
Petersen A., Mundt C. Investigations on the carbohydrate moieties of glycoprotein allergens J. Chromat. B 2001; 756:141-150
Powers D.B. et al. Expression of single-chain Fv-Fc fusions in Pichia pastoris J. Immunol Meth. 2001; 251:123-135.
Quevillon E. et al. InterProScan: protein domains identifier. [online, last accessed 23rd January 2009] http://www.ebi.ac.uk/Tools/InterProScan/ Nucleic Acids Res. 2005; 33(Web Server issue):W116-W120
Reid, M.J. et al. Seasonal asthma in northern California: allergic causes and efficacy of immunotherapy. J. Allergy Clin. Immunol. 1986; 78:590-600
Rudensky, et al. Sequence analysis of peptides bound to MHC class II molecules. Nature 1991; 353:622-627.
Sambrook et al, Molecular Cloning: A Laboratory Manual, Cold Spring Harbor Press, NY, 1989; 9.31-9.58,1989.
Schmidt M. and Hoffman D.R. Expression systems for production of recombinant allergens. Int. Arch. Allergy Immunol. 2002; 128: 264-270.
Steinberger P. et al. Construction of a Combinatorial IgE Library from a Allergic Patient. J. Biol. Chem. 1996; 271: 10967-10972.
Tomiya, N. et al. Comparing N-glycan processing in mammalian cell lines to native and engineered lepidopteran insect cell lines. Glycoconjugate J. 2004; 21: 343-360.
Valenta R. et al. The Immunoglobulin E-Allergen Interaction : A Target for Therapy of Type I Allergic Diseases. Int. Arch. Immunol. 1998; 116: 167-176.
Varney V.A. et al. Usefulness of immunotherapy in patients with severe summer hay fever uncontrolled by antiallergic drugs. British Medical J. 1991; 302: 265-269.
Varney V.A. et al. Influence of grass pollen immunotherapy on cellular infiltration and cytokine mRNA expression during allergen-induced late-phase cutaneous responses. J. Clin. Invest. 1993; 92:644-651.
Wang P. et al. A Systematic Assessment of MHC Class II Peptide Binding Predictions and Evaluation of a Consensus Approach. PloS Comp Biol 2008; 4:1-10.
Wetterwald A. et al. Isotypic and idiotypic characterization of anti-bee venom phospholipase A2 antibodies. Int. Arch. Allergy Appl. Immunol. 1985; 77:195-197.
Winningham K.M. et al. Hymenoptera venom protease allergens. NCBI nucleic acid database entry AY285998.UniProtKB database entry Q7Z269.
Winter G. and Milstein C. Man-made antibodies Nature 1991; 349:93-299
   J. Allergy Clin. Immunol. 2004; 114:928-933.
Yamamoto T. et al. Identification of proteins from venom of the paralytic spider wasp Cyphonyx dorsalis. Insect Biochem. Mol. Biol. 2007; 37:278-286.
Zhang, Q. et al. Immune epitope database analysis resource (IEDB-AR) Nucleic Acid Res. 2008; 36:W513-W518.

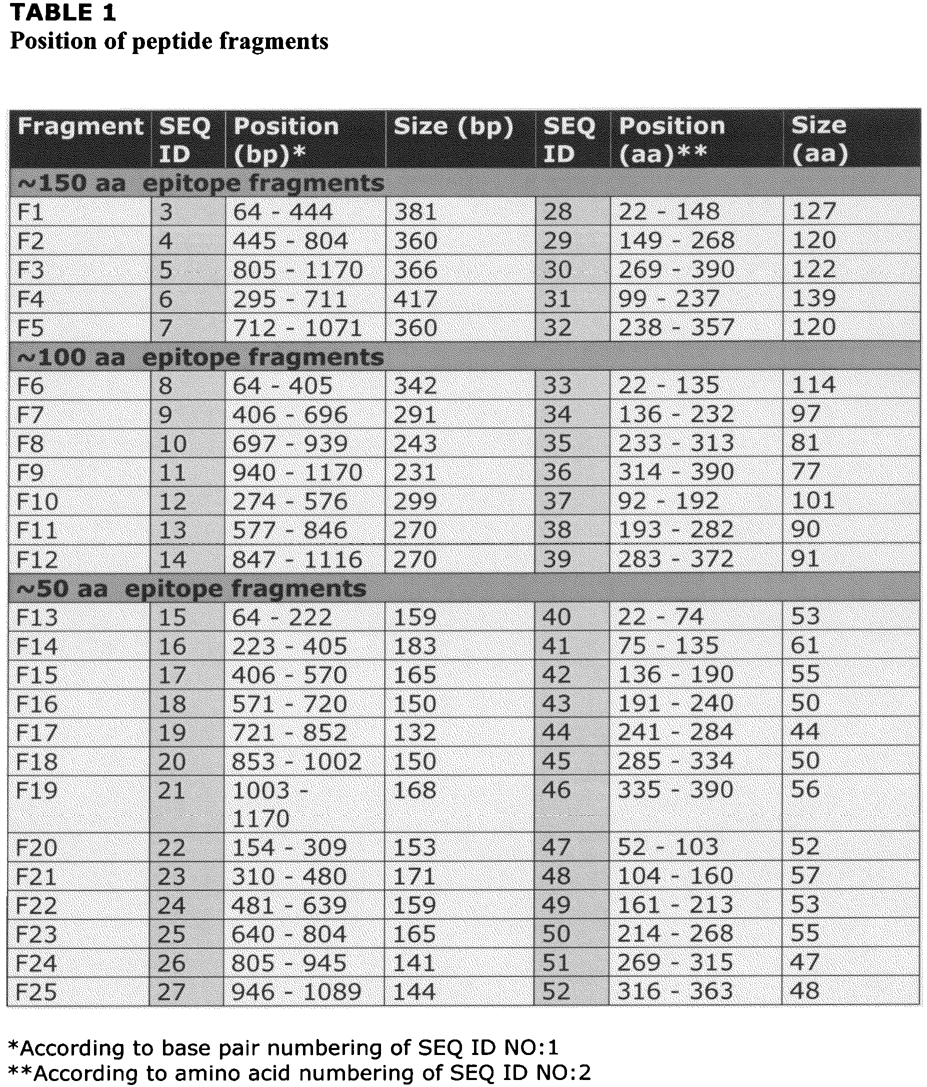

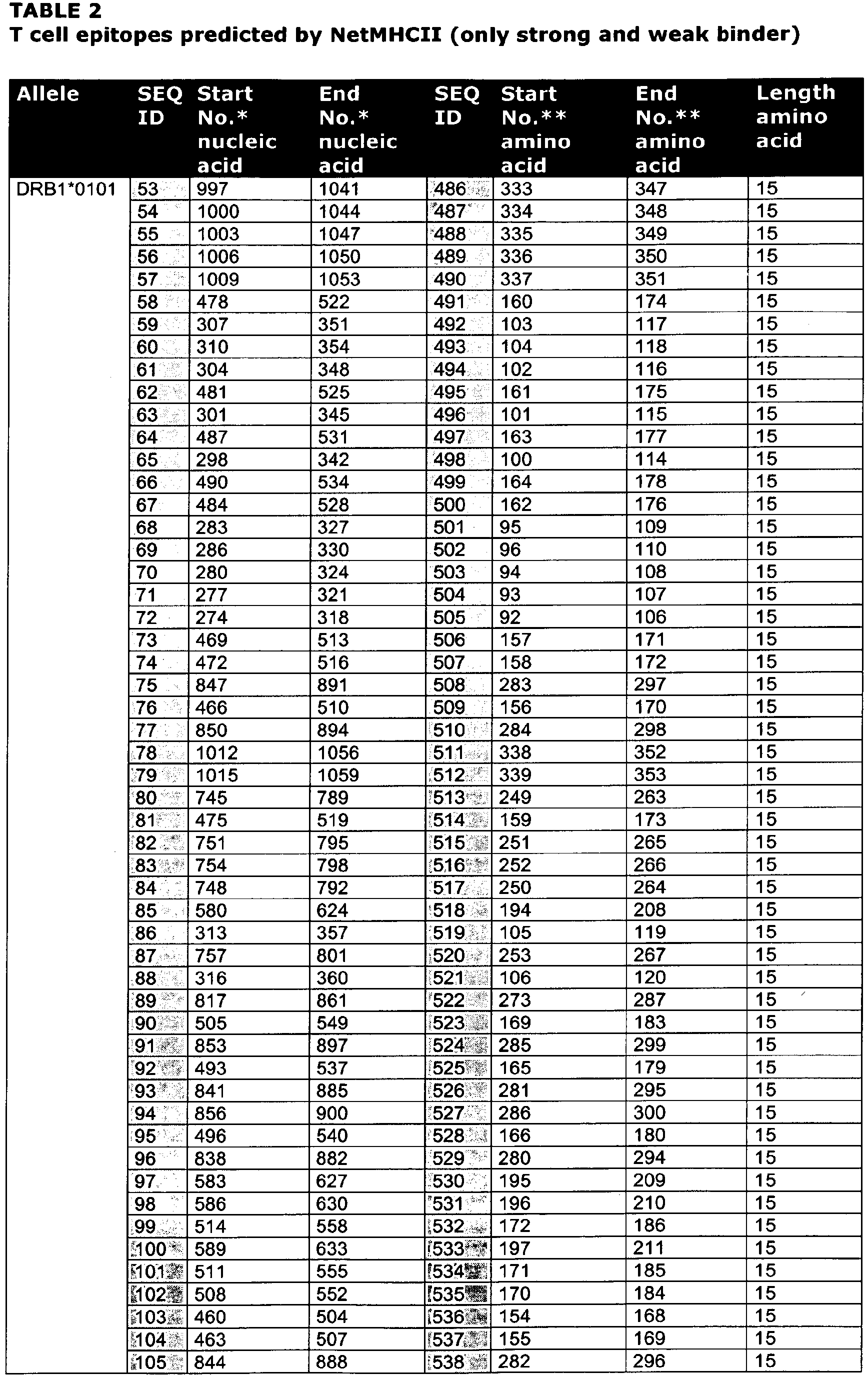

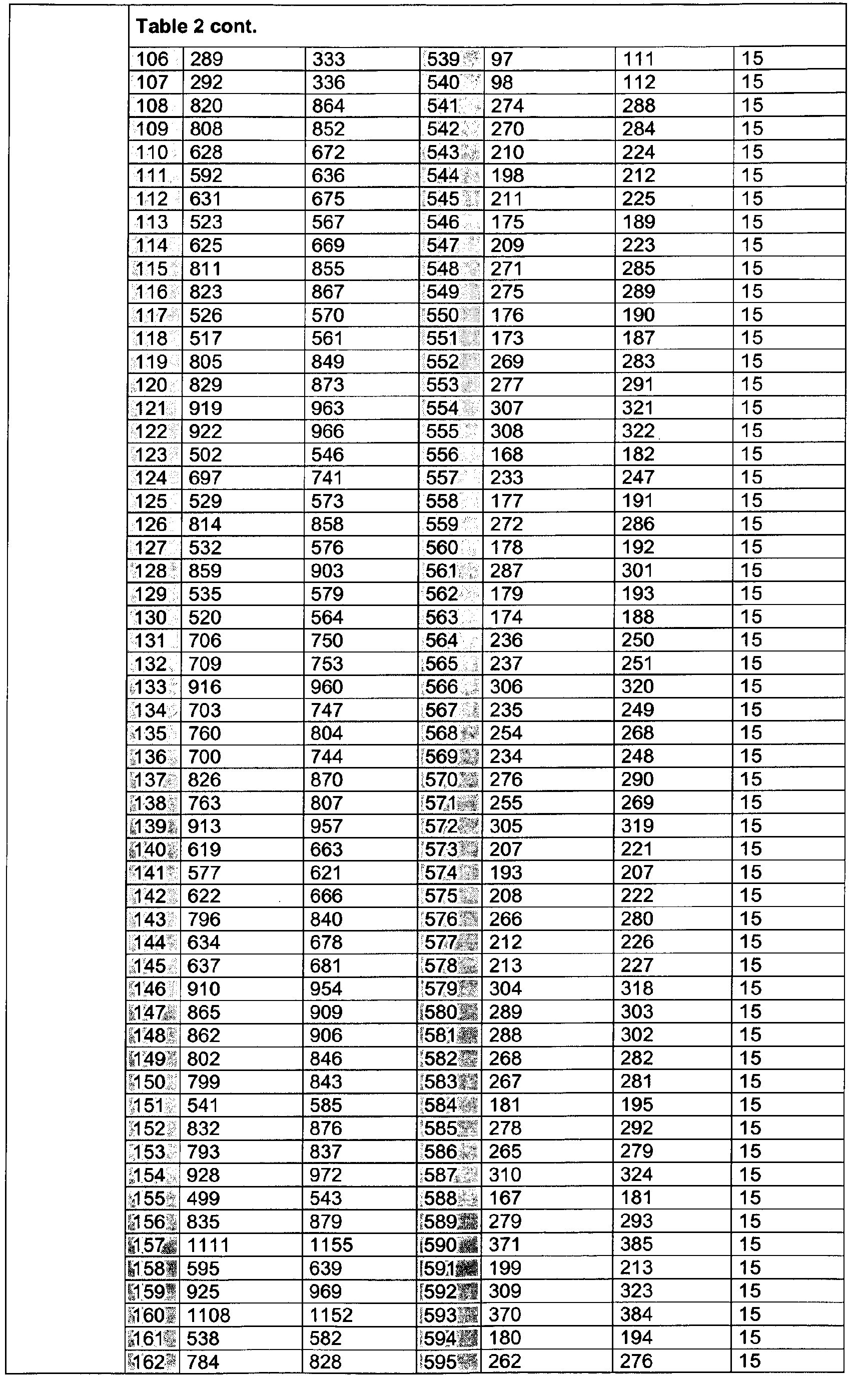

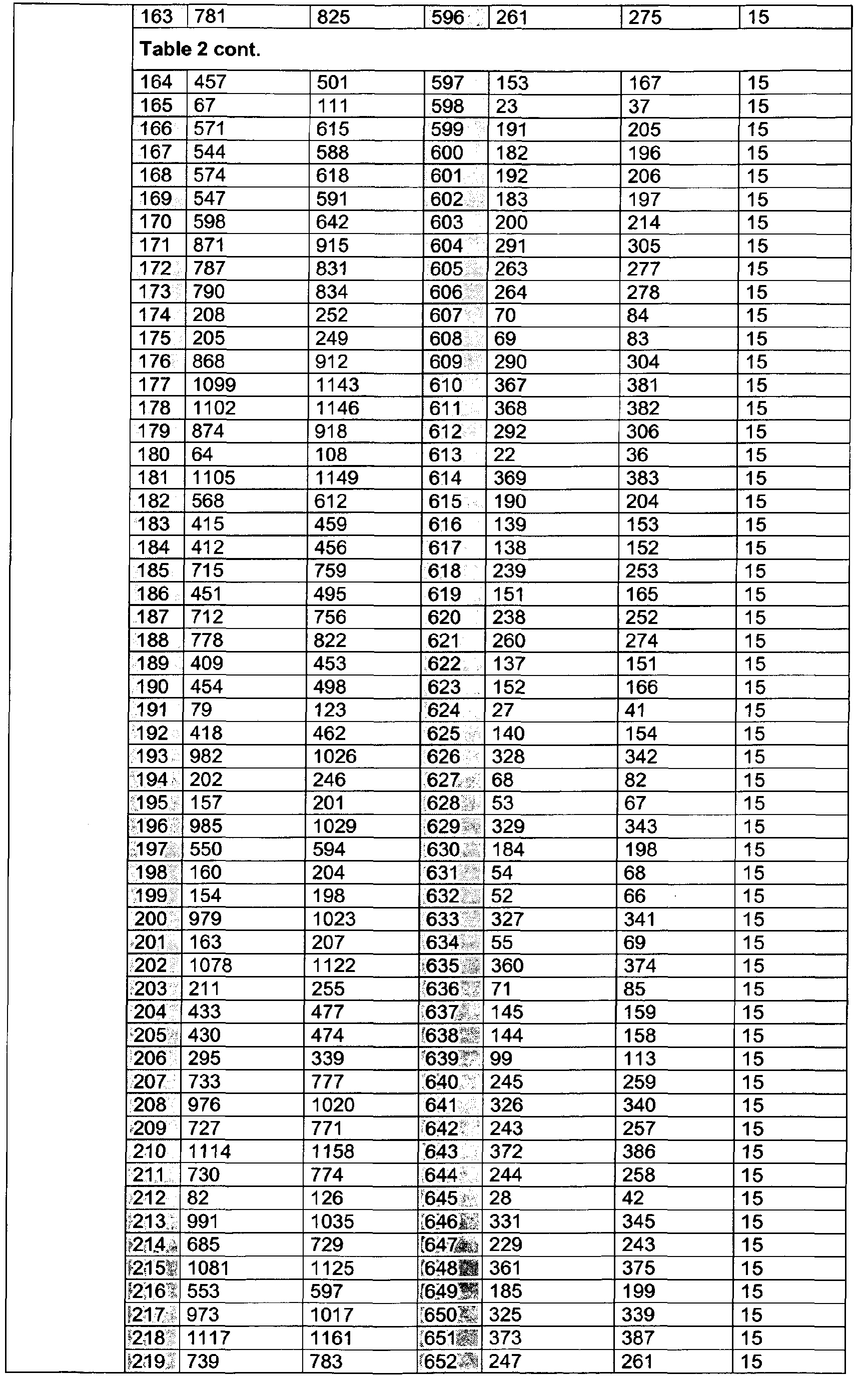

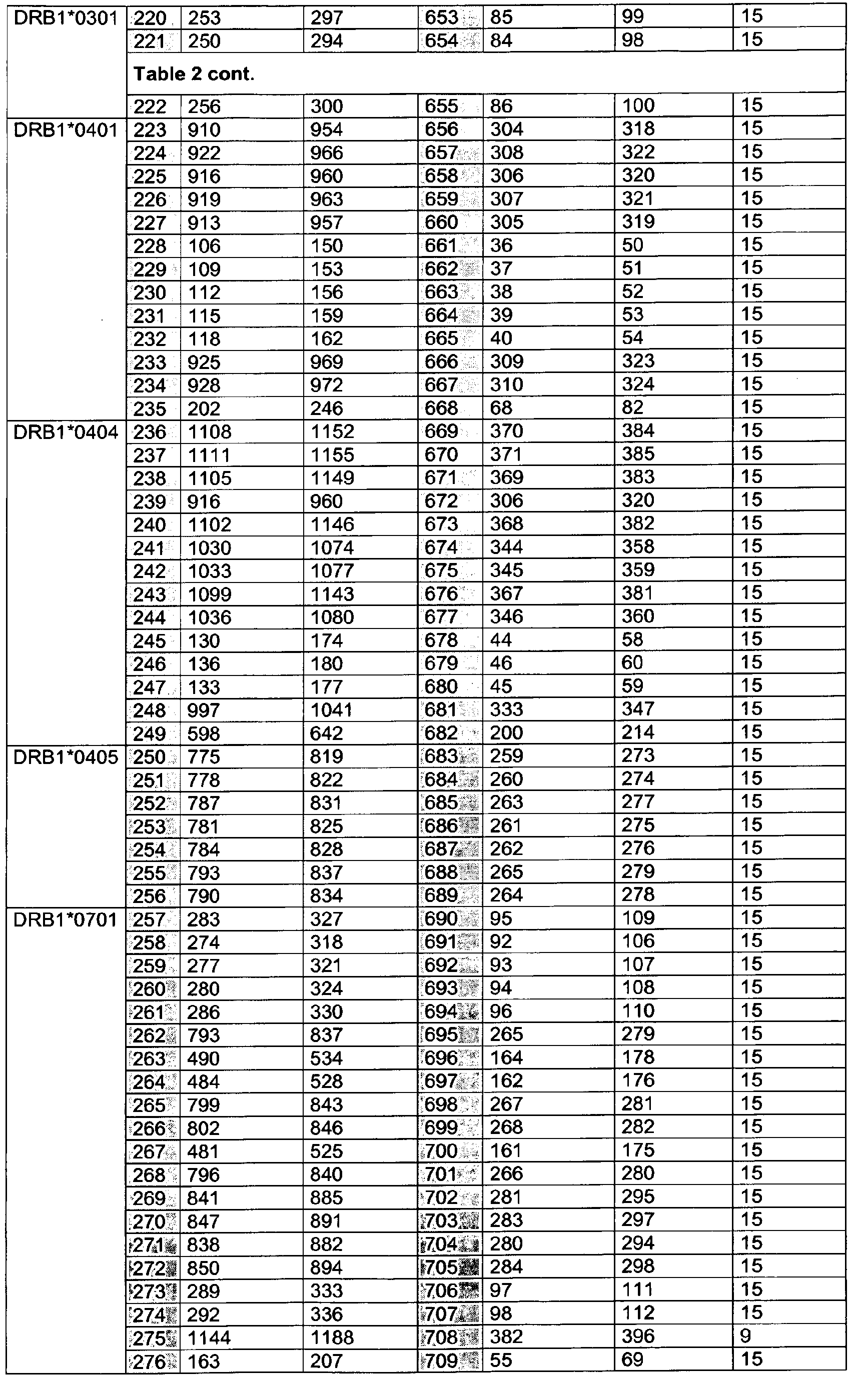

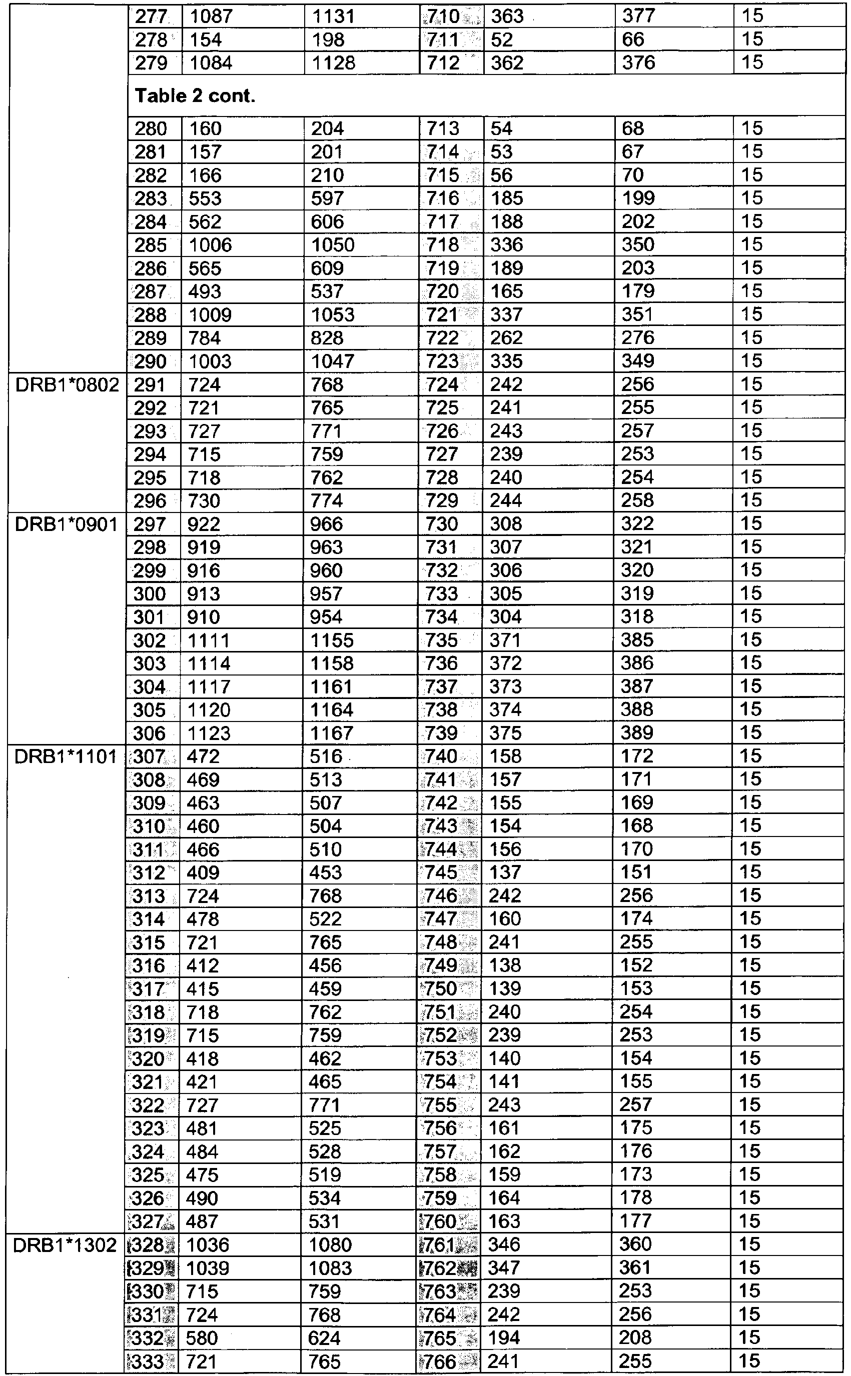

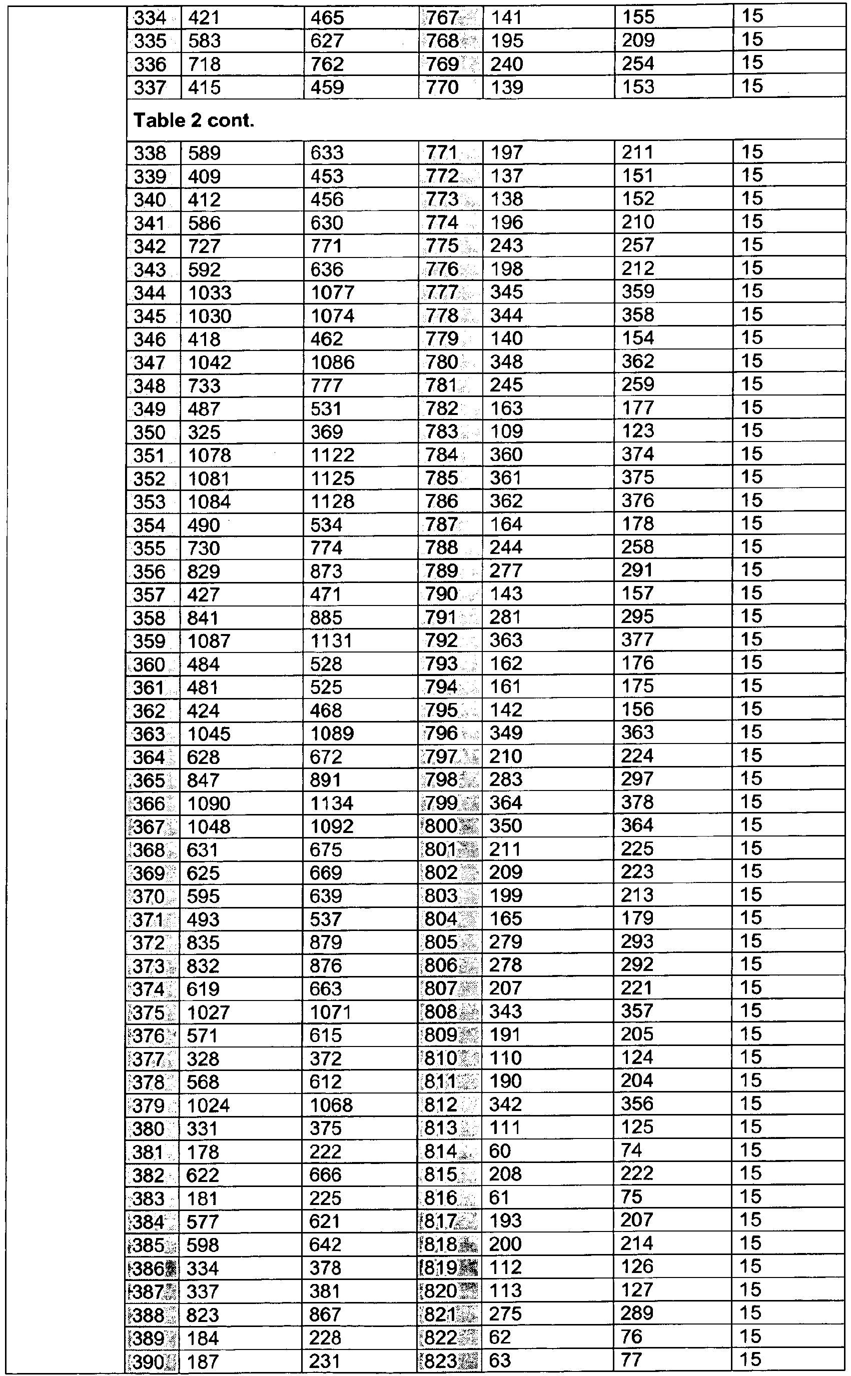

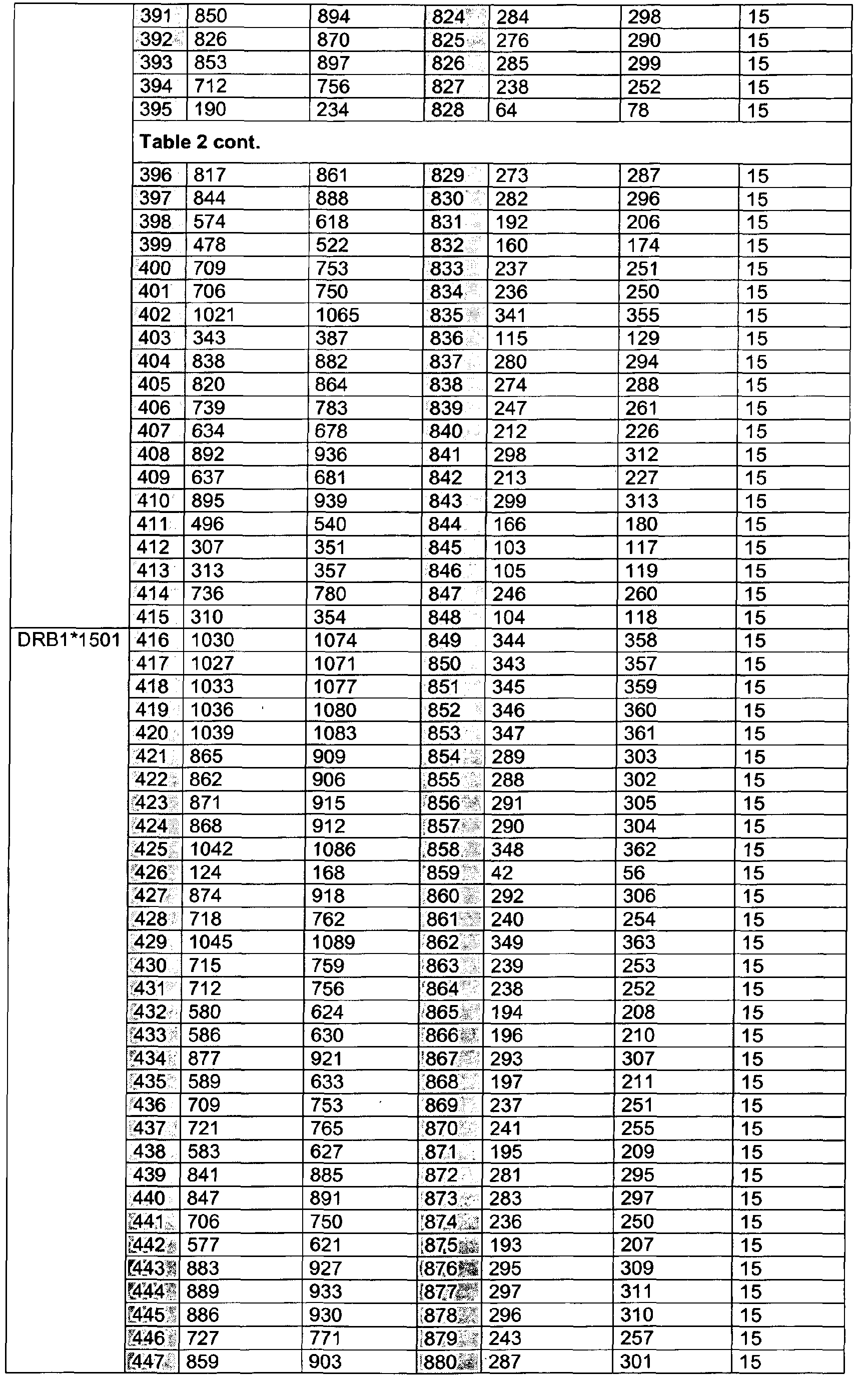

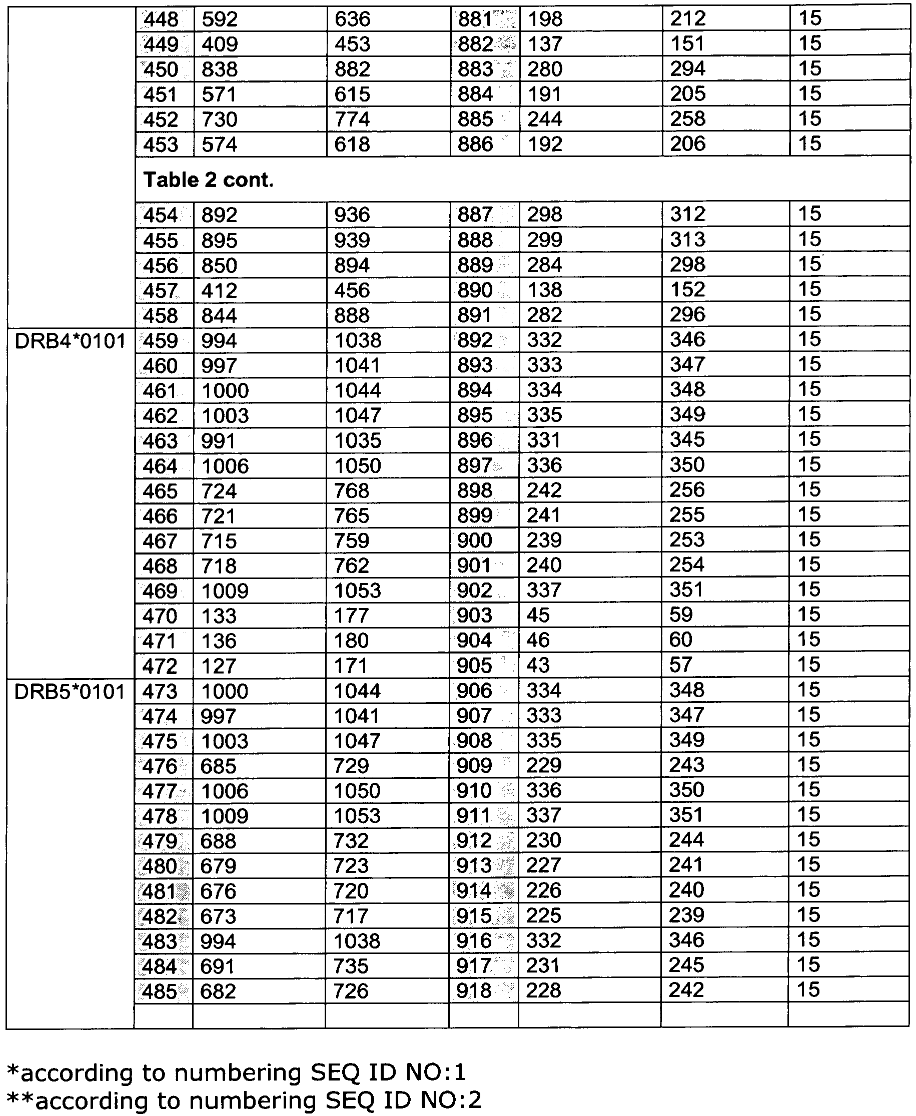

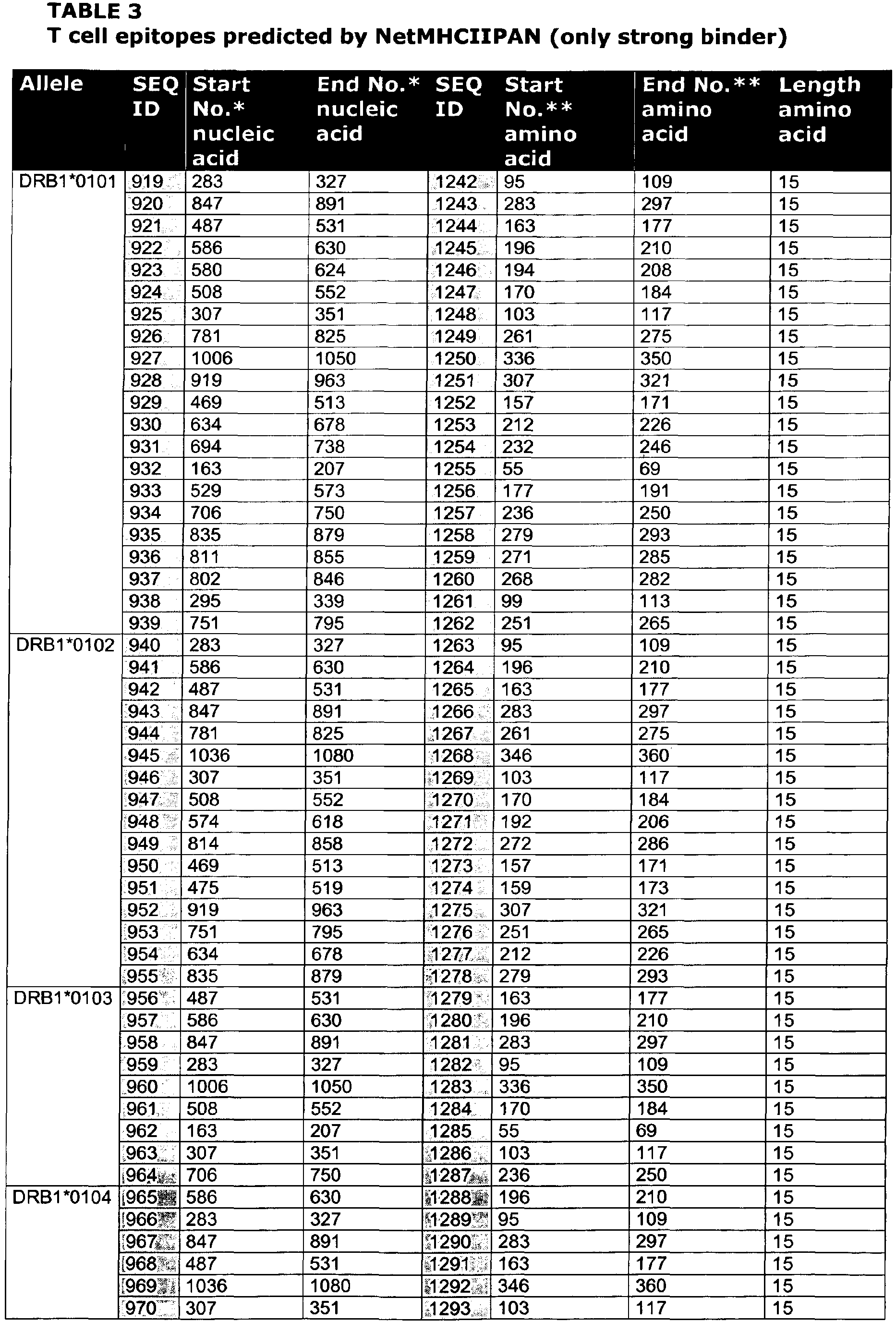

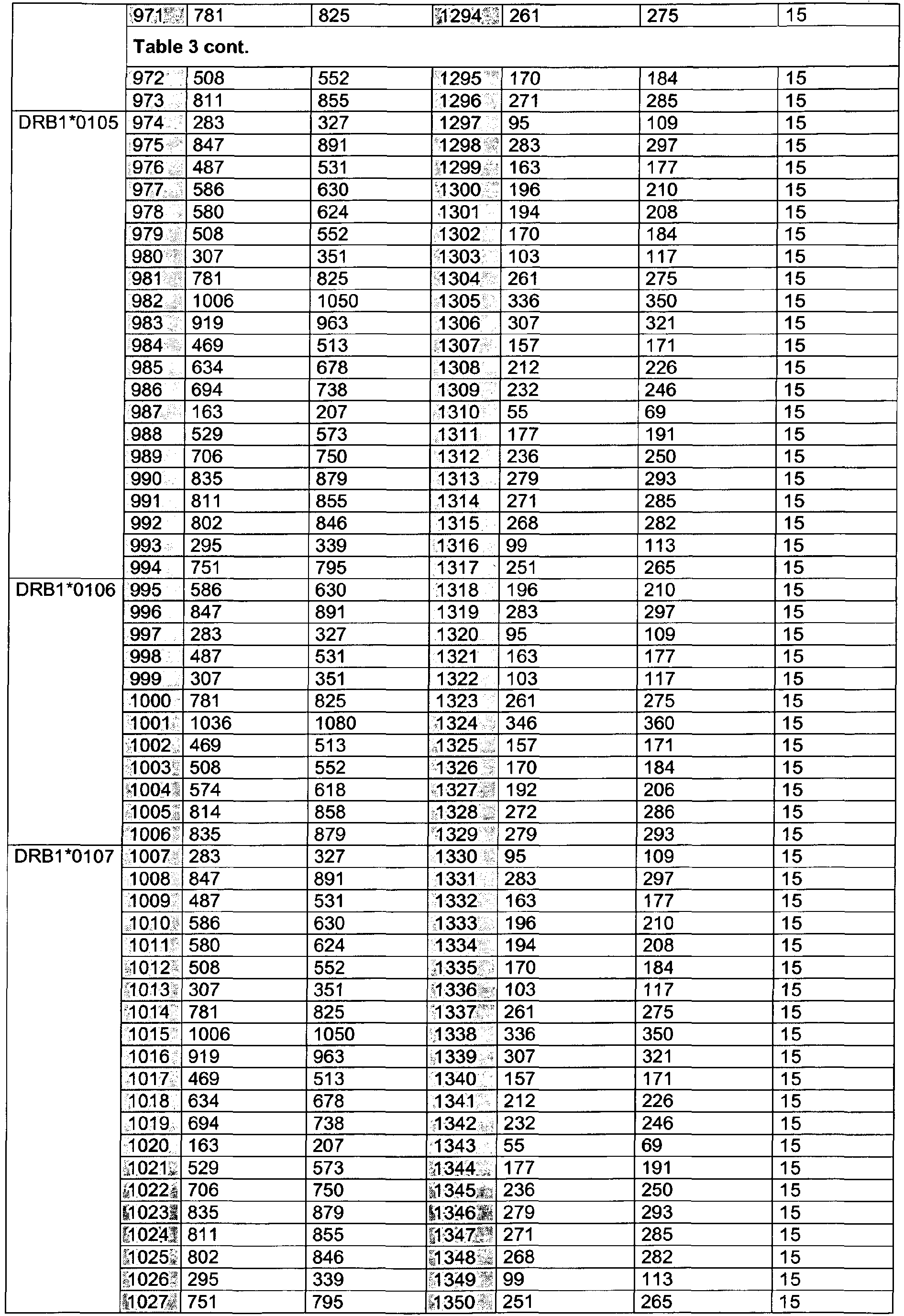

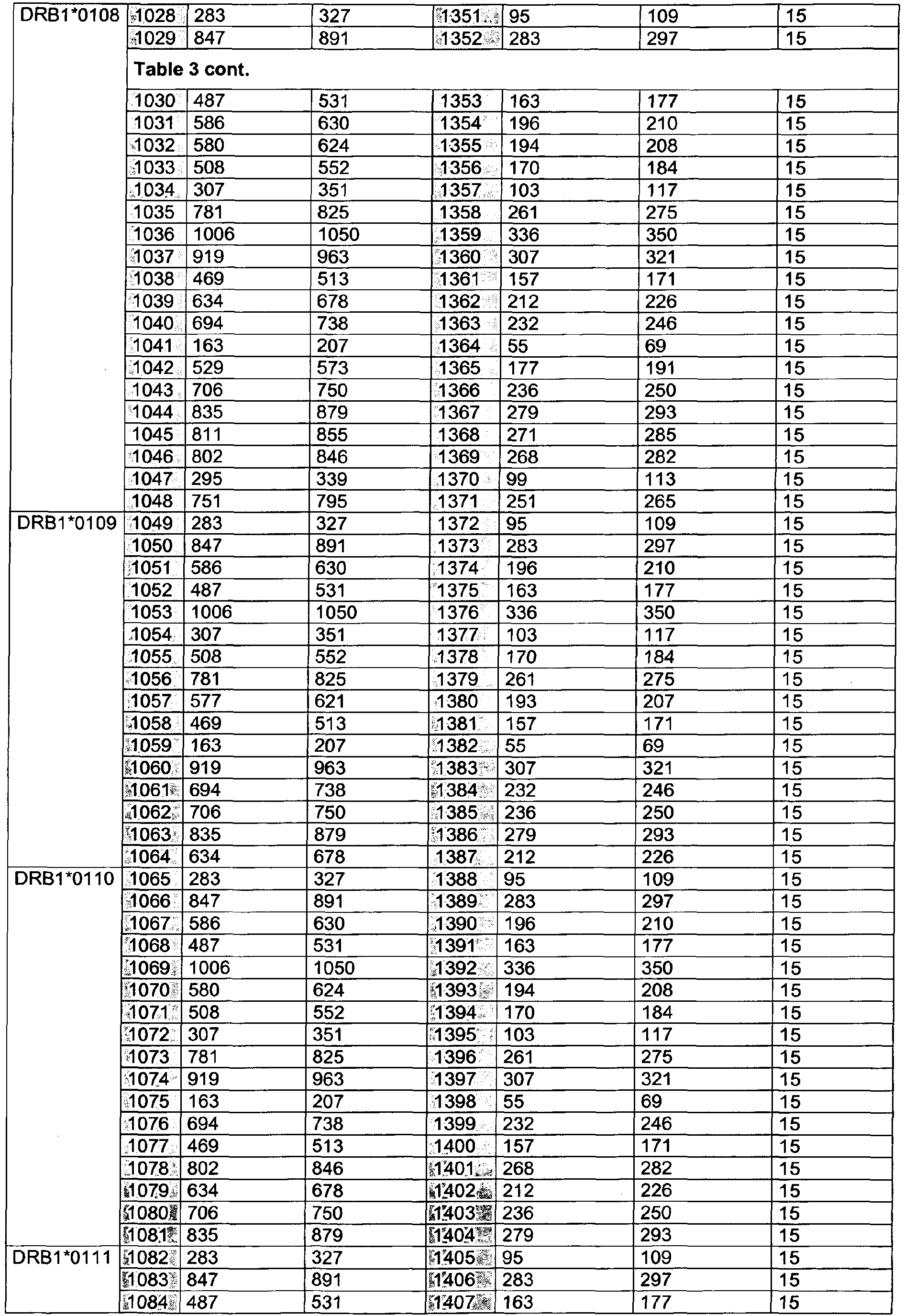

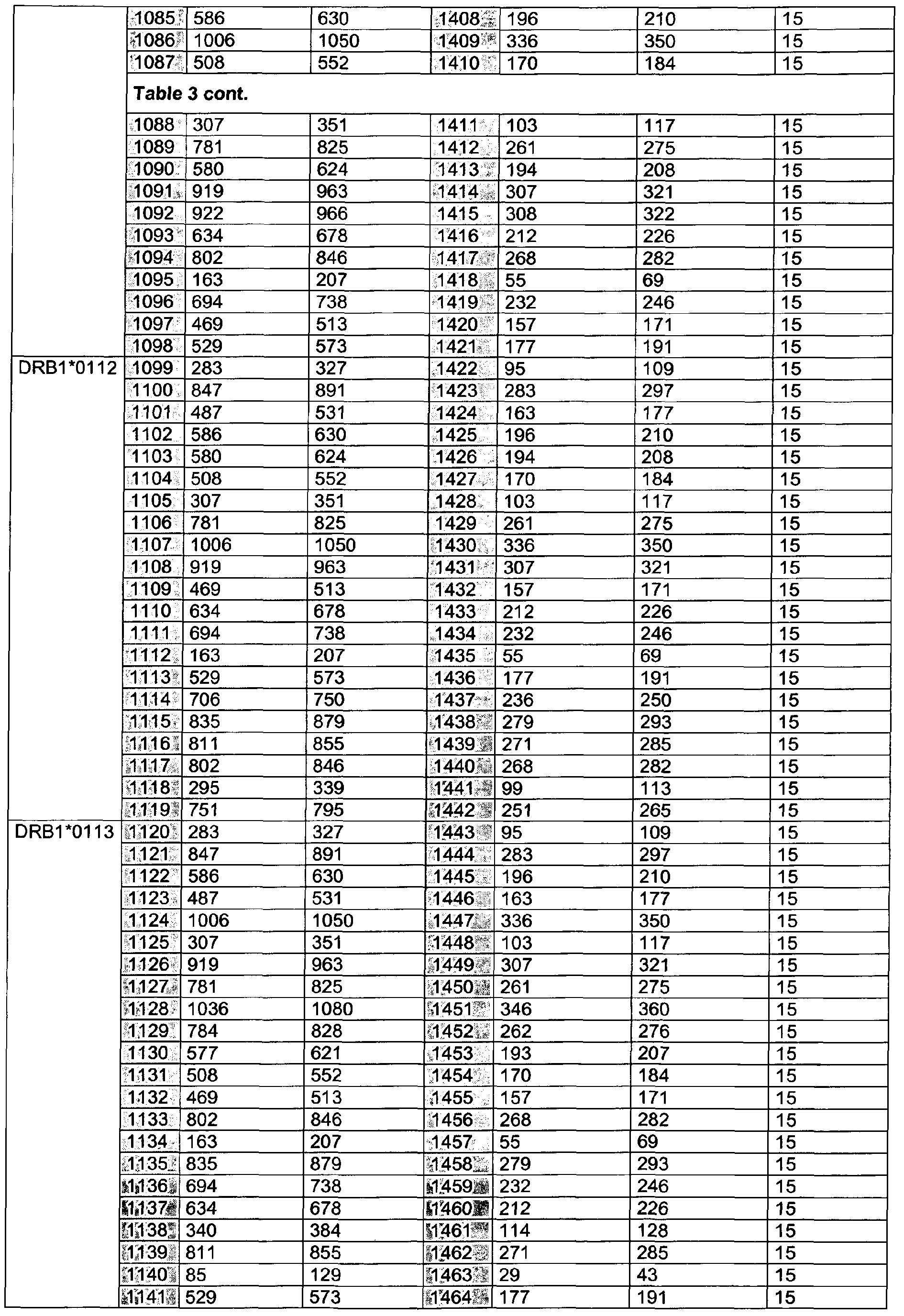

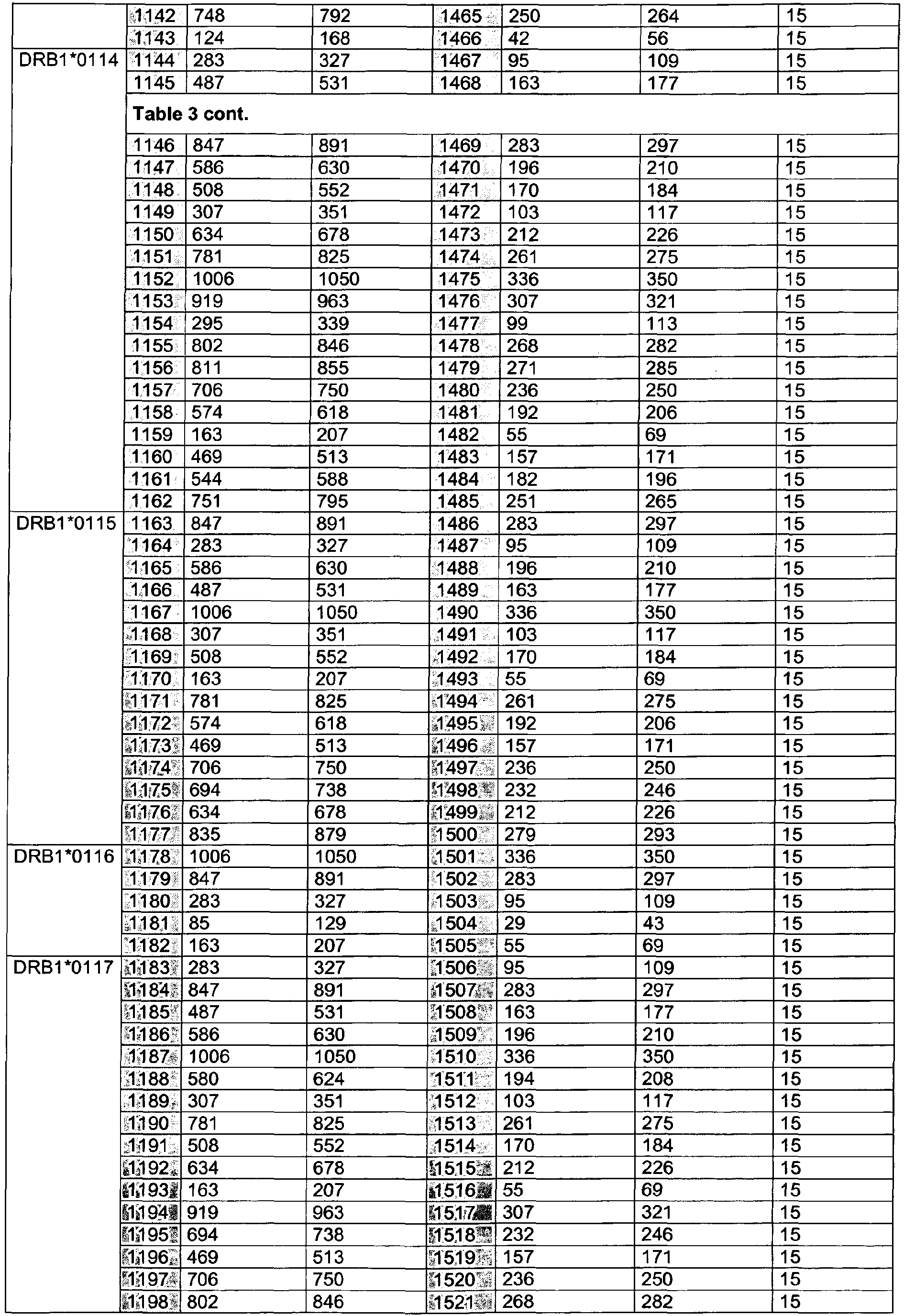

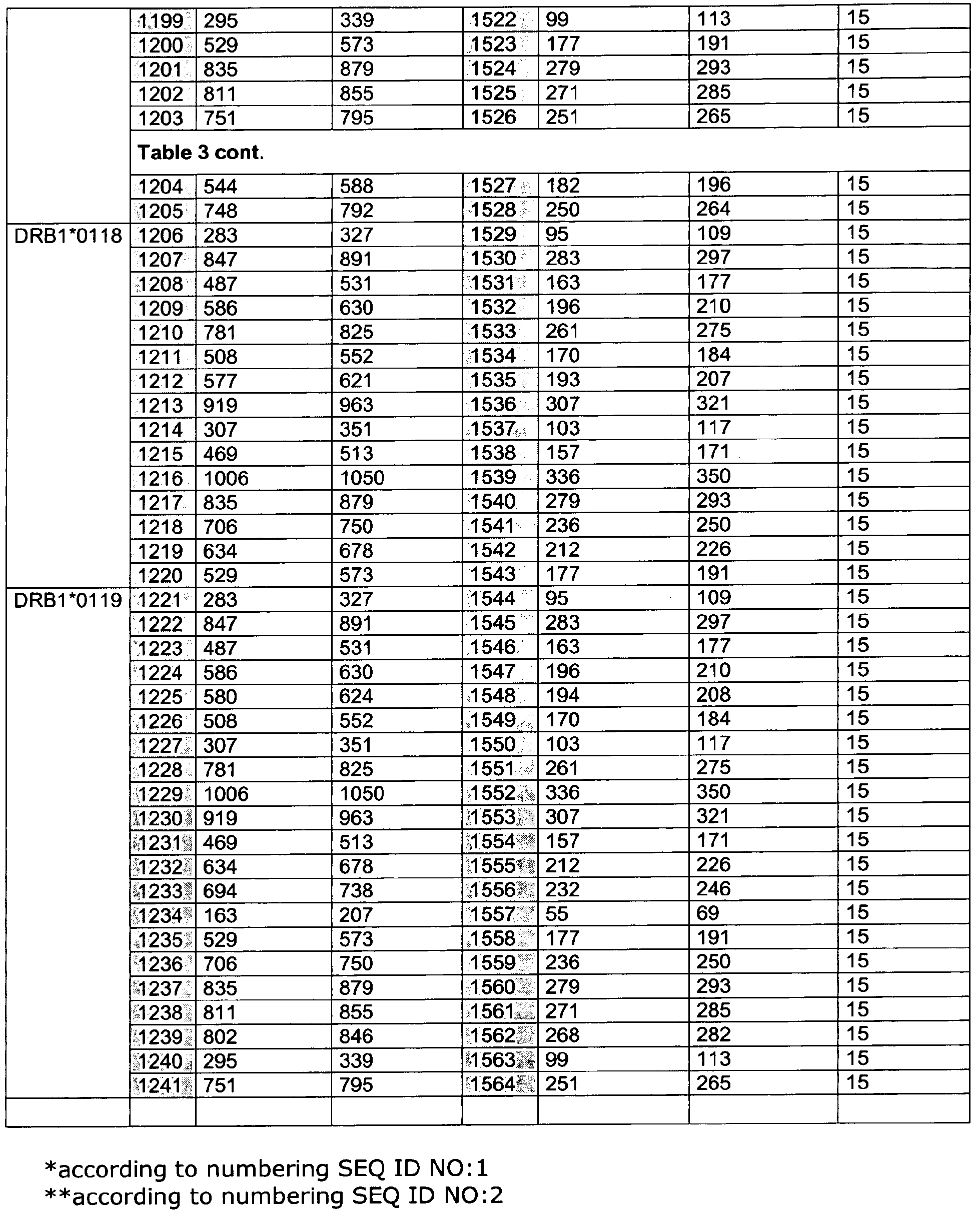

**TABLE 4**

| **Calculation of putative surface epitopes per protein size ratio** | | | | |
|---|---|---|---|---|
| Antigen | Size | Surface (Å²) | Surface epitopes* | Surface epitopes /kda |
| Ambt5 | 4.3 kDa | 2438.3 | 2.57 | 0.6 |
| Api m 1 | 16-20 kDa | 7606.4 | 8.0 | 0.4 |
| Api m 2 | 43 kDa | 15905.5 | 16.74 | 0.39 |
| Api m 4 | 3 kDa | 3885.7 | 4.09 | 1.36 |
| Ara t 8 | 14.2 kDa | 7080.1 | 7.45 | 0.52 |
| Asp f 1 | 16.8 kDa | 16037.6 | 16.88 | 1.0 |
| Asp f 6 | 23.3 kDa | 8793.2 | 9.26 | 0.4 |
| Bet v 1 | 17.4 kDa | 5215.3 | 5.49 | 0.32 |
| Bet v 2 | 14.3 kDa | 6493.9 | 6.84 | 0.48 |
| Bos d 4 | 14.2 kDa | 7246.9 | 7.63 | 0.54 |
| Bos d 5 | 18.2 kDa | 9546.5 | 10.05 | 0.55 |
| Bos d 5 | 18.2 kDa | 9618.4 | 10.12 | 0.56 |
| Der f2 | 15.8 kDa | 7785.2 | 8.19 | 0.52 |
| Der p2 | 16 kDa | 7588.8 | 7.99 | 0.5 |
| Equ c 1 | 20 kDa | 8907.4 | 9.38 | 0.47 |
| Gal d 3 | 75.8 kDa | 15952.9 | 16.79 | 0.22 |
| Gal d 4 | 16.2 kDa | 6951.3 | 7.32 | 0.45 |
| Hev b 8 | 14 kDa | 11982 | 12.61 | 0.9 |
| Mus m 1 | 18.7 kDa | 8943.5 | 9.41 | 0.5 |
| Phl p 1 | 26.1 kDa | 12145.6 | 12.78 | 0.49 |
| Phl p 2 | 10.8 kDa | 6099.5 | 6.42 | 0.59 |
| Phl p 6 | 11.8 kDa | 5429.5 | 5.72 | 0.48 |
| Pru av 1 | 17.7 kDa | 9742.8 | 10.26 | 0.58 |
| Ves v 5 | 25.8 kDa | 11657.1 | 12.27 | 0.47 |
| Zea m 14 | 11.7 kDa | 5099.5 | 5.37 | 0.46 |
| | | | | |
| | | | Average value | 0.55 +/- 0.23 |

| | | | | |
|---|---|---|---|---|
| *Estimated IgE epitope area: 950 Å² | | | | |

**TABLE 5**

| **Calculation of the average number of IgE epitopes on allergens** | | | | | | | | | |
|---|---|---|---|---|---|---|---|---|---|
| Antigen | Protein | Organism | Common | PDB code | Size (kDa) | Surface (Å²) | Size/ Surface | Possible B-cell epitopes | Identified IgE binding peptides |
| Alt a 1 | - | Alt. alternata | Fungi | - | 15.2 | - | - | - | 2 |
| Ara h 1 | Vicilin | Arachis hypogaea | Peanut | - | 67.7 | - | - | - | 21 |
| Ara h 2 | Conglutin | Arachis hypogaea | Peanut | - | 17.5 | - | - | - | 10 |
| Asp f 1 | Mitogillin | Asp. fumigatus | Fungi | 1AQZ | 16.8 | 16037.6 | 1.0 | 16-17 | 13 |
| Asp f2 | - | Asp. fumigatus | Fungi | - | 31.2 | - | - | - | 9 |
| Asp f3 | Peroximal protein | Asp. fumigatus | Fungi | - | 18.4 | - | - | - | 7 |
| Aspf13 | Oryzin | Asp. fumigatus | Fungi | | 28.7 | - | - | - | 5 |
| Bet v 1 | PR10 | Betulla verrucosa | Birch | 1BV1 | 17.4 | 5215.3 | 3.3 | 5-6 | |
| Bet v 2 | Profilin | Betulla verrucosa | Birch | 1CQA | 14.3 | 6493.9 | 2.2 | 6-7 | 3 |
| Bos d 5 | b-Lactoglobulin | Bos domesticus | Cow | 1B8E | 18.2 | 9546.5 | 1.9 | 9-10 | 7 |
| Bos d5 | b-Lactoglobulin | Bos domesticus | Cow | 1QG5 | 18.2 | 9618.4 | 1.9 | 9-10 | 7 |
| Cry j 2 | Pectinase | Cryp. japonica | Sugi | - | 42.2 | - | - | - | 4 |
| Gal d 1 | Ovomucoid | Gallus domesticus | Chicken | - | 20.1 | - | - | - | 9 (8 IgG) |
| Hev b1 | Elongaton factor | Hevea brasiliensis | Latex | - | 14.6 | - | - | - | 8 |
| Hev b3 | SRPP | Hevea brasiliensis | Latex | - | 22.3 | - | - | - | 11 |
| Hev b5 | - | Hevea brasiliensis | Latex | - | 15.9 | - | - | - | 11 |
| Jun a 1 | Pectate lyase | Juniperus ashei | Cedar | - | 37.6 | - | - | - | 4 |
| Jun a 3 | - | Juniperus ashei | Cedar | - | 21 | - | - | - | 5 |
| Parj 1 | Lipid transfer prot.1 | Parietaria judaica | Weed | - | 15 | - | - | - | 5 |
| Par j 2 | Lipid transfer prot.2 | Parietaria judaica | Weed | - | 11.3 | - | - | - | 8 |
| Pen n18 | Serine protease | Pen. notatum | Fungi | - | 52.4 | - | - | - | 9 |

**TABLE 6**

| **Prediction of linear B cell epitopes by BepiPred 1.0b** | | | |
|---|---|---|---|
| Putative B-cell epitope | SEQ ID | Amino acid positions predicted* | Sequence |
| **1** | **1565** | **22** | **T** |
| **2** | **1566** | **32.34** | **K L K** |
| **3** | **1567** | **44-50.52** | **P D F P N Y . M** |
| **4** | **1568** | **59-61.63** | **S A R . N** |
| **5** | **1569** | **74-81** | **D V P P S S N C** |
| **6** | **1570** | **91** | **D** |
| **7** | **1571** | **122-124** | **N T Y** |
| **8** | **1572** | **143-152.154-159** | **W K N P S R I V G G . E T G V N E** |
| **9** | **1573** | **212-220** | **T W A I N D T R A** |
| **10** | **1574** | **234-238** | **N Y R P** K |
| **11** | **1575** | **328-334.336-344** | **A C Q F D S G . P I L W Q N P K S** |
| **12** | **1576** | **358-366** | **T C A D E A P G V** |
| **13** | **1577** | **379-385** | **R S T P G E I** |

| | | | |
|---|---|---|---|
| *numbering according to SEQ ID NO.2 | | | |

## Claims

1. An isolated nucleic acid molecule comprising the nucleotide sequence as shown in SEQ ID NO: 1, or
isolated nucleic acid molecules that are more than 90% identical to the nucleotide sequence as shown in SEQ ID NO: 1, or
isolated fragments of the nucleic acid molecule comprising the nucleotide sequence as shown in SEQ ID NO: 1, wherein said fragments are between 150 and 1172 nucleotides in length.

2. The nucleic acid of claim 1, wherein the nucleotide sequence encodes polypeptides comprising one or more B cell epitopes of the *Vespula* venom protease Ves v 4, one or more T cell epitopes of the *Vespula* venom protease Ves v 4 or one or more B cell epitopes and one or more T cell epitopes of the *Vespula* venom protease Ves v 4.

3. The nucleic acid of claims 1 or 2, wherein the nucleic acid has been modified by site-directed mutagenesis, wherein the modification preferably affects one or more of the native N-glycosylation sites, and wherein the nucleic acid preferably encodes a Ves v 4 chimeric or fusion protein.

4. A vector comprising one or more nucleic acid molecule(s) of claims 1 to 3 operationally associated with a promoter.

5. A host cell comprising the vector of claim 4.

6. An isolated polypeptide comprising the amino acid sequence as shown in SEQ ID NO:2
or an isolated polypeptide that is more than 90% identical, more preferably more than 95% identical and most preferably more than 99% identical to the amino sequence as shown in SEQ ID NO: 2,
or an isolated fragment of the amino acid sequence as shown in SEQ ID NO:2, wherein said fragment is a partial segment of the polypeptide sequence shown in SEQ ID NO:2 which is at least 50 amino acids long.

7. The polypeptide of claim 6, containing a polypeptide fragment, wherein the polypeptide fragment is selected from the group consisting of amino acid residues 22 to 74, 75 to 135, 136 to 190, 191 to 240, 241 to 284, 285 to 334, and 335 to 390 of said polypeptide, wherein the numbering corresponds to the region of said polypeptide having the sequence shown in SEQ ID NO: 2,
or
wherein the polypeptide fragment is selected from the group consisting of amino acid
residues 22 to 135, 136 to 232, 233 to 313, and 314 to 390 of said polypeptide, wherein the numbering corresponds to the region of said polypeptide having the sequence shown in SEQ ID NO: 2,
or
wherein the polypeptide fragment is selected from the group consisting of amino acid residues 22 to 148, 149 to 268, and 269 to 390 of said polypeptide, wherein the numbering corresponds to the region of said polypeptide having the sequence shown in SEQ ID NO: 2.

8. The polypeptide of claim 7, wherein the N-glycosylation of the polypeptide has been modified, and
wherein the modified N-glycosylation is selected from the group consisting of absence of detectable core alpha(1,3)-fucosylation, mutated N-glycosylation sites, and absence of N-glycosylation.

9. A derivative of the polypeptide of claims 7 to 8, wherein the derivative is selected from the group consisting of chimeric or fusion protein, a mutant comprising one amino acid substitution, a mutant comprising one amino acid substitution that increase resistance of the polypeptide to proteolytic degradation and a mutant comprising one amino acid substitution that introduce one canonical protease sensitive site, and wherein the chimeric or fusion protein comprises the polypeptide of claims 7 to 8 and a non-Ves v 4 polypeptide selected from the group consisting of poly-Histidine tag (His tag), glutathione-S-transferase (GST), maltose binding protein (MBP), a chitin binding domain (CBD), 13-galactosidase, an IgG-Fc, a therapeutic polypeptide, preferably a cytokine, and other vespid venom proteins.

10. A pharmaceutical composition comprising one or more of the polypeptide(s) of claims 7 to 9 and a pharmaceutically acceptable carrier.

11. The pharmaceutical composition of claim 10, wherein the one or more polypeptide(s) map the total length of the Ves v 4 polypeptide, wherein the pharmaceutical composition further comprises one or more additional polypeptide(s) selected from the group consisting of Ves v 1 polypeptide (phospolipase Al), Ves v 2a polypeptide (hyaluronidase), Ves v 2b polypeptide, Ves v 3 polypeptide (dipeptidylpeptidase), Ves v 5 polypeptide, glycosylated IgE-binding proteins.

12. The polypeptide of claims 7 to 9 or the pharmaceutical composition of claims 10 or 11 for use as a medicament.

13. The polypeptide of claims 7 to 9
or polypeptide fragments, wherein the
polypeptide fragment selected from the group consisting of amino acid residues 22 to 74, 75 to 135, 136 to 190, 191 to 240, 241 to 284, 285 to 334, and 335 to 390 of said polypeptide, wherein the numbering corresponds to the region of said polypeptide having the sequence shown in SEQ ID NO: 2,
or
wherein the polypeptide fragment is selected from the group consisting of amino acid
residues 22 to 135, 136 to 232, 233 to 313, and 314 to 390 of said polypeptide, wherein the numbering corresponds to the region of said polypeptide having the sequence shown in SEQ ID NO: 2,
or
wherein the polypeptide fragment is selected from the group consisting of amino acid residues 22 to 148, 149 to 268, and 269 to 390 of said polypeptide, wherein the numbering corresponds to the region of said polypeptide having the sequence shown in SEQ ID NO: 2
or
the pharmaceutical composition of claims 10 or 11
for use in the treatment of allergy against *Vespula* venom.

14. The polypeptide or the pharmaceutical composition of claim 13 for use in the treatment of allergy against the *Vespula* venom protease Ves v 4.

15. The polypeptide or the pharmaceutical composition for use in the treatment of allergy against *Vespula* venom or against the *Vespula* venom protease Ves v 4 of claims 13 or 14, wherein the polypeptide or the pharmaceutical composition are administered in an amount sufficient to reduce or inhibit an immune reaction against the Ves v 4 polypeptide, and
wherein the polypeptide and pharmaceutical composition is administered over a period of time in gradually increasing doses.

16. Use of the polypeptide of claims 7 to 9 or of the pharmaceutical composition of claims 10 or 11 for the generation of a medicament for the treatment of allergy against *Vespula* venom, preferably of allergy against the *Vespula* venom protease Ves v 4.

17. An in vitro method for decreasing the allergenicity of a Ves v 4 poly-peptide comprising the steps of
a) providing a nucleic acid molecule of claims 1 to 3 or a nucleic acid molecule encoding a polypeptide of claims 7 to 9,
b) applying site-directed mutagenesis to said nucleic acid molecule so that one or more IgE epitope(s) on the encoded polypeptide is altered to give a modified allergen, and
c) determining the reduction in allergenicity of said modified allergen.

18. An in vitro method for detecting serum IgE or IgG antibodies specific for the polypeptides of claims 7 to 9, comprising the steps of
a) providing serum from an allergic patient, and
b) detecting said IgE or IgG antibodies in said serum by a detection method,
wherein said detection method is selected from the group consisting of radio-allergosorbent test (RAST), enzyme-linked immunosorbent assays (ELISA), immunoelectrophoresis, immunoblot, immunodotblotting, bead array technology, fluid phase systems, and in vitro mediator release assays (MRA).

19. The method of claim 18, wherein said detecting comprises in vitro measuring the ratio of serum IgE and IgG4 antibodies specific for the polypeptides of claims 7 to 9, and
wherein said method further comprises the step of
c) evaluating the success of immunotherapeutical treatment.

## Patentansprüche

1. Isoliertes Nukleinsäuremolekül, welches die Nuk-. leotidsequenz wie in SEQ ID NO:1 gezeigt umfasst, oder
isolierte Nukleinsäuremoleküle, die zu mehr als 90 % identisch mit der Nukleotidsequenz wie in SEQ ID NO:1 gezeigt sind, oder
isolierte Fragmente des Nukleinsäuremoleküls, welche die Nukleotidsequenz wie in SEQ ID NO:1 gezeigt umfassen, wobei die Fragmente eine Länge zwischen 150 und 1172 Nukleotiden aufweisen.

2. Nukleinsäure nach Anspruch 1, wobei die Nukleotidsequenz Polypeptide codiert, welche ein oder mehrere B-Zell-Epitope der *Vespula*-Venom-Protease Ves v4, ein oder mehrere T-Zell-Epitope der *Vespula*-Venom-Protease Ves v4 oder ein oder mehrere B-Zell-Epitope und ein oder mehrere T-Zell-Epitope der *Vespula*-Venom-Protease Ves v4 umfassen.

3. Nukleinsäure nach den Ansprüchen 1 oder 2, wobei die Nukleinsäure durch ortsspezifische Mutagenese modifiziert worden ist, wobei die Modifizierung bevorzugt eine oder mehrere der nativen N-Glykosylierungsstellen beeinflusst, und wobei die Nukleinsäure bevorzugt ein chimäres Ves v4- oder Fusionsprotein codiert.

4. Vektor, welcher ein oder mehrere Nukleinsäuremoleküle nach den Ansprüchen 1 bis 3 umfasst, funktionsmäßig mit einem Promotor verbunden.

5. Wirtszelle, welche den Vektor nach Anspruch 4 umfasst.

6. Isoliertes Polypeptid, welches die Aminosäuresequenz wie in SEQ ID NO:2 gezeigt umfasst,
oder isoliertes Polypeptid, das zu mehr als 90 % identisch, mehr bevorzugt zu mehr als 95 % identisch und am meisten bevorzugt zu mehr als 99 % identisch mit der Aminosequenz wie in SEQ ID NO:2 gezeigt ist,
oder isoliertes Fragment der Aminosäuresequenz wie in SEQ ID NO:2 gezeigt, wobei das Fragment ein partielles Segment der in SEQ ID NO:2 gezeigten Polypeptidsequenz ist, welche mindestens 50 Aminosäuren lang ist.

7. Polypeptid nach Anspruch 6, welches ein Polypeptidfragment enthält, wobei das Polypeptidfragment ausgewählt ist aus der Gruppe bestehend aus den Aminosäureresten 22 bis 74, 75 bis 135, 136 bis 190, 191 bis 240, 241 bis 284, 285 bis 334 und 335 bis 390 des Polypeptids, wobei die Nummerierung der Region des Polypeptids mit der in SEQ ID NO:2 gezeigten Sequenz entspricht,
oder
wobei das Polypeptidfragment ausgewählt ist aus der Gruppe bestehend aus den Aminosäureresten 22 bis 135, 136 bis 232, 233 bis 313 und 314 bis 390 des Polypeptids, wobei die Nummerierung der Region des Polypeptids mit der in SEQ ID NO:2 gezeigten Sequenz entspricht,
oder
wobei das Polypeptidfragment ausgewählt ist aus der Gruppe bestehend aus den Aminosäureresten 22 bis 148, 149 bis 268 und 269 bis 390 des Polypeptids, wobei die Nummerierung der Region des Polypeptids mit der in SEQ ID NO:2 gezeigten Sequenz entspricht.

8. Polypeptid nach Anspruch 7, wobei die N-Glykosylierung des Polypeptids modifiziert worden ist, und
wobei die modifizierte N-Glykosylierung bevorzugt ausgewählt ist aus der Gruppe bestehend aus der Abwesenheit einer erfassbaren Core-Alpha(1,3)-Fukosylation, mutierten N-Glykosylierungsstellen und Abwesenheit einer N-Glykosylierung.

9. Derivat des Polypeptids nach den Ansprüchen 7 bis 8, wobei das Derivat ausgewählt ist aus der Gruppe bestehend aus einem chimären oder Fusionsprotein, einer Mutante, welche eine Aminosäuresubstitution umfasst, einer Mutante, welche eine Aminosäuresubstitution umfasst, die die Beständigkeit des Polypeptids gegen proteolytische Zersetzung erhöht, und einer Mutante, welche eine Aminosäuresubstitution umfasst, die eine kanonische protease-sensitive Stelle einführt, und wobei das chimäre oder Fusionsprotein das Polypeptid nach den Ansprüchen 7 bis 8 und ein Nicht-Ves v4-Polypeptid ausgewählt aus der Gruppe bestehend aus Polyhistidin-Tag (His-Tag), Glutathion-S-Transferase (GST), Maltose-Bindungsprotein (MBP), einer Chitin bindenden Domäne (CBD), 13-Galactosidase, einem IgG-Fc, einem therapeutischen Polypeptid, bevorzugt einem Zytokin und weiteren Vespid-Venomproteinen umfasst.

10. Pharmazeutische Zusammensetzung, welche ein oder mehrere der Polypeptide nach den Ansprüchen 7 bis 9 und einen pharmazeutisch akzeptablen Träger umfasst.

11. Pharmazeutische Zusammensetzung nach Anspruch 10, wobei das eine oder die mehreren Polypeptide die Gesamtlänge des Ves v4-Polypeptids abbilden, wobei die pharmazeutische Zusammensetzung ferner ein oder mehrere weitere Polypeptide ausgewählt aus der Gruppe bestehend aus Ves v1-Polypeptid (Phospolipase A1), Ves v2a-Polypeptid (Hyaluronidase), Ves v2b-Polypeptid, Ves v3-Polypeptid (Dipeptidylpeptidase), Ves v5-Polypeptid, glykosylierten IgE-Bindungsproteinen umfasst.

12. Polypeptid nach den Ansprüchen 7 bis 9 oder pharmazeutische Zusammensetzung nach den Ansprüchen 10 oder 11 zur Verwendung als Medikament.

13. Polypeptid nach den Ansprüchen 7 bis 9 oder Polypeptidfragmente, wobei das Polypeptidfragment ausgewählt ist aus der Gruppe bestehend aus den Aminosäureresten 22 bis 74, 75 bis 135, 136 bis 190, 191 bis 240, 241 bis 284, 285 bis 334 und 335 bis 390 des Polypeptids, wobei die Nummerierung der Region des Polypeptids mit der in SEQ ID NO:2 gezeigten Sequenz entspricht,
oder
wobei das Polypeptidfragment ausgewählt ist aus der Gruppe bestehend aus den Aminosäureresten 22 bis 135, 136 bis 232, 233 bis 313 und 314 bis 390 des Polypeptids, wobei die Nummerierung der Region des Polypeptids mit der in SEQ ID NO:2 gezeigten Sequenz entspricht,
oder
wobei das Polypeptidfragment ausgewählt ist aus der Gruppe bestehend aus den Aminosäureresten 22 bis 148, 149 bis 268 und 269 bis 390 des Polypeptids, wobei die Nummerierung der Region des Polypeptids mit der in SEQ ID NO:2 gezeigten Sequenz entspricht,
oder pharmazeutische Zusammensetzung nach den Ansprüchen 10 oder 11
zur Verwendung bei der Behandlung einer Allergie auf *Vespula-*Gift*.*

14. Polypeptid oder pharmazeutische Zusammensetzung nach Anspruch 13 zur Verwendung bei der Behandlung einer Allergie auf die *Vespula*-Venom-Protease Ves v4.

15. Polypeptid oder pharmazeutische Zusammensetzung zur Verwendung bei der Behandlung einer Allergie auf *Vespula-Gift* oder auf *Vespula*-Venom-Protease Ves v4 nach den Ansprüchen 13 oder 14, wobei das Polypeptid oder die pharmazeutische Zusammensetzung in einer Menge verabreicht werden, welche ausreichend ist, eine Immunreaktion gegen das Ves v4-Polypeptid zu reduzieren oder zu hemmen, und wobei das Polypeptid und die pharmazeutische Zusammensetzung über eine Zeit in graduell zunehmenden Dosen verabreicht werden.

16. Verwendung des Polypeptids nach den Ansprüchen 7 bis 9 oder der pharmazeutischen Zusammensetzung nach den Ansprüchen 10 oder 11 für die Herstellung eines Medikaments zur Verwendung bei der Behandlung einer Allergie auf *Vespula*-Gift, bevorzugt einer Allergie auf die *Vespula*-Venom-Protease Ves v4.

17. In-Vitro-Verfahren zur Verringerung der Allergenität eines Ves v4-Polypeptids, welches die folgenden Schritte umfasst:
a) Vorsehen eines Nukleinsäuremoleküls nach den Ansprüchen 1 bis 3 oder eines Nukleinsäuremoleküls, das ein Polypeptid nach den Ansprüchen 7 bis 9 codiert,
b) Anwenden von ortsspezifischer Mutagenese auf das Nukleinsäuremolekül, so dass ein oder mehrere IgE-Epitope des codierten Polypeptids verändert werden, um ein modifiziertes Allergen zu ergeben, und
c) Bestimmen der Verringerung der Allergenität des modifizierten Allergens.

18. In-Vitro-Verfahren zum Nachweisen von Serum-IgE- oder IgG-Antikörpern, die spezifisch für die Polypeptide nach den Ansprüchen 7 bis 9 sind, welches die folgenden Schritte umfasst:
a) Vorsehen eines Serums für einen allergischen Patienten, und
b) Nachweisen der Serum-IgE- oder IgG-Antikörper in dem Serum durch ein Nachweisverfahren,
wobei das Nachweisverfahren ausgewählt wird aus der Gruppe bestehend aus dem Radioallergosorbent-Test (RAST), dem enzymgekoppelten Immunosorbent-Assay (ELISA), der Immunelektrophorese, dem Immunblot, dem Immundotblotting, der Bead-Array-Technik, Fluidphasensystemen und In-Vitro-Mediator-Release-Assays (MRA).

19. Verfahren nach Anspruch 18, wobei das Nachweisen das In-Vitro-Messen des Verhältnisses der Serum-IgE- oder IgG-Antikörper, die spezifisch für die Polypeptide nach den Ansprüchen 7 bis 9 sind, umfasst, und
wobei das Verfahren ferner den folgenden Schritt umfasst:
c) Auswerten des Erfolgs der immuntherapeutischen Behandlung.

## Revendications

1. Molécule d'acide nucléique isolée comprenant la séquence de nucléotides montrée dans SEQ ID NO:1, ou
des molécules d'acide nucléique isolées qui sont identiques par plus de 90 % à la séquence de nucléotides montrée dans SEQ ID NO:1, ou
des fragments isolés de la molécule d'acide nucléique comprenant la séquence de nucléotides montrée dans SEQ ID NO:1, les fragments ayant une longueur entre 150 et 1172 nucléotides.

2. Acide nucléique selon la revendication 1, dans lequel la séquence de nucléotides code pour des polypeptides comprenant un ou plusieurs épitopes des cellules B de la protéase de venin de *Vespula* Ves v4, un ou plusieurs épitopes des cellules T de la protéase de venin de *Vespula* Ves v4 ou un ou plusieurs épitopes des cellules B et un ou plusieurs épitopes des cellules T de la protéase de venin de *Vespula* Ves v4.

3. Acide nucléique selon la revendication 1 ou 2, dans lequel l'acide nucléique a été modifié par mutagenèse dirigée par le site, dans lequel la modification de préférence affecte un ou plusieurs des sites de N-glycosylation natifs, et dans lequel l'acide nucléique de préférence code pour une protéine Ves v4 chimérique ou de fusion.

4. Vecteur comprenant une ou plusieurs molécules d'acide nucléique selon la revendication 1 à 3 fonctionnellement associées à un promoteur.

5. Cellule hôte comprenant le vecteur selon la revendication 4.

6. Polypeptide isolé comprenant la séquence d'acide aminé montrée dans SEQ ID NO:2
ou un polypeptide isolé qui est identique par plus de 90 %, de préférence plus haute identique par plus de 95 % et de préférence la plus haute identique par plus de 99 % à la séquence d'acide aminé montrée dans SEQ ID NO:2,
ou un fragment isolé de la séquence d'acide aminé montrée dans SEQ ID NO:2, dans lequel le fragment est un segment partielle de la séquence de polypeptide montrée dans SEQ ID NO:2 qui a une longueur d'au moins de 50 acides aminés.

7. Polypeptide selon la revendication 6, comportant un fragment de polypeptide, dans lequel le fragment de polypeptide est choisi à partir du groupe consistant en les résidus d'acides aminés 22 à 74, 75 à 135, 136 à 190, 191 à 240, 241 à 284, 285 à 334 et 335 à 390 du polypeptide, dans lequel la numérotation correspond à la région du polypeptide ayant la séquence montrée dans SEQ ID NO:2,
ou
dans lequel le fragment de polypeptide est choisi à partir du groupe consistant en les résidus d'acides aminés 22 à 135, 136 à 232, 233 à 313 et 314 à 390 du polypeptide, dans lequel la numérotation correspond à la région du polypeptide ayant la séquence montrée dans SEQ ID NO:2
ou
dans lequel le fragment de polypeptide est choisi à partir du groupe consistant en les résidus d'acides aminés 22 à 148, 149 à 268, et 269 à 390 du polypeptide, dans lequel la numérotation correspond à la région du polypeptide ayant la séquence montrée dans SEQ ID NO:2.

8. Polypeptide selon la revendication 7, dans lequel la N-glycosylation du polypeptide a été modifiée, et
dans lequel la N-glycosylation modifiée de préférence est choisie à partir du groupe consistant en absence de core alpha(1,3)-fucosylation détectable, sites mutés de N-glycosylation, et absence de N-glycosylation.

9. Dérivé du polypeptide selon la revendication 7 à 8, dans lequel le dérivé est choisi à partir du groupe consistant en une protéine chimérique ou de fusion, un mutant comprenant une substitution d'acide aminé, un mutant comprenant une substitution d'acide aminé qui augmente la résistance du polypeptide à la dégradation protéolytique et un mutant comprenant une substitution d'acide aminé qui introduit un site sensible de protéase canonique, et dans lequel la protéine chimérique ou de fusion comprend le polypeptide selon la revendication 7 à 8 et un polypeptide non-Ves v4 choisi à partir du groupe consistant en une étiquette poly-histidine (étiquette His), glutathione-S-transférase (GST), la protéine de maltose binding (MBP), un domaine de chitine binding (CBD), 13-galactosidase, un IgG-Fc, un polypeptide thérapeutique, de préférence une cytokine, et d'autres protéines de venin vespide.

10. Composition pharmaceutique comprenant un ou plusieurs des polypeptides selon la revendication 7 à 9 et un excipient pharmaceutiquement acceptable.

11. Composition pharmaceutique selon la revendication 10, dans lequel l'un ou les plusieurs polypeptides sont représentatifs de la longueur totale du polypeptide Ves v4, dans lequel la composition pharmaceutique en outre comprend un ou plusieurs polypeptides additionnels choisis à partir du groupe consistant en polypeptide Ves v1 (phospolipase A1), polypeptide Ves v2a (hyaluronidase), polypeptide Ves v2b, polypeptide Ves v3 (dipeptidylpeptidase), polypeptide Ves v5, protéines IgE-binding glycosylées.

12. Polypeptide selon la revendication 7 à 9 ou composition pharmaceutique selon la revendication 10 ou 11 pour l'utilisation comme médicament.

13. Polypeptide selon les revendications 7 à 9 ou fragments de polypeptide, dans lequel le fragment de polypeptide est choisi à partir du groupe consistant en les résidus d'acides aminés 22 à 74, 75 à 135, 136 à 190, 191 à 240, 241 à 284, 285 à 334 et 335 à 390 du polypeptide, dans lequel la numérotation correspond à la région du polypeptide ayant la séquence montrée dans SEQ ID NO:2,
ou
dans lequel le fragment de polypeptide est choisi à partir du groupe consistant en les résidus d'acides aminés 22 à 135, 136 à 232, 233 à 313 et 314 à 390 du polypeptide, dans lequel la numérotation correspond à la région du polypeptide ayant la séquence montrée dans SEQ ID NO:2
ou
dans lequel le fragment de polypeptide est choisi à partir du groupe consistant en les résidus d'acides aminés 22 à 148, 149 à 268 et 269 à 390 du polypeptide, dans lequel la numérotation correspond à la région du polypeptide ayant la séquence montrée dans SEQ ID NO:2
ou
ou composition pharmaceutique selon les revendications 10 ou 11
pour l'utilisation lors du traitement d'une allergie au venin *Vespula.*

14. Polypeptide ou composition pharmaceutique selon la revendication 13 pour l'utilisation lors du traitement d'une allergie à la protéase de venin de *Vespula* Ves v4.

15. Polypeptide ou composition pharmaceutique pour l'utilisation lors du traitement d'une allergie au venin de *Vespula* ou à la protéase de venin de *Vespula* Ves v4 selon les revendications 13 ou 14, dans lequel le polypeptide ou la composition pharmaceutique sont administrés à une quantité suffisante pour réduire ou inhiber une réponse immune contre le polypeptide Ves v4, et dans lequel le polypeptide et la composition pharmaceutique sont administrés pendant une durée de temps à des doses s'augmentant graduellement.

16. Utilisation du polypeptide selon les revendications 7 à 9 ou de la composition pharmaceutique selon les revendications 10 ou 11 pour la génération d'un médicament pour l'utilisation lors du traitement d'une allergie à un venin de *Vespula,* de préférence d'une allergie à la protéase du venin de *Vespula* Ves v4.

17. Procédé in vitro pour la réduction de l'allergénicité d'un polypeptide Ves v4, comprenant les étapes suivantes:
a) prévoir d'une molécule d'acide nucléique selon les revendications 1 à 3 ou d'une molécule d'acide nucléique, qui code pour un polypeptide selon les revendications 7 à 9,
b) appliquer la mutagenèse dirigée par le site sur la molécule d'acide nucléique, de façon qu'un ou plusieurs épitopes IgE du polypeptide codé soient modifiés pour obtenir un allergène modifié, et
c) déterminer la réduction de l'allergénicité de l'allergène modifié.

18. Procédé in vitro pour détecter des anticorps de sérum IgE ou IgG, qui sont spécifiques pour les polypeptides selon les revendications 7 à 9, comprenant les étapes suivantes:
a) prévoir un sérum pour un patient allergique, et
b) détecter les anticorps de sérum IgE ou IgG dans le sérum par un procédé de détection,
dans lequel le procédé de détection est choisi à partir du groupe consistant en radio-allergosorbent test (RAST), enzyme-linked immunosorbent assay (ELISA), immunoélectrophorèse, immunoblot, immundotblotting, bead-array technique, systèmes de phases fluides et in vitro médiator release assays (MRA).

19. Procédé selon la revendication 18, dans lequel la détection comprend le mesurage in vitro de la proportion des anticorps de sérum IgE ou IgG, qui sont spécifiques pour les polypeptides selon les revendications 7 à 9, et
dans lequel le procédé en outre comprend l'étape suivante:
c) évaluer le succès du traitement immunothérapeutique.


### SEQUENCE LISTING

<110> PLS-Design GmbH
   Seismann, Henning
   Braren, Ingke
   Grunwald, Thomas
<120> CLONING AND RECOMBINANT PRODUCTION OF VESPULA VENOM PROTEASE AND METHODS OF USE THEREOF
<130> PLSD001PEP
<140> EP 09003033.9
   <141> 2009-03-03
<160> 1577
<170> PatentIn version 3.5
<210> 1
   <211> 1173
   <212> DNA
   <213> Vespula vulgaris
<400> 1
<210> 2
   <211> 390
   <212> PRT
   <213> Vespula vulgaris
<220>
   <221> Signal_sequence
   <222> (1)..(21)
<220>
   <221> CUB_domain
   <222> (27)..(135)
<220>
   <221> Trypsin-like_serine_protease
   <222> (141)..(381)
<400> 2
<210> 3
   <211> 381
   <212> DNA
   <213> Vespula vulgaris
<400> 3
   actatcgatc tcaattgtgg ctacacccaa aaattaaaat cagatgttaa ttattgcgtg      60
   tataatcctg attttccgaa ttattacatg ggagaacata attgtcggtg gagtgctagg     120
   agcaatactc gaattaaatt gaattgcaca gttttcgatg ttcctccgag ttcgaattgt     180
   tcattggatt ttatgaaagt caaagtcgac gatgacatcg agtacgtttt ctgtggatta     240
   aattcttttg cagtggaatc aatagcatcg aaaatgacta taaaattcca ttcaagatac     300
   aatacttatg gaggcaaatt tcgatgtaat ctcagatcag tcaaagagaa atgtagatgt     360
   ggttggaaga atccgtcgag a                                               381
<210> 4
   <211> 360
   <212> DNA
   <213> Vespula vulgaris
<400> 4
   atcgtaggcg gtgttgaaac aggagtgaat gaatatccta tgatggctgg tataatacat      60
   cttgcaacgc gttttctcta ttgcggtgca actataataa ctccgcaaca tgtattaacg     120
   gctgctcatt gcgttgcgag gtataaaagg attttatata ttctaggagt tgttgttgga     180
   gaacataata catgggcaat taacgataca aaggcaactc aactttatct tattgatgat     240
   ataatcgtac atccgaatta taggccaaaa ttaaacgatt tagctgttat aaaattgcag     300
   aagaggttaa aatattctat gagaattggt ccagcttgtc ttccgttcta ttacatgcag     360
<210> 5
   <211> 366
   <212> DNA
   <213> Vespula vulgaris
<400> 5
   cgaaactttg tagacactgt tgttacagct gtaggatggg gtcttacgaa tttttatggt      60
   gtcaagtctg aagttttgag aaaagtcgat ttgcatgtag tttcaatgaa gaaatgcgtc     120
   aagtatcact ttctggctac acctaagcaa ctttgtacat tcgatatggg aaaggatgct     180
   tgccagttcg acagtggtgg tccaatttta tggcaaaatc caaaaagcaa acgtattttc     240
   cttcttggag tcatcaatta tggaagaaca tgtgccgatg aagctcctgg tgttaacttg     300
   agagtcacta gttatttaga ttttattaca agatcaacac ctggagaaat atattgtcag     360
   gcgtat                                                                366
<210> 6
   <211> 417
   <212> DNA
   <213> Vespula vulgaris
<400> 6
   tgtggattaa attcttttgc agtggaatca atagcatcga aaatgactat aaaattccat 60
   tcaagataca atacttatgg aggcaaattt cgatgtaatc tcagatcagt caaagagaaa 120
   tgtagatgtg gttggaagaa tccgtcgaga atcgtaggcg gtgttgaaac aggagtgaat 180
   gaatatccta tgatggctgg tataatacat cttgcaacgc gttttctcta ttgcggtgca 240
   actataataa ctccgcaaca tgtattaacg gctgctcatt gcgttgcgag gtataaaagg 300
   attttatata ttctaggagt tgttgttgga gaacataata catgggcaat taacgataca 360
   aaggcaactc aactttatct tattgatgat ataatcgtac atccgaatta taggcca 417
<210> 7
   <211> 360
   <212> DNA
   <213> Vespula vulgaris
<400> 7
   aaattaaacg atttagctgt tataaaattg cagaagaggt taaaatattc tatgagaatt 60
   ggtccagctt gtcttccgtt ctattacatg cagcgaaact ttgtagacac tgttgttaca 120
   gctgtaggat ggggtcttac gaatttttat ggtgtcaagt ctgaagtttt gagaaaagtc 180
   gatttgcatg tagtttcaat gaagaaatgc gtcaagtatc actttctggc tacacctaag 240
   caactttgta cattcgatat gggaaaggat gcttgccagt tcgacagtgg tggtccaatt 300
   ttatggcaaa atccaaaaag caaacgtatt ttccttcttg gagtcatcaa ttatggaaga 360
<210> 8
   <211> 342
   <212> DNA
   <213> Vespula vulgaris
<400> 8
   actatcgatc tcaattgtgg ctacacccaa aaattaaaat cagatgttaa ttattgcgtg 60
   tataatcctg attttccgaa ttattacatg ggagaacata attgtcggtg gagtgctagg 120
   agcaatactc gaattaaatt gaattgcaca gttttcgatg ttcctccgag ttcgaattgt 180
   tcattggatt ttatgaaagt caaagtcgac gatgacatcg agtacgtttt ctgtggatta 240
   aattcttttg cagtggaatc aatagcatcg aaaatgacta taaaattcca ttcaagatac 300
   aatacttatg gaggcaaatt tcgatgtaat ctcagatcag tc 342
<210> 9
   <211> 291
   <212> DNA
   <213> Vespula vulgaris
<400> 9
   aaagagaaat gtagatgtgg ttggaagaat ccgtcgagaa tcgtaggcgg tgttgaaaca 60
   ggagtgaatg aatatcctat gatggctggt ataatacatc ttgcaacgcg ttttctctat 120
   tgcggtgcaa ctataataac tccgcaacat gtattaacgg ctgctcattg cgttgcgagg 180
   tataaaagga ttttatatat tctaggagtt gttgttggag aacataatac atgggcaatt 240
   aacgatacaa aggcaactca actttatctt attgatgata taatcgtaca t 291
<210> 10
   <211> 243
   <212> DNA
   <213> Vespula vulgaris
<400> 10
   ccgaattata ggccaaaatt aaacgattta gctgttataa aattgcagaa gaggttaaaa 60
   tattctatga gaattggtcc agcttgtctt ccgttctatt acatgcagcg aaactttgta 120
   gacactgttg ttacagctgt aggatggggt cttacgaatt tttatggtgt caagtctgaa 180
   gttttgagaa aagtcgattt gcatgtagtt tcaatgaaga aatgcgtcaa gtatcacttt 240
   ctg 243
<210> 11
   <211> 231
   <212> DNA
   <213> Vespula vulgaris
<400> 11
   gctacaccta agcaactttg tacattcgat atgggaaagg atgcttgcca gttcgacagt 60
   ggtggtccaa ttttatggca aaatccaaaa agcaaacgta ttttccttct tggagtcatc 120
   aattatggaa gaacatgtgc cgatgaagct cctggtgtta acttgagagt cactagttat 180
   ttagatttta ttacaagatc aacacctgga gaaatatatt gtcaggcgta t 231
<210> 12
   <211> 299
   <212> DNA
   <213> Vespula vulgaris
<400> 12
   gatgacatcg agtacgtttt ctgtggatta aattcttttg cagtggaatc aatagcatcg 60
   aaaatgacta taaaattcca ttcaagatac aatacttatg gaggcaaatt tcgatgtaat 120
   ctcagatcag tcaaagagaa atgtagatgt ggttggaaga atccgtcgag aatcgtaggc 180
   ggtgttgaaa caggagtgaa tgaatatcct atgatggctg gtataataca tcttgcaacg 240
   cgttttctct attgcggtgc aactataata actccgcaac atgtattaac ggctgctca 299
<210> 13
   <211> 270
   <212> DNA
   <213> Vespula vulgaris
<400> 13
   gttgcgaggt ataaaaggat tttatatatt ctaggagttg ttgttggaga acataataca 60
   tgggcaatta acgatacaaa ggcaactcaa ctttatctta ttgatgatat aatcgtacat 120
   ccgaattata ggccaaaatt aaacgattta gctgttataa aattgcagaa gaggttaaaa 180
   tattctatga gaattggtcc agcttgtctt ccgttctatt acatgcagcg aaactttgta 240
   gacactgttg ttacagctgt aggatggggt 270
<210> 14
   <211> 270
   <212> DNA
   <213> Vespula vulgaris
<400> 14
   cttacgaatt tttatggtgt caagtctgaa gttttgagaa aagtcgattt gcatgtagtt 60
   tcaatgaaga aatgcgtcaa gtatcacttt ctggctacac ctaagcaact ttgtacattc 120
   gatatgggaa aggatgcttg ccagttcgac agtggtggtc caattttatg gcaaaatcca 180
   aaaagcaaac gtattttcct tcttggagtc atcaattatg gaagaacatg tgccgatgaa 240
   gctcctggtg ttaacttgag agtcactagt 270
<210> 15
   <211> 159
   <212> DNA
   <213> Vespula vulgaris
<400> 15
   actatcgatc tcaattgtgg ctacacccaa aaattaaaat cagatgttaa ttattgcgtg 60
   tataatcctg attttccgaa ttattacatg ggagaacata attgtcggtg gagtgctagg 120
   agcaatactc gaattaaatt gaattgcaca gttttcgat 159
<210> 16
   <211> 183
   <212> DNA
   <213> Vespula vulgaris
<400> 16
   gttcctccga gttcgaattg ttcattggat tttatgaaag tcaaagtcga cgatgacatc 60
   gagtacgttt tctgtggatt aaattctttt gcagtggaat caatagcatc gaaaatgact 120
   ataaaattcc attcaagata caatacttat ggaggcaaat ttcgatgtaa tctcagatca 180
   gtc 183
<210> 17
   <211> 165
   <212> DNA
   <213> Vespula vulgaris
<400> 17
   aaagagaaat gtagatgtgg ttggaagaat ccgtcgagaa tcgtaggcgg tgttgaaaca 60
   ggagtgaatg aatatcctat gatggctggt ataatacatc ttgcaacgcg ttttctctat 120
   tgcggtgcaa ctataataac tccgcaacat gtattaacgg ctgct 165
<210> 18
   <211> 150
   <212> DNA
   <213> Vespula vulgaris
<400> 18
   cattgcgttg cgaggtataa aaggatttta tatattctag gagttgttgt tggagaacat 60
   aatacatggg caattaacga tacaaaggca actcaacttt atcttattga tgatataatc 120
   gtacatccga attataggcc aaaattaaac 150
<210> 19
   <211> 132
   <212> DNA
   <213> Vespula vulgaris
<400> 19
   gatttagctg ttataaaatt gcagaagagg ttaaaatatt ctatgagaat tggtccagct 60
   tgtcttccgt tctattacat gcagcgaaac tttgtagaca ctgttgttac agctgtagga 120
   tggggtctta cg 132
<210> 20
   <211> 150
   <212> DNA
   <213> Vespula vulgaris
<400> 20
   aatttttatg gtgtcaagtc tgaagttttg agaaaagtcg atttgcatgt agtttcaatg 60
   aagaaatgcg tcaagtatca ctttctggct acacctaagc aactttgtac attcgatatg 120
   ggaaaggatg cttgccagtt cgacagtggt 150
<210> 21
   <211> 168
   <212> DNA
   <213> Vespula vulgaris
<400> 21
   ggtccaattt tatggcaaaa tccaaaaagc aaacgtattt tccttcttgg agtcatcaat 60
   tatggaagaa catgtgccga tgaagctcct ggtgttaact tgagagtcac tagttattta 120
   gattttatta caagatcaac acctggagaa atatattgtc aggcgtat 168
<210> 22
   <211> 153
   <212> DNA
   <213> Vespula vulgaris
<400> 22
   ggagaacata attgtcggtg gagtgctagg agcaatactc gaattaaatt gaattgcaca 60
   gttttcgatg ttcctccgag ttcgaattgt tcattggatt ttatgaaagt caaagtcgac 120
   gatgacatcg agtacgtttt ctgtggatta aat 153
<210> 23
   <211> 171
   <212> DNA
   <213> Vespula vulgaris
<400> 23
   tttgcagtgg aatcaatagc atcgaaaatg actataaaat tccattcaag atacaatact      60
   tatggaggca aatttcgatg taatctcaga tcagtcaaag agaaatgtag atgtggttgg     120
   aagaatccgt cgagaatcgt aggcggtgtt gaaacaggag tgaatgaata t              171
<210> 24
   <211> 159
   <212> DNA
   <213> Vespula vulgaris
<400> 24
   cctatgatgg ctggtataat acatcttgca acgcgttttc tctattgcgg tgcaactata      60
   ataactccgc aacatgtatt aacggctgct cattgcgttg cgaggtataa aaggatttta     120
   tatattctag gagttgttgt tggagaacat aatacatgg                            159
<210> 25
   <211> 165
   <212> DNA
   <213> Vespula vulgaris
<400> 25
   gcaattaacg atacaaaggc aactcaactt tatcttattg atgatataat cgtacatccg      60
   aattataggc caaaattaaa cgatttagct gttataaaat tgcagaagag gttaaaatat     120
   tctatgagaa ttggtccagc ttgtcttccg ttctattaca tgcag                     165
<210> 26
   <211> 141
   <212> DNA
   <213> Vespula vulgaris
<400> 26
   cgaaactttg tagacactgt tgttacagct gtaggatggg gtcttacgaa tttttatggt      60
   gtcaagtctg aagttttgag aaaagtcgat ttgcatgtag tttcaatgaa gaaatgcgtc     120
   aagtatcact ttctggctac a                                               141
<210> 27
   <211> 144
   <212> DNA
   <213> Vespula vulgaris
<400> 27
   cctaagcaac tttgtacatt cgatatggga aaggatgctt gccagttcga cagtggtggt       60
   ccaattttat ggcaaaatcc aaaaagcaaa cgtattttcc ttcttggagt catcaattat      120
   ggaagaacat gtgccgatga agct                                             144
<210> 28
   <211> 127
   <212> PRT
   <213> Vespula vulgaris
<400> 28
   Thr Ile Asp Leu Asn Cys Gly Tyr Thr Gln Lys Leu Lys Ser Asp Val
   1               5                   10                  15
   Asn Tyr Cys Val Tyr Asn Pro Asp Phe Pro Asn Tyr Tyr Met Gly Glu
               20                  25                  30
   His Asn Cys Arg Trp Ser Ala Arg Ser Asn Thr Arg Ile Lys Leu Asn
           35                  40                  45
   Cys Thr Val Phe Asp Val Pro Pro Ser Ser Asn Cys Ser Leu Asp Phe
       50                  55                  60
   Met Lys Val Lys Val Asp Asp Asp Ile Glu Tyr Val Phe Cys Gly Leu
   65                  70                  75                  80
   Asn Ser Phe Ala Val Glu Ser Ile Ala Ser Lys Met Thr Ile Lys Phe
                   85                  90                  95
   His Ser Arg Tyr Asn Thr Tyr Gly Gly Lys Phe Arg Cys Asn Leu Arg
               100                 105                 110
   Ser Val Lys Glu Lys Cys Arg Cys Gly Trp Lys Asn Pro Ser Arg
           115                 120                 125
<210> 29
   <211> 120
   <212> PRT
   <213> Vespula vulgaris
<400> 29
   Ile Val Gly Gly Val Glu Thr Gly Val Asn Glu Tyr Pro Met Met Ala
   1               5                   10                  15
   Gly Ile Ile His Leu Ala Thr Arg Phe Leu Tyr Cys Gly Ala Thr Ile
               20                  25                  30
   Ile Thr Pro Gln His Val Leu Thr Ala Ala His Cys Val Ala Arg Tyr
           35                  40                  45
   Lys Arg Ile Leu Tyr Ile Leu Gly Val Val Val Gly Glu His Asn Thr
       50                  55                  60
   Trp Ala Ile Asn Asp Thr Lys Ala Thr Gln Leu Tyr Leu Ile Asp Asp
   65                  70                  75                  80
   Ile Ile Val His Pro Asn Tyr Arg Pro Lys Leu Asn Asp Leu Ala Val
                   85                  90                  95
   Ile Lys Leu Gln Lys Arg Leu Lys Tyr Ser Met Arg Ile Gly Pro Ala
               100                 105                 110
   Cys Leu Pro Phe Tyr Tyr Met Gln
           115                 120
<210> 30
   <211> 122
   <212> PRT
   <213> Vespula vulgaris
<400> 30
   Arg Asn Phe Val Asp Thr Val Val Thr Ala Val Gly Trp Gly Leu Thr
   1               5                   10                  15
   Asn Phe Tyr Gly Val Lys Ser Glu Val Leu Arg Lys Val Asp Leu His
               20                  25                  30
   Val Val Ser Met Lys Lys Cys Val Lys Tyr His Phe Leu Ala Thr Pro
           35                  40                  45
   Lys Gln Leu Cys Thr Phe Asp Met Gly Lys Asp Ala Cys Gln Phe Asp
       50                  55                  60
   Ser Gly Gly Pro Ile Leu Trp Gln Asn Pro Lys Ser Lys Arg Ile Phe
   65                  70                  75                  80
   Leu Leu Gly Val Ile Asn Tyr Gly Arg Thr Cys Ala Asp Glu Ala Pro
                   85                  90                  95
   Gly Val Asn Leu Arg Val Thr Ser Tyr Leu Asp Phe Ile Thr Arg Ser
               100                 105                 110
   Thr Pro Gly Glu Ile Tyr Cys Gln Ala Tyr
           115                 120
<210> 31
   <211> 139
   <212> PRT
   <213> Vespula vulgaris
<400> 31
   Cys Gly Leu Asn Ser Phe Ala Val Glu Ser Ile Ala Ser Lys Met Thr
   1               5                   10                  15
   Ile Lys Phe His Ser Arg Tyr Asn Thr Tyr Gly Gly Lys Phe Arg Cys
               20                  25                  30
   Asn Leu Arg Ser Val Lys Glu Lys Cys Arg Cys Gly Trp Lys Asn Pro
           35                  40                  45
   Ser Arg Ile Val Gly Gly Val Glu Thr Gly Val Asn Glu Tyr Pro Met
       50                  55                  60
   Met Ala Gly Ile Ile His Leu Ala Thr Arg Phe Leu Tyr Cys Gly Ala
   65                  70                  75                  80
   Thr Ile Ile Thr Pro Gln His Val Leu Thr Ala Ala His Cys Val Ala
                   85                  90                  95
   Arg Tyr Lys Arg Ile Leu Tyr Ile Leu Gly Val Val Val Gly Glu His
               100                 105                 110
   Asn Thr Trp Ala Ile Asn Asp Thr Lys Ala Thr Gln Leu Tyr Leu Ile
           115                 120                 125
   Asp Asp Ile Ile Val His Pro Asn Tyr Arg Pro
       130                 135
<210> 32
   <211> 120
   <212> PRT
   <213> Vespula vulgaris
<400> 32
   Lys Leu Asn Asp Leu Ala Val Ile Lys Leu Gln Lys Arg Leu Lys Tyr
   1               5                   10                  15
   Ser Met Arg Ile Gly Pro Ala Cys Leu Pro Phe Tyr Tyr Met Gln Arg
               20                  25                  30
   Asn Phe Val Asp Thr Val Val Thr Ala Val Gly Trp Gly Leu Thr Asn
           35                  40                  45
   Phe Tyr Gly Val Lys Ser Glu Val Leu Arg Lys Val Asp Leu His Val
       50                  55                  60
   Val Ser Met Lys Lys Cys Val Lys Tyr His Phe Leu Ala Thr Pro Lys
   65                  70                  75                  80
   Gln Leu Cys Thr Phe Asp Met Gly Lys Asp Ala Cys Gln Phe Asp Ser
                   85                  90                  95
   Gly Gly Pro Ile Leu Trp Gln Asn Pro Lys Ser Lys Arg Ile Phe Leu
               100                 105                 110
   Leu Gly Val Ile Asn Tyr Gly Arg
           115                 120
<210> 33
   <211> 114
   <212> PRT
   <213> Vespula vulgaris
<400> 33
   Thr Ile Asp Leu Asn Cys Gly Tyr Thr Gln Lys Leu Lys Ser Asp Val
   1               5                   10                  15
   Asn Tyr Cys Val Tyr Asn Pro Asp Phe Pro Asn Tyr Tyr Met Gly Glu
               20                  25                  30
   His Asn Cys Arg Trp Ser Ala Arg Ser Asn Thr Arg Ile Lys Leu Asn
           35                  40                  45
   Cys Thr Val Phe Asp Val Pro Pro Ser Ser Asn Cys Ser Leu Asp Phe
       50                  55                  60
   Met Lys Val Lys Val Asp Asp Asp Ile Glu Tyr Val Phe Cys Gly Leu
   65                  70                  75                  80
   Asn Ser Phe Ala Val Glu Ser Ile Ala Ser Lys Met Thr Ile Lys Phe
                   85                  90                  95
   His Ser Arg Tyr Asn Thr Tyr Gly Gly Lys Phe Arg Cys Asn Leu Arg
               100                 105                 110
   Ser Val
<210> 34
   <211> 97
   <212> PRT
   <213> Vespula vulgaris
<400> 34
   Lys Glu Lys Cys Arg Cys Gly Trp Lys Asn Pro Ser Arg Ile Val Gly
   1               5                   10                  15
   Gly Val Glu Thr Gly Val Asn Glu Tyr Pro Met Met Ala Gly Ile Ile
               20                  25                  30
   His Leu Ala Thr Arg Phe Leu Tyr Cys Gly Ala Thr Ile Ile Thr Pro
           35                  40                  45
   Gln His Val Leu Thr Ala Ala His Cys Val Ala Arg Tyr Lys Arg Ile
       50                  55                  60
   Leu Tyr Ile Leu Gly Val Val Val Gly Glu His Asn Thr Trp Ala Ile
   65                  70                  75                      80
   Asn Asp Thr Lys Ala Thr Gln Leu Tyr Leu Ile Asp Asp Ile Ile Val
                   85                  90                  95
   His
<210> 35
   <211> 81
   <212> PRT
   <213> Vespula vulgaris
<400> 35
   Pro Asn Tyr Arg Pro Lys Leu Asn Asp Leu Ala Val Ile Lys Leu Gln
   1               5                   10                  15
   Lys Arg Leu Lys Tyr Ser Met Arg Ile Gly Pro Ala Cys Leu Pro Phe
               20                  25                  30
   Tyr Tyr Met Gln Arg Asn Phe Val Asp Thr Val Val Thr Ala Val Gly
           35                  40                  45
   Trp Gly Leu Thr Asn Phe Tyr Gly Val Lys Ser Glu Val Leu Arg Lys
       50                  55                  60
   Val Asp Leu His Val Val Ser Met Lys Lys Cys Val Lys Tyr His Phe
   65                  70                  75                      80
   Leu
<210> 36
   <211> 77
   <212> PRT
   <213> Vespula vulgaris
<400> 36
   Ala Thr Pro Lys Gln Leu Cys Thr Phe Asp Met Gly Lys Asp Ala Cys
   1               5                   10                  15
   Gln Phe Asp Ser Gly Gly Pro Ile Leu Trp Gln Asn Pro Lys Ser Lys
               20                  25                  30
   Arg Ile Phe Leu Leu Gly Val Ile Asn Tyr Gly Arg Thr Cys Ala Asp
           35                  40                  45
   Glu Ala Pro Gly Val Asn Leu Arg Val Thr Ser Tyr Leu Asp Phe Ile
       50                  55                  60
   Thr Arg Ser Thr Pro Gly Glu Ile Tyr Cys Gln Ala Tyr
   65                  70                  75
<210> 37
   <211> 101
   <212> PRT
   <213> Vespula vulgaris
<400> 37
   Asp Asp Ile Glu Tyr Val Phe Cys Gly Leu Asn Ser Phe Ala Val Glu
   1               5                   10                  15
   Ser Ile Ala Ser Lys Met Thr Ile Lys Phe His Ser Arg Tyr Asn Thr
               20                  25                  30
   Tyr Gly Gly Lys Phe Arg Cys Asn Leu Arg Ser Val Lys Glu Lys Cys
           35                  40                  45
   Arg Cys Gly Trp Lys Asn Pro Ser Arg Ile Val Gly Gly Val Glu Thr
       50                  55                  60
   Gly Val Asn Glu Tyr Pro Met Met Ala Gly Ile Ile His Leu Ala Thr
   65                  70                  75                  80
   Arg Phe Leu Tyr Cys Gly Ala Thr Ile Ile Thr Pro Gln His Val Leu
                   85                  90                  95
   Thr Ala Ala His Cys
               100
<210> 38
   <211> 90
   <212> PRT
   <213> Vespula vulgaris
<400> 38
   Val Ala Arg Tyr Lys Arg Ile Leu Tyr Ile Leu Gly Val Val Val Gly
   1               5                   10                  15
   Glu His Asn Thr Trp Ala Ile Asn Asp Thr Lys Ala Thr Gln Leu Tyr
               20                  25                  30
   Leu Ile Asp Asp Ile Ile Val His Pro Asn Tyr Arg Pro Lys Leu Asn
           35                  40                  45
   Asp Leu Ala Val Ile Lys Leu Gln Lys Arg Leu Lys Tyr Ser Met Arg
       50                  55                  60
   Ile Gly Pro Ala Cys Leu Pro Phe Tyr Tyr Met Gln Arg Asn Phe Val
   65                  70                  75                  80
   Asp Thr Val Val Thr Ala Val Gly Trp Gly
                       85                  90
<210> 39
   <211> 91
   <212> PRT
   <213> Vespula vulgaris
<400> 39
   Leu Thr Asn Phe Tyr Gly Val Lys Ser Glu Val Leu Arg Lys Val Asp
   1               5                   10                  15
   Leu His Val Val Ser Met Lys Lys Cys Val Lys Tyr His Phe Leu Ala
               20                  25                  30
   Thr Pro Lys Gln Leu Cys Thr Phe Asp Met Gly Lys Asp Ala Cys Gln
           35                  40                  45
   Phe Asp Ser Gly Gly Pro Ile Leu Trp Gln Asn Pro Lys Ser Lys Arg
       50                  55                  60
   Ile Phe Leu Leu Gly Val Ile Asn Tyr Gly Arg Thr Cys Ala Asp Glu
   65                  70                  75                  80
   Ala Pro Gly Val Asn Leu Arg Val Thr Ser Tyr
                   85                  90
<210> 40
   <211> 53
   <212> PRT
   <213> Vespula vulgaris
<400> 40
   Thr Ile Asp Leu Asn Cys Gly Tyr Thr Gln Lys Leu Lys Ser Asp Val
   1               5                   10                  15
   Asn Tyr Cys Val Tyr Asn Pro Asp Phe Pro Asn Tyr Tyr Met Gly Glu
               20                  25                  30
   His Asn Cys Arg Trp Ser Ala Arg Ser Asn Thr Arg Ile Lys Leu Asn
           35                  40                  45
   Cys Thr Val Phe Asp
       50
<210> 41
   <211> 61
   <212> PRT
   <213> Vespula vulgaris
<400> 41
   Val Pro Pro Ser Ser Asn Cys Ser Leu Asp Phe Met Lys Val Lys Val
   1               5                   10                  15
   Asp Asp Asp Ile Glu Tyr Val Phe Cys Gly Leu Asn Ser Phe Ala Val
               20                  25                  30
   Glu Ser Ile Ala Ser Lys Met Thr Ile Lys Phe His Ser Arg Tyr Asn
           35                  40                  45
   Thr Tyr Gly Gly Lys Phe Arg Cys Asn Leu Arg Ser Val
       50                  55                  60
<210> 42
   <211> 55
   <212> PRT
   <213> Vespula vulgaris
<400> 42
   Lys Glu Lys Cys Arg Cys Gly Trp Lys Asn Pro Ser Arg Ile Val Gly
   1               5                   10                  15
   Gly Val Glu Thr Gly Val Asn Glu Tyr Pro Met Met Ala Gly Ile Ile
               20                  25                  30
   His Leu Ala Thr Arg Phe Leu Tyr Cys Gly Ala Thr Ile Ile Thr Pro
           35                  40                  45
   Gln His Val Leu Thr Ala Ala
       50                  55
<210> 43
   <211> 50
   <212> PRT
   <213> Vespula vulgaris
<400> 43
   His Cys Val Ala Arg Tyr Lys Arg Ile Leu Tyr Ile Leu Gly Val Val
   1               5                   10                  15
   Val Gly Glu His Asn Thr Trp Ala Ile Asn Asp Thr Lys Ala Thr Gln
               20                  25                  30
   Leu Tyr Leu Ile Asp Asp Ile Ile Val His Pro Asn Tyr Arg Pro Lys
           35                  40                  45
   Leu Asn
       50
<210> 44
   <211> 44
   <212> PRT
   <213> Vespula vulgaris
<400> 44
   Asp Leu Ala Val Ile Lys Leu Gln Lys Arg Leu Lys Tyr Ser Met Arg
   1               5                   10                  15
   Ile Gly Pro Ala Cys Leu Pro Phe Tyr Tyr Met Gln Arg Asn Phe Val
               20                  25                  30
   Asp Thr Val Val Thr Ala Val Gly Trp Gly Leu Thr
           35                  40
<210> 45
   <211> 50
   <212> PRT
   <213> Vespula vulgaris
<400> 45
   Asn Phe Tyr Gly Val Lys Ser Glu Val Leu Arg Lys Val Asp Leu His
   1               5                   10                  15
   Val Val Ser Met Lys Lys Cys Val Lys Tyr His Phe Leu Ala Thr Pro
               20                  25                  30
   Lys Gln Leu Cys Thr Phe Asp Met Gly Lys Asp Ala Cys Gln Phe Asp
           35                  40                  45
   Ser Gly
       50
<210> 46
   <211> 56
   <212> PRT
   <213> Vespula vulgaris
<400> 46
   Gly Pro Ile Leu Trp Gln Asn Pro Lys Ser Lys Arg Ile Phe Leu Leu
   1               5                   10                  15
   Gly Val Ile Asn Tyr Gly Arg Thr Cys Ala Asp Glu Ala Pro Gly Val
               20                  25                  30
   Asn Leu Arg Val Thr Ser Tyr Leu Asp Phe Ile Thr Arg Ser Thr Pro
           35                  40                  45
   Gly Glu Ile Tyr Cys Gln Ala Tyr
       50                  55
<210> 47
   <211> 52
   <212> PRT
   <213> Vespula vulgaris
<400> 47
   Gly Glu His Asn Cys Arg Trp Ser Ala Arg Ser Asn Thr Arg Ile Lys
   1               5                   10                  15
   Leu Asn Cys Thr Val Phe Asp Val Pro Pro Ser Ser Asn Cys Ser Leu
               20                  25                  30
   Asp Phe Met Lys Val Lys Val Asp Asp Asp Ile Glu Tyr Val Phe Cys
           35                  40                  45
   Gly Leu Asn Ser
       50
<210> 48
   <211> 57
   <212> PRT
   <213> Vespula vulgaris
<400> 48
   Phe Ala Val Glu Ser Ile Ala Ser Lys Met Thr Ile Lys Phe His Ser
   1               5                   10                  15
   Arg Tyr Asn Thr Tyr Gly Gly Lys Phe Arg Cys Asn Leu Arg Ser Val
               20                  25                  30
   Lys Glu Lys Cys Arg Cys Gly Trp Lys Asn Pro Ser Arg Ile Val Gly
           35                  40                  45
   Gly Val Glu Thr Gly Val Asn Glu Tyr
       50                  55
<210> 49
   <211> 53
   <212> PRT
   <213> Vespula vulgaris
<400> 49
   Pro Met Met Ala Gly Ile Ile His Leu Ala Thr Arg Phe Leu Tyr Cys
   1               5                   10                  15
   Gly Ala Thr Ile Ile Thr Pro Gln His Val Leu Thr Ala Ala His Cys
               20                  25                  30
   Val Ala Arg Tyr Lys Arg Ile Leu Tyr Ile Leu Gly Val Val Val Gly
           35                  40                  45
   Glu His Asn Thr Trp
       50
<210> 50
   <211> 55
   <212> PRT
   <213> Vespula vulgaris
<400> 50
   Ala Ile Asn Asp Thr Lys Ala Thr Gln Leu Tyr Leu Ile Asp Asp Ile
   1 5 10 15
   Ile Val His Pro Asn Tyr Arg Pro Lys Leu Asn Asp Leu Ala Val Ile
   20 25 30
   Lys Leu Gln Lys Arg Leu Lys Tyr Ser Met Arg Ile Gly Pro Ala Cys
   35 40 45
   Leu Pro Phe Tyr Tyr Met Gln
   50 55
<210> 51
   <211> 47
   <212> PRT
   <213> Vespula vulgaris
<400> 51
   Arg Asn Phe Val Asp Thr Val Val Thr Ala Val Gly Trp Gly Leu Thr
   1 5 10 15
   Asn Phe Tyr Gly Val Lys Ser Glu Val Leu Arg Lys Val Asp Leu His
   20 25 30
   Val Val Ser Met Lys Lys Cys Val Lys Tyr His Phe Leu Ala Thr
   35 40 45
<210> 52
   <211> 48
   <212> PRT
   <213> Vespula vulgaris
<400> 52
   Pro Lys Gln Leu Cys Thr Phe Asp Met Gly Lys Asp Ala Cys Gln Phe
   1 5 10 15
   Asp Ser Gly Gly Pro Ile Leu Trp Gln Asn Pro Lys Ser Lys Arg Ile
   20 25 30
   Phe Leu Leu Gly Val Ile Asn Tyr Gly Arg Thr Cys Ala Asp Glu Ala
   35 40 45
<210> 53
   <211> 45
   <212> DNA
   <213> Vespula vulgaris
<400> 53
   agtggtggtc caattttatg gcaaaatcca aaaagcaaac gtatt 45
<210> 54
   <211> 45
   <212> DNA
   <213> Vespula vulgaris
<400> 54
   ggtggtccaa ttttatggca aaatccaaaa agcaaacgta ttttc 45
<210> 55
   <211> 45
   <212> DNA
   <213> Vespula vulgaris
<400> 55
   ggtccaattt tatggcaaaa tccaaaaagc aaacgtattt tcctt 45
<210> 56
   <211> 45
   <212> DNA
   <213> Vespula vulgaris
<400> 56
   ccaattttat ggcaaaatcc aaaaagcaaa cgtattttcc ttctt 45
<210> 57
   <211> 45
   <212> DNA
   <213> Vespula vulgaris
<400> 57
   attttatggc aaaatccaaa aagcaaacgt attttccttc ttgga 45
<210> 58
   <211> 45
   <212> DNA
   <213> Vespula vulgaris
<400> 58
   tatcctatga tggctggtat aatacatctt gcaacgcgtt ttctc 45
<210> 59
   <211> 45
   <212> DNA
   <213> Vespula vulgaris
<400> 59
   tcttttgcag tggaatcaat agcatcgaaa atgactataa aattc 45
<210> 60
   <211> 45
   <212> DNA
   <213> Vespula vulgaris
<400> 60
   tttgcagtgg aatcaatagc atcgaaaatg actataaaat tccat 45
<210> 61
   <211> 45
   <212> DNA
   <213> Vespula vulgaris
<400> 61
   aattcttttg cagtggaatc aatagcatcg aaaatgacta taaaa 45
<210> 62
   <211> 45
   <212> DNA
   <213> Vespula vulgaris
<400> 62
   cctatgatgg ctggtataat acatcttgca acgcgttttc tctat 45
<210> 63
   <211> 45
   <212> DNA
   <213> Vespula vulgaris
<400> 63
   ttaaattctt ttgcagtgga atcaatagca tcgaaaatga ctata 45
<210> 64
   <211> 45
   <212> DNA
   <213> Vespula vulgaris
<400> 64
   atggctggta taatacatct tgcaacgcgt tttctctatt gcggt 45
<210> 65
   <211> 45
   <212> DNA
   <213> Vespula vulgaris
<400> 65
   ggattaaatt cttttgcagt ggaatcaata gcatcgaaaa tgact 45
<210> 66
   <211> 45
   <212> DNA
   <213> Vespula vulgaris
<400> 66
   gctggtataa tacatcttgc aacgcgtttt ctctattgcg gtgca 45
<210> 67
   <211> 45
   <212> DNA
   <213> Vespula vulgaris
<400> 67
   atgatggctg gtataataca tcttgcaacg cgttttctct attgc 45
<210> 68
   <211> 45
   <212> DNA
   <213> Vespula vulgaris
<400> 68
   gagtacgttt tctgtggatt aaattctttt gcagtggaat caata 45
<210> 69
   <211> 45
   <212> DNA
   <213> Vespula vulgaris
<400> 69
   tacgttttct gtggattaaa ttcttttgca gtggaatcaa tagca 45
<210> 70
   <211> 45
   <212> DNA
   <213> Vespula vulgaris
<400> 70
   atcgagtacg ttttctgtgg attaaattct tttgcagtgg aatca 45
<210> 71
   <211> 45
   <212> DNA
   <213> Vespula vulgaris
<400> 71
   gacatcgagt acgttttctg tggattaaat tcttttgcag tggaa 45
<210> 72
   <211> 45
   <212> DNA
   <213> Vespula vulgaris
<400> 72
   gatgacatcg agtacgtttt ctgtggatta aattcttttg cagtg 45
<210> 73
   <211> 45
   <212> DNA
   <213> Vespula vulgaris
<400> 73
   gtgaatgaat atcctatgat ggctggtata atacatcttg caacg 45
<210> 74
   <211> 45
   <212> DNA
   <213> Vespula vulgaris
<400> 74
   aatgaatatc ctatgatggc tggtataata catcttgcaa cgcgt 45
<210> 75
   <211> 45
   <212> DNA
   <213> Vespula vulgaris
<400> 75
   cttacgaatt tttatggtgt caagtctgaa gttttgagaa aagtc 45
<210> 76
   <211> 45
   <212> DNA
   <213> Vespula vulgaris
<400> 76
   ggagtgaatg aatatcctat gatggctggt ataatacatc ttgca 45
<210> 77
   <211> 45
   <212> DNA
   <213> Vespula vulgaris
<400> 77
   acgaattttt atggtgtcaa gtctgaagtt ttgagaaaag tcgat 45
<210> 78
   <211> 45
   <212> DNA
   <213> Vespula vulgaris
<400> 78
   ttatggcaaa atccaaaaag caaacgtatt ttccttcttg gagtc 45
<210> 79
   <211> 45
   <212> DNA
   <213> Vespula vulgaris
<400> 79
   tggcaaaatc caaaaagcaa acgtattttc cttcttggag tcatc 45
<210> 80
   <211> 45
   <212> DNA
   <213> Vespula vulgaris
<400> 80
   aagaggttaa aatattctat gagaattggt ccagcttgtc ttccg 45
<210> 81
   <211> 45
   <212> DNA
   <213> Vespula vulgaris
<400> 81
   gaatatccta tgatggctgg tataatacat cttgcaacgc gtttt 45
<210> 82
   <211> 45
   <212> DNA
   <213> Vespula vulgaris
<400> 82
   ttaaaatatt ctatgagaat tggtccagct tgtcttccgt tctat 45
<210> 83
   <211> 45
   <212> DNA
   <213> Vespula vulgaris
<400> 83
   aaatattcta tgagaattgg tccagcttgt cttccgttct attac 45
<210> 84
   <211> 45
   <212> DNA
   <213> Vespula vulgaris
<400> 84
   aggttaaaat attctatgag aattggtcca gcttgtcttc cgttc 45
<210> 85
   <211> 45
   <212> DNA
   <213> Vespula vulgaris
<400> 85
   gcgaggtata aaaggatttt atatattcta ggagttgttg ttgga 45
<210> 86
   <211> 45
   <212> DNA
   <213> Vespula vulgaris
<400> 86
   gcagtggaat caatagcatc gaaaatgact ataaaattcc attca 45
<210> 87
   <211> 45
   <212> DNA
   <213> Vespula vulgaris
<400> 87
   tattctatga gaattggtcc agcttgtctt ccgttctatt acatg 45
<210> 88
   <211> 45
   <212> DNA
   <213> Vespula vulgaris
<400> 88
   gtggaatcaa tagcatcgaa aatgactata aaattccatt caaga 45
<210> 89
   <211> 45
   <212> DNA
   <213> Vespula vulgaris
<400> 89
   gacactgttg ttacagctgt aggatggggt cttacgaatt tttat 45
<210> 90
   <211> 45
   <212> DNA
   <213> Vespula vulgaris
<400> 90
   cttgcaacgc gttttctcta ttgcggtgca actataataa ctccg 45
<210> 91
   <211> 45
   <212> DNA
   <213> Vespula vulgaris
<400> 91
   aatttttatg gtgtcaagtc tgaagttttg agaaaagtcg atttg 45
<210> 92
   <211> 45
   <212> DNA
   <213> Vespula vulgaris
<400> 92
   ggtataatac atcttgcaac gcgttttctc tattgcggtg caact 45
<210> 93
   <211> 45
   <212> DNA
   <213> Vespula vulgaris
<400> 93
   tggggtctta cgaattttta tggtgtcaag tctgaagttt tgaga 45
<210> 94
   <211> 45
   <212> DNA
   <213> Vespula vulgaris
<400> 94
   ttttatggtg tcaagtctga agttttgaga aaagtcgatt tgcat 45
<210> 95
   <211> 45
   <212> DNA
   <213> Vespula vulgaris
<400> 95
   ataatacatc ttgcaacgcg ttttctctat tgcggtgcaa ctata 45
<210> 96
   <211> 45
   <212> DNA
   <213> Vespula vulgaris
<400> 96
   ggatggggtc ttacgaattt ttatggtgtc aagtctgaag ttttg 45
<210> 97
   <211> 45
   <212> DNA
   <213> Vespula vulgaris
<400> 97
   aggtataaaa ggattttata tattctagga gttgttgttg gagaa 45
<210> 98
   <211> 45
   <212> DNA
   <213> Vespula vulgaris
<400> 98
   tataaaagga ttttatatat tctaggagtt gttgttggag aacat 45
<210> 99
   <211> 45
   <212> DNA
   <213> Vespula vulgaris
<400> 99
   cgttttctct attgcggtgc aactataata actccgcaac atgta 45
<210> 100
   <211> 45
   <212> DNA
   <213> Vespula vulgaris
<400> 100
   aaaaggattt tatatattct aggagttgtt gttggagaac ataat 45
<210> 101
   <211> 45
   <212> DNA
   <213> Vespula vulgaris
<400> 101
   acgcgttttc tctattgcgg tgcaactata ataactccgc aacat 45
<210> 102
   <211> 45
   <212> DNA
   <213> Vespula vulgaris
<400> 102
   gcaacgcgtt ttctctattg cggtgcaact ataataactc cgcaa 45
<210> 103
   <211> 45
   <212> DNA
   <213> Vespula vulgaris
<400> 103
   gaaacaggag tgaatgaata tcctatgatg gctggtataa tacat 45
<210> 104
   <211> 45
   <212> DNA
   <213> Vespula vulgaris
<400> 104
   acaggagtga atgaatatcc tatgatggct ggtataatac atctt 45
<210> 105
   <211> 45
   <212> DNA
   <213> Vespula vulgaris
<400> 105
   ggtcttacga atttttatgg tgtcaagtct gaagttttga gaaaa 45
<210> 106
   <211> 45
   <212> DNA
   <213> Vespula vulgaris
<400> 106
   gttttctgtg gattaaattc ttttgcagtg gaatcaatag catcg 45
<210> 107
   <211> 45
   <212> DNA
   <213> Vespula vulgaris
<400> 107
   ttctgtggat taaattcttt tgcagtggaa tcaatagcat cgaaa 45
<210> 108
   <211> 45
   <212> DNA
   <213> Vespula vulgaris
<400> 108
   actgttgtta cagctgtagg atggggtctt acgaattttt atggt 45
<210> 109
   <211> 45
   <212> DNA
   <213> Vespula vulgaris
<400> 109
   aactttgtag acactgttgt tacagctgta ggatggggtc ttacg 45
<210> 110
   <211> 45
   <212> DNA
   <213> Vespula vulgaris
<400> 110
   cataatacat gggcaattaa cgatacaaag gcaactcaac tttat 45
<210> 111
   <211> 45
   <212> DNA
   <213> Vespula vulgaris
<400> 111
   aggattttat atattctagg agttgttgtt ggagaacata ataca 45
<210> 112
   <211> 45
   <212> DNA
   <213> Vespula vulgaris
<400> 112
   aatacatggg caattaacga tacaaaggca actcaacttt atctt 45
<210> 113
   <211> 45
   <212> DNA
   <213> Vespula vulgaris
<400> 113
   tattgcggtg caactataat aactccgcaa catgtattaa cggct 45
<210> 114
   <211> 45
   <212> DNA
   <213> Vespula vulgaris
<400> 114
   gaacataata catgggcaat taacgataca aaggcaactc aactt 45
<210> 115
   <211> 45
   <212> DNA
   <213> Vespula vulgaris
<400> 115
   tttgtagaca ctgttgttac agctgtagga tggggtctta cgaat 45
<210> 116
   <211> 45
   <212> DNA
   <213> Vespula vulgaris
<400> 116
   gttgttacag ctgtaggatg gggtcttacg aatttttatg gtgtc 45
<210> 117
   <211> 45
   <212> DNA
   <213> Vespula vulgaris
<400> 117
   tgcggtgcaa ctataataac tccgcaacat gtattaacgg ctgct 45
<210> 118
   <211> 45
   <212> DNA
   <213> Vespula vulgaris
<400> 118
   tttctctatt gcggtgcaac tataataact ccgcaacatg tatta 45
<210> 119
   <211> 45
   <212> DNA
   <213> Vespula vulgaris
<400> 119
   cgaaactttg tagacactgt tgttacagct gtaggatggg gtctt 45
<210> 120
   <211> 45
   <212> DNA
   <213> Vespula vulgaris
<400> 120
   acagctgtag gatggggtct tacgaatttt tatggtgtca agtct 45
<210> 121
   <211> 45
   <212> DNA
   <213> Vespula vulgaris
<400> 121
   tgcgtcaagt atcactttct ggctacacct aagcaacttt gtaca 45
<210> 122
   <211> 45
   <212> DNA
   <213> Vespula vulgaris
<400> 122
   gtcaagtatc actttctggc tacacctaag caactttgta cattc 45
<210> 123
   <211> 45
   <212> DNA
   <213> Vespula vulgaris
<400> 123
   catcttgcaa cgcgttttct ctattgcggt gcaactataa taact 45
<210> 124
   <211> 45
   <212> DNA
   <213> Vespula vulgaris
<400> 124
   ccgaattata ggccaaaatt aaacgattta gctgttataa aattg 45
<210> 125
   <211> 45
   <212> DNA
   <213> Vespula vulgaris
<400> 125
   ggtgcaacta taataactcc gcaacatgta ttaacggctg ctcat 45
<210> 126
   <211> 45
   <212> DNA
   <213> Vespula vulgaris
<400> 126
   gtagacactg ttgttacagc tgtaggatgg ggtcttacga atttt 45
<210> 127
   <211> 45
   <212> DNA
   <213> Vespula vulgaris
<400> 127
   gcaactataa taactccgca acatgtatta acggctgctc attgc 45
<210> 128
   <211> 45
   <212> DNA
   <213> Vespula vulgaris
<400> 128
   tatggtgtca agtctgaagt tttgagaaaa gtcgatttgc atgta 45
<210> 129
   <211> 45
   <212> DNA
   <213> Vespula vulgaris
<400> 129
   actataataa ctccgcaaca tgtattaacg gctgctcatt gcgtt 45
<210> 130
   <211> 45
   <212> DNA
   <213> Vespula vulgaris
<400> 130
   ctctattgcg gtgcaactat aataactccg caacatgtat taacg 45
<210> 131
   <211> 45
   <212> DNA
   <213> Vespula vulgaris
<400> 131
   aggccaaaat taaacgattt agctgttata aaattgcaga agagg 45
<210> 132
   <211> 45
   <212> DNA
   <213> Vespula vulgaris
<400> 132
   ccaaaattaa acgatttagc tgttataaaa ttgcagaaga ggtta 45
<210> 133
   <211> 45
   <212> DNA
   <213> Vespula vulgaris
<400> 133
   aaatgcgtca agtatcactt tctggctaca cctaagcaac tttgt 45
<210> 134
   <211> 45
   <212> DNA
   <213> Vespula vulgaris
<400> 134
   tataggccaa aattaaacga tttagctgtt ataaaattgc agaag 45
<210> 135
   <211> 45
   <212> DNA
   <213> Vespula vulgaris
<400> 135
   tctatgagaa ttggtccagc ttgtcttccg ttctattaca tgcag 45
<210> 136
   <211> 45
   <212> DNA
   <213> Vespula vulgaris
<400> 136
   aattataggc caaaattaaa cgatttagct gttataaaat tgcag 45
<210> 137
   <211> 45
   <212> DNA
   <213> Vespula vulgaris
<400> 137
   gttacagctg taggatgggg tcttacgaat ttttatggtg tcaag 45
<210> 138
   <211> 45
   <212> DNA
   <213> Vespula vulgaris
<400> 138
   atgagaattg gtccagcttg tcttccgttc tattacatgc agcga 45
<210> 139
   <211> 45
   <212> DNA
   <213> Vespula vulgaris
<400> 139
   aagaaatgcg tcaagtatca ctttctggct acacctaagc aactt 45
<210> 140
   <211> 45
   <212> DNA
   <213> Vespula vulgaris
<400> 140
   gttggagaac ataatacatg ggcaattaac gatacaaagg caact 45
<210> 141
   <211> 45
   <212> DNA
   <213> Vespula vulgaris
<400> 141
   gttgcgaggt ataaaaggat tttatatatt ctaggagttg ttgtt 45
<210> 142
   <211> 45
   <212> DNA
   <213> Vespula vulgaris
<400> 142
   ggagaacata atacatgggc aattaacgat acaaaggcaa ctcaa 45
<210> 143
   <211> 45
   <212> DNA
   <213> Vespula vulgaris
<400> 143
   tacatgcagc gaaactttgt agacactgtt gttacagctg tagga 45
<210> 144
   <211> 45
   <212> DNA
   <213> Vespula vulgaris
<400> 144
   acatgggcaa ttaacgatac aaaggcaact caactttatc ttatt 45
<210> 145
   <211> 45
   <212> DNA
   <213> Vespula vulgaris
<400> 145
   tgggcaatta acgatacaaa ggcaactcaa ctttatctta ttgat 45
<210> 146
   <211> 45
   <212> DNA
   <213> Vespula vulgaris
<400> 146
   atgaagaaat gcgtcaagta tcactttctg gctacaccta agcaa 45
<210> 147
   <211> 45
   <212> DNA
   <213> Vespula vulgaris
<400> 147
   gtcaagtctg aagttttgag aaaagtcgat ttgcatgtag tttca 45
<210> 148
   <211> 45
   <212> DNA
   <213> Vespula vulgaris
<400> 148
   ggtgtcaagt ctgaagtttt gagaaaagtc gatttgcatg tagtt 45
<210> 149
   <211> 45
   <212> DNA
   <213> Vespula vulgaris
<400> 149
   cagcgaaact ttgtagacac tgttgttaca gctgtaggat ggggt 45
<210> 150
   <211> 45
   <212> DNA
   <213> Vespula vulgaris
<400> 150
   atgcagcgaa actttgtaga cactgttgtt acagctgtag gatgg 45
<210> 151
   <211> 45
   <212> DNA
   <213> Vespula vulgaris
<400> 151
   ataactccgc aacatgtatt aacggctgct cattgcgttg cgagg 45
<210> 152
   <211> 45
   <212> DNA
   <213> Vespula vulgaris
<400> 152
   gctgtaggat ggggtcttac gaatttttat ggtgtcaagt ctgaa 45
<210> 153
   <211> 45
   <212> DNA
   <213> Vespula vulgaris
<400> 153
   tattacatgc agcgaaactt tgtagacact gttgttacag ctgta 45
<210> 154
   <211> 45
   <212> DNA
   <213> Vespula vulgaris
<400> 154
   tatcactttc tggctacacc taagcaactt tgtacattcg atatg 45
<210> 155
   <211> 45
   <212> DNA
   <213> Vespula vulgaris
<400> 155
   atacatcttg caacgcgttt tctctattgc ggtgcaacta taata 45
<210> 156
   <211> 45
   <212> DNA
   <213> Vespula vulgaris
<400> 156
   gtaggatggg gtcttacgaa tttttatggt gtcaagtctg aagtt 45
<210> 157
   <211> 45
   <212> DNA
   <213> Vespula vulgaris
<400> 157
   actagttatt tagattttat tacaagatca acacctggag aaata 45
<210> 158
   <211> 45
   <212> DNA
   <213> Vespula vulgaris
<400> 158
   attttatata ttctaggagt tgttgttgga gaacataata catgg 45
<210> 159
   <211> 45
   <212> DNA
   <213> Vespula vulgaris
<400> 159
   aagtatcact ttctggctac acctaagcaa ctttgtacat tcgat 45
<210> 160
   <211> 45
   <212> DNA
   <213> Vespula vulgaris
<400> 160
   gtcactagtt atttagattt tattacaaga tcaacacctg gagaa 45
<210> 161
   <211> 45
   <212> DNA
   <213> Vespula vulgaris
<400> 161
   ataataactc cgcaacatgt attaacggct gctcattgcg ttgcg 45
<210> 162
   <211> 45
   <212> DNA
   <213> Vespula vulgaris
<400> 162
   cttccgttct attacatgca gcgaaacttt gtagacactg ttgtt 45
<210> 163
   <211> 45
   <212> DNA
   <213> Vespula vulgaris
<400> 163
   tgtcttccgt tctattacat gcagcgaaac tttgtagaca ctgtt 45
<210> 164
   <211> 45
   <212> DNA
   <213> Vespula vulgaris
<400> 164
   gttgaaacag gagtgaatga atatcctatg atggctggta taata 45
<210> 165
   <211> 45
   <212> DNA
   <213> Vespula vulgaris
<400> 165
   atcgatctca attgtggcta cacccaaaaa ttaaaatcag atgtt 45
<210> 166
   <211> 45
   <212> DNA
   <213> Vespula vulgaris
<400> 166
   cattgcgttg cgaggtataa aaggatttta tatattctag gagtt 45
<210> 167
   <211> 45
   <212> DNA
   <213> Vespula vulgaris
<400> 167
   actccgcaac atgtattaac ggctgctcat tgcgttgcga ggtat 45
<210> 168
   <211> 45
   <212> DNA
   <213> Vespula vulgaris
<400> 168
   tgcgttgcga ggtataaaag gattttatat attctaggag ttgtt 45
<210> 169
   <211> 45
   <212> DNA
   <213> Vespula vulgaris
<400> 169
   ccgcaacatg tattaacggc tgctcattgc gttgcgaggt ataaa 45
<210> 170
   <211> 45
   <212> DNA
   <213> Vespula vulgaris
<400> 170
   ttatatattc taggagttgt tgttggagaa cataatacat gggca 45
<210> 171
   <211> 45
   <212> DNA
   <213> Vespula vulgaris
<400> 171
   tctgaagttt tgagaaaagt cgatttgcat gtagtttcaa tgaag 45
<210> 172
   <211> 45
   <212> DNA
   <213> Vespula vulgaris
<400> 172
   ccgttctatt acatgcagcg aaactttgta gacactgttg ttaca 45
<210> 173
   <211> 45
   <212> DNA
   <213> Vespula vulgaris
<400> 173
   ttctattaca tgcagcgaaa ctttgtagac actgttgtta cagct 45
<210> 174
   <211> 45
   <212> DNA
   <213> Vespula vulgaris
<400> 174
   tgcacagttt tcgatgttcc tccgagttcg aattgttcat tggat 45
<210> 175
   <211> 45
   <212> DNA
   <213> Vespula vulgaris
<400> 175
   aattgcacag ttttcgatgt tcctccgagt tcgaattgtt cattg 45
<210> 176
   <211> 45
   <212> DNA
   <213> Vespula vulgaris
<400> 176
   aagtctgaag ttttgagaaa agtcgatttg catgtagttt caatg 45
<210> 177
   <211> 45
   <212> DNA
   <213> Vespula vulgaris
<400> 177
   aacttgagag tcactagtta tttagatttt attacaagat caaca 45
<210> 178
   <211> 45
   <212> DNA
   <213> Vespula vulgaris
<400> 178
   ttgagagtca ctagttattt agattttatt acaagatcaa cacct 45
<210> 179
   <211> 45
   <212> DNA
   <213> Vespula vulgaris
<400> 179
   gaagttttga gaaaagtcga tttgcatgta gtttcaatga agaaa 45
<210> 180
   <211> 45
   <212> DNA
   <213> Vespula vulgaris
<400> 180
   actatcgatc tcaattgtgg ctacacccaa aaattaaaat cagat 45
<210> 181
   <211> 45
   <212> DNA
   <213> Vespula vulgaris
<400> 181
   agagtcacta gttatttaga ttttattaca agatcaacac ctgga 45
<210> 182
   <211> 45
   <212> DNA
   <213> Vespula vulgaris
<400> 182
   gctcattgcg ttgcgaggta taaaaggatt ttatatattc tagga 45
<210> 183
   <211> 45
   <212> DNA
   <213> Vespula vulgaris
<400> 183
   tgtagatgtg gttggaagaa tccgtcgaga atcgtaggcg gtgtt 45
<210> 184
   <211> 45
   <212> DNA
   <213> Vespula vulgaris
<400> 184
   aaatgtagat gtggttggaa gaatccgtcg agaatcgtag gcggt 45
<210> 185
   <211> 45
   <212> DNA
   <213> Vespula vulgaris
<400> 185
   ttaaacgatt tagctgttat aaaattgcag aagaggttaa aatat 45
<210> 186
   <211> 45
   <212> DNA
   <213> Vespula vulgaris
<400> 186
   ggcggtgttg aaacaggagt gaatgaatat cctatgatgg ctggt 45
<210> 187
   <211> 45
   <212> DNA
   <213> Vespula vulgaris
<400> 187
   aaattaaacg atttagctgt tataaaattg cagaagaggt taaaa 45
<210> 188
   <211> 45
   <212> DNA
   <213> Vespula vulgaris
<400> 188
   gcttgtcttc cgttctatta catgcagcga aactttgtag acact 45
<210> 189
   <211> 45
   <212> DNA
   <213> Vespula vulgaris
<400> 189
   gagaaatgta gatgtggttg gaagaatccg tcgagaatcg taggc 45
<210> 190
   <211> 45
   <212> DNA
   <213> Vespula vulgaris
<400> 190
   ggtgttgaaa caggagtgaa tgaatatcct atgatggctg gtata 45
<210> 191
   <211> 45
   <212> DNA
   <213> Vespula vulgaris
<400> 191
   tgtggctaca cccaaaaatt aaaatcagat gttaattatt gcgtg 45
<210> 192
   <211> 45
   <212> DNA
   <213> Vespula vulgaris
<400> 192
   agatgtggtt ggaagaatcc gtcgagaatc gtaggcggtg ttgaa 45
<210> 193
   <211> 45
   <212> DNA
   <213> Vespula vulgaris
<400> 193
   gcttgccagt tcgacagtgg tggtccaatt ttatggcaaa atcca 45
<210> 194
   <211> 45
   <212> DNA
   <213> Vespula vulgaris
<400> 194
   ttgaattgca cagttttcga tgttcctccg agttcgaatt gttca 45
<210> 195
   <211> 45
   <212> DNA
   <213> Vespula vulgaris
<400> 195
   gaacataatt gtcggtggag tgctaggagc aatactcgaa ttaaa 45
<210> 196
   <211> 45
   <212> DNA
   <213> Vespula vulgaris
<400> 196
   tgccagttcg acagtggtgg tccaatttta tggcaaaatc caaaa 45
<210> 197
   <211> 45
   <212> DNA
   <213> Vespula vulgaris
<400> 197
   caacatgtat taacggctgc tcattgcgtt gcgaggtata aaagg 45
<210> 198
   <211> 45
   <212> DNA
   <213> Vespula vulgaris
<400> 198
   cataattgtc ggtggagtgc taggagcaat actcgaatta aattg 45
<210> 199
   <211> 45
   <212> DNA
   <213> Vespula vulgaris
<400> 199
   ggagaacata attgtcggtg gagtgctagg agcaatactc gaatt 45
<210> 200
   <211> 45
   <212> DNA
   <213> Vespula vulgaris
<400> 200
   gatgcttgcc agttcgacag tggtggtcca attttatggc aaaat 45
<210> 201
   <211> 45
   <212> DNA
   <213> Vespula vulgaris
<400> 201
   aattgtcggt ggagtgctag gagcaatact cgaattaaat tgaat 45
<210> 202
   <211> 45
   <212> DNA
   <213> Vespula vulgaris
<400> 202
   gccgatgaag ctcctggtgt taacttgaga gtcactagtt attta 45
<210> 203
   <211> 45
   <212> DNA
   <213> Vespula vulgaris
<400> 203
   acagttttcg atgttcctcc gagttcgaat tgttcattgg atttt 45
<210> 204
   <211> 45
   <212> DNA
   <213> Vespula vulgaris
<400> 204
   aatccgtcga gaatcgtagg cggtgttgaa acaggagtga atgaa 45
<210> 205
   <211> 45
   <212> DNA
   <213> Vespula vulgaris
<400> 205
   aagaatccgt cgagaatcgt aggcggtgtt gaaacaggag tgaat 45
<210> 206
   <211> 45
   <212> DNA
   <213> Vespula vulgaris
<400> 206
   tgtggattaa attcttttgc agtggaatca atagcatcga aaatg 45
<210> 207
   <211> 45
   <212> DNA
   <213> Vespula vulgaris
<400> 207
   ataaaattgc agaagaggtt aaaatattct atgagaattg gtcca 45
<210> 208
   <211> 45
   <212> DNA
   <213> Vespula vulgaris
<400> 208
   aaggatgctt gccagttcga cagtggtggt ccaattttat ggcaa 45
<210> 209
   <211> 45
   <212> DNA
   <213> Vespula vulgaris
<400> 209
   gctgttataa aattgcagaa gaggttaaaa tattctatga gaatt 45
<210> 210
   <211> 45
   <212> DNA
   <213> Vespula vulgaris
<400> 210
   agttatttag attttattac aagatcaaca cctggagaaa tatat 45
<210> 211
   <211> 45
   <212> DNA
   <213> Vespula vulgaris
<400> 211
   gttataaaat tgcagaagag gttaaaatat tctatgagaa ttggt 45
<210> 212
   <211> 45
   <212> DNA
   <213> Vespula vulgaris
<400> 212
   ggctacaccc aaaaattaaa atcagatgtt aattattgcg tgtat 45
<210> 213
   <211> 45
   <212> DNA
   <213> Vespula vulgaris
<400> 213
   ttcgacagtg gtggtccaat tttatggcaa aatccaaaaa gcaaa 45
<210> 214
   <211> 45
   <212> DNA
   <213> Vespula vulgaris
<400> 214
   ataatcgtac atccgaatta taggccaaaa ttaaacgatt tagct 45
<210> 215
   <211> 45
   <212> DNA
   <213> Vespula vulgaris
<400> 215
   gatgaagctc ctggtgttaa cttgagagtc actagttatt tagat 45
<210> 216
   <211> 45
   <212> DNA
   <213> Vespula vulgaris
<400> 216
   catgtattaa cggctgctca ttgcgttgcg aggtataaaa ggatt 45
<210> 217
   <211> 45
   <212> DNA
   <213> Vespula vulgaris
<400> 217
   ggaaaggatg cttgccagtt cgacagtggt ggtccaattt tatgg 45
<210> 218
   <211> 45
   <212> DNA
   <213> Vespula vulgaris
<400> 218
   tatttagatt ttattacaag atcaacacct ggagaaatat attgt 45
<210> 219
   <211> 45
   <212> DNA
   <213> Vespula vulgaris
<400> 219
   ttgcagaaga ggttaaaata ttctatgaga attggtccag cttgt 45
<210> 220
   <211> 45
   <212> DNA
   <213> Vespula vulgaris
<400> 220
   tttatgaaag tcaaagtcga cgatgacatc gagtacgttt tctgt 45
<210> 221
   <211> 45
   <212> DNA
   <213> Vespula vulgaris
<400> 221
   gattttatga aagtcaaagt cgacgatgac atcgagtacg ttttc 45
<210> 222
   <211> 45
   <212> DNA
   <213> Vespula vulgaris
<400> 222
   atgaaagtca aagtcgacga tgacatcgag tacgttttct gtgga 45
<210> 223
   <211> 45
   <212> DNA
   <213> Vespula vulgaris
<400> 223
   atgaagaaat gcgtcaagta tcactttctg gctacaccta agcaa 45
<210> 224
   <211> 45
   <212> DNA
   <213> Vespula vulgaris
<400> 224
   gtcaagtatc actttctggc tacacctaag caactttgta cattc 45
<210> 225
   <211> 45
   <212> DNA
   <213> Vespula vulgaris
<400> 225
   aaatgcgtca agtatcactt tctggctaca cctaagcaac tttgt 45
<210> 226
   <211> 45
   <212> DNA
   <213> Vespula vulgaris
<400> 226
   tgcgtcaagt atcactttct ggctacacct aagcaacttt gtaca 45
<210> 227
   <211> 45
   <212> DNA
   <213> Vespula vulgaris
<400> 227
   aagaaatgcg tcaagtatca ctttctggct acacctaagc aactt 45
<210> 228
   <211> 45
   <212> DNA
   <213> Vespula vulgaris
<400> 228
   gatgttaatt attgcgtgta taatcctgat tttccgaatt attac 45
<210> 229
   <211> 45
   <212> DNA
   <213> Vespula vulgaris
<400> 229
   gttaattatt gcgtgtataa tcctgatttt ccgaattatt acatg 45
<210> 230
   <211> 45
   <212> DNA
   <213> Vespula vulgaris
<400> 230
   aattattgcg tgtataatcc tgattttccg aattattaca tggga 45
<210> 231
   <211> 45
   <212> DNA
   <213> Vespula vulgaris
<400> 231
   tattgcgtgt ataatcctga ttttccgaat tattacatgg gagaa 45
<210> 232
   <211> 45
   <212> DNA
   <213> Vespula vulgaris
<400> 232
   tgcgtgtata atcctgattt tccgaattat tacatgggag aacat 45
<210> 233
   <211> 45
   <212> DNA
   <213> Vespula vulgaris
<400> 233
   aagtatcact ttctggctac acctaagcaa ctttgtacat tcgat 45
<210> 234
   <211> 45
   <212> DNA
   <213> Vespula vulgaris
<400> 234
   tatcactttc tggctacacc taagcaactt tgtacattcg atatg 45
<210> 235
   <211> 45
   <212> DNA
   <213> Vespula vulgaris
<400> 235
   ttgaattgca cagttttcga tgttcctccg agttcgaatt gttca 45
<210> 236
   <211> 45
   <212> DNA
   <213> Vespula vulgaris
<400> 236
   gtcactagtt atttagattt tattacaaga tcaacacctg gagaa 45
<210> 237
   <211> 45
   <212> DNA
   <213> Vespula vulgaris
<400> 237
   actagttatt tagattttat tacaagatca acacctggag aaata 45
<210> 238
   <211> 45
   <212> DNA
   <213> Vespula vulgaris
<400> 238
   agagtcacta gttatttaga ttttattaca agatcaacac ctgga 45
<210> 239
   <211> 45
   <212> DNA
   <213> Vespula vulgaris
<400> 239
   aaatgcgtca agtatcactt tctggctaca cctaagcaac tttgt 45
<210> 240
   <211> 45
   <212> DNA
   <213> Vespula vulgaris
<400> 240
   ttgagagtca ctagttattt agattttatt acaagatcaa cacct 45
<210> 241
   <211> 45
   <212> DNA
   <213> Vespula vulgaris
<400> 241
   agcaaacgta ttttccttct tggagtcatc aattatggaa gaaca 45
<210> 242
   <211> 45
   <212> DNA
   <213> Vespula vulgaris
<400> 242
   aaacgtattt tccttcttgg agtcatcaat tatggaagaa catgt 45
<210> 243
   <211> 45
   <212> DNA
   <213> Vespula vulgaris
<400> 243
   aacttgagag tcactagtta tttagatttt attacaagat caaca 45
<210> 244
   <211> 45
   <212> DNA
   <213> Vespula vulgaris
<400> 244
   cgtattttcc ttcttggagt catcaattat ggaagaacat gtgcc 45
<210> 245
   <211> 45
   <212> DNA
   <213> Vespula vulgaris
<400> 245
   cctgattttc cgaattatta catgggagaa cataattgtc ggtgg 45
<210> 246
   <211> 45
   <212> DNA
   <213> Vespula vulgaris
<400> 246
   tttccgaatt attacatggg agaacataat tgtcggtgga gtgct 45
<210> 247
   <211> 45
   <212> DNA
   <213> Vespula vulgaris
<400> 247
   gattttccga attattacat gggagaacat aattgtcggt ggagt 45
<210> 248
   <211> 45
   <212> DNA
   <213> Vespula vulgaris
<400> 248
   agtggtggtc caattttatg gcaaaatcca aaaagcaaac gtatt 45
<210> 249
   <211> 45
   <212> DNA
   <213> Vespula vulgaris
<400> 249
   ttatatattc taggagttgt tgttggagaa cataatacat gggca 45
<210> 250
   <211> 45
   <212> DNA
   <213> Vespula vulgaris
<400> 250
   ccagcttgtc ttccgttcta ttacatgcag cgaaactttg tagac 45
<210> 251
   <211> 45
   <212> DNA
   <213> Vespula vulgaris
<400> 251
   gcttgtcttc cgttctatta catgcagcga aactttgtag acact 45
<210> 252
   <211> 45
   <212> DNA
   <213> Vespula vulgaris
<400> 252
   ccgttctatt acatgcagcg aaactttgta gacactgttg ttaca 45
<210> 253
   <211> 45
   <212> DNA
   <213> Vespula vulgaris
<400> 253
   tgtcttccgt tctattacat gcagcgaaac tttgtagaca ctgtt 45
<210> 254
   <211> 45
   <212> DNA
   <213> Vespula vulgaris
<400> 254
   cttccgttct attacatgca gcgaaacttt gtagacactg ttgtt 45
<210> 255
   <211> 45
   <212> DNA
   <213> Vespula vulgaris
<400> 255
   tattacatgc agcgaaactt tgtagacact gttgttacag ctgta 45
<210> 256
   <211> 45
   <212> DNA
   <213> Vespula vulgaris
<400> 256
   ttctattaca tgcagcgaaa ctttgtagac actgttgtta cagct 45
<210> 257
   <211> 45
   <212> DNA
   <213> Vespula vulgaris
<400> 257
   gagtacgttt tctgtggatt aaattctttt gcagtggaat caata 45
<210> 258
   <211> 45
   <212> DNA
   <213> Vespula vulgaris
<400> 258
   gatgacatcg agtacgtttt ctgtggatta aattcttttg cagtg 45
<210> 259
   <211> 45
   <212> DNA
   <213> Vespula vulgaris
<400> 259
   gacatcgagt acgttttctg tggattaaat tcttttgcag tggaa 45
<210> 260
   <211> 45
   <212> DNA
   <213> Vespula vulgaris
<400> 260
   atcgagtacg ttttctgtgg attaaattct tttgcagtgg aatca 45
<210> 261
   <211> 45
   <212> DNA
   <213> Vespula vulgaris
<400> 261
   tacgttttct gtggattaaa ttcttttgca gtggaatcaa tagca 45
<210> 262
   <211> 45
   <212> DNA
   <213> Vespula vulgaris
<400> 262
   tattacatgc agcgaaactt tgtagacact gttgttacag ctgta 45
<210> 263
   <211> 45
   <212> DNA
   <213> Vespula vulgaris
<400> 263
   gctggtataa tacatcttgc aacgcgtttt ctctattgcg gtgca 45
<210> 264
   <211> 45
   <212> DNA
   <213> Vespula vulgaris
<400> 264
   atgatggctg gtataataca tcttgcaacg cgttttctct attgc 45
<210> 265
   <211> 45
   <212> DNA
   <213> Vespula vulgaris
<400> 265
   atgcagcgaa actttgtaga cactgttgtt acagctgtag gatgg 45
<210> 266
   <211> 45
   <212> DNA
   <213> Vespula vulgaris
<400> 266
   cagcgaaact ttgtagacac tgttgttaca gctgtaggat ggggt 45
<210> 267
   <211> 45
   <212> DNA
   <213> Vespula vulgaris
<400> 267
   cctatgatgg ctggtataat acatcttgca acgcgttttc tctat 45
<210> 268
   <211> 45
   <212> DNA
   <213> Vespula vulgaris
<400> 268
   tacatgcagc gaaactttgt agacactgtt gttacagctg tagga 45
<210> 269
   <211> 45
   <212> DNA
   <213> Vespula vulgaris
<400> 269
   tggggtctta cgaattttta tggtgtcaag tctgaagttt tgaga 45
<210> 270
   <211> 45
   <212> DNA
   <213> Vespula vulgaris
<400> 270
   cttacgaatt tttatggtgt caagtctgaa gttttgagaa aagtc 45
<210> 271
   <211> 45
   <212> DNA
   <213> Vespula vulgaris
<400> 271
   ggatggggtc ttacgaattt ttatggtgtc aagtctgaag ttttg 45
<210> 272
   <211> 45
   <212> DNA
   <213> Vespula vulgaris
<400> 272
   acgaattttt atggtgtcaa gtctgaagtt ttgagaaaag tcgat 45
<210> 273
   <211> 45
   <212> DNA
   <213> Vespula vulgaris
<400> 273
   gttttctgtg gattaaattc ttttgcagtg gaatcaatag catcg 45
<210> 274
   <211> 45
   <212> DNA
   <213> Vespula vulgaris
<400> 274
   ttctgtggat taaattcttt tgcagtggaa tcaatagcat cgaaa 45
<210> 275
   <211> 30
   <212> DNA
   <213> Vespula vulgaris
<400> 275
   cctggagaaa tatattgtca ggcgtattaa 30
<210> 276
   <211> 45
   <212> DNA
   <213> Vespula vulgaris
<400> 276
   aattgtcggt ggagtgctag gagcaatact cgaattaaat tgaat 45
<210> 277
   <211> 45
   <212> DNA
   <213> Vespula vulgaris
<400> 277
   gctcctggtg ttaacttgag agtcactagt tatttagatt ttatt 45
<210> 278
   <211> 45
   <212> DNA
   <213> Vespula vulgaris
<400> 278
   ggagaacata attgtcggtg gagtgctagg agcaatactc gaatt 45
<210> 279
   <211> 45
   <212> DNA
   <213> Vespula vulgaris
<400> 279
   gaagctcctg gtgttaactt gagagtcact agttatttag atttt 45
<210> 280
   <211> 45
   <212> DNA
   <213> Vespula vulgaris
<400> 280
   cataattgtc ggtggagtgc taggagcaat actcgaatta aattg 45
<210> 281
   <211> 45
   <212> DNA
   <213> Vespula vulgaris
<400> 281
   gaacataatt gtcggtggag tgctaggagc aatactcgaa ttaaa 45
<210> 282
   <211> 45
   <212> DNA
   <213> Vespula vulgaris
<400> 282
   tgtcggtgga gtgctaggag caatactcga attaaattga attgc 45
<210> 283
   <211> 45
   <212> DNA
   <213> Vespula vulgaris
<400> 283
   catgtattaa cggctgctca ttgcgttgcg aggtataaaa ggatt 45
<210> 284
   <211> 45
   <212> DNA
   <213> Vespula vulgaris
<400> 284
   acggctgctc attgcgttgc gaggtataaa aggattttat atatt 45
<210> 285
   <211> 45
   <212> DNA
   <213> Vespula vulgaris
<400> 285
   ccaattttat ggcaaaatcc aaaaagcaaa cgtattttcc ttctt 45
<210> 286
   <211> 45
   <212> DNA
   <213> Vespula vulgaris
<400> 286
   gctgctcatt gcgttgcgag gtataaaagg attttatata ttcta 45
<210> 287
   <211> 45
   <212> DNA
   <213> Vespula vulgaris
<400> 287
   ggtataatac atcttgcaac gcgttttctc tattgcggtg caact 45
<210> 288
   <211> 45
   <212> DNA
   <213> Vespula vulgaris
<400> 288
   attttatggc aaaatccaaa aagcaaacgt attttccttc ttgga 45
<210> 289
   <211> 45
   <212> DNA
   <213> Vespula vulgaris
<400> 289
   cttccgttct attacatgca gcgaaacttt gtagacactg ttgtt 45
<210> 290
   <211> 45
   <212> DNA
   <213> Vespula vulgaris
<400> 290
   ggtccaattt tatggcaaaa tccaaaaagc aaacgtattt tcctt 45
<210> 291
   <211> 45
   <212> DNA
   <213> Vespula vulgaris
<400> 291
   ttagctgtta taaaattgca gaagaggtta aaatattcta tgaga 45
<210> 292
   <211> 45
   <212> DNA
   <213> Vespula vulgaris
<400> 292
   gatttagctg ttataaaatt gcagaagagg ttaaaatatt ctatg 45
<210> 293
   <211> 45
   <212> DNA
   <213> Vespula vulgaris
<400> 293
   gctgttataa aattgcagaa gaggttaaaa tattctatga gaatt 45
<210> 294
   <211> 45
   <212> DNA
   <213> Vespula vulgaris
<400> 294
   ttaaacgatt tagctgttat aaaattgcag aagaggttaa aatat 45
<210> 295
   <211> 45
   <212> DNA
   <213> Vespula vulgaris
<400> 295
   aacgatttag ctgttataaa attgcagaag aggttaaaat attct 45
<210> 296
   <211> 45
   <212> DNA
   <213> Vespula vulgaris
<400> 296
   gttataaaat tgcagaagag gttaaaatat tctatgagaa ttggt 45
<210> 297
   <211> 45
   <212> DNA
   <213> Vespula vulgaris
<400> 297
   gtcaagtatc actttctggc tacacctaag caactttgta cattc 45
<210> 298
   <211> 45
   <212> DNA
   <213> Vespula vulgaris
<400> 298
   tgcgtcaagt atcactttct ggctacacct aagcaacttt gtaca 45
<210> 299
   <211> 45
   <212> DNA
   <213> Vespula vulgaris
<400> 299
   aaatgcgtca agtatcactt tctggctaca cctaagcaac tttgt 45
<210> 300
   <211> 45
   <212> DNA
   <213> Vespula vulgaris
<400> 300
   aagaaatgcg tcaagtatca ctttctggct acacctaagc aactt 45
<210> 301
   <211> 45
   <212> DNA
   <213> Vespula vulgaris
<400> 301
   atgaagaaat gcgtcaagta tcactttctg gctacaccta agcaa 45
<210> 302
   <211> 45
   <212> DNA
   <213> Vespula vulgaris
<400> 302
   actagttatt tagattttat tacaagatca acacctggag aaata 45
<210> 303
   <211> 45
   <212> DNA
   <213> Vespula vulgaris
<400> 303
   agttatttag attttattac aagatcaaca cctggagaaa tatat 45
<210> 304
   <211> 45
   <212> DNA
   <213> Vespula vulgaris
<400> 304
   tatttagatt ttattacaag atcaacacct ggagaaatat attgt 45
<210> 305
   <211> 45
   <212> DNA
   <213> Vespula vulgaris
<400> 305
   ttagatttta ttacaagatc aacacctgga gaaatatatt gtcag 45
<210> 306
   <211> 45
   <212> DNA
   <213> Vespula vulgaris
<400> 306
   gattttatta caagatcaac acctggagaa atatattgtc aggcg 45
<210> 307
   <211> 45
   <212> DNA
   <213> Vespula vulgaris
<400> 307
   aatgaatatc ctatgatggc tggtataata catcttgcaa cgcgt 45
<210> 308
   <211> 45
   <212> DNA
   <213> Vespula vulgaris
<400> 308
   gtgaatgaat atcctatgat ggctggtata atacatcttg caacg 45
<210> 309
   <211> 45
   <212> DNA
   <213> Vespula vulgaris
<400> 309
   acaggagtga atgaatatcc tatgatggct ggtataatac atctt 45
<210> 310
   <211> 45
   <212> DNA
   <213> Vespula vulgaris
<400> 310
   gaaacaggag tgaatgaata tcctatgatg gctggtataa tacat 45
<210> 311
   <211> 45
   <212> DNA
   <213> Vespula vulgaris
<400> 311
   ggagtgaatg aatatcctat gatggctggt ataatacatc ttgca 45
<210> 312
   <211> 45
   <212> DNA
   <213> Vespula vulgaris
<400> 312
   gagaaatgta gatgtggttg gaagaatccg tcgagaatcg taggc 45
<210> 313
   <211> 45
   <212> DNA
   <213> Vespula vulgaris
<400> 313
   ttagctgtta taaaattgca gaagaggtta aaatattcta tgaga 45
<210> 314
   <211> 45
   <212> DNA
   <213> Vespula vulgaris
<400> 314
   tatcctatga tggctggtat aatacatctt gcaacgcgtt ttctc 45
<210> 315
   <211> 45
   <212> DNA
   <213> Vespula vulgaris
<400> 315
   gatttagctg ttataaaatt gcagaagagg ttaaaatatt ctatg 45
<210> 316
   <211> 45
   <212> DNA
   <213> Vespula vulgaris
<400> 316
   aaatgtagat gtggttggaa gaatccgtcg agaatcgtag gcggt 45
<210> 317
   <211> 45
   <212> DNA
   <213> Vespula vulgaris
<400> 317
   tgtagatgtg gttggaagaa tccgtcgaga atcgtaggcg gtgtt 45
<210> 318
   <211> 45
   <212> DNA
   <213> Vespula vulgaris
<400> 318
   aacgatttag ctgttataaa attgcagaag aggttaaaat attct 45
<210> 319
   <211> 45
   <212> DNA
   <213> Vespula vulgaris
<400> 319
   ttaaacgatt tagctgttat aaaattgcag aagaggttaa aatat 45
<210> 320
   <211> 45
   <212> DNA
   <213> Vespula vulgaris
<400> 320
   agatgtggtt ggaagaatcc gtcgagaatc gtaggcggtg ttgaa 45
<210> 321
   <211> 45
   <212> DNA
   <213> Vespula vulgaris
<400> 321
   tgtggttgga agaatccgtc gagaatcgta ggcggtgttg aaaca 45
<210> 322
   <211> 45
   <212> DNA
   <213> Vespula vulgaris
<400> 322
   gctgttataa aattgcagaa gaggttaaaa tattctatga gaatt 45
<210> 323
   <211> 45
   <212> DNA
   <213> Vespula vulgaris
<400> 323
   cctatgatgg ctggtataat acatcttgca acgcgttttc tctat 45
<210> 324
   <211> 45
   <212> DNA
   <213> Vespula vulgaris
<400> 324
   atgatggctg gtataataca tcttgcaacg cgttttctct attgc 45
<210> 325
   <211> 45
   <212> DNA
   <213> Vespula vulgaris
<400> 325
   gaatatccta tgatggctgg tataatacat cttgcaacgc gtttt 45
<210> 326
   <211> 45
   <212> DNA
   <213> Vespula vulgaris
<400> 326
   gctggtataa tacatcttgc aacgcgtttt ctctattgcg gtgca 45
<210> 327
   <211> 45
   <212> DNA
   <213> Vespula vulgaris
<400> 327
   atggctggta taatacatct tgcaacgcgt tttctctatt gcggt 45
<210> 328
   <211> 45
   <212> DNA
   <213> Vespula vulgaris
<400> 328
   cgtattttcc ttcttggagt catcaattat ggaagaacat gtgcc 45
<210> 329
   <211> 45
   <212> DNA
   <213> Vespula vulgaris
<400> 329
   attttccttc ttggagtcat caattatgga agaacatgtg ccgat 45
<210> 330
   <211> 45
   <212> DNA
   <213> Vespula vulgaris
<400> 330
   ttaaacgatt tagctgttat aaaattgcag aagaggttaa aatat 45
<210> 331
   <211> 45
   <212> DNA
   <213> Vespula vulgaris
<400> 331
   ttagctgtta taaaattgca gaagaggtta aaatattcta tgaga 45
<210> 332
   <211> 45
   <212> DNA
   <213> Vespula vulgaris
<400> 332
   gcgaggtata aaaggatttt atatattcta ggagttgttg ttgga 45
<210> 333
   <211> 45
   <212> DNA
   <213> Vespula vulgaris
<400> 333
   gatttagctg ttataaaatt gcagaagagg ttaaaatatt ctatg 45
<210> 334
   <211> 45
   <212> DNA
   <213> Vespula vulgaris
<400> 334
   tgtggttgga agaatccgtc gagaatcgta ggcggtgttg aaaca 45
<210> 335
   <211> 45
   <212> DNA
   <213> Vespula vulgaris
<400> 335
   aggtataaaa ggattttata tattctagga gttgttgttg gagaa 45
<210> 336
   <211> 45
   <212> DNA
   <213> Vespula vulgaris
<400> 336
   aacgatttag ctgttataaa attgcagaag aggttaaaat attct 45
<210> 337
   <211> 45
   <212> DNA
   <213> Vespula vulgaris
<400> 337
   tgtagatgtg gttggaagaa tccgtcgaga atcgtaggcg gtgtt 45
<210> 338
   <211> 45
   <212> DNA
   <213> Vespula vulgaris
<400> 338
   aaaaggattt tatatattct aggagttgtt gttggagaac ataat 45
<210> 339
   <211> 45
   <212> DNA
   <213> Vespula vulgaris
<400> 339
   gagaaatgta gatgtggttg gaagaatccg tcgagaatcg taggc 45
<210> 340
   <211> 45
   <212> DNA
   <213> Vespula vulgaris
<400> 340
   aaatgtagat gtggttggaa gaatccgtcg agaatcgtag gcggt 45
<210> 341
   <211> 45
   <212> DNA
   <213> Vespula vulgaris
<400> 341
   tataaaagga ttttatatat tctaggagtt gttgttggag aacat 45
<210> 342
   <211> 45
   <212> DNA
   <213> Vespula vulgaris
<400> 342
   gctgttataa aattgcagaa gaggttaaaa tattctatga gaatt 45
<210> 343
   <211> 45
   <212> DNA
   <213> Vespula vulgaris
<400> 343
   aggattttat atattctagg agttgttgtt ggagaacata ataca 45
<210> 344
   <211> 45
   <212> DNA
   <213> Vespula vulgaris
<400> 344
   aaacgtattt tccttcttgg agtcatcaat tatggaagaa catgt 45
<210> 345
   <211> 45
   <212> DNA
   <213> Vespula vulgaris
<400> 345
   agcaaacgta ttttccttct tggagtcatc aattatggaa gaaca 45
<210> 346
   <211> 45
   <212> DNA
   <213> Vespula vulgaris
<400> 346
   agatgtggtt ggaagaatcc gtcgagaatc gtaggcggtg ttgaa 45
<210> 347
   <211> 45
   <212> DNA
   <213> Vespula vulgaris
<400> 347
   ttccttcttg gagtcatcaa ttatggaaga acatgtgccg atgaa 45
<210> 348
   <211> 45
   <212> DNA
   <213> Vespula vulgaris
<400> 348
   ataaaattgc agaagaggtt aaaatattct atgagaattg gtcca 45
<210> 349
   <211> 45
   <212> DNA
   <213> Vespula vulgaris
<400> 349
   atggctggta taatacatct tgcaacgcgt tttctctatt gcggt 45
<210> 350
   <211> 45
   <212> DNA
   <213> Vespula vulgaris
<400> 350
   atagcatcga aaatgactat aaaattccat tcaagataca atact 45
<210> 351
   <211> 45
   <212> DNA
   <213> Vespula vulgaris
<400> 351
   gccgatgaag ctcctggtgt taacttgaga gtcactagtt attta 45
<210> 352
   <211> 45
   <212> DNA
   <213> Vespula vulgaris
<400> 352
   gatgaagctc ctggtgttaa cttgagagtc actagttatt tagat 45
<210> 353
   <211> 45
   <212> DNA
   <213> Vespula vulgaris
<400> 353
   gaagctcctg gtgttaactt gagagtcact agttatttag atttt 45
<210> 354
   <211> 45
   <212> DNA
   <213> Vespula vulgaris
<400> 354
   gctggtataa tacatcttgc aacgcgtttt ctctattgcg gtgca 45
<210> 355
   <211> 45
   <212> DNA
   <213> Vespula vulgaris
<400> 355
   gttataaaat tgcagaagag gttaaaatat tctatgagaa ttggt 45
<210> 356
   <211> 45
   <212> DNA
   <213> Vespula vulgaris
<400> 356
   acagctgtag gatggggtct tacgaatttt tatggtgtca agtct 45
<210> 357
   <211> 45
   <212> DNA
   <213> Vespula vulgaris
<400> 357
   tggaagaatc cgtcgagaat cgtaggcggt gttgaaacag gagtg 45
<210> 358
   <211> 45
   <212> DNA
   <213> Vespula vulgaris
<400> 358
   tggggtctta cgaattttta tggtgtcaag tctgaagttt tgaga 45
<210> 359
   <211> 45
   <212> DNA
   <213> Vespula vulgaris
<400> 359
   gctcctggtg ttaacttgag agtcactagt tatttagatt ttatt 45
<210> 360
   <211> 45
   <212> DNA
   <213> Vespula vulgaris
<400> 360
   atgatggctg gtataataca tcttgcaacg cgttttctct attgc 45
<210> 361
   <211> 45
   <212> DNA
   <213> Vespula vulgaris
<400> 361
   cctatgatgg ctggtataat acatcttgca acgcgttttc tctat 45
<210> 362
   <211> 45
   <212> DNA
   <213> Vespula vulgaris
<400> 362
   ggttggaaga atccgtcgag aatcgtaggc ggtgttgaaa cagga 45
<210> 363
   <211> 45
   <212> DNA
   <213> Vespula vulgaris
<400> 363
   cttcttggag tcatcaatta tggaagaaca tgtgccgatg aagct 45
<210> 364
   <211> 45
   <212> DNA
   <213> Vespula vulgaris
<400> 364
   cataatacat gggcaattaa cgatacaaag gcaactcaac tttat 45
<210> 365
   <211> 45
   <212> DNA
   <213> Vespula vulgaris
<400> 365
   cttacgaatt tttatggtgt caagtctgaa gttttgagaa aagtc 45
<210> 366
   <211> 45
   <212> DNA
   <213> Vespula vulgaris
<400> 366
   cctggtgtta acttgagagt cactagttat ttagatttta ttaca 45
<210> 367
   <211> 45
   <212> DNA
   <213> Vespula vulgaris
<400> 367
   cttggagtca tcaattatgg aagaacatgt gccgatgaag ctcct 45
<210> 368
   <211> 45
   <212> DNA
   <213> Vespula vulgaris
<400> 368
   aatacatggg caattaacga tacaaaggca actcaacttt atctt 45
<210> 369
   <211> 45
   <212> DNA
   <213> Vespula vulgaris
<400> 369
   gaacataata catgggcaat taacgataca aaggcaactc aactt 45
<210> 370
   <211> 45
   <212> DNA
   <213> Vespula vulgaris
<400> 370
   attttatata ttctaggagt tgttgttgga gaacataata catgg 45
<210> 371
   <211> 45
   <212> DNA
   <213> Vespula vulgaris
<400> 371
   ggtataatac atcttgcaac gcgttttctc tattgcggtg caact 45
<210> 372
   <211> 45
   <212> DNA
   <213> Vespula vulgaris
<400> 372
   gtaggatggg gtcttacgaa tttttatggt gtcaagtctg aagtt 45
<210> 373
   <211> 45
   <212> DNA
   <213> Vespula vulgaris
<400> 373
   gctgtaggat ggggtcttac gaatttttat ggtgtcaagt ctgaa 45
<210> 374
   <211> 45
   <212> DNA
   <213> Vespula vulgaris
<400> 374
   gttggagaac ataatacatg ggcaattaac gatacaaagg caact 45
<210> 375
   <211> 45
   <212> DNA
   <213> Vespula vulgaris
<400> 375
   aaaagcaaac gtattttcct tcttggagtc atcaattatg gaaga 45
<210> 376
   <211> 45
   <212> DNA
   <213> Vespula vulgaris
<400> 376
   cattgcgttg cgaggtataa aaggatttta tatattctag gagtt 45
<210> 377
   <211> 45
   <212> DNA
   <213> Vespula vulgaris
<400> 377
   gcatcgaaaa tgactataaa attccattca agatacaata cttat 45
<210> 378
   <211> 45
   <212> DNA
   <213> Vespula vulgaris
<400> 378
   gctcattgcg ttgcgaggta taaaaggatt ttatatattc tagga 45
<210> 379
   <211> 45
   <212> DNA
   <213> Vespula vulgaris
<400> 379
   ccaaaaagca aacgtatttt ccttcttgga gtcatcaatt atgga 45
<210> 380
   <211> 45
   <212> DNA
   <213> Vespula vulgaris
<400> 380
   tcgaaaatga ctataaaatt ccattcaaga tacaatactt atgga 45
<210> 381
   <211> 45
   <212> DNA
   <213> Vespula vulgaris
<400> 381
   gctaggagca atactcgaat taaattgaat tgcacagttt tcgat 45
<210> 382
   <211> 45
   <212> DNA
   <213> Vespula vulgaris
<400> 382
   ggagaacata atacatgggc aattaacgat acaaaggcaa ctcaa 45
<210> 383
   <211> 45
   <212> DNA
   <213> Vespula vulgaris
<400> 383
   aggagcaata ctcgaattaa attgaattgc acagttttcg atgtt 45
<210> 384
   <211> 45
   <212> DNA
   <213> Vespula vulgaris
<400> 384
   gttgcgaggt ataaaaggat tttatatatt ctaggagttg ttgtt 45
<210> 385
   <211> 45
   <212> DNA
   <213> Vespula vulgaris
<400> 385
   ttatatattc taggagttgt tgttggagaa cataatacat gggca 45
<210> 386
   <211> 45
   <212> DNA
   <213> Vespula vulgaris
<400> 386
   aaaatgacta taaaattcca ttcaagatac aatacttatg gaggc 45
<210> 387
   <211> 45
   <212> DNA
   <213> Vespula vulgaris
<400> 387
   atgactataa aattccattc aagatacaat acttatggag gcaaa 45
<210> 388
   <211> 45
   <212> DNA
   <213> Vespula vulgaris
<400> 388
   gttgttacag ctgtaggatg gggtcttacg aatttttatg gtgtc 45
<210> 389
   <211> 45
   <212> DNA
   <213> Vespula vulgaris
<400> 389
   agcaatactc gaattaaatt gaattgcaca gttttcgatg ttcct 45
<210> 390
   <211> 45
   <212> DNA
   <213> Vespula vulgaris
<400> 390
   aatactcgaa ttaaattgaa ttgcacagtt ttcgatgttc ctccg 45
<210> 391
   <211> 45
   <212> DNA
   <213> Vespula vulgaris
<400> 391
   acgaattttt atggtgtcaa gtctgaagtt ttgagaaaag tcgat 45
<210> 392
   <211> 45
   <212> DNA
   <213> Vespula vulgaris
<400> 392
   gttacagctg taggatgggg tcttacgaat ttttatggtg tcaag 45
<210> 393
   <211> 45
   <212> DNA
   <213> Vespula vulgaris
<400> 393
   aatttttatg gtgtcaagtc tgaagttttg agaaaagtcg atttg 45
<210> 394
   <211> 45
   <212> DNA
   <213> Vespula vulgaris
<400> 394
   aaattaaacg atttagctgt tataaaattg cagaagaggt taaaa 45
<210> 395
   <211> 45
   <212> DNA
   <213> Vespula vulgaris
<400> 395
   actcgaatta aattgaattg cacagttttc gatgttcctc cgagt 45
<210> 396
   <211> 45
   <212> DNA
   <213> Vespula vulgaris
<400> 396
   gacactgttg ttacagctgt aggatggggt cttacgaatt tttat 45
<210> 397
   <211> 45
   <212> DNA
   <213> Vespula vulgaris
<400> 397
   ggtcttacga atttttatgg tgtcaagtct gaagttttga gaaaa 45
<210> 398
   <211> 45
   <212> DNA
   <213> Vespula vulgaris
<400> 398
   tgcgttgcga ggtataaaag gattttatat attctaggag ttgtt 45
<210> 399
   <211> 45
   <212> DNA
   <213> Vespula vulgaris
<400> 399
   tatcctatga tggctggtat aatacatctt gcaacgcgtt ttctc 45
<210> 400
   <211> 45
   <212> DNA
   <213> Vespula vulgaris
<400> 400
   ccaaaattaa acgatttagc tgttataaaa ttgcagaaga ggtta 45
<210> 401
   <211> 45
   <212> DNA
   <213> Vespula vulgaris
<400> 401
   aggccaaaat taaacgattt agctgttata aaattgcaga agagg 45
<210> 402
   <211> 45
   <212> DNA
   <213> Vespula vulgaris
<400> 402
   aatccaaaaa gcaaacgtat tttccttctt ggagtcatca attat 45
<210> 403
   <211> 45
   <212> DNA
   <213> Vespula vulgaris
<400> 403
   ataaaattcc attcaagata caatacttat ggaggcaaat ttcga 45
<210> 404
   <211> 45
   <212> DNA
   <213> Vespula vulgaris
<400> 404
   ggatggggtc ttacgaattt ttatggtgtc aagtctgaag ttttg 45
<210> 405
   <211> 45
   <212> DNA
   <213> Vespula vulgaris
<400> 405
   actgttgtta cagctgtagg atggggtctt acgaattttt atggt 45
<210> 406
   <211> 45
   <212> DNA
   <213> Vespula vulgaris
<400> 406
   ttgcagaaga ggttaaaata ttctatgaga attggtccag cttgt 45
<210> 407
   <211> 45
   <212> DNA
   <213> Vespula vulgaris
<400> 407
   acatgggcaa ttaacgatac aaaggcaact caactttatc ttatt 45
<210> 408
   <211> 45
   <212> DNA
   <213> Vespula vulgaris
<400> 408
   gatttgcatg tagtttcaat gaagaaatgc gtcaagtatc acttt 45
<210> 409
   <211> 45
   <212> DNA
   <213> Vespula vulgaris
<400> 409
   tgggcaatta acgatacaaa ggcaactcaa ctttatctta ttgat 45
<210> 410
   <211> 45
   <212> DNA
   <213> Vespula vulgaris
<400> 410
   ttgcatgtag tttcaatgaa gaaatgcgtc aagtatcact ttctg 45
<210> 411
   <211> 45
   <212> DNA
   <213> Vespula vulgaris
<400> 411
   ataatacatc ttgcaacgcg ttttctctat tgcggtgcaa ctata 45
<210> 412
   <211> 45
   <212> DNA
   <213> Vespula vulgaris
<400> 412
   tcttttgcag tggaatcaat agcatcgaaa atgactataa aattc 45
<210> 413
   <211> 45
   <212> DNA
   <213> Vespula vulgaris
<400> 413
   gcagtggaat caatagcatc gaaaatgact ataaaattcc attca 45
<210> 414
   <211> 45
   <212> DNA
   <213> Vespula vulgaris
<400> 414
   aaattgcaga agaggttaaa atattctatg agaattggtc cagct 45
<210> 415
   <211> 45
   <212> DNA
   <213> Vespula vulgaris
<400> 415
   tttgcagtgg aatcaatagc atcgaaaatg actataaaat tccat 45
<210> 416
   <211> 45
   <212> DNA
   <213> Vespula vulgaris
<400> 416
   agcaaacgta ttttccttct tggagtcatc aattatggaa gaaca 45
<210> 417
   <211> 45
   <212> DNA
   <213> Vespula vulgaris
<400> 417
   aaaagcaaac gtattttcct tcttggagtc atcaattatg gaaga 45
<210> 418
   <211> 45
   <212> DNA
   <213> Vespula vulgaris
<400> 418
   aaacgtattt tccttcttgg agtcatcaat tatggaagaa catgt 45
<210> 419
   <211> 45
   <212> DNA
   <213> Vespula vulgaris
<400> 419
   cgtattttcc ttcttggagt catcaattat ggaagaacat gtgcc 45
<210> 420
   <211> 45
   <212> DNA
   <213> Vespula vulgaris
<400> 420
   attttccttc ttggagtcat caattatgga agaacatgtg ccgat 45
<210> 421
   <211> 45
   <212> DNA
   <213> Vespula vulgaris
<400> 421
   gtcaagtctg aagttttgag aaaagtcgat ttgcatgtag tttca 45
<210> 422
   <211> 45
   <212> DNA
   <213> Vespula vulgaris
<400> 422
   ggtgtcaagt ctgaagtttt gagaaaagtc gatttgcatg tagtt 45
<210> 423
   <211> 45
   <212> DNA
   <213> Vespula vulgaris
<400> 423
   tctgaagttt tgagaaaagt cgatttgcat gtagtttcaa tgaag 45
<210> 424
   <211> 45
   <212> DNA
   <213> Vespula vulgaris
<400> 424
   aagtctgaag ttttgagaaa agtcgatttg catgtagttt caatg 45
<210> 425
   <211> 45
   <212> DNA
   <213> Vespula vulgaris
<400> 425
   ttccttcttg gagtcatcaa ttatggaaga acatgtgccg atgaa 45
<210> 426
   <211> 45
   <212> DNA
   <213> Vespula vulgaris
<400> 426
   tataatcctg attttccgaa ttattacatg ggagaacata attgt 45
<210> 427
   <211> 45
   <212> DNA
   <213> Vespula vulgaris
<400> 427
   gaagttttga gaaaagtcga tttgcatgta gtttcaatga agaaa 45
<210> 428
   <211> 45
   <212> DNA
   <213> Vespula vulgaris
<400> 428
   aacgatttag ctgttataaa attgcagaag aggttaaaat attct 45
<210> 429
   <211> 45
   <212> DNA
   <213> Vespula vulgaris
<400> 429
   cttcttggag tcatcaatta tggaagaaca tgtgccgatg aagct 45
<210> 430
   <211> 45
   <212> DNA
   <213> Vespula vulgaris
<400> 430
   ttaaacgatt tagctgttat aaaattgcag aagaggttaa aatat 45
<210> 431
   <211> 45
   <212> DNA
   <213> Vespula vulgaris
<400> 431
   aaattaaacg atttagctgt tataaaattg cagaagaggt taaaa 45
<210> 432
   <211> 45
   <212> DNA
   <213> Vespula vulgaris
<400> 432
   gcgaggtata aaaggatttt atatattcta ggagttgttg ttgga 45
<210> 433
   <211> 45
   <212> DNA
   <213> Vespula vulgaris
<400> 433
   tataaaagga ttttatatat tctaggagtt gttgttggag aacat 45
<210> 434
   <211> 45
   <212> DNA
   <213> Vespula vulgaris
<400> 434
   gttttgagaa aagtcgattt gcatgtagtt tcaatgaaga aatgc 45
<210> 435
   <211> 45
   <212> DNA
   <213> Vespula vulgaris
<400> 435
   aaaaggattt tatatattct aggagttgtt gttggagaac ataat 45
<210> 436
   <211> 45
   <212> DNA
   <213> Vespula vulgaris
<400> 436
   ccaaaattaa acgatttagc tgttataaaa ttgcagaaga ggtta 45
<210> 437
   <211> 45
   <212> DNA
   <213> Vespula vulgaris
<400> 437
   gatttagctg ttataaaatt gcagaagagg ttaaaatatt ctatg 45
<210> 438
   <211> 45
   <212> DNA
   <213> Vespula vulgaris
<400> 438
   aggtataaaa ggattttata tattctagga gttgttgttg gagaa 45
<210> 439
   <211> 45
   <212> DNA
   <213> Vespula vulgaris
<400> 439
   tggggtctta cgaattttta tggtgtcaag tctgaagttt tgaga 45
<210> 440
   <211> 45
   <212> DNA
   <213> Vespula vulgaris
<400> 440
   cttacgaatt tttatggtgt caagtctgaa gttttgagaa aagtc 45
<210> 441
   <211> 45
   <212> DNA
   <213> Vespula vulgaris
<400> 441
   aggccaaaat taaacgattt agctgttata aaattgcaga agagg 45
<210> 442
   <211> 45
   <212> DNA
   <213> Vespula vulgaris
<400> 442
   gttgcgaggt ataaaaggat tttatatatt ctaggagttg ttgtt 45
<210> 443
   <211> 45
   <212> DNA
   <213> Vespula vulgaris
<400> 443
   agaaaagtcg atttgcatgt agtttcaatg aagaaatgcg tcaag 45
<210> 444
   <211> 45
   <212> DNA
   <213> Vespula vulgaris
<400> 444
   gtcgatttgc atgtagtttc aatgaagaaa tgcgtcaagt atcac 45
<210> 445
   <211> 45
   <212> DNA
   <213> Vespula vulgaris
<400> 445
   aaagtcgatt tgcatgtagt ttcaatgaag aaatgcgtca agtat 45
<210> 446
   <211> 45
   <212> DNA
   <213> Vespula vulgaris
<400> 446
   gctgttataa aattgcagaa gaggttaaaa tattctatga gaatt 45
<210> 447
   <211> 45
   <212> DNA
   <213> Vespula vulgaris
<400> 447
   tatggtgtca agtctgaagt tttgagaaaa gtcgatttgc atgta 45
<210> 448
   <211> 45
   <212> DNA
   <213> Vespula vulgaris
<400> 448
   aggattttat atattctagg agttgttgtt ggagaacata ataca 45
<210> 449
   <211> 45
   <212> DNA
   <213> Vespula vulgaris
<400> 449
   gagaaatgta gatgtggttg gaagaatccg tcgagaatcg taggc 45
<210> 450
   <211> 45
   <212> DNA
   <213> Vespula vulgaris
<400> 450
   ggatggggtc ttacgaattt ttatggtgtc aagtctgaag ttttg 45
<210> 451
   <211> 45
   <212> DNA
   <213> Vespula vulgaris
<400> 451
   cattgcgttg cgaggtataa aaggatttta tatattctag gagtt 45
<210> 452
   <211> 45
   <212> DNA
   <213> Vespula vulgaris
<400> 452
   gttataaaat tgcagaagag gttaaaatat tctatgagaa ttggt 45
<210> 453
   <211> 45
   <212> DNA
   <213> Vespula vulgaris
<400> 453
   tgcgttgcga ggtataaaag gattttatat attctaggag ttgtt 45
<210> 454
   <211> 45
   <212> DNA
   <213> Vespula vulgaris
<400> 454
   gatttgcatg tagtttcaat gaagaaatgc gtcaagtatc acttt 45
<210> 455
   <211> 45
   <212> DNA
   <213> Vespula vulgaris
<400> 455
   ttgcatgtag tttcaatgaa gaaatgcgtc aagtatcact ttctg 45
<210> 456
   <211> 45
   <212> DNA
   <213> Vespula vulgaris
<400> 456
   acgaattttt atggtgtcaa gtctgaagtt ttgagaaaag tcgat 45
<210> 457
   <211> 45
   <212> DNA
   <213> Vespula vulgaris
<400> 457
   aaatgtagat gtggttggaa gaatccgtcg agaatcgtag gcggt 45
<210> 458
   <211> 45
   <212> DNA
   <213> Vespula vulgaris
<400> 458
   ggtcttacga atttttatgg tgtcaagtct gaagttttga gaaaa 45
<210> 459
   <211> 45
   <212> DNA
   <213> Vespula vulgaris
<400> 459
   gacagtggtg gtccaatttt atggcaaaat ccaaaaagca aacgt 45
<210> 460
   <211> 45
   <212> DNA
   <213> Vespula vulgaris
<400> 460
   agtggtggtc caattttatg gcaaaatcca aaaagcaaac gtatt 45
<210> 461
   <211> 45
   <212> DNA
   <213> Vespula vulgaris
<400> 461
   ggtggtccaa ttttatggca aaatccaaaa agcaaacgta ttttc 45
<210> 462
   <211> 45
   <212> DNA
   <213> Vespula vulgaris
<400> 462
   ggtccaattt tatggcaaaa tccaaaaagc aaacgtattt tcctt 45
<210> 463
   <211> 45
   <212> DNA
   <213> Vespula vulgaris
<400> 463
   ttcgacagtg gtggtccaat tttatggcaa aatccaaaaa gcaaa 45
<210> 464
   <211> 45
   <212> DNA
   <213> Vespula vulgaris
<400> 464
   ccaattttat ggcaaaatcc aaaaagcaaa cgtattttcc ttctt 45
<210> 465
   <211> 45
   <212> DNA
   <213> Vespula vulgaris
<400> 465
   ttagctgtta taaaattgca gaagaggtta aaatattcta tgaga 45
<210> 466
   <211> 45
   <212> DNA
   <213> Vespula vulgaris
<400> 466
   gatttagctg ttataaaatt gcagaagagg ttaaaatatt ctatg 45
<210> 467
   <211> 45
   <212> DNA
   <213> Vespula vulgaris
<400> 467
   ttaaacgatt tagctgttat aaaattgcag aagaggttaa aatat 45
<210> 468
   <211> 45
   <212> DNA
   <213> Vespula vulgaris
<400> 468
   aacgatttag ctgttataaa attgcagaag aggttaaaat attct 45
<210> 469
   <211> 45
   <212> DNA
   <213> Vespula vulgaris
<400> 469
   attttatggc aaaatccaaa aagcaaacgt attttccttc ttgga 45
<210> 470
   <211> 45
   <212> DNA
   <213> Vespula vulgaris
<400> 470
   gattttccga attattacat gggagaacat aattgtcggt ggagt 45
<210> 471
   <211> 45
   <212> DNA
   <213> Vespula vulgaris
<400> 471
   tttccgaatt attacatggg agaacataat tgtcggtgga gtgct 45
<210> 472
   <211> 45
   <212> DNA
   <213> Vespula vulgaris
<400> 472
   aatcctgatt ttccgaatta ttacatggga gaacataatt gtcgg 45
<210> 473
   <211> 45
   <212> DNA
   <213> Vespula vulgaris
<400> 473
   ggtggtccaa ttttatggca aaatccaaaa agcaaacgta ttttc 45
<210> 474
   <211> 45
   <212> DNA
   <213> Vespula vulgaris
<400> 474
   agtggtggtc caattttatg gcaaaatcca aaaagcaaac gtatt 45
<210> 475
   <211> 45
   <212> DNA
   <213> Vespula vulgaris
<400> 475
   ggtccaattt tatggcaaaa tccaaaaagc aaacgtattt tcctt 45
<210> 476
   <211> 45
   <212> DNA
   <213> Vespula vulgaris
<400> 476
   ataatcgtac atccgaatta taggccaaaa ttaaacgatt tagct 45
<210> 477
   <211> 45
   <212> DNA
   <213> Vespula vulgaris
<400> 477
   ccaattttat ggcaaaatcc aaaaagcaaa cgtattttcc ttctt 45
<210> 478
   <211> 45
   <212> DNA
   <213> Vespula vulgaris
<400> 478
   attttatggc aaaatccaaa aagcaaacgt attttccttc ttgga 45
<210> 479
   <211> 45
   <212> DNA
   <213> Vespula vulgaris
<400> 479
   atcgtacatc cgaattatag gccaaaatta aacgatttag ctgtt 45
<210> 480
   <211> 45
   <212> DNA
   <213> Vespula vulgaris
<400> 480
   gatgatataa tcgtacatcc gaattatagg ccaaaattaa acgat 45
<210> 481
   <211> 45
   <212> DNA
   <213> Vespula vulgaris
<400> 481
   attgatgata taatcgtaca tccgaattat aggccaaaat taaac 45
<210> 482
   <211> 45
   <212> DNA
   <213> Vespula vulgaris
<400> 482
   cttattgatg atataatcgt acatccgaat tataggccaa aatta 45
<210> 483
   <211> 45
   <212> DNA
   <213> Vespula vulgaris
<400> 483
   gacagtggtg gtccaatttt atggcaaaat ccaaaaagca aacgt 45
<210> 484
   <211> 45
   <212> DNA
   <213> Vespula vulgaris
<400> 484
   gtacatccga attataggcc aaaattaaac gatttagctg ttata 45
<210> 485
   <211> 45
   <212> DNA
   <213> Vespula vulgaris
<400> 485
   gatataatcg tacatccgaa ttataggcca aaattaaacg attta 45
<210> 486
   <211> 15
   <212> PRT
   <213> Vespula vulgaris
<400> 486
   Ser Gly Gly Pro Ile Leu Trp Gln Asn Pro Lys Ser Lys Arg Ile
   1 5 10 15
<210> 487
   <211> 15
   <212> PRT
   <213> Vespula vulgaris
<400> 487
   Gly Gly Pro Ile Leu Trp Gln Asn Pro Lys Ser Lys Arg Ile Phe
   1 5 10 15
<210> 488
   <211> 15
   <212> PRT
   <213> Vespula vulgaris
<400> 488
   Gly Pro Ile Leu Trp Gln Asn Pro Lys Ser Lys Arg Ile Phe Leu
   1 5 10 15
<210> 489
   <211> 15
   <212> PRT
   <213> Vespula vulgaris
<400> 489
   Pro Ile Leu Trp Gln Asn Pro Lys Ser Lys Arg Ile Phe Leu Leu
   1 5 10 15
<210> 490
   <211> 15
   <212> PRT
   <213> Vespula vulgaris
<400> 490
   Ile Leu Trp Gln Asn Pro Lys Ser Lys Arg Ile Phe Leu Leu Gly
   1 5 10 15
<210> 491
   <211> 15
   <212> PRT
   <213> Vespula vulgaris
<400> 491
   Tyr Pro Met Met Ala Gly Ile Ile His Leu Ala Thr Arg Phe Leu
   1 5 10 15
<210> 492
   <211> 15
   <212> PRT
   <213> Vespula vulgaris
<400> 492
   Ser Phe Ala Val Glu Ser Ile Ala Ser Lys Met Thr Ile Lys Phe
   1 5 10 15
<210> 493
   <211> 15
   <212> PRT
   <213> Vespula vulgaris
<400> 493
   Phe Ala Val Glu Ser Ile Ala Ser Lys Met Thr Ile Lys Phe His
   1 5 10 15
<210> 494
   <211> 15
   <212> PRT
   <213> Vespula vulgaris
<400> 494
   Asn Ser Phe Ala Val Glu Ser Ile Ala Ser Lys Met Thr Ile Lys
   1 5 10 15
<210> 495
   <211> 15
   <212> PRT
   <213> Vespula vulgaris
<400> 495
   Pro Met Met Ala Gly Ile Ile His Leu Ala Thr Arg Phe Leu Tyr
   1 5 10 15
<210> 496
   <211> 15
   <212> PRT
   <213> Vespula vulgaris
<400> 496
   Leu Asn Ser Phe Ala Val Glu Ser Ile Ala Ser Lys Met Thr Ile
   1 5 10 15
<210> 497
   <211> 15
   <212> PRT
   <213> Vespula vulgaris
<400> 497
   Met Ala Gly Ile Ile His Leu Ala Thr Arg Phe Leu Tyr Cys Gly
   1               5                   10                  15
<210> 498
   <211> 15
   <212> PRT
   <213> Vespula vulgaris
<400> 498
   Gly Leu Asn Ser Phe Ala Val Glu Ser Ile Ala Ser Lys Met Thr
   1               5                   10                  15
<210> 499
   <211> 15
   <212> PRT
   <213> Vespula vulgaris
<400> 499
   Ala Gly Ile Ile His Leu Ala Thr Arg Phe Leu Tyr Cys Gly Ala
   1               5                   10                  15
<210> 500
   <211> 15
   <212> PRT
   <213> Vespula vulgaris
<400> 500
   Met Met Ala Gly Ile Ile His Leu Ala Thr Arg Phe Leu Tyr Cys
   1               5                   10                  15
<210> 501
   <211> 15
   <212> PRT
   <213> Vespula vulgaris
<400> 501
   Glu Tyr Val Phe Cys Gly Leu Asn Ser Phe Ala Val Glu Ser Ile
   1               5                   10                  15
<210> 502
   <211> 15
   <212> PRT
   <213> Vespula vulgaris
<400> 502
   Tyr Val Phe Cys Gly Leu Asn Ser Phe Ala Val Glu Ser Ile Ala
   1               5                   10                  15
<210> 503
   <211> 15
   <212> PRT
   <213> Vespula vulgaris
<400> 503
   Ile Glu Tyr Val Phe Cys Gly Leu Asn Ser Phe Ala Val Glu Ser
   1 5 10 15
<210> 504
   <211> 15
   <212> PRT
   <213> Vespula vulgaris
<400> 504
   Asp Ile Glu Tyr Val Phe Cys Gly Leu Asn Ser Phe Ala Val Glu
   1 5 10 15
<210> 505
   <211> 15
   <212> PRT
   <213> Vespula vulgaris
<400> 505
   Asp Asp Ile Glu Tyr Val Phe Cys Gly Leu Asn Ser Phe Ala Val
   1 5 10 15
<210> 506
   <211> 15
   <212> PRT
   <213> Vespula vulgaris
<400> 506
   Val Asn Glu Tyr Pro Met Met Ala Gly Ile Ile His Leu Ala Thr
   1 5 10 15
<210> 507
   <211> 15
   <212> PRT
   <213> Vespula vulgaris
<400> 507
   Asn Glu Tyr Pro Met Met Ala Gly Ile Ile His Leu Ala Thr Arg
   1 5 10 15
<210> 508
   <211> 15
   <212> PRT
   <213> Vespula vulgaris
<400> 508
   Leu Thr Asn Phe Tyr Gly Val Lys Ser Glu Val Leu Arg Lys Val
   1 5 10 15
<210> 509
   <211> 15
   <212> PRT
   <213> Vespula vulgaris
<400> 509
   Gly Val Asn Glu Tyr Pro Met Met Ala Gly Ile Ile His Leu Ala
   1 5 10 15
<210> 510
   <211> 15
   <212> PRT
   <213> Vespula vulgaris
<400> 510
   Thr Asn Phe Tyr Gly Val Lys Ser Glu Val Leu Arg Lys Val Asp
   1 5 10 15
<210> 511
   <211> 15
   <212> PRT
   <213> Vespula vulgaris
<400> 511
   Leu Trp Gln Asn Pro Lys Ser Lys Arg Ile Phe Leu Leu Gly Val
   1 5 10 15
<210> 512
   <211> 15
   <212> PRT
   <213> Vespula vulgaris
<400> 512
   Trp Gln Asn Pro Lys Ser Lys Arg Ile Phe Leu Leu Gly Val Ile
   1 5 10 15
<210> 513
   <211> 15
   <212> PRT
   <213> Vespula vulgaris
<400> 513
   Lys Arg Leu Lys Tyr Ser Met Arg Ile Gly Pro Ala Cys Leu Pro
   1 5 10 15
<210> 514
   <211> 15
   <212> PRT
   <213> Vespula vulgaris
<400> 514
   Glu Tyr Pro Met Met Ala Gly Ile Ile His Leu Ala Thr Arg Phe
   1 5 10 15
<210> 515
   <211> 15
   <212> PRT
   <213> Vespula vulgaris
<400> 515
   Leu Lys Tyr Ser Met Arg Ile Gly Pro Ala Cys Leu Pro Phe Tyr
   1 5 10 15
<210> 516
   <211> 15
   <212> PRT
   <213> Vespula vulgaris
<400> 516
   Lys Tyr Ser Met Arg Ile Gly Pro Ala Cys Leu Pro Phe Tyr Tyr
   1 5 10 15
<210> 517
   <211> 15
   <212> PRT
   <213> Vespula vulgaris
<400> 517
   Arg Leu Lys Tyr Ser Met Arg Ile Gly Pro Ala Cys Leu Pro Phe
   1 5 10 15
<210> 518
   <211> 15
   <212> PRT
   <213> Vespula vulgaris
<400> 518
   Ala Arg Tyr Lys Arg Ile Leu Tyr Ile Leu Gly Val Val Val Gly
   1 5 10 15
<210> 519
   <211> 15
   <212> PRT
   <213> Vespula vulgaris
<400> 519
   Ala Val Glu Ser Ile Ala Ser Lys Met Thr Ile Lys Phe His Ser
   1 5 10 15
<210> 520
   <211> 15
   <212> PRT
   <213> Vespula vulgaris
<400> 520
   Tyr Ser Met Arg Ile Gly Pro Ala Cys Leu Pro Phe Tyr Tyr Met
   1 5 10 15
<210> 521
   <211> 15
   <212> PRT
   <213> Vespula vulgaris
<400> 521
   Val Glu Ser Ile Ala Ser Lys Met Thr Ile Lys Phe His Ser Arg
   1 5 10 15
<210> 522
   <211> 15
   <212> PRT
   <213> Vespula vulgaris
<400> 522
   Asp Thr Val Val Thr Ala Val Gly Trp Gly Leu Thr Asn Phe Tyr
   1 5 10 15
<210> 523
   <211> 15
   <212> PRT
   <213> Vespula vulgaris
<400> 523
   Leu Ala Thr Arg Phe Leu Tyr Cys Gly Ala Thr Ile Ile Thr Pro
   1 5 10 15
<210> 524
   <211> 15
   <212> PRT
   <213> Vespula vulgaris
<400> 524
   Asn Phe Tyr Gly Val Lys Ser Glu Val Leu Arg Lys Val Asp Leu
   1 5 10 15
<210> 525
   <211> 15
   <212> PRT
   <213> Vespula vulgaris
<400> 525
   Gly Ile Ile His Leu Ala Thr Arg Phe Leu Tyr Cys Gly Ala Thr
   1 5 10 15
<210> 526
   <211> 15
   <212> PRT
   <213> Vespula vulgaris
<400> 526
   Trp Gly Leu Thr Asn Phe Tyr Gly Val Lys Ser Glu Val Leu Arg
   1 5 10 15
<210> 527
   <211> 15
   <212> PRT
   <213> Vespula vulgaris
<400> 527
   Phe Tyr Gly Val Lys Ser Glu Val Leu Arg Lys Val Asp Leu His
   1 5 10 15
<210> 528
   <211> 15
   <212> PRT
   <213> Vespula vulgaris
<400> 528
   Ile Ile His Leu Ala Thr Arg Phe Leu Tyr Cys Gly Ala Thr Ile
   1 5 10 15
<210> 529
   <211> 15
   <212> PRT
   <213> Vespula vulgaris
<400> 529
   Gly Trp Gly Leu Thr Asn Phe Tyr Gly Val Lys Ser Glu Val Leu
   1 5 10 15
<210> 530
   <211> 15
   <212> PRT
   <213> Vespula vulgaris
<400> 530
   Arg Tyr Lys Arg Ile Leu Tyr Ile Leu Gly Val Val Val Gly Glu
   1 5 10 15
<210> 531
   <211> 15
   <212> PRT
   <213> Vespula vulgaris
<400> 531
   Tyr Lys Arg Ile Leu Tyr Ile Leu Gly Val Val Val Gly Glu His
   1 5 10 15
<210> 532
   <211> 15
   <212> PRT
   <213> Vespula vulgaris
<400> 532
   Arg Phe Leu Tyr Cys Gly Ala Thr Ile Ile Thr Pro Gln His Val
   1 5 10 15
<210> 533
   <211> 15
   <212> PRT
   <213> Vespula vulgaris
<400> 533
   Lys Arg Ile Leu Tyr Ile Leu Gly Val Val Val Gly Glu His Asn
   1 5 10 15
<210> 534
   <211> 15
   <212> PRT
   <213> Vespula vulgaris
<400> 534
   Thr Arg Phe Leu Tyr Cys Gly Ala Thr Ile Ile Thr Pro Gln His
   1 5 10 15
<210> 535
   <211> 15
   <212> PRT
   <213> Vespula vulgaris
<400> 535
   Ala Thr Arg Phe Leu Tyr Cys Gly Ala Thr Ile Ile Thr Pro Gln
   1 5 10 15
<210> 536
   <211> 15
   <212> PRT
   <213> Vespula vulgaris
<400> 536
   Glu Thr Gly Val Asn Glu Tyr Pro Met Met Ala Gly Ile Ile His
   1 5 10 15
<210> 537
   <211> 15
   <212> PRT
   <213> Vespula vulgaris
<400> 537
   Thr Gly Val Asn Glu Tyr Pro Met Met Ala Gly Ile Ile His Leu
   1 5 10 15
<210> 538
   <211> 15
   <212> PRT
   <213> Vespula vulgaris
<400> 538
   Gly Leu Thr Asn Phe Tyr Gly Val Lys Ser Glu Val Leu Arg Lys
   1 5 10 15
<210> 539
   <211> 15
   <212> PRT
   <213> Vespula vulgaris
<400> 539
   Val Phe Cys Gly Leu Asn Ser Phe Ala Val Glu Ser Ile Ala Ser
   1 5 10 15
<210> 540
   <211> 15
   <212> PRT
   <213> Vespula vulgaris
<400> 540
   Phe Cys Gly Leu Asn Ser Phe Ala Val Glu Ser Ile Ala Ser Lys
   1 5 10 15
<210> 541
   <211> 15
   <212> PRT
   <213> Vespula vulgaris
<400> 541
   Thr Val Val Thr Ala Val Gly Trp Gly Leu Thr Asn Phe Tyr Gly
   1 5 10 15
<210> 542
   <211> 15
   <212> PRT
   <213> Vespula vulgaris
<400> 542
   Asn Phe Val Asp Thr Val Val Thr Ala Val Gly Trp Gly Leu Thr
   1 5 10 15
<210> 543
   <211> 15
   <212> PRT
   <213> Vespula vulgaris
<400> 543
   His Asn Thr Trp Ala Ile Asn Asp Thr Lys Ala Thr Gln Leu Tyr
   1 5 10 15
<210> 544
   <211> 15
   <212> PRT
   <213> Vespula vulgaris
<400> 544
   Arg Ile Leu Tyr Ile Leu Gly Val Val Val Gly Glu His Asn Thr
   1 5 10 15
<210> 545
   <211> 15
   <212> PRT
   <213> Vespula vulgaris
<400> 545
   Asn Thr Trp Ala Ile Asn Asp Thr Lys Ala Thr Gln Leu Tyr Leu
   1 5 10 15
<210> 546
   <211> 15
   <212> PRT
   <213> Vespula vulgaris
<400> 546
   Tyr Cys Gly Ala Thr Ile Ile Thr Pro Gln His Val Leu Thr Ala
   1 5 10 15
<210> 547
   <211> 15
   <212> PRT
   <213> Vespula vulgaris
<400> 547
   Glu His Asn Thr Trp Ala Ile Asn Asp Thr Lys Ala Thr Gln Leu
   1 5 10 15
<210> 548
   <211> 15
   <212> PRT
   <213> Vespula vulgaris
<400> 548
   Phe Val Asp Thr Val Val Thr Ala Val Gly Trp Gly Leu Thr Asn
   1 5 10 15
<210> 549
   <211> 15
   <212> PRT
   <213> Vespula vulgaris
<400> 549
   Val Val Thr Ala Val Gly Trp Gly Leu Thr Asn Phe Tyr Gly Val
   1 5 10 15
<210> 550
   <211> 15
   <212> PRT
   <213> Vespula vulgaris
<400> 550
   Cys Gly Ala Thr Ile Ile Thr Pro Gln His Val Leu Thr Ala Ala
   1 5 10 15
<210> 551
   <211> 15
   <212> PRT
   <213> Vespula vulgaris
<400> 551
   Phe Leu Tyr Cys Gly Ala Thr Ile Ile Thr Pro Gln His Val Leu
   1 5 10 15
<210> 552
   <211> 15
   <212> PRT
   <213> Vespula vulgaris
<400> 552
   Arg Asn Phe Val Asp Thr Val Val Thr Ala Val Gly Trp Gly Leu
   1 5 10 15
<210> 553
   <211> 15
   <212> PRT
   <213> Vespula vulgaris
<400> 553
   Thr Ala Val Gly Trp Gly Leu Thr Asn Phe Tyr Gly Val Lys Ser
   1 5 10 15
<210> 554
   <211> 15
   <212> PRT
   <213> Vespula vulgaris
<400> 554
   Cys Val Lys Tyr His Phe Leu Ala Thr Pro Lys Gln Leu Cys Thr
   1 5 10 15
<210> 555
   <211> 15
   <212> PRT
   <213> Vespula vulgaris
<400> 555
   Val Lys Tyr His Phe Leu Ala Thr Pro Lys Gln Leu Cys Thr Phe
   1 5 10 15
<210> 556
   <211> 15
   <212> PRT
   <213> Vespula vulgaris
<400> 556
   His Leu Ala Thr Arg Phe Leu Tyr Cys Gly Ala Thr Ile Ile Thr
   1 5 10 15
<210> 557
   <211> 15
   <212> PRT
   <213> Vespula vulgaris
<400> 557
   Pro Asn Tyr Arg Pro Lys Leu Asn Asp Leu Ala Val Ile Lys Leu
   1 5 10 15
<210> 558
   <211> 15
   <212> PRT
   <213> Vespula vulgaris
<400> 558
   Gly Ala Thr Ile Ile Thr Pro Gln His Val Leu Thr Ala Ala His
   1 5 10 15
<210> 559
   <211> 15
   <212> PRT
   <213> Vespula vulgaris
<400> 559
   Val Asp Thr Val Val Thr Ala Val Gly Trp Gly Leu Thr Asn Phe
   1 5 10 15
<210> 560
   <211> 15
   <212> PRT
   <213> Vespula vulgaris
<400> 560
   Ala Thr Ile Ile Thr Pro Gln His Val Leu Thr Ala Ala His Cys
   1 5 10 15
<210> 561
   <211> 15
   <212> PRT
   <213> Vespula vulgaris
<400> 561
   Tyr Gly Val Lys Ser Glu Val Leu Arg Lys Val Asp Leu His Val
   1 5 10 15
<210> 562
   <211> 15
   <212> PRT
   <213> Vespula vulgaris
<400> 562
   Thr Ile Ile Thr Pro Gln His Val Leu Thr Ala Ala His Cys Val
   1 5 10 15
<210> 563
   <211> 15
   <212> PRT
   <213> Vespula vulgaris
<400> 563
   Leu Tyr Cys Gly Ala Thr Ile Ile Thr Pro Gln His Val Leu Thr
   1 5 10 15
<210> 564
   <211> 15
   <212> PRT
   <213> Vespula vulgaris
<400> 564
   Arg Pro Lys Leu Asn Asp Leu Ala Val Ile Lys Leu Gln Lys Arg
   1 5 10 15
<210> 565
   <211> 15
   <212> PRT
   <213> Vespula vulgaris
<400> 565
   Pro Lys Leu Asn Asp Leu Ala Val Ile Lys Leu Gln Lys Arg Leu
   1 5 10 15
<210> 566
   <211> 15
   <212> PRT
   <213> Vespula vulgaris
<400> 566
   Lys Cys Val Lys Tyr His Phe Leu Ala Thr Pro Lys Gln Leu Cys
   1 5 10 15
<210> 567
   <211> 15
   <212> PRT
   <213> Vespula vulgaris
<400> 567
   Tyr Arg Pro Lys Leu Asn Asp Leu Ala Val Ile Lys Leu Gln Lys
   1 5 10 15
<210> 568
   <211> 15
   <212> PRT
   <213> Vespula vulgaris
<400> 568
   Ser Met Arg Ile Gly Pro Ala Cys Leu Pro Phe Tyr Tyr Met Gln
   1 5 10 15
<210> 569
   <211> 15
   <212> PRT
   <213> Vespula vulgaris
<400> 569
   Asn Tyr Arg Pro Lys Leu Asn Asp Leu Ala Val Ile Lys Leu Gln
   1 5 10 15
<210> 570
   <211> 15
   <212> PRT
   <213> Vespula vulgaris
<400> 570
   Val Thr Ala Val Gly Trp Gly Leu Thr Asn Phe Tyr Gly Val Lys
   1 5 10 15
<210> 571
   <211> 15
   <212> PRT
   <213> Vespula vulgaris
<400> 571
   Met Arg Ile Gly Pro Ala Cys Leu Pro Phe Tyr Tyr Met Gln Arg
   1 5 10 15
<210> 572
   <211> 15
   <212> PRT
   <213> Vespula vulgaris
<400> 572
   Lys Lys Cys Val Lys Tyr His Phe Leu Ala Thr Pro Lys Gln Leu
   1 5 10 15
<210> 573
   <211> 15
   <212> PRT
   <213> Vespula vulgaris
<400> 573
   Val Gly Glu His Asn Thr Trp Ala Ile Asn Asp Thr Lys Ala Thr
   1 5 10 15
<210> 574
   <211> 15
   <212> PRT
   <213> Vespula vulgaris
<400> 574
   Val Ala Arg Tyr Lys Arg Ile Leu Tyr Ile Leu Gly Val Val Val
   1 5 10 15
<210> 575
   <211> 15
   <212> PRT
   <213> Vespula vulgaris
<400> 575
   Gly Glu His Asn Thr Trp Ala Ile Asn Asp Thr Lys Ala Thr Gln
   1 5 10 15
<210> 576
   <211> 15
   <212> PRT
   <213> Vespula vulgaris
<400> 576
   Tyr Met Gln Arg Asn Phe Val Asp Thr Val Val Thr Ala Val Gly
   1 5 10 15
<210> 577
   <211> 15
   <212> PRT
   <213> Vespula vulgaris
<400> 577
   Thr Trp Ala Ile Asn Asp Thr Lys Ala Thr Gln Leu Tyr Leu Ile
   1 5 10 15
<210> 578
   <211> 15
   <212> PRT
   <213> Vespula vulgaris
<400> 578
   Trp Ala Ile Asn Asp Thr Lys Ala Thr Gln Leu Tyr Leu Ile Asp
   1 5 10 15
<210> 579
   <211> 15
   <212> PRT
   <213> Vespula vulgaris
<400> 579
   Met Lys Lys Cys Val Lys Tyr His Phe Leu Ala Thr Pro Lys Gln
   1 5 10 15
<210> 580
   <211> 15
   <212> PRT
   <213> Vespula vulgaris
<400> 580
   Val Lys Ser Glu Val Leu Arg Lys Val Asp Leu His Val Val Ser
   1 5 10 15
<210> 581
   <211> 15
   <212> PRT
   <213> Vespula vulgaris
<400> 581
   Gly Val Lys Ser Glu Val Leu Arg Lys Val Asp Leu His Val Val
   1 5 10 15
<210> 582
   <211> 15
   <212> PRT
   <213> Vespula vulgaris
<400> 582
   Gln Arg Asn Phe Val Asp Thr Val Val Thr Ala Val Gly Trp Gly
   1 5 10 15
<210> 583
   <211> 15
   <212> PRT
   <213> Vespula vulgaris
<400> 583
   Met Gln Arg Asn Phe Val Asp Thr Val Val Thr Ala Val Gly Trp
   1 5 10 15
<210> 584
   <211> 15
   <212> PRT
   <213> Vespula vulgaris
<400> 584
   Ile Thr Pro Gln His Val Leu Thr Ala Ala His Cys Val Ala Arg
   1 5 10 15
<210> 585
   <211> 15
   <212> PRT
   <213> Vespula vulgaris
<400> 585
   Ala Val Gly Trp Gly Leu Thr Asn Phe Tyr Gly Val Lys Ser Glu
   1 5 10 15
<210> 586
   <211> 15
   <212> PRT
   <213> Vespula vulgaris
<400> 586
   Tyr Tyr Met Gln Arg Asn Phe Val Asp Thr Val Val Thr Ala Val
   1 5 10 15
<210> 587
   <211> 15
   <212> PRT
   <213> Vespula vulgaris
<400> 587
   Tyr His Phe Leu Ala Thr Pro Lys Gln Leu Cys Thr Phe Asp Met
   1 5 10 15
<210> 588
   <211> 15
   <212> PRT
   <213> Vespula vulgaris
<400> 588
   Ile His Leu Ala Thr Arg Phe Leu Tyr Cys Gly Ala Thr Ile Ile
   1 5 10 15
<210> 589
   <211> 15
   <212> PRT
   <213> Vespula vulgaris
<400> 589
   Val Gly Trp Gly Leu Thr Asn Phe Tyr Gly Val Lys Ser Glu Val
   1               5                   10                  15
<210> 590
   <211> 15
   <212> PRT
   <213> Vespula vulgaris
<400> 590
   Thr Ser Tyr Leu Asp Phe Ile Thr Arg Ser Thr Pro Gly Glu Ile
   1               5                   10                  15
<210> 591
   <211> 15
   <212> PRT
   <213> Vespula vulgaris
<400> 591
   Ile Leu Tyr Ile Leu Gly Val Val Val Gly Glu His Asn Thr Trp
   1               5                   10                  15
<210> 592
   <211> 15
   <212> PRT
   <213> Vespula vulgaris
<400> 592
   Lys Tyr His Phe Leu Ala Thr Pro Lys Gln Leu Cys Thr Phe Asp
   1               5                   10                  15
<210> 593
   <211> 15
   <212> PRT
   <213> Vespula vulgaris
<400> 593
   Val Thr Ser Tyr Leu Asp Phe Ile Thr Arg Ser Thr Pro Gly Glu
   1               5                   10                  15
<210> 594
   <211> 15
   <212> PRT
   <213> Vespula vulgaris
<400> 594
   Ile Ile Thr Pro Gln His Val Leu Thr Ala Ala His Cys Val Ala
   1               5                   10                  15
<210> 595
   <211> 15
   <212> PRT
   <213> Vespula vulgaris
<400> 595
   Leu Pro Phe Tyr Tyr Met Gln Arg Asn Phe Val Asp Thr Val Val
   1 5 10 15
<210> 596
   <211> 15
   <212> PRT
   <213> Vespula vulgaris
<400> 596
   Cys Leu Pro Phe Tyr Tyr Met Gln Arg Asn Phe Val Asp Thr Val
   1 5 10 15
<210> 597
   <211> 15
   <212> PRT
   <213> Vespula vulgaris
<400> 597
   Val Glu Thr Gly Val Asn Glu Tyr Pro Met Met Ala Gly Ile Ile
   1 5 10 15
<210> 598
   <211> 15
   <212> PRT
   <213> Vespula vulgaris
<400> 598
   Ile Asp Leu Asn Cys Gly Tyr Thr Gln Lys Leu Lys Ser Asp Val
   1 5 10 15
<210> 599
   <211> 15
   <212> PRT
   <213> Vespula vulgaris
<400> 599
   His Cys Val Ala Arg Tyr Lys Arg Ile Leu Tyr Ile Leu Gly Val
   1 5 10 15
<210> 600
   <211> 15
   <212> PRT
   <213> Vespula vulgaris
<400> 600
   Thr Pro Gln His Val Leu Thr Ala Ala His Cys Val Ala Arg Tyr
   1 5 10 15
<210> 601
   <211> 15
   <212> PRT
   <213> Vespula vulgaris
<400> 601
   Cys Val Ala Arg Tyr Lys Arg Ile Leu Tyr Ile Leu Gly Val Val
   1 5 10 15
<210> 602
   <211> 15
   <212> PRT
   <213> Vespula vulgaris
<400> 602
   Pro Gln His Val Leu Thr Ala Ala His Cys Val Ala Arg Tyr Lys
   1 5 10 15
<210> 603
   <211> 15
   <212> PRT
   <213> Vespula vulgaris
<400> 603
   Leu Tyr Ile Leu Gly Val Val Val Gly Glu His Asn Thr Trp Ala
   1 5 10 15
<210> 604
   <211> 15
   <212> PRT
   <213> Vespula vulgaris
<400> 604
   Ser Glu Val Leu Arg Lys Val Asp Leu His Val Val Ser Met Lys
   1 5 10 15
<210> 605
   <211> 15
   <212> PRT
   <213> Vespula vulgaris
<400> 605
   Pro Phe Tyr Tyr Met Gln Arg Asn Phe Val Asp Thr Val Val Thr
   1 5 10 15
<210> 606
   <211> 15
   <212> PRT
   <213> Vespula vulgaris
<400> 606
   Phe Tyr Tyr Met Gln Arg Asn Phe Val Asp Thr Val Val Thr Ala
   1 5 10 15
<210> 607
   <211> 15
   <212> PRT
   <213> Vespula vulgaris
<400> 607
   Cys Thr Val Phe Asp Val Pro Pro Ser Ser Asn Cys Ser Leu Asp
   1               5                   10                  15
<210> 608
   <211> 15
   <212> PRT
   <213> Vespula vulgaris
<400> 608
   Asn Cys Thr Val Phe Asp Val Pro Pro Ser Ser Asn Cys Ser Leu
   1               5                   10                  15
<210> 609
   <211> 15
   <212> PRT
   <213> Vespula vulgaris
<400> 609
   Lys Ser Glu Val Leu Arg Lys Val Asp Leu His Val Val Ser Met
   1               5                   10                  15
<210> 610
   <211> 15
   <212> PRT
   <213> Vespula vulgaris
<400> 610
   Asn Leu Arg Val Thr Ser Tyr Leu Asp Phe Ile Thr Arg Ser Thr
   1               5                   10                  15
<210> 611
   <211> 15
   <212> PRT
   <213> Vespula vulgaris
<400> 611
   Leu Arg Val Thr Ser Tyr Leu Asp Phe Ile Thr Arg Ser Thr Pro
   1               5                   10                  15
<210> 612
   <211> 15
   <212> PRT
   <213> Vespula vulgaris
<400> 612
   Glu Val Leu Arg Lys Val Asp Leu His Val Val Ser Met Lys Lys
   1 5 10 15
<210> 613
   <211> 15
   <212> PRT
   <213> Vespula vulgaris
<400> 613
   Thr Ile Asp Leu Asn Cys Gly Tyr Thr Gln Lys Leu Lys Ser Asp
   1 5 10 15
<210> 614
   <211> 15
   <212> PRT
   <213> Vespula vulgaris
<400> 614
   Arg Val Thr Ser Tyr Leu Asp Phe Ile Thr Arg Ser Thr Pro Gly
   1 5 10 15
<210> 615
   <211> 15
   <212> PRT
   <213> Vespula vulgaris
<400> 615
   Ala His Cys Val Ala Arg Tyr Lys Arg Ile Leu Tyr Ile Leu Gly
   1 5 10 15
<210> 616
   <211> 15
   <212> PRT
   <213> Vespula vulgaris
<400> 616
   Cys Arg Cys Gly Trp Lys Asn Pro Ser Arg Ile Val Gly Gly Val
   1 5 10 15
<210> 617
   <211> 15
   <212> PRT
   <213> Vespula vulgaris
<400> 617
   Lys Cys Arg Cys Gly Trp Lys Asn Pro Ser Arg Ile Val Gly Gly
   1 5 10 15
<210> 618
   <211> 15
   <212> PRT
   <213> Vespula vulgaris
<400> 618
   Leu Asn Asp Leu Ala Val Ile Lys Leu Gln Lys Arg Leu Lys Tyr
   1 5 10 15
<210> 619
   <211> 15
   <212> PRT
   <213> Vespula vulgaris
<400> 619
   Gly Gly Val Glu Thr Gly Val Asn Glu Tyr Pro Met Met Ala Gly
   1 5 10 15
<210> 620
   <211> 15
   <212> PRT
   <213> Vespula vulgaris
<400> 620
   Lys Leu Asn Asp Leu Ala Val Ile Lys Leu Gln Lys Arg Leu Lys
   1 5 10 15
<210> 621
   <211> 15
   <212> PRT
   <213> Vespula vulgaris
<400> 621
   Ala Cys Leu Pro Phe Tyr Tyr Met Gln Arg Asn Phe Val Asp Thr
   1 5 10 15
<210> 622
   <211> 15
   <212> PRT
   <213> Vespula vulgaris
<400> 622
   Glu Lys Cys Arg Cys Gly Trp Lys Asn Pro Ser Arg Ile Val Gly
   1 5 10 15
<210> 623
   <211> 15
   <212> PRT
   <213> Vespula vulgaris
<400> 623
   Gly Val Glu Thr Gly Val Asn Glu Tyr Pro Met Met Ala Gly Ile
   1 5 10 15
<210> 624
   <211> 15
   <212> PRT
   <213> Vespula vulgaris
<400> 624
   Cys Gly Tyr Thr Gln Lys Leu Lys Ser Asp Val Asn Tyr Cys Val
   1 5 10 15
<210> 625
   <211> 15
   <212> PRT
   <213> Vespula vulgaris
<400> 625
   Arg Cys Gly Trp Lys Asn Pro Ser Arg Ile Val Gly Gly Val Glu
   1 5 10 15
<210> 626
   <211> 15
   <212> PRT
   <213> Vespula vulgaris
<400> 626
   Ala Cys Gln Phe Asp Ser Gly Gly Pro Ile Leu Trp Gln Asn Pro
   1 5 10 15
<210> 627
   <211> 15
   <212> PRT
   <213> Vespula vulgaris
<400> 627
   Leu Asn Cys Thr Val Phe Asp Val Pro Pro Ser Ser Asn Cys Ser
   1 5 10 15
<210> 628
   <211> 15
   <212> PRT
   <213> Vespula vulgaris
<400> 628
   Glu His Asn Cys Arg Trp Ser Ala Arg Ser Asn Thr Arg Ile Lys
   1 5 10 15
<210> 629
   <211> 15
   <212> PRT
   <213> Vespula vulgaris
<400> 629
   Cys Gln Phe Asp Ser Gly Gly Pro Ile Leu Trp Gln Asn Pro Lys
   1 5 10 15
<210> 630
   <211> 15
   <212> PRT
   <213> Vespula vulgaris
<400> 630
   Gln His Val Leu Thr Ala Ala His Cys Val Ala Arg Tyr Lys Arg
   1 5 10 15
<210> 631
   <211> 15
   <212> PRT
   <213> Vespula vulgaris
<400> 631
   His Asn Cys Arg Trp Ser Ala Arg Ser Asn Thr Arg Ile Lys Leu
   1 5 10 15
<210> 632
   <211> 15
   <212> PRT
   <213> Vespula vulgaris
<400> 632
   Gly Glu His Asn Cys Arg Trp Ser Ala Arg Ser Asn Thr Arg Ile
   1 5 10 15
<210> 633
   <211> 15
   <212> PRT
   <213> Vespula vulgaris
<400> 633
   Asp Ala Cys Gln Phe Asp Ser Gly Gly Pro Ile Leu Trp Gln Asn
   1 5 10 15
<210> 634
   <211> 15
   <212> PRT
   <213> Vespula vulgaris
<400> 634
   Asn Cys Arg Trp Ser Ala Arg Ser Asn Thr Arg Ile Lys Leu Asn
   1 5 10 15
<210> 635
   <211> 15
   <212> PRT
   <213> Vespula vulgaris
<400> 635
   Ala Asp Glu Ala Pro Gly Val Asn Leu Arg Val Thr Ser Tyr Leu
   1 5 10 15
<210> 636
   <211> 15
   <212> PRT
   <213> Vespula vulgaris
<400> 636
   Thr Val Phe Asp Val Pro Pro Ser Ser Asn Cys Ser Leu Asp Phe
   1 5 10 15
<210> 637
   <211> 15
   <212> PRT
   <213> Vespula vulgaris
<400> 637
   Asn Pro Ser Arg Ile Val Gly Gly Val Glu Thr Gly Val Asn Glu
   1 5 10 15
<210> 638
   <211> 15
   <212> PRT
   <213> Vespula vulgaris
<400> 638
   Lys Asn Pro Ser Arg Ile Val Gly Gly Val Glu Thr Gly Val Asn
   1 5 10 15
<210> 639
   <211> 15
   <212> PRT
   <213> Vespula vulgaris
<400> 639
   Cys Gly Leu Asn Ser Phe Ala Val Glu Ser Ile Ala Ser Lys Met
   1 5 10 15
<210> 640
   <211> 15
   <212> PRT
   <213> Vespula vulgaris
<400> 640
   Ile Lys Leu Gln Lys Arg Leu Lys Tyr Ser Met Arg Ile Gly Pro
   1 5 10 15
<210> 641
   <211> 15
   <212> PRT
   <213> Vespula vulgaris
<400> 641
   Lys Asp Ala Cys Gln Phe Asp Ser Gly Gly Pro Ile Leu Trp Gln
   1 5 10 15
<210> 642
   <211> 15
   <212> PRT
   <213> Vespula vulgaris
<400> 642
   Ala Val Ile Lys Leu Gln Lys Arg Leu Lys Tyr Ser Met Arg Ile
   1 5 10 15
<210> 643
   <211> 15
   <212> PRT
   <213> Vespula vulgaris
<400> 643
   Ser Tyr Leu Asp Phe Ile Thr Arg Ser Thr Pro Gly Glu Ile Tyr
   1 5 10 15
<210> 644
   <211> 15
   <212> PRT
   <213> Vespula vulgaris
<400> 644
   Val Ile Lys Leu Gln Lys Arg Leu Lys Tyr Ser Met Arg Ile Gly
   1 5 10 15
<210> 645
   <211> 15
   <212> PRT
   <213> Vespula vulgaris
<400> 645
   Gly Tyr Thr Gln Lys Leu Lys Ser Asp Val Asn Tyr Cys Val Tyr
   1 5 10 15
<210> 646
   <211> 15
   <212> PRT
   <213> Vespula vulgaris
<400> 646
   Phe Asp Ser Gly Gly Pro Ile Leu Trp Gln Asn Pro Lys Ser Lys
   1 5 10 15
<210> 647
   <211> 15
   <212> PRT
   <213> Vespula vulgaris
<400> 647
   Ile Ile Val His Pro Asn Tyr Arg Pro Lys Leu Asn Asp Leu Ala
   1 5 10 15
<210> 648
   <211> 15
   <212> PRT
   <213> Vespula vulgaris
<400> 648
   Asp Glu Ala Pro Gly Val Asn Leu Arg Val Thr Ser Tyr Leu Asp
   1 5 10 15
<210> 649
   <211> 15
   <212> PRT
   <213> Vespula vulgaris
<400> 649
   His Val Leu Thr Ala Ala His Cys Val Ala Arg Tyr Lys Arg Ile
   1 5 10 15
<210> 650
   <211> 15
   <212> PRT
   <213> Vespula vulgaris
<400> 650
   Gly Lys Asp Ala Cys Gln Phe Asp Ser Gly Gly Pro Ile Leu Trp
   1 5 10 15
<210> 651
   <211> 15
   <212> PRT
   <213> Vespula vulgaris
<400> 651
   Tyr Leu Asp Phe Ile Thr Arg Ser Thr Pro Gly Glu Ile Tyr Cys
   1 5 10 15
<210> 652
   <211> 15
   <212> PRT
   <213> Vespula vulgaris
<400> 652
   Leu Gln Lys Arg Leu Lys Tyr Ser Met Arg Ile Gly Pro Ala Cys
   1 5 10 15
<210> 653
   <211> 15
   <212> PRT
   <213> Vespula vulgaris
<400> 653
   Phe Met Lys Val Lys Val Asp Asp Asp Ile Glu Tyr Val Phe Cys
   1 5 10 15
<210> 654
   <211> 15
   <212> PRT
   <213> Vespula vulgaris
<400> 654
   Asp Phe Met Lys Val Lys Val Asp Asp Asp Ile Glu Tyr Val Phe
   1 5 10 15
<210> 655
   <211> 15
   <212> PRT
   <213> Vespula vulgaris
<400> 655
   Met Lys Val Lys Val Asp Asp Asp Ile Glu Tyr Val Phe Cys Gly
   1 5 10 15
<210> 656
   <211> 15
   <212> PRT
   <213> Vespula vulgaris
<400> 656
   Met Lys Lys Cys Val Lys Tyr His Phe Leu Ala Thr Pro Lys Gln
   1 5 10 15
<210> 657
   <211> 15
   <212> PRT
   <213> Vespula vulgaris
<400> 657
   Val Lys Tyr His Phe Leu Ala Thr Pro Lys Gln Leu Cys Thr Phe
   1 5 10 15
<210> 658
   <211> 15
   <212> PRT
   <213> Vespula vulgaris
<400> 658
   Lys Cys Val Lys Tyr His Phe Leu Ala Thr Pro Lys Gln Leu Cys
   1 5 10 15
<210> 659
   <211> 15
   <212> PRT
   <213> Vespula vulgaris
<400> 659
   Cys Val Lys Tyr His Phe Leu Ala Thr Pro Lys Gln Leu Cys Thr
   1 5 10 15
<210> 660
   <211> 15
   <212> PRT
   <213> Vespula vulgaris
<400> 660
   Lys Lys Cys Val Lys Tyr His Phe Leu Ala Thr Pro Lys Gln Leu
   1 5 10 15
<210> 661
   <211> 15
   <212> PRT
   <213> Vespula vulgaris
<400> 661
   Asp Val Asn Tyr Cys Val Tyr Asn Pro Asp Phe Pro Asn Tyr Tyr
   1 5 10 15
<210> 662
   <211> 15
   <212> PRT
   <213> Vespula vulgaris
<400> 662
   Val Asn Tyr Cys Val Tyr Asn Pro Asp Phe Pro Asn Tyr Tyr Met
   1 5 10 15
<210> 663
   <211> 15
   <212> PRT
   <213> Vespula vulgaris
<400> 663
   Asn Tyr Cys Val Tyr Asn Pro Asp Phe Pro Asn Tyr Tyr Met Gly
   1 5 10 15
<210> 664
   <211> 15
   <212> PRT
   <213> Vespula vulgaris
<400> 664
   Tyr Cys Val Tyr Asn Pro Asp Phe Pro Asn Tyr Tyr Met Gly Glu
   1 5 10 15
<210> 665
   <211> 15
   <212> PRT
   <213> Vespula vulgaris
<400> 665
   Cys Val Tyr Asn Pro Asp Phe Pro Asn Tyr Tyr Met Gly Glu His
   1 5 10 15
<210> 666
   <211> 15
   <212> PRT
   <213> Vespula vulgaris
<400> 666
   Lys Tyr His Phe Leu Ala Thr Pro Lys Gln Leu Cys Thr Phe Asp
   1 5 10 15
<210> 667
   <211> 15
   <212> PRT
   <213> Vespula vulgaris
<400> 667
   Tyr His Phe Leu Ala Thr Pro Lys Gln Leu Cys Thr Phe Asp Met
   1 5 10 15
<210> 668
   <211> 15
   <212> PRT
   <213> Vespula vulgaris
<400> 668
   Leu Asn Cys Thr Val Phe Asp Val Pro Pro Ser Ser Asn Cys Ser
   1 5 10 15
<210> 669
   <211> 15
   <212> PRT
   <213> Vespula vulgaris
<400> 669
   Val Thr Ser Tyr Leu Asp Phe Ile Thr Arg Ser Thr Pro Gly Glu
   1 5 10 15
<210> 670
   <211> 15
   <212> PRT
   <213> Vespula vulgaris
<400> 670
   Thr Ser Tyr Leu Asp Phe Ile Thr Arg Ser Thr Pro Gly Glu Ile
   1 5 10 15
<210> 671
   <211> 15
   <212> PRT
   <213> Vespula vulgaris
<400> 671
   Arg Val Thr Ser Tyr Leu Asp Phe Ile Thr Arg Ser Thr Pro Gly
   1 5 10 15
<210> 672
   <211> 15
   <212> PRT
   <213> Vespula vulgaris
<400> 672
   Lys Cys Val Lys Tyr His Phe Leu Ala Thr Pro Lys Gln Leu Cys
   1 5 10 15
<210> 673
   <211> 15
   <212> PRT
   <213> Vespula vulgaris
<400> 673
   Leu Arg Val Thr Ser Tyr Leu Asp Phe Ile Thr Arg Ser Thr Pro
   1 5 10 15
<210> 674
   <211> 15
   <212> PRT
   <213> Vespula vulgaris
<400> 674
   Ser Lys Arg Ile Phe Leu Leu Gly Val Ile Asn Tyr Gly Arg Thr
   1 5 10 15
<210> 675
   <211> 15
   <212> PRT
   <213> Vespula vulgaris
<400> 675
   Lys Arg Ile Phe Leu Leu Gly Val Ile Asn Tyr Gly Arg Thr Cys
   1 5 10 15
<210> 676
   <211> 15
   <212> PRT
   <213> Vespula vulgaris
<400> 676
   Asn Leu Arg Val Thr Ser Tyr Leu Asp Phe Ile Thr Arg Ser Thr
   1 5 10 15
<210> 677
   <211> 15
   <212> PRT
   <213> Vespula vulgaris
<400> 677
   Arg Ile Phe Leu Leu Gly Val Ile Asn Tyr Gly Arg Thr Cys Ala
   1 5 10 15
<210> 678
   <211> 15
   <212> PRT
   <213> Vespula vulgaris
<400> 678
   Pro Asp Phe Pro Asn Tyr Tyr Met Gly Glu His Asn Cys Arg Trp
   1 5 10 15
<210> 679
   <211> 15
   <212> PRT
   <213> Vespula vulgaris
<400> 679
   Phe Pro Asn Tyr Tyr Met Gly Glu His Asn Cys Arg Trp Ser Ala
   1 5 10 15
<210> 680
   <211> 15
   <212> PRT
   <213> Vespula vulgaris
<400> 680
   Asp Phe Pro Asn Tyr Tyr Met Gly Glu His Asn Cys Arg Trp Ser
   1 5 10 15
<210> 681
   <211> 15
   <212> PRT
   <213> Vespula vulgaris
<400> 681
   Ser Gly Gly Pro Ile Leu Trp Gln Asn Pro Lys Ser Lys Arg Ile
   1 5 10 15
<210> 682
   <211> 15
   <212> PRT
   <213> Vespula vulgaris
<400> 682
   Leu Tyr Ile Leu Gly Val Val Val Gly Glu His Asn Thr Trp Ala
   1 5 10 15
<210> 683
   <211> 15
   <212> PRT
   <213> Vespula vulgaris
<400> 683
   Pro Ala Cys Leu Pro Phe Tyr Tyr Met Gln Arg Asn Phe Val Asp
   1 5 10 15
<210> 684
   <211> 15
   <212> PRT
   <213> Vespula vulgaris
<400> 684
   Ala Cys Leu Pro Phe Tyr Tyr Met Gln Arg Asn Phe Val Asp Thr
   1 5 10 15
<210> 685
   <211> 15
   <212> PRT
   <213> Vespula vulgaris
<400> 685
   Pro Phe Tyr Tyr Met Gln Arg Asn Phe Val Asp Thr Val Val Thr
   1 5 10 15
<210> 686
   <211> 15
   <212> PRT
   <213> Vespula vulgaris
<400> 686
   Cys Leu Pro Phe Tyr Tyr Met Gln Arg Asn Phe Val Asp Thr Val
   1 5 10 15
<210> 687
   <211> 15
   <212> PRT
   <213> Vespula vulgaris
<400> 687
   Leu Pro Phe Tyr Tyr Met Gln Arg Asn Phe Val Asp Thr Val Val
   1 5 10 15
<210> 688
   <211> 15
   <212> PRT
   <213> Vespula vulgaris
<400> 688
   Tyr Tyr Met Gln Arg Asn Phe Val Asp Thr Val Val Thr Ala Val
   1 5 10 15
<210> 689
   <211> 15
   <212> PRT
   <213> Vespula vulgaris
<400> 689
   Phe Tyr Tyr Met Gln Arg Asn Phe Val Asp Thr Val Val Thr Ala
   1 5 10 15
<210> 690
   <211> 15
   <212> PRT
   <213> Vespula vulgaris
<400> 690
   Glu Tyr Val Phe Cys Gly Leu Asn Ser Phe Ala Val Glu Ser Ile
   1 5 10 15
<210> 691
   <211> 15
   <212> PRT
   <213> Vespula vulgaris
<400> 691
   Asp Asp Ile Glu Tyr Val Phe Cys Gly Leu Asn Ser Phe Ala Val
   1 5 10 15
<210> 692
   <211> 15
   <212> PRT
   <213> Vespula vulgaris
<400> 692
   Asp Ile Glu Tyr Val Phe Cys Gly Leu Asn Ser Phe Ala Val Glu
   1 5 10 15
<210> 693
   <211> 15
   <212> PRT
   <213> Vespula vulgaris
<400> 693
   Ile Glu Tyr Val Phe Cys Gly Leu Asn Ser Phe Ala Val Glu Ser
   1 5 10 15
<210> 694
   <211> 15
   <212> PRT
   <213> Vespula vulgaris
<400> 694
   Tyr Val Phe Cys Gly Leu Asn Ser Phe Ala Val Glu Ser Ile Ala
   1 5 10 15
<210> 695
   <211> 15
   <212> PRT
   <213> Vespula vulgaris
<400> 695
   Tyr Tyr Met Gln Arg Asn Phe Val Asp Thr Val Val Thr Ala Val
   1 5 10 15
<210> 696
   <211> 15
   <212> PRT
   <213> Vespula vulgaris
<400> 696
   Ala Gly Ile Ile His Leu Ala Thr Arg Phe Leu Tyr Cys Gly Ala
   1 5 10 15
<210> 697
   <211> 15
   <212> PRT
   <213> Vespula vulgaris
<400> 697
   Met Met Ala Gly Ile Ile His Leu Ala Thr Arg Phe Leu Tyr Cys
   1 5 10 15
<210> 698
   <211> 15
   <212> PRT
   <213> Vespula vulgaris
<400> 698
   Met Gln Arg Asn Phe Val Asp Thr Val Val Thr Ala Val Gly Trp
   1 5 10 15
<210> 699
   <211> 15
   <212> PRT
   <213> Vespula vulgaris
<400> 699
   Gln Arg Asn Phe Val Asp Thr Val Val Thr Ala Val Gly Trp Gly
   1 5 10 15
<210> 700
   <211> 15
   <212> PRT
   <213> Vespula vulgaris
<400> 700
   Pro Met Met Ala Gly Ile Ile His Leu Ala Thr Arg Phe Leu Tyr
   1 5 10 15
<210> 701
   <211> 15
   <212> PRT
   <213> Vespula vulgaris
<400> 701
   Tyr Met Gln Arg Asn Phe Val Asp Thr Val Val Thr Ala Val Gly
   1 5 10 15
<210> 702
   <211> 15
   <212> PRT
   <213> Vespula vulgaris
<400> 702
   Trp Gly Leu Thr Asn Phe Tyr Gly Val Lys Ser Glu Val Leu Arg
   1 5 10 15
<210> 703
   <211> 15
   <212> PRT
   <213> Vespula vulgaris
<400> 703
   Leu Thr Asn Phe Tyr Gly Val Lys Ser Glu Val Leu Arg Lys Val
   1 5 10 15
<210> 704
   <211> 15
   <212> PRT
   <213> Vespula vulgaris
<400> 704
   Gly Trp Gly Leu Thr Asn Phe Tyr Gly Val Lys Ser Glu Val Leu
   1 5 10 15
<210> 705
   <211> 15
   <212> PRT
   <213> Vespula vulgaris
<400> 705
   Thr Asn Phe Tyr Gly Val Lys Ser Glu Val Leu Arg Lys Val Asp
   1 5 10 15
<210> 706
   <211> 15
   <212> PRT
   <213> Vespula vulgaris
<400> 706
   Val Phe Cys Gly Leu Asn Ser Phe Ala Val Glu Ser Ile Ala Ser
   1 5 10 15
<210> 707
   <211> 15
   <212> PRT
   <213> Vespula vulgaris
<400> 707
   Phe Cys Gly Leu Asn Ser Phe Ala Val Glu Ser Ile Ala Ser Lys
   1 5 10 15
<210> 708
   <211> 9
   <212> PRT
   <213> Vespula vulgaris
<400> 708
   Pro Gly Glu Ile Tyr Cys Gln Ala Tyr
   1 5
<210> 709
   <211> 15
   <212> PRT
   <213> Vespula vulgaris
<400> 709
   Asn Cys Arg Trp Ser Ala Arg Ser Asn Thr Arg Ile Lys Leu Asn
   1 5 10 15
<210> 710
   <211> 15
   <212> PRT
   <213> Vespula vulgaris
<400> 710
   Ala Pro Gly Val Asn Leu Arg Val Thr Ser Tyr Leu Asp Phe Ile
   1 5 10 15
<210> 711
   <211> 15
   <212> PRT
   <213> Vespula vulgaris
<400> 711
   Gly Glu His Asn Cys Arg Trp Ser Ala Arg Ser Asn Thr Arg Ile
   1 5 10 15
<210> 712
   <211> 15
   <212> PRT
   <213> Vespula vulgaris
<400> 712
   Glu Ala Pro Gly Val Asn Leu Arg Val Thr Ser Tyr Leu Asp Phe
   1 5 10 15
<210> 713
   <211> 15
   <212> PRT
   <213> Vespula vulgaris
<400> 713
   His Asn Cys Arg Trp Ser Ala Arg Ser Asn Thr Arg Ile Lys Leu
   1 5 10 15
<210> 714
   <211> 15
   <212> PRT
   <213> Vespula vulgaris
<400> 714
   Glu His Asn Cys Arg Trp Ser Ala Arg Ser Asn Thr Arg Ile Lys
   1 5 10 15
<210> 715
   <211> 15
   <212> PRT
   <213> Vespula vulgaris
<400> 715
   Cys Arg Trp Ser Ala Arg Ser Asn Thr Arg Ile Lys Leu Asn Cys
   1 5 10 15
<210> 716
   <211> 15
   <212> PRT
   <213> Vespula vulgaris
<400> 716
   His Val Leu Thr Ala Ala His Cys Val Ala Arg Tyr Lys Arg Ile
   1 5 10 15
<210> 717
   <211> 15
   <212> PRT
   <213> Vespula vulgaris
<400> 717
   Thr Ala Ala His Cys Val Ala Arg Tyr Lys Arg Ile Leu Tyr Ile
   1 5 10 15
<210> 718
   <211> 15
   <212> PRT
   <213> Vespula vulgaris
<400> 718
   Pro Ile Leu Trp Gln Asn Pro Lys Ser Lys Arg Ile Phe Leu Leu
   1 5 10 15
<210> 719
   <211> 15
   <212> PRT
   <213> Vespula vulgaris
<400> 719
   Ala Ala His Cys Val Ala Arg Tyr Lys Arg Ile Leu Tyr Ile Leu
   1 5 10 15
<210> 720
   <211> 15
   <212> PRT
   <213> Vespula vulgaris
<400> 720
   Gly Ile Ile His Leu Ala Thr Arg Phe Leu Tyr Cys Gly Ala Thr
   1 5 10 15
<210> 721
   <211> 15
   <212> PRT
   <213> Vespula vulgaris
<400> 721
   Ile Leu Trp Gln Asn Pro Lys Ser Lys Arg Ile Phe Leu Leu Gly
   1 5 10 15
<210> 722
   <211> 15
   <212> PRT
   <213> Vespula vulgaris
<400> 722
   Leu Pro Phe Tyr Tyr Met Gln Arg Asn Phe Val Asp Thr Val Val
   1 5 10 15
<210> 723
   <211> 15
   <212> PRT
   <213> Vespula vulgaris
<400> 723
   Gly Pro Ile Leu Trp Gln Asn Pro Lys Ser Lys Arg Ile Phe Leu
   1 5 10 15
<210> 724
   <211> 15
   <212> PRT
   <213> Vespula vulgaris
<400> 724
   Leu Ala Val Ile Lys Leu Gln Lys Arg Leu Lys Tyr Ser Met Arg
   1 5 10 15
<210> 725
   <211> 15
   <212> PRT
   <213> Vespula vulgaris
<400> 725
   Asp Leu Ala Val Ile Lys Leu Gln Lys Arg Leu Lys Tyr Ser Met
   1 5 10 15
<210> 726
   <211> 15
   <212> PRT
   <213> Vespula vulgaris
<400> 726
   Ala Val Ile Lys Leu Gln Lys Arg Leu Lys Tyr Ser Met Arg Ile
   1 5 10 15
<210> 727
   <211> 15
   <212> PRT
   <213> Vespula vulgaris
<400> 727
   Leu Asn Asp Leu Ala Val Ile Lys Leu Gln Lys Arg Leu Lys Tyr
   1 5 10 15
<210> 728
   <211> 15
   <212> PRT
   <213> Vespula vulgaris
<400> 728
   Asn Asp Leu Ala Val Ile Lys Leu Gln Lys Arg Leu Lys Tyr Ser
   1 5 10 15
<210> 729
   <211> 15
   <212> PRT
   <213> Vespula vulgaris
<400> 729
   Val Ile Lys Leu Gln Lys Arg Leu Lys Tyr Ser Met Arg Ile Gly
   1 5 10 15
<210> 730
   <211> 15
   <212> PRT
   <213> Vespula vulgaris
<400> 730
   Val Lys Tyr His Phe Leu Ala Thr Pro Lys Gln Leu Cys Thr Phe
   1 5 10 15
<210> 731
   <211> 15
   <212> PRT
   <213> Vespula vulgaris
<400> 731
   Cys Val Lys Tyr His Phe Leu Ala Thr Pro Lys Gln Leu Cys Thr
   1 5 10 15
<210> 732
   <211> 15
   <212> PRT
   <213> Vespula vulgaris
<400> 732
   Lys Cys Val Lys Tyr His Phe Leu Ala Thr Pro Lys Gln Leu Cys
   1 5 10 15
<210> 733
   <211> 15
   <212> PRT
   <213> Vespula vulgaris
<400> 733
   Lys Lys Cys Val Lys Tyr His Phe Leu Ala Thr Pro Lys Gln Leu
   1 5 10 15
<210> 734
   <211> 15
   <212> PRT
   <213> Vespula vulgaris
<400> 734
   Met Lys Lys Cys Val Lys Tyr His Phe Leu Ala Thr Pro Lys Gln
   1 5 10 15
<210> 735
   <211> 15
   <212> PRT
   <213> Vespula vulgaris
<400> 735
   Thr Ser Tyr Leu Asp Phe Ile Thr Arg Ser Thr Pro Gly Glu Ile
   1 5 10 15
<210> 736
   <211> 15
   <212> PRT
   <213> Vespula vulgaris
<400> 736
   Ser Tyr Leu Asp Phe Ile Thr Arg Ser Thr Pro Gly Glu Ile Tyr
   1 5 10 15
<210> 737
   <211> 15
   <212> PRT
   <213> Vespula vulgaris
<400> 737
   Tyr Leu Asp Phe Ile Thr Arg Ser Thr Pro Gly Glu Ile Tyr Cys
   1 5 10 15
<210> 738
   <211> 15
   <212> PRT
   <213> Vespula vulgaris
<400> 738
   Leu Asp Phe Ile Thr Arg Ser Thr Pro Gly Glu Ile Tyr Cys Gln
   1 5 10 15
<210> 739
   <211> 15
   <212> PRT
   <213> Vespula vulgaris
<400> 739
   Asp Phe Ile Thr Arg Ser Thr Pro Gly Glu Ile Tyr Cys Gln Ala
   1 5 10 15
<210> 740
   <211> 15
   <212> PRT
   <213> Vespula vulgaris
<400> 740
   Asn Glu Tyr Pro Met Met Ala Gly Ile Ile His Leu Ala Thr Arg
   1 5 10 15
<210> 741
   <211> 15
   <212> PRT
   <213> Vespula vulgaris
<400> 741
   Val Asn Glu Tyr Pro Met Met Ala Gly Ile Ile His Leu Ala Thr
   1 5 10 15
<210> 742
   <211> 15
   <212> PRT
   <213> Vespula vulgaris
<400> 742
   Thr Gly Val Asn Glu Tyr Pro Met Met Ala Gly Ile Ile His Leu
   1 5 10 15
<210> 743
   <211> 15
   <212> PRT
   <213> Vespula vulgaris
<400> 743
   Glu Thr Gly Val Asn Glu Tyr Pro Met Met Ala Gly Ile Ile His
   1 5 10 15
<210> 744
   <211> 15
   <212> PRT
   <213> Vespula vulgaris
<400> 744
   Gly Val Asn Glu Tyr Pro Met Met Ala Gly Ile Ile His Leu Ala
   1 5 10 15
<210> 745
   <211> 15
   <212> PRT
   <213> Vespula vulgaris
<400> 745
   Glu Lys Cys Arg Cys Gly Trp Lys Asn Pro Ser Arg Ile Val Gly
   1 5 10 15
<210> 746
   <211> 15
   <212> PRT
   <213> Vespula vulgaris
<400> 746
   Leu Ala Val Ile Lys Leu Gln Lys Arg Leu Lys Tyr Ser Met Arg
   1 5 10 15
<210> 747
   <211> 15
   <212> PRT
   <213> Vespula vulgaris
<400> 747
   Tyr Pro Met Met Ala Gly Ile Ile His Leu Ala Thr Arg Phe Leu
   1 5 10 15
<210> 748
   <211> 15
   <212> PRT
   <213> Vespula vulgaris
<400> 748
   Asp Leu Ala Val Ile Lys Leu Gln Lys Arg Leu Lys Tyr Ser Met
   1 5 10 15
<210> 749
   <211> 15
   <212> PRT
   <213> Vespula vulgaris
<400> 749
   Lys Cys Arg Cys Gly Trp Lys Asn Pro Ser Arg Ile Val Gly Gly
   1 5 10 15
<210> 750
   <211> 15
   <212> PRT
   <213> Vespula vulgaris
<400> 750
   Cys Arg Cys Gly Trp Lys Asn Pro Ser Arg Ile Val Gly Gly Val
   1 5 10 15
<210> 751
   <211> 15
   <212> PRT
   <213> Vespula vulgaris
<400> 751
   Asn Asp Leu Ala Val Ile Lys Leu Gln Lys Arg Leu Lys Tyr Ser
   1 5 10 15
<210> 752
   <211> 15
   <212> PRT
   <213> Vespula vulgaris
<400> 752
   Leu Asn Asp Leu Ala Val Ile Lys Leu Gln Lys Arg Leu Lys Tyr
   1 5 10 15
<210> 753
   <211> 15
   <212> PRT
   <213> Vespula vulgaris
<400> 753
   Arg Cys Gly Trp Lys Asn Pro Ser Arg Ile Val Gly Gly Val Glu
   1 5 10 15
<210> 754
   <211> 15
   <212> PRT
   <213> Vespula vulgaris
<400> 754
   Cys Gly Trp Lys Asn Pro Ser Arg Ile Val Gly Gly Val Glu Thr
   1 5 10 15
<210> 755
   <211> 15
   <212> PRT
   <213> Vespula vulgaris
<400> 755
   Ala Val Ile Lys Leu Gln Lys Arg Leu Lys Tyr Ser Met Arg Ile
   1 5 10 15
<210> 756
   <211> 15
   <212> PRT
   <213> Vespula vulgaris
<400> 756
   Pro Met Met Ala Gly Ile Ile His Leu Ala Thr Arg Phe Leu Tyr
   1 5 10 15
<210> 757
   <211> 15
   <212> PRT
   <213> Vespula vulgaris
<400> 757
   Met Met Ala Gly Ile Ile His Leu Ala Thr Arg Phe Leu Tyr Cys
   1 5 10 15
<210> 758
   <211> 15
   <212> PRT
   <213> Vespula vulgaris
<400> 758
   Glu Tyr Pro Met Met Ala Gly Ile Ile His Leu Ala Thr Arg Phe
   1 5 10 15
<210> 759
   <211> 15
   <212> PRT
   <213> Vespula vulgaris
<400> 759
   Ala Gly Ile Ile His Leu Ala Thr Arg Phe Leu Tyr Cys Gly Ala
   1 5 10 15
<210> 760
   <211> 15
   <212> PRT
   <213> Vespula vulgaris
<400> 760
   Met Ala Gly Ile Ile His Leu Ala Thr Arg Phe Leu Tyr Cys Gly
   1 5 10 15
<210> 761
   <211> 15
   <212> PRT
   <213> Vespula vulgaris
<400> 761
   Arg Ile Phe Leu Leu Gly Val Ile Asn Tyr Gly Arg Thr Cys Ala
   1 5 10 15
<210> 762
   <211> 15
   <212> PRT
   <213> Vespula vulgaris
<400> 762
   Ile Phe Leu Leu Gly Val Ile Asn Tyr Gly Arg Thr Cys Ala Asp
   1 5 10 15
<210> 763
   <211> 15
   <212> PRT
   <213> Vespula vulgaris
<400> 763
   Leu Asn Asp Leu Ala Val Ile Lys Leu Gln Lys Arg Leu Lys Tyr
   1 5 10 15
<210> 764
   <211> 15
   <212> PRT
   <213> Vespula vulgaris
<400> 764
   Leu Ala Val Ile Lys Leu Gln Lys Arg Leu Lys Tyr Ser Met Arg
   1 5 10 15
<210> 765
   <211> 15
   <212> PRT
   <213> Vespula vulgaris
<400> 765
   Ala Arg Tyr Lys Arg Ile Leu Tyr Ile Leu Gly Val Val Val Gly
   1 5 10 15
<210> 766
   <211> 15
   <212> PRT
   <213> Vespula vulgaris
<400> 766
   Asp Leu Ala Val Ile Lys Leu Gln Lys Arg Leu Lys Tyr Ser Met
   1 5 10 15
<210> 767
   <211> 15
   <212> PRT
   <213> Vespula vulgaris
<400> 767
   Cys Gly Trp Lys Asn Pro Ser Arg Ile Val Gly Gly Val Glu Thr
   1 5 10 15
<210> 768
   <211> 15
   <212> PRT
   <213> Vespula vulgaris
<400> 768
   Arg Tyr Lys Arg Ile Leu Tyr Ile Leu Gly Val Val Val Gly Glu
   1               5                   10                  15
<210> 769
   <211> 15
   <212> PRT
   <213> Vespula vulgaris
<400> 769
   Asn Asp Leu Ala Val Ile Lys Leu Gln Lys Arg Leu Lys Tyr Ser
   1               5                   10                  15
<210> 770
   <211> 15
   <212> PRT
   <213> Vespula vulgaris
<400> 770
   Cys Arg Cys Gly Trp Lys Asn Pro Ser Arg Ile Val Gly Gly Val
   1               5                   10                  15
<210> 771
   <211> 15
   <212> PRT
   <213> Vespula vulgaris
<400> 771
   Lys Arg Ile Leu Tyr Ile Leu Gly Val Val Val Gly Glu His Asn
   1               5                   10                  15
<210> 772
   <211> 15
   <212> PRT
   <213> Vespula vulgaris
<400> 772
   Glu Lys Cys Arg Cys Gly Trp Lys Asn Pro Ser Arg Ile Val Gly
   1               5                   10                  15
<210> 773
   <211> 15
   <212> PRT
   <213> Vespula vulgaris
<400> 773
   Lys Cys Arg Cys Gly Trp Lys Asn Pro Ser Arg Ile Val Gly Gly
   1               5                   10                  15
<210> 774
   <211> 15
   <212> PRT
   <213> Vespula vulgaris
<400> 774
   Tyr Lys Arg Ile Leu Tyr Ile Leu Gly Val Val Val Gly Glu His
   1               5                   10                  15
<210> 775
   <211> 15
   <212> PRT
   <213> Vespula vulgaris
<400> 775
   Ala Val Ile Lys Leu Gln Lys Arg Leu Lys Tyr Ser Met Arg Ile
   1               5                   10                  15
<210> 776
   <211> 15
   <212> PRT
   <213> Vespula vulgaris
<400> 776
   Arg Ile Leu Tyr Ile Leu Gly Val Val Val Gly Glu His Asn Thr
   1               5                   10                  15
<210> 777
   <211> 15
   <212> PRT
   <213> Vespula vulgaris
<400> 777
   Lys Arg Ile Phe Leu Leu Gly Val Ile Asn Tyr Gly Arg Thr Cys
   1               5                   10                  15
<210> 778
   <211> 15
   <212> PRT
   <213> Vespula vulgaris
<400> 778
   Ser Lys Arg Ile Phe Leu Leu Gly Val Ile Asn Tyr Gly Arg Thr
   1               5                   10                  15
<210> 779
   <211> 15
   <212> PRT
   <213> Vespula vulgaris
<400> 779
   Arg Cys Gly Trp Lys Asn Pro Ser Arg Ile Val Gly Gly Val Glu
   1               5                   10                  15
<210> 780
   <211> 15
   <212> PRT
   <213> Vespula vulgaris
<400> 780
   Phe Leu Leu Gly Val Ile Asn Tyr Gly Arg Thr Cys Ala Asp Glu
   1               5                   10                  15
<210> 781
   <211> 15
   <212> PRT
   <213> Vespula vulgaris
<400> 781
   Ile Lys Leu Gln Lys Arg Leu Lys Tyr Ser Met Arg Ile Gly Pro
   1               5                   10                  15
<210> 782
   <211> 15
   <212> PRT
   <213> Vespula vulgaris
<400> 782
   Met Ala Gly Ile Ile His Leu Ala Thr Arg Phe Leu Tyr Cys Gly
   1               5                   10                  15
<210> 783
   <211> 15
   <212> PRT
   <213> Vespula vulgaris
<400> 783
   Ile Ala Ser Lys Met Thr Ile Lys Phe His Ser Arg Tyr Asn Thr
   1               5                   10                  15
<210> 784
   <211> 15
   <212> PRT
   <213> Vespula vulgaris
<400> 784
   Ala Asp Glu Ala Pro Gly Val Asn Leu Arg Val Thr Ser Tyr Leu
   1               5                   10                  15
<210> 785
   <211> 15
   <212> PRT
   <213> Vespula vulgaris
<400> 785
   Asp Glu Ala Pro Gly Val Asn Leu Arg Val Thr Ser Tyr Leu Asp
   1               5                   10                  15
<210> 786
   <211> 15
   <212> PRT
   <213> Vespula vulgaris
<400> 786
   Glu Ala Pro Gly Val Asn Leu Arg Val Thr Ser Tyr Leu Asp Phe
   1 5 10 15
<210> 787
   <211> 15
   <212> PRT
   <213> Vespula vulgaris
<400> 787
   Ala Gly Ile Ile His Leu Ala Thr Arg Phe Leu Tyr Cys Gly Ala
   1 5 10 15
<210> 788
   <211> 15
   <212> PRT
   <213> Vespula vulgaris
<400> 788
   Val Ile Lys Leu Gln Lys Arg Leu Lys Tyr Ser Met Arg Ile Gly
   1 5 10 15
<210> 789
   <211> 15
   <212> PRT
   <213> Vespula vulgaris
<400> 789
   Thr Ala Val Gly Trp Gly Leu Thr Asn Phe Tyr Gly Val Lys Ser
   1 5 10 15
<210> 790
   <211> 15
   <212> PRT
   <213> Vespula vulgaris
<400> 790
   Trp Lys Asn Pro Ser Arg Ile Val Gly Gly Val Glu Thr Gly Val
   1 5 10 15
<210> 791
   <211> 15
   <212> PRT
   <213> Vespula vulgaris
<400> 791
   Trp Gly Leu Thr Asn Phe Tyr Gly Val Lys Ser Glu Val Leu Arg
   1 5 10 15
<210> 792
   <211> 15
   <212> PRT
   <213> Vespula vulgaris
<400> 792
   Ala Pro Gly Val Asn Leu Arg Val Thr Ser Tyr Leu Asp Phe Ile
   1 5 10 15
<210> 793
   <211> 15
   <212> PRT
   <213> Vespula vulgaris
<400> 793
   Met Met Ala Gly Ile Ile His Leu Ala Thr Arg Phe Leu Tyr Cys
   1 5 10 15
<210> 794
   <211> 15
   <212> PRT
   <213> Vespula vulgaris
<400> 794
   Pro Met Met Ala Gly Ile Ile His Leu Ala Thr Arg Phe Leu Tyr
   1 5 10 15
<210> 795
   <211> 15
   <212> PRT
   <213> Vespula vulgaris
<400> 795
   Gly Trp Lys Asn Pro Ser Arg Ile Val Gly Gly Val Glu Thr Gly
   1 5 10 15
<210> 796
   <211> 15
   <212> PRT
   <213> Vespula vulgaris
<400> 796
   Leu Leu Gly Val Ile Asn Tyr Gly Arg Thr Cys Ala Asp Glu Ala
   1 5 10 15
<210> 797
   <211> 15
   <212> PRT
   <213> Vespula vulgaris
<400> 797
   His Asn Thr Trp Ala Ile Asn Asp Thr Lys Ala Thr Gln Leu Tyr
   1 5 10 15
<210> 798
   <211> 15
   <212> PRT
   <213> Vespula vulgaris
<400> 798
   Leu Thr Asn Phe Tyr Gly Val Lys Ser Glu Val Leu Arg Lys Val
   1 5 10 15
<210> 799
   <211> 15
   <212> PRT
   <213> Vespula vulgaris
<400> 799
   Pro Gly Val Asn Leu Arg Val Thr Ser Tyr Leu Asp Phe Ile Thr
   1 5 10 15
<210> 800
   <211> 15
   <212> PRT
   <213> Vespula vulgaris
<400> 800
   Leu Gly Val Ile Asn Tyr Gly Arg Thr Cys Ala Asp Glu Ala Pro
   1 5 10 15
<210> 801
   <211> 15
   <212> PRT
   <213> Vespula vulgaris
<400> 801
   Asn Thr Trp Ala Ile Asn Asp Thr Lys Ala Thr Gln Leu Tyr Leu
   1 5 10 15
<210> 802
   <211> 15
   <212> PRT
   <213> Vespula vulgaris
<400> 802
   Glu His Asn Thr Trp Ala Ile Asn Asp Thr Lys Ala Thr Gln Leu
   1 5 10 15
<210> 803
   <211> 15
   <212> PRT
   <213> Vespula vulgaris
<400> 803
   Ile Leu Tyr Ile Leu Gly Val Val Val Gly Glu His Asn Thr Trp
   1 5 10 15
<210> 804
   <211> 15
   <212> PRT
   <213> Vespula vulgaris
<400> 804
   Gly Ile Ile His Leu Ala Thr Arg Phe Leu Tyr Cys Gly Ala Thr
   1 5 10 15
<210> 805
   <211> 15
   <212> PRT
   <213> Vespula vulgaris
<400> 805
   Val Gly Trp Gly Leu Thr Asn Phe Tyr Gly Val Lys Ser Glu Val
   1 5 10 15
<210> 806
   <211> 15
   <212> PRT
   <213> Vespula vulgaris
<400> 806
   Ala Val Gly Trp Gly Leu Thr Asn Phe Tyr Gly Val Lys Ser Glu
   1 5 10 15
<210> 807
   <211> 15
   <212> PRT
   <213> Vespula vulgaris
<400> 807
   Val Gly Glu His Asn Thr Trp Ala Ile Asn Asp Thr Lys Ala Thr
   1 5 10 15
<210> 808
   <211> 15
   <212> PRT
   <213> Vespula vulgaris
<400> 808
   Lys Ser Lys Arg Ile Phe Leu Leu Gly Val Ile Asn Tyr Gly Arg
   1 5 10 15
<210> 809
   <211> 15
   <212> PRT
   <213> Vespula vulgaris
<400> 809
   His Cys Val Ala Arg Tyr Lys Arg Ile Leu Tyr Ile Leu Gly Val
   1 5 10 15
<210> 810
   <211> 15
   <212> PRT
   <213> Vespula vulgaris
<400> 810
   Ala Ser Lys Met Thr Ile Lys Phe His Ser Arg Tyr Asn Thr Tyr
   1 5 10 15
<210> 811
   <211> 15
   <212> PRT
   <213> Vespula vulgaris
<400> 811
   Ala His Cys Val Ala Arg Tyr Lys Arg Ile Leu Tyr Ile Leu Gly
   1 5 10 15
<210> 812
   <211> 15
   <212> PRT
   <213> Vespula vulgaris
<400> 812
   Pro Lys Ser Lys Arg Ile Phe Leu Leu Gly Val Ile Asn Tyr Gly
   1 5 10 15
<210> 813
   <211> 15
   <212> PRT
   <213> Vespula vulgaris
<400> 813
   Ser Lys Met Thr Ile Lys Phe His Ser Arg Tyr Asn Thr Tyr Gly
   1 5 10 15
<210> 814
   <211> 15
   <212> PRT
   <213> Vespula vulgaris
<400> 814
   Ala Arg Ser Asn Thr Arg Ile Lys Leu Asn Cys Thr Val Phe Asp
   1 5 10 15
<210> 815
   <211> 15
   <212> PRT
   <213> Vespula vulgaris
<400> 815
   Gly Glu His Asn Thr Trp Ala Ile Asn Asp Thr Lys Ala Thr Gln
   1 5 10 15
<210> 816
   <211> 15
   <212> PRT
   <213> Vespula vulgaris
<400> 816
   Arg Ser Asn Thr Arg Ile Lys Leu Asn Cys Thr Val Phe Asp Val
   1 5 10 15
<210> 817
   <211> 15
   <212> PRT
   <213> Vespula vulgaris
<400> 817
   Val Ala Arg Tyr Lys Arg Ile Leu Tyr Ile Leu Gly Val Val Val
   1 5 10 15
<210> 818
   <211> 15
   <212> PRT
   <213> Vespula vulgaris
<400> 818
   Leu Tyr Ile Leu Gly Val Val Val Gly Glu His Asn Thr Trp Ala
   1 5 10 15
<210> 819
   <211> 15
   <212> PRT
   <213> Vespula vulgaris
<400> 819
   Lys Met Thr Ile Lys Phe His Ser Arg Tyr Asn Thr Tyr Gly Gly
   1 5 10 15
<210> 820
   <211> 15
   <212> PRT
   <213> Vespula vulgaris
<400> 820
   Met Thr Ile Lys Phe His Ser Arg Tyr Asn Thr Tyr Gly Gly Lys
   1               5                   10                  15
<210> 821
   <211> 15
   <212> PRT
   <213> Vespula vulgaris
<400> 821
   Val Val Thr Ala Val Gly Trp Gly Leu Thr Asn Phe Tyr Gly Val
   1               5                   10                  15
<210> 822
   <211> 15
   <212> PRT
   <213> Vespula vulgaris
<400> 822
   Ser Asn Thr Arg Ile Lys Leu Asn Cys Thr Val Phe Asp Val Pro
   1               5                   10                  15
<210> 823
   <211> 15
   <212> PRT
   <213> Vespula vulgaris
<400> 823
   Asn Thr Arg Ile Lys Leu Asn Cys Thr Val Phe Asp Val Pro Pro
   1               5                   10                  15
<210> 824
   <211> 15
   <212> PRT
   <213> Vespula vulgaris
<400> 824
   Thr Asn Phe Tyr Gly Val Lys Ser Glu Val Leu Arg Lys Val Asp
   1               5                   10                  15
<210> 825
   <211> 15
   <212> PRT
   <213> Vespula vulgaris
<400> 825
   Val Thr Ala Val Gly Trp Gly Leu Thr Asn Phe Tyr Gly Val Lys
   1               5                   10                  15
<210> 826
   <211> 15
   <212> PRT
   <213> Vespula vulgaris
<400> 826
   Asn Phe Tyr Gly Val Lys Ser Glu Val Leu Arg Lys Val Asp Leu
   1 5 10 15
<210> 827
   <211> 15
   <212> PRT
   <213> Vespula vulgaris
<400> 827
   Lys Leu Asn Asp Leu Ala Val Ile Lys Leu Gln Lys Arg Leu Lys
   1 5 10 15
<210> 828
   <211> 15
   <212> PRT
   <213> Vespula vulgaris
<400> 828
   Thr Arg Ile Lys Leu Asn Cys Thr Val Phe Asp Val Pro Pro Ser
   1 5 10 15
<210> 829
   <211> 15
   <212> PRT
   <213> Vespula vulgaris
<400> 829
   Asp Thr Val Val Thr Ala Val Gly Trp Gly Leu Thr Asn Phe Tyr
   1 5 10 15
<210> 830
   <211> 15
   <212> PRT
   <213> Vespula vulgaris
<400> 830
   Gly Leu Thr Asn Phe Tyr Gly Val Lys Ser Glu Val Leu Arg Lys
   1 5 10 15
<210> 831
   <211> 15
   <212> PRT
   <213> Vespula vulgaris
<400> 831
   Cys Val Ala Arg Tyr Lys Arg Ile Leu Tyr Ile Leu Gly Val Val
   1 5 10 15
<210> 832
   <211> 15
   <212> PRT
   <213> Vespula vulgaris
<400> 832
   Tyr Pro Met Met Ala Gly Ile Ile His Leu Ala Thr Arg Phe Leu
   1 5 10 15
<210> 833
   <211> 15
   <212> PRT
   <213> Vespula vulgaris
<400> 833
   Pro Lys Leu Asn Asp Leu Ala Val Ile Lys Leu Gln Lys Arg Leu
   1 5 10 15
<210> 834
   <211> 15
   <212> PRT
   <213> Vespula vulgaris
<400> 834
   Arg Pro Lys Leu Asn Asp Leu Ala Val Ile Lys Leu Gln Lys Arg
   1 5 10 15
<210> 835
   <211> 15
   <212> PRT
   <213> Vespula vulgaris
<400> 835
   Asn Pro Lys Ser Lys Arg Ile Phe Leu Leu Gly Val Ile Asn Tyr
   1 5 10 15
<210> 836
   <211> 15
   <212> PRT
   <213> Vespula vulgaris
<400> 836
   Ile Lys Phe His Ser Arg Tyr Asn Thr Tyr Gly Gly Lys Phe Arg
   1 5 10 15
<210> 837
   <211> 15
   <212> PRT
   <213> Vespula vulgaris
<400> 837
   Gly Trp Gly Leu Thr Asn Phe Tyr Gly Val Lys Ser Glu Val Leu
   1 5 10 15
<210> 838
   <211> 15
   <212> PRT
   <213> Vespula vulgaris
<400> 838
   Thr Val Val Thr Ala Val Gly Trp Gly Leu Thr Asn Phe Tyr Gly
   1 5 10 15
<210> 839
   <211> 15
   <212> PRT
   <213> Vespula vulgaris
<400> 839
   Leu Gln Lys Arg Leu Lys Tyr Ser Met Arg Ile Gly Pro Ala Cys
   1 5 10 15
<210> 840
   <211> 15
   <212> PRT
   <213> Vespula vulgaris
<400> 840
   Thr Trp Ala Ile Asn Asp Thr Lys Ala Thr Gln Leu Tyr Leu Ile
   1 5 10 15
<210> 841
   <211> 15
   <212> PRT
   <213> Vespula vulgaris
<400> 841
   Asp Leu His Val Val Ser Met Lys Lys Cys Val Lys Tyr His Phe
   1 5 10 15
<210> 842
   <211> 15
   <212> PRT
   <213> Vespula vulgaris
<400> 842
   Trp Ala Ile Asn Asp Thr Lys Ala Thr Gln Leu Tyr Leu Ile Asp
   1 5 10 15
<210> 843
   <211> 15
   <212> PRT
   <213> Vespula vulgaris
<400> 843
   Leu His Val Val Ser Met Lys Lys Cys Val Lys Tyr His Phe Leu
   1               5                   10                  15
<210> 844
   <211> 15
   <212> PRT
   <213> Vespula vulgaris
<400> 844
   Ile Ile His Leu Ala Thr Arg Phe Leu Tyr Cys Gly Ala Thr Ile
   1               5                   10                  15
<210> 845
   <211> 15
   <212> PRT
   <213> Vespula vulgaris
<400> 845
   Ser Phe Ala Val Glu Ser Ile Ala Ser Lys Met Thr Ile Lys Phe
   1               5                   10                  15
<210> 846
   <211> 15
   <212> PRT
   <213> Vespula vulgaris
<400> 846
   Ala Val Glu Ser Ile Ala Ser Lys Met Thr Ile Lys Phe His Ser
   1               5                   10                  15
<210> 847
   <211> 15
   <212> PRT
   <213> Vespula vulgaris
<400> 847
   Lys Leu Gln Lys Arg Leu Lys Tyr Ser Met Arg Ile Gly Pro Ala
   1               5                   10                  15
<210> 848
   <211> 15
   <212> PRT
   <213> Vespula vulgaris
<400> 848
   Phe Ala Val Glu Ser Ile Ala Ser Lys Met Thr Ile Lys Phe His
   1               5                   10                  15
<210> 849
   <211> 15
   <212> PRT
   <213> Vespula vulgaris
<400> 849
   Ser Lys Arg Ile Phe Leu Leu Gly Val Ile Asn Tyr Gly Arg Thr
   1               5                   10                  15
<210> 850
   <211> 15
   <212> PRT
   <213> Vespula vulgaris
<400> 850
   Lys Ser Lys Arg Ile Phe Leu Leu Gly Val Ile Asn Tyr Gly Arg
   1               5                   10                  15
<210> 851
   <211> 15
   <212> PRT
   <213> Vespula vulgaris
<400> 851
   Lys Arg Ile Phe Leu Leu Gly Val Ile Asn Tyr Gly Arg Thr Cys
   1               5                   10                  15
<210> 852
   <211> 15
   <212> PRT
   <213> Vespula vulgaris
<400> 852
   Arg Ile Phe Leu Leu Gly Val Ile Asn Tyr Gly Arg Thr Cys Ala
   1               5                   10                  15
<210> 853
   <211> 15
   <212> PRT
   <213> Vespula vulgaris
<400> 853
   Ile Phe Leu Leu Gly Val Ile Asn Tyr Gly Arg Thr Cys Ala Asp
   1               5                   10                  15
<210> 854
   <211> 15
   <212> PRT
   <213> Vespula vulgaris
<400> 854
   Val Lys Ser Glu Val Leu Arg Lys Val Asp Leu His Val Val Ser
   1               5                   10                  15
<210> 855
   <211> 15
   <212> PRT
   <213> Vespula vulgaris
<400> 855
   Gly Val Lys Ser Glu Val Leu Arg Lys Val Asp Leu His Val Val
   1 5 10 15
<210> 856
   <211> 15
   <212> PRT
   <213> Vespula vulgaris
<400> 856
   Ser Glu Val Leu Arg Lys Val Asp Leu His Val Val Ser Met Lys
   1 5 10 15
<210> 857
   <211> 15
   <212> PRT
   <213> Vespula vulgaris
<400> 857
   Lys Ser Glu Val Leu Arg Lys Val Asp Leu His Val Val Ser Met
   1 5 10 15
<210> 858
   <211> 15
   <212> PRT
   <213> Vespula vulgaris
<400> 858
   Phe Leu Leu Gly Val Ile Asn Tyr Gly Arg Thr Cys Ala Asp Glu
   1 5 10 15
<210> 859
   <211> 15
   <212> PRT
   <213> Vespula vulgaris
<400> 859
   Tyr Asn Pro Asp Phe Pro Asn Tyr Tyr Met Gly Glu His Asn Cys
   1 5 10 15
<210> 860
   <211> 15
   <212> PRT
   <213> Vespula vulgaris
<400> 860
   Glu Val Leu Arg Lys Val Asp Leu His Val Val Ser Met Lys Lys
   1 5 10 15
<210> 861
   <211> 15
   <212> PRT
   <213> Vespula vulgaris
<400> 861
   Asn Asp Leu Ala Val Ile Lys Leu Gln Lys Arg Leu Lys Tyr Ser
   1 5 10 15
<210> 862
   <211> 15
   <212> PRT
   <213> Vespula vulgaris
<400> 862
   Leu Leu Gly Val Ile Asn Tyr Gly Arg Thr Cys Ala Asp Glu Ala
   1 5 10 15
<210> 863
   <211> 15
   <212> PRT
   <213> Vespula vulgaris
<400> 863
   Leu Asn Asp Leu Ala Val Ile Lys Leu Gln Lys Arg Leu Lys Tyr
   1 5 10 15
<210> 864
   <211> 15
   <212> PRT
   <213> Vespula vulgaris
<400> 864
   Lys Leu Asn Asp Leu Ala Val Ile Lys Leu Gln Lys Arg Leu Lys
   1 5 10 15
<210> 865
   <211> 15
   <212> PRT
   <213> Vespula vulgaris
<400> 865
   Ala Arg Tyr Lys Arg Ile Leu Tyr Ile Leu Gly Val Val Val Gly
   1 5 10 15
<210> 866
   <211> 15
   <212> PRT
   <213> Vespula vulgaris
<400> 866
   Tyr Lys Arg Ile Leu Tyr Ile Leu Gly Val Val Val Gly Glu His
   1 5 10 15
<210> 867
   <211> 15
   <212> PRT
   <213> Vespula vulgaris
<400> 867
   Val Leu Arg Lys Val Asp Leu His Val Val Ser Met Lys Lys Cys
   1 5 10 15
<210> 868
   <211> 15
   <212> PRT
   <213> Vespula vulgaris
<400> 868
   Lys Arg Ile Leu Tyr Ile Leu Gly Val Val Val Gly Glu His Asn
   1 5 10 15
<210> 869
   <211> 15
   <212> PRT
   <213> Vespula vulgaris
<400> 869
   Pro Lys Leu Asn Asp Leu Ala Val Ile Lys Leu Gln Lys Arg Leu
   1 5 10 15
<210> 870
   <211> 15
   <212> PRT
   <213> Vespula vulgaris
<400> 870
   Asp Leu Ala Val Ile Lys Leu Gln Lys Arg Leu Lys Tyr Ser Met
   1 5 10 15
<210> 871
   <211> 15
   <212> PRT
   <213> Vespula vulgaris
<400> 871
   Arg Tyr Lys Arg Ile Leu Tyr Ile Leu Gly Val Val Val Gly Glu
   1 5 10 15
<210> 872
   <211> 15
   <212> PRT
   <213> Vespula vulgaris
<400> 872
   Trp Gly Leu Thr Asn Phe Tyr Gly Val Lys Ser Glu Val Leu Arg
   1 5 10 15
<210> 873
   <211> 15
   <212> PRT
   <213> Vespula vulgaris
<400> 873
   Leu Thr Asn Phe Tyr Gly Val Lys Ser Glu Val Leu Arg Lys Val
   1 5 10 15
<210> 874
   <211> 15
   <212> PRT
   <213> Vespula vulgaris
<400> 874
   Arg Pro Lys Leu Asn Asp Leu Ala Val Ile Lys Leu Gln Lys Arg
   1 5 10 15
<210> 875
   <211> 15
   <212> PRT
   <213> Vespula vulgaris
<400> 875
   Val Ala Arg Tyr Lys Arg Ile Leu Tyr Ile Leu Gly Val Val Val
   1 5 10 15
<210> 876
   <211> 15
   <212> PRT
   <213> Vespula vulgaris
<400> 876
   Arg Lys Val Asp Leu His Val Val Ser Met Lys Lys Cys Val Lys
   1 5 10 15
<210> 877
   <211> 15
   <212> PRT
   <213> Vespula vulgaris
<400> 877
   Val Asp Leu His Val Val Ser Met Lys Lys Cys Val Lys Tyr His
   1 5 10 15
<210> 878
   <211> 15
   <212> PRT
   <213> Vespula vulgaris
<400> 878
   Lys Val Asp Leu His Val Val Ser Met Lys Lys Cys Val Lys Tyr
   1 5 10 15
<210> 879
   <211> 15
   <212> PRT
   <213> Vespula vulgaris
<400> 879
   Ala Val Ile Lys Leu Gln Lys Arg Leu Lys Tyr Ser Met Arg Ile
   1 5 10 15
<210> 880
   <211> 15
   <212> PRT
   <213> Vespula vulgaris
<400> 880
   Tyr Gly Val Lys Ser Glu Val Leu Arg Lys Val Asp Leu His Val
   1 5 10 15
<210> 881
   <211> 15
   <212> PRT
   <213> Vespula vulgaris
<400> 881
   Arg Ile Leu Tyr Ile Leu Gly Val Val Val Gly Glu His Asn Thr
   1 5 10 15
<210> 882
   <211> 15
   <212> PRT
   <213> Vespula vulgaris
<400> 882
   Glu Lys Cys Arg Cys Gly Trp Lys Asn Pro Ser Arg Ile Val Gly
   1 5 10 15
<210> 883
   <211> 15
   <212> PRT
   <213> Vespula vulgaris
<400> 883
   Gly Trp Gly Leu Thr Asn Phe Tyr Gly Val Lys Ser Glu Val Leu
   1 5 10 15
<210> 884
   <211> 15
   <212> PRT
   <213> Vespula vulgaris
<400> 884
   His Cys Val Ala Arg Tyr Lys Arg Ile Leu Tyr Ile Leu Gly Val
   1               5                   10                  15
<210> 885
   <211> 15
   <212> PRT
   <213> Vespula vulgaris
<400> 885
   Val Ile Lys Leu Gln Lys Arg Leu Lys Tyr Ser Met Arg Ile Gly
   1               5                   10                  15
<210> 886
   <211> 15
   <212> PRT
   <213> Vespula vulgaris
<400> 886
   Cys Val Ala Arg Tyr Lys Arg Ile Leu Tyr Ile Leu Gly Val Val
   1               5                   10                  15
<210> 887
   <211> 15
   <212> PRT
   <213> Vespula vulgaris
<400> 887
   Asp Leu His Val Val Ser Met Lys Lys Cys Val Lys Tyr His Phe
   1               5                   10                  15
<210> 888
   <211> 15
   <212> PRT
   <213> Vespula vulgaris
<400> 888
   Leu His Val Val Ser Met Lys Lys Cys Val Lys Tyr His Phe Leu
   1               5                   10                  15
<210> 889
   <211> 15
   <212> PRT
   <213> Vespula vulgaris
<400> 889
   Thr Asn Phe Tyr Gly Val Lys Ser Glu Val Leu Arg Lys Val Asp
   1               5                   10                  15
<210> 890
   <211> 15
   <212> PRT
   <213> Vespula vulgaris
<400> 890
   Lys Cys Arg Cys Gly Trp Lys Asn Pro Ser Arg Ile Val Gly Gly
   1 5 10 15
<210> 891
   <211> 15
   <212> PRT
   <213> Vespula vulgaris
<400> 891
   Gly Leu Thr Asn Phe Tyr Gly Val Lys Ser Glu Val Leu Arg Lys
   1 5 10 15
<210> 892
   <211> 15
   <212> PRT
   <213> Vespula vulgaris
<400> 892
   Asp Ser Gly Gly Pro Ile Leu Trp Gln Asn Pro Lys Ser Lys Arg
   1 5 10 15
<210> 893
   <211> 15
   <212> PRT
   <213> Vespula vulgaris
<400> 893
   Ser Gly Gly Pro Ile Leu Trp Gln Asn Pro Lys Ser Lys Arg Ile
   1 5 10 15
<210> 894
   <211> 15
   <212> PRT
   <213> Vespula vulgaris
<400> 894
   Gly Gly Pro Ile Leu Trp Gln Asn Pro Lys Ser Lys Arg Ile Phe
   1 5 10 15
<210> 895
   <211> 15
   <212> PRT
   <213> Vespula vulgaris
<400> 895
   Gly Pro Ile Leu Trp Gln Asn Pro Lys Ser Lys Arg Ile Phe Leu
   1 5 10 15
<210> 896
   <211> 15
   <212> PRT
   <213> Vespula vulgaris
<400> 896
   Phe Asp Ser Gly Gly Pro Ile Leu Trp Gln Asn Pro Lys Ser Lys
   1 5 10 15
<210> 897
   <211> 15
   <212> PRT
   <213> Vespula vulgaris
<400> 897
   Pro Ile Leu Trp Gln Asn Pro Lys Ser Lys Arg Ile Phe Leu Leu
   1 5 10 15
<210> 898
   <211> 15
   <212> PRT
   <213> Vespula vulgaris
<400> 898
   Leu Ala Val Ile Lys Leu Gln Lys Arg Leu Lys Tyr Ser Met Arg
   1 5 10 15
<210> 899
   <211> 15
   <212> PRT
   <213> Vespula vulgaris
<400> 899
   Asp Leu Ala Val Ile Lys Leu Gln Lys Arg Leu Lys Tyr Ser Met
   1 5 10 15
<210> 900
   <211> 15
   <212> PRT
   <213> Vespula vulgaris
<400> 900
   Leu Asn Asp Leu Ala Val Ile Lys Leu Gln Lys Arg Leu Lys Tyr
   1 5 10 15
<210> 901
   <211> 15
   <212> PRT
   <213> Vespula vulgaris
<400> 901
   Asn Asp Leu Ala Val Ile Lys Leu Gln Lys Arg Leu Lys Tyr Ser
   1 5 10 15
<210> 902
   <211> 15
   <212> PRT
   <213> Vespula vulgaris
<400> 902
   Ile Leu Trp Gln Asn Pro Lys Ser Lys Arg Ile Phe Leu Leu Gly
   1 5 10 15
<210> 903
   <211> 15
   <212> PRT
   <213> Vespula vulgaris
<400> 903
   Asp Phe Pro Asn Tyr Tyr Met Gly Glu His Asn Cys Arg Trp Ser
   1 5 10 15
<210> 904
   <211> 15
   <212> PRT
   <213> Vespula vulgaris
<400> 904
   Phe Pro Asn Tyr Tyr Met Gly Glu His Asn Cys Arg Trp Ser Ala
   1 5 10 15
<210> 905
   <211> 15
   <212> PRT
   <213> Vespula vulgaris
<400> 905
   Asn Pro Asp Phe Pro Asn Tyr Tyr Met Gly Glu His Asn Cys Arg
   1 5 10 15
<210> 906
   <211> 15
   <212> PRT
   <213> Vespula vulgaris
<400> 906
   Gly Gly Pro Ile Leu Trp Gln Asn Pro Lys Ser Lys Arg Ile Phe
   1 5 10 15
<210> 907
   <211> 15
   <212> PRT
   <213> Vespula vulgaris
<400> 907
   Ser Gly Gly Pro Ile Leu Trp Gln Asn Pro Lys Ser Lys Arg Ile
   1 5 10 15
<210> 908
   <211> 15
   <212> PRT
   <213> Vespula vulgaris
<400> 908
   Gly Pro Ile Leu Trp Gln Asn Pro Lys Ser Lys Arg Ile Phe Leu
   1 5 10 15
<210> 909
   <211> 15
   <212> PRT
   <213> Vespula vulgaris
<400> 909
   Ile Ile Val His Pro Asn Tyr Arg Pro Lys Leu Asn Asp Leu Ala
   1 5 10 15
<210> 910
   <211> 15
   <212> PRT
   <213> Vespula vulgaris
<400> 910
   Pro Ile Leu Trp Gln Asn Pro Lys Ser Lys Arg Ile Phe Leu Leu
   1 5 10 15
<210> 911
   <211> 15
   <212> PRT
   <213> Vespula vulgaris
<400> 911
   Ile Leu Trp Gln Asn Pro Lys Ser Lys Arg Ile Phe Leu Leu Gly
   1 5 10 15
<210> 912
   <211> 15
   <212> PRT
   <213> Vespula vulgaris
<400> 912
   Ile Val His Pro Asn Tyr Arg Pro Lys Leu Asn Asp Leu Ala Val
   1 5 10 15
<210> 913
   <211> 15
   <212> PRT
   <213> Vespula vulgaris
<400> 913
   Asp Asp Ile Ile Val His Pro Asn Tyr Arg Pro Lys Leu Asn Asp
   1 5 10 15
<210> 914
   <211> 15
   <212> PRT
   <213> Vespula vulgaris
<400> 914
   Ile Asp Asp Ile Ile Val His Pro Asn Tyr Arg Pro Lys Leu Asn
   1 5 10 15
<210> 915
   <211> 15
   <212> PRT
   <213> Vespula vulgaris
<400> 915
   Leu Ile Asp Asp Ile Ile Val His Pro Asn Tyr Arg Pro Lys Leu
   1 5 10 15
<210> 916
   <211> 15
   <212> PRT
   <213> Vespula vulgaris
<400> 916
   Asp Ser Gly Gly Pro Ile Leu Trp Gln Asn Pro Lys Ser Lys Arg
   1 5 10 15
<210> 917
   <211> 15
   <212> PRT
   <213> Vespula vulgaris
<400> 917
   Val His Pro Asn Tyr Arg Pro Lys Leu Asn Asp Leu Ala Val Ile
   1 5 10 15
<210> 918
   <211> 15
   <212> PRT
   <213> Vespula vulgaris
<400> 918
   Asp Ile Ile Val His Pro Asn Tyr Arg Pro Lys Leu Asn Asp Leu
   1 5 10 15
<210> 919
   <211> 45
   <212> DNA
   <213> Vespula vulgaris
<400> 919
   gagtacgttt tctgtggatt aaattctttt gcagtggaat caata 45
<210> 920
   <211> 45
   <212> DNA
   <213> Vespula vulgaris
<400> 920
   cttacgaatt tttatggtgt caagtctgaa gttttgagaa aagtc 45
<210> 921
   <211> 45
   <212> DNA
   <213> Vespula vulgaris
<400> 921
   atggctggta taatacatct tgcaacgcgt tttctctatt gcggt 45
<210> 922
   <211> 45
   <212> DNA
   <213> Vespula vulgaris
<400> 922
   tataaaagga ttttatatat tctaggagtt gttgttggag aacat 45
<210> 923
   <211> 45
   <212> DNA
   <213> Vespula vulgaris
<400> 923
   gcgaggtata aaaggatttt atatattcta ggagttgttg ttgga 45
<210> 924
   <211> 45
   <212> DNA
   <213> Vespula vulgaris
<400> 924
   gcaacgcgtt ttctctattg cggtgcaact ataataactc cgcaa 45
<210> 925
   <211> 45
   <212> DNA
   <213> Vespula vulgaris
<400> 925
   tcttttgcag tggaatcaat agcatcgaaa atgactataa aattc 45
<210> 926
   <211> 45
   <212> DNA
   <213> Vespula vulgaris
<400> 926
   tgtcttccgt tctattacat gcagcgaaac tttgtagaca ctgtt 45
<210> 927
   <211> 45
   <212> DNA
   <213> Vespula vulgaris
<400> 927
   ccaattttat ggcaaaatcc aaaaagcaaa cgtattttcc ttctt 45
<210> 928
   <211> 45
   <212> DNA
   <213> Vespula vulgaris
<400> 928
   tgcgtcaagt atcactttct ggctacacct aagcaacttt gtaca 45
<210> 929
   <211> 45
   <212> DNA
   <213> Vespula vulgaris
<400> 929
   gtgaatgaat atcctatgat ggctggtata atacatcttg caacg 45
<210> 930
   <211> 45
   <212> DNA
   <213> Vespula vulgaris
<400> 930
   acatgggcaa ttaacgatac aaaggcaact caactttatc ttatt 45
<210> 931
   <211> 45
   <212> DNA
   <213> Vespula vulgaris
<400> 931
   catccgaatt ataggccaaa attaaacgat ttagctgtta taaaa 45
<210> 932
   <211> 45
   <212> DNA
   <213> Vespula vulgaris
<400> 932
   aattgtcggt ggagtgctag gagcaatact cgaattaaat tgaat 45
<210> 933
   <211> 45
   <212> DNA
   <213> Vespula vulgaris
<400> 933
   ggtgcaacta taataactcc gcaacatgta ttaacggctg ctcat 45
<210> 934
   <211> 45
   <212> DNA
   <213> Vespula vulgaris
<400> 934
   aggccaaaat taaacgattt agctgttata aaattgcaga agagg 45
<210> 935
   <211> 45
   <212> DNA
   <213> Vespula vulgaris
<400> 935
   gtaggatggg gtcttacgaa tttttatggt gtcaagtctg aagtt 45
<210> 936
   <211> 45
   <212> DNA
   <213> Vespula vulgaris
<400> 936
   tttgtagaca ctgttgttac agctgtagga tggggtctta cgaat 45
<210> 937
   <211> 45
   <212> DNA
   <213> Vespula vulgaris
<400> 937
   cagcgaaact ttgtagacac tgttgttaca gctgtaggat ggggt 45
<210> 938
   <211> 45
   <212> DNA
   <213> Vespula vulgaris
<400> 938
   tgtggattaa attcttttgc agtggaatca atagcatcga aaatg 45
<210> 939
   <211> 45
   <212> DNA
   <213> Vespula vulgaris
<400> 939
   ttaaaatatt ctatgagaat tggtccagct tgtcttccgt tctat 45
<210> 940
   <211> 45
   <212> DNA
   <213> Vespula vulgaris
<400> 940
   gagtacgttt tctgtggatt aaattctttt gcagtggaat caata 45
<210> 941
   <211> 45
   <212> DNA
   <213> Vespula vulgaris
<400> 941
   tataaaagga ttttatatat tctaggagtt gttgttggag aacat 45
<210> 942
   <211> 45
   <212> DNA
   <213> Vespula vulgaris
<400> 942
   atggctggta taatacatct tgcaacgcgt tttctctatt gcggt 45
<210> 943
   <211> 45
   <212> DNA
   <213> Vespula vulgaris
<400> 943
   cttacgaatt tttatggtgt caagtctgaa gttttgagaa aagtc 45
<210> 944
   <211> 45
   <212> DNA
   <213> Vespula vulgaris
<400> 944
   tgtcttccgt tctattacat gcagcgaaac tttgtagaca ctgtt 45
<210> 945
   <211> 45
   <212> DNA
   <213> Vespula vulgaris
<400> 945
   cgtattttcc ttcttggagt catcaattat ggaagaacat gtgcc 45
<210> 946
   <211> 45
   <212> DNA
   <213> Vespula vulgaris
<400> 946
   tcttttgcag tggaatcaat agcatcgaaa atgactataa aattc 45
<210> 947
   <211> 45
   <212> DNA
   <213> Vespula vulgaris
<400> 947
   gcaacgcgtt ttctctattg cggtgcaact ataataactc cgcaa 45
<210> 948
   <211> 45
   <212> DNA
   <213> Vespula vulgaris
<400> 948
   tgcgttgcga ggtataaaag gattttatat attctaggag ttgtt 45
<210> 949
   <211> 45
   <212> DNA
   <213> Vespula vulgaris
<400> 949
   gtagacactg ttgttacagc tgtaggatgg ggtcttacga atttt 45
<210> 950
   <211> 45
   <212> DNA
   <213> Vespula vulgaris
<400> 950
   gtgaatgaat atcctatgat ggctggtata atacatcttg caacg 45
<210> 951
   <211> 45
   <212> DNA
   <213> Vespula vulgaris
<400> 951
   gaatatccta tgatggctgg tataatacat cttgcaacgc gtttt 45
<210> 952
   <211> 45
   <212> DNA
   <213> Vespula vulgaris
<400> 952
   tgcgtcaagt atcactttct ggctacacct aagcaacttt gtaca 45
<210> 953
   <211> 45
   <212> DNA
   <213> Vespula vulgaris
<400> 953
   ttaaaatatt ctatgagaat tggtccagct tgtcttccgt tctat 45
<210> 954
   <211> 45
   <212> DNA
   <213> Vespula vulgaris
<400> 954
   acatgggcaa ttaacgatac aaaggcaact caactttatc ttatt 45
<210> 955
   <211> 45
   <212> DNA
   <213> Vespula vulgaris
<400> 955
   gtaggatggg gtcttacgaa tttttatggt gtcaagtctg aagtt 45
<210> 956
   <211> 45
   <212> DNA
   <213> Vespula vulgaris
<400> 956
   atggctggta taatacatct tgcaacgcgt tttctctatt gcggt 45
<210> 957
   <211> 45
   <212> DNA
   <213> Vespula vulgaris
<400> 957
   tataaaagga ttttatatat tctaggagtt gttgttggag aacat 45
<210> 958
   <211> 45
   <212> DNA
   <213> Vespula vulgaris
<400> 958
   cttacgaatt tttatggtgt caagtctgaa gttttgagaa aagtc 45
<210> 959
   <211> 45
   <212> DNA
   <213> Vespula vulgaris
<400> 959
   gagtacgttt tctgtggatt aaattctttt gcagtggaat caata 45
<210> 960
   <211> 45
   <212> DNA
   <213> Vespula vulgaris
<400> 960
   ccaattttat ggcaaaatcc aaaaagcaaa cgtattttcc ttctt 45
<210> 961
   <211> 45
   <212> DNA
   <213> Vespula vulgaris
<400> 961
   gcaacgcgtt ttctctattg cggtgcaact ataataactc cgcaa 45
<210> 962
   <211> 45
   <212> DNA
   <213> Vespula vulgaris
<400> 962
   aattgtcggt ggagtgctag gagcaatact cgaattaaat tgaat 45
<210> 963
   <211> 45
   <212> DNA
   <213> Vespula vulgaris
<400> 963
   tcttttgcag tggaatcaat agcatcgaaa atgactataa aattc 45
<210> 964
   <211> 45
   <212> DNA
   <213> Vespula vulgaris
<400> 964
   aggccaaaat taaacgattt agctgttata aaattgcaga agagg 45
<210> 965
   <211> 45
   <212> DNA
   <213> Vespula vulgaris
<400> 965
   tataaaagga ttttatatat tctaggagtt gttgttggag aacat 45
<210> 966
   <211> 45
   <212> DNA
   <213> Vespula vulgaris
<400> 966
   gagtacgttt tctgtggatt aaattctttt gcagtggaat caata 45
<210> 967
   <211> 45
   <212> DNA
   <213> Vespula vulgaris
<400> 967
   cttacgaatt tttatggtgt caagtctgaa gttttgagaa aagtc 45
<210> 968
   <211> 45
   <212> DNA
   <213> Vespula vulgaris
<400> 968
   atggctggta taatacatct tgcaacgcgt tttctctatt gcggt 45
<210> 969
   <211> 45
   <212> DNA
   <213> Vespula vulgaris
<400> 969
   cgtattttcc ttcttggagt catcaattat ggaagaacat gtgcc 45
<210> 970
   <211> 45
   <212> DNA
   <213> Vespula vulgaris
<400> 970
   tcttttgcag tggaatcaat agcatcgaaa atgactataa aattc 45
<210> 971
   <211> 45
   <212> DNA
   <213> Vespula vulgaris
<400> 971
   tgtcttccgt tctattacat gcagcgaaac tttgtagaca ctgtt 45
<210> 972
   <211> 45
   <212> DNA
   <213> Vespula vulgaris
<400> 972
   gcaacgcgtt ttctctattg cggtgcaact ataataactc cgcaa 45
<210> 973
   <211> 45
   <212> DNA
   <213> Vespula vulgaris
<400> 973
   tttgtagaca ctgttgttac agctgtagga tggggtctta cgaat 45
<210> 974
   <211> 45
   <212> DNA
   <213> Vespula vulgaris
<400> 974
   gagtacgttt tctgtggatt aaattctttt gcagtggaat caata 45
<210> 975
   <211> 45
   <212> DNA
   <213> Vespula vulgaris
<400> 975
   cttacgaatt tttatggtgt caagtctgaa gttttgagaa aagtc 45
<210> 976
   <211> 45
   <212> DNA
   <213> Vespula vulgaris
<400> 976
   atggctggta taatacatct tgcaacgcgt tttctctatt gcggt 45
<210> 977
   <211> 45
   <212> DNA
   <213> Vespula vulgaris
<400> 977
   tataaaagga ttttatatat tctaggagtt gttgttggag aacat 45
<210> 978
   <211> 45
   <212> DNA
   <213> Vespula vulgaris
<400> 978
   gcgaggtata aaaggatttt atatattcta ggagttgttg ttgga 45
<210> 979
   <211> 45
   <212> DNA
   <213> Vespula vulgaris
<400> 979
   gcaacgcgtt ttctctattg cggtgcaact ataataactc cgcaa 45
<210> 980
   <211> 45
   <212> DNA
   <213> Vespula vulgaris
<400> 980
   tcttttgcag tggaatcaat agcatcgaaa atgactataa aattc 45
<210> 981
   <211> 45
   <212> DNA
   <213> Vespula vulgaris
<400> 981
   tgtcttccgt tctattacat gcagcgaaac tttgtagaca ctgtt 45
<210> 982
   <211> 45
   <212> DNA
   <213> Vespula vulgaris
<400> 982
   ccaattttat ggcaaaatcc aaaaagcaaa cgtattttcc ttctt 45
<210> 983
   <211> 45
   <212> DNA
   <213> Vespula vulgaris
<400> 983
   tgcgtcaagt atcactttct ggctacacct aagcaacttt gtaca 45
<210> 984
   <211> 45
   <212> DNA
   <213> Vespula vulgaris
<400> 984
   gtgaatgaat atcctatgat ggctggtata atacatcttg caacg 45
<210> 985
   <211> 45
   <212> DNA
   <213> Vespula vulgaris
<400> 985
   acatgggcaa ttaacgatac aaaggcaact caactttatc ttatt 45
<210> 986
   <211> 45
   <212> DNA
   <213> Vespula vulgaris
<400> 986
   catccgaatt ataggccaaa attaaacgat ttagctgtta taaaa 45
<210> 987
   <211> 45
   <212> DNA
   <213> Vespula vulgaris
<400> 987
   aattgtcggt ggagtgctag gagcaatact cgaattaaat tgaat 45
<210> 988
   <211> 45
   <212> DNA
   <213> Vespula vulgaris
<400> 988
   ggtgcaacta taataactcc gcaacatgta ttaacggctg ctcat 45
<210> 989
   <211> 45
   <212> DNA
   <213> Vespula vulgaris
<400> 989
   aggccaaaat taaacgattt agctgttata aaattgcaga agagg 45
<210> 990
   <211> 45
   <212> DNA
   <213> Vespula vulgaris
<400> 990
   gtaggatggg gtcttacgaa tttttatggt gtcaagtctg aagtt 45
<210> 991
   <211> 45
   <212> DNA
   <213> Vespula vulgaris
<400> 991
   tttgtagaca ctgttgttac agctgtagga tggggtctta cgaat 45
<210> 992
   <211> 45
   <212> DNA
   <213> Vespula vulgaris
<400> 992
   cagcgaaact ttgtagacac tgttgttaca gctgtaggat ggggt 45
<210> 993
   <211> 45
   <212> DNA
   <213> Vespula vulgaris
<400> 993
   tgtggattaa attcttttgc agtggaatca atagcatcga aaatg 45
<210> 994
   <211> 45
   <212> DNA
   <213> Vespula vulgaris
<400> 994
   ttaaaatatt ctatgagaat tggtccagct tgtcttccgt tctat 45
<210> 995
   <211> 45
   <212> DNA
   <213> Vespula vulgaris
<400> 995
   tataaaagga ttttatatat tctaggagtt gttgttggag aacat 45
<210> 996
   <211> 45
   <212> DNA
   <213> Vespula vulgaris
<400> 996
   cttacgaatt tttatggtgt caagtctgaa gttttgagaa aagtc 45
<210> 997
   <211> 45
   <212> DNA
   <213> Vespula vulgaris
<400> 997
   gagtacgttt tctgtggatt aaattctttt gcagtggaat caata 45
<210> 998
   <211> 45
   <212> DNA
   <213> Vespula vulgaris
<400> 998
   atggctggta taatacatct tgcaacgcgt tttctctatt gcggt 45
<210> 999
   <211> 45
   <212> DNA
   <213> Vespula vulgaris
<400> 999
   tcttttgcag tggaatcaat agcatcgaaa atgactataa aattc 45
<210> 1000
   <211> 45
   <212> DNA
   <213> Vespula vulgaris
<400> 1000
   tgtcttccgt tctattacat gcagcgaaac tttgtagaca ctgtt 45
<210> 1001
   <211> 45
   <212> DNA
   <213> Vespula vulgaris
<400> 1001
   cgtattttcc ttcttggagt catcaattat ggaagaacat gtgcc 45
<210> 1002
   <211> 45
   <212> DNA
   <213> Vespula vulgaris
<400> 1002
   gtgaatgaat atcctatgat ggctggtata atacatcttg caacg 45
<210> 1003
   <211> 45
   <212> DNA
   <213> Vespula vulgaris
<400> 1003
   gcaacgcgtt ttctctattg cggtgcaact ataataactc cgcaa 45
<210> 1004
   <211> 45
   <212> DNA
   <213> Vespula vulgaris
<400> 1004
   tgcgttgcga ggtataaaag gattttatat attctaggag ttgtt 45
<210> 1005
   <211> 45
   <212> DNA
   <213> Vespula vulgaris
<400> 1005
   gtagacactg ttgttacagc tgtaggatgg ggtcttacga atttt 45
<210> 1006
   <211> 45
   <212> DNA
   <213> Vespula vulgaris
<400> 1006
   gtaggatggg gtcttacgaa tttttatggt gtcaagtctg aagtt 45
<210> 1007
   <211> 45
   <212> DNA
   <213> Vespula vulgaris
<400> 1007
   gagtacgttt tctgtggatt aaattctttt gcagtggaat caata 45
<210> 1008
   <211> 45
   <212> DNA
   <213> Vespula vulgaris
<400> 1008
   cttacgaatt tttatggtgt caagtctgaa gttttgagaa aagtc 45
<210> 1009
   <211> 45
   <212> DNA
   <213> Vespula vulgaris
<400> 1009
   atggctggta taatacatct tgcaacgcgt tttctctatt gcggt 45
<210> 1010
   <211> 45
   <212> DNA
   <213> Vespula vulgaris
<400> 1010
   tataaaagga ttttatatat tctaggagtt gttgttggag aacat 45
<210> 1011
   <211> 45
   <212> DNA
   <213> Vespula vulgaris
<400> 1011
   gcgaggtata aaaggatttt atatattcta ggagttgttg ttgga 45
<210> 1012
   <211> 45
   <212> DNA
   <213> Vespula vulgaris
<400> 1012
   gcaacgcgtt ttctctattg cggtgcaact ataataactc cgcaa 45
<210> 1013
   <211> 45
   <212> DNA
   <213> Vespula vulgaris
<400> 1013
   tcttttgcag tggaatcaat agcatcgaaa atgactataa aattc 45
<210> 1014
   <211> 45
   <212> DNA
   <213> Vespula vulgaris
<400> 1014
   tgtcttccgt tctattacat gcagcgaaac tttgtagaca ctgtt 45
<210> 1015
   <211> 45
   <212> DNA
   <213> Vespula vulgaris
<400> 1015
   ccaattttat ggcaaaatcc aaaaagcaaa cgtattttcc ttctt 45
<210> 1016
   <211> 45
   <212> DNA
   <213> Vespula vulgaris
<400> 1016
   tgcgtcaagt atcactttct ggctacacct aagcaacttt gtaca 45
<210> 1017
   <211> 45
   <212> DNA
   <213> Vespula vulgaris
<400> 1017
   gtgaatgaat atcctatgat ggctggtata atacatcttg caacg 45
<210> 1018
   <211> 45
   <212> DNA
   <213> Vespula vulgaris
<400> 1018
   acatgggcaa ttaacgatac aaaggcaact caactttatc ttatt 45
<210> 1019
   <211> 45
   <212> DNA
   <213> Vespula vulgaris
<400> 1019
   catccgaatt ataggccaaa attaaacgat ttagctgtta taaaa 45
<210> 1020
   <211> 45
   <212> DNA
   <213> Vespula vulgaris
<400> 1020
   aattgtcggt ggagtgctag gagcaatact cgaattaaat tgaat 45
<210> 1021
   <211> 45
   <212> DNA
   <213> Vespula vulgaris
<400> 1021
   ggtgcaacta taataactcc gcaacatgta ttaacggctg ctcat 45
<210> 1022
   <211> 45
   <212> DNA
   <213> Vespula vulgaris
<400> 1022
   aggccaaaat taaacgattt agctgttata aaattgcaga agagg 45
<210> 1023
   <211> 45
   <212> DNA
   <213> Vespula vulgaris
<400> 1023
   gtaggatggg gtcttacgaa tttttatggt gtcaagtctg aagtt 45
<210> 1024
   <211> 45
   <212> DNA
   <213> Vespula vulgaris
<400> 1024
   tttgtagaca ctgttgttac agctgtagga tggggtctta cgaat 45
<210> 1025
   <211> 45
   <212> DNA
   <213> Vespula vulgaris
<400> 1025
   cagcgaaact ttgtagacac tgttgttaca gctgtaggat ggggt 45
<210> 1026
   <211> 45
   <212> DNA
   <213> Vespula vulgaris
<400> 1026
   tgtggattaa attcttttgc agtggaatca atagcatcga aaatg 45
<210> 1027
   <211> 45
   <212> DNA
   <213> Vespula vulgaris
<400> 1027
   ttaaaatatt ctatgagaat tggtccagct tgtcttccgt tctat 45
<210> 1028
   <211> 45
   <212> DNA
   <213> Vespula vulgaris
<400> 1028
   gagtacgttt tctgtggatt aaattctttt gcagtggaat caata 45
<210> 1029
   <211> 45
   <212> DNA
   <213> Vespula vulgaris
<400> 1029
   cttacgaatt tttatggtgt caagtctgaa gttttgagaa aagtc 45
<210> 1030
   <211> 45
   <212> DNA
   <213> Vespula vulgaris
<400> 1030
   atggctggta taatacatct tgcaacgcgt tttctctatt gcggt 45
<210> 1031
   <211> 45
   <212> DNA
   <213> Vespula vulgaris
<400> 1031
   tataaaagga ttttatatat tctaggagtt gttgttggag aacat 45
<210> 1032
   <211> 45
   <212> DNA
   <213> Vespula vulgaris
<400> 1032
   gcgaggtata aaaggatttt atatattcta ggagttgttg ttgga 45
<210> 1033
   <211> 45
   <212> DNA
   <213> Vespula vulgaris
<400> 1033
   gcaacgcgtt ttctctattg cggtgcaact ataataactc cgcaa 45
<210> 1034
   <211> 45
   <212> DNA
   <213> Vespula vulgaris
<400> 1034
   tcttttgcag tggaatcaat agcatcgaaa atgactataa aattc 45
<210> 1035
   <211> 45
   <212> DNA
   <213> Vespula vulgaris
<400> 1035
   tgtcttccgt tctattacat gcagcgaaac tttgtagaca ctgtt 45
<210> 1036
   <211> 45
   <212> DNA
   <213> Vespula vulgaris
<400> 1036
   ccaattttat ggcaaaatcc aaaaagcaaa cgtattttcc ttctt 45
<210> 1037
   <211> 45
   <212> DNA
   <213> Vespula vulgaris
<400> 1037
   tgcgtcaagt atcactttct ggctacacct aagcaacttt gtaca 45
<210> 1038
   <211> 45
   <212> DNA
   <213> Vespula vulgaris
<400> 1038
   gtgaatgaat atcctatgat ggctggtata atacatcttg caacg 45
<210> 1039
   <211> 45
   <212> DNA
   <213> Vespula vulgaris
<400> 1039
   acatgggcaa ttaacgatac aaaggcaact caactttatc ttatt 45
<210> 1040
   <211> 45
   <212> DNA
   <213> Vespula vulgaris
<400> 1040
   catccgaatt ataggccaaa attaaacgat ttagctgtta taaaa 45
<210> 1041
   <211> 45
   <212> DNA
   <213> Vespula vulgaris
<400> 1041
   aattgtcggt ggagtgctag gagcaatact cgaattaaat tgaat 45
<210> 1042
   <211> 45
   <212> DNA
   <213> Vespula vulgaris
<400> 1042
   ggtgcaacta taataactcc gcaacatgta ttaacggctg ctcat 45
<210> 1043
   <211> 45
   <212> DNA
   <213> Vespula vulgaris
<400> 1043
   aggccaaaat taaacgattt agctgttata aaattgcaga agagg 45
<210> 1044
   <211> 45
   <212> DNA
   <213> Vespula vulgaris
<400> 1044
   gtaggatggg gtcttacgaa tttttatggt gtcaagtctg aagtt 45
<210> 1045
   <211> 45
   <212> DNA
   <213> Vespula vulgaris
<400> 1045
   tttgtagaca ctgttgttac agctgtagga tggggtctta cgaat 45
<210> 1046
   <211> 45
   <212> DNA
   <213> Vespula vulgaris
<400> 1046
   cagcgaaact ttgtagacac tgttgttaca gctgtaggat ggggt 45
<210> 1047
   <211> 45
   <212> DNA
   <213> Vespula vulgaris
<400> 1047
   tgtggattaa attcttttgc agtggaatca atagcatcga aaatg 45
<210> 1048
   <211> 45
   <212> DNA
   <213> Vespula vulgaris
<400> 1048
   ttaaaatatt ctatgagaat tggtccagct tgtcttccgt tctat 45
<210> 1049
   <211> 45
   <212> DNA
   <213> Vespula vulgaris
<400> 1049
   gagtacgttt tctgtggatt aaattctttt gcagtggaat caata 45
<210> 1050
   <211> 45
   <212> DNA
   <213> Vespula vulgaris
<400> 1050
   cttacgaatt tttatggtgt caagtctgaa gttttgagaa aagtc 45
<210> 1051
   <211> 45
   <212> DNA
   <213> Vespula vulgaris
<400> 1051
   tataaaagga ttttatatat tctaggagtt gttgttggag aacat 45
<210> 1052
   <211> 45
   <212> DNA
   <213> Vespula vulgaris
<400> 1052
   atggctggta taatacatct tgcaacgcgt tttctctatt gcggt 45
<210> 1053
   <211> 45
   <212> DNA
   <213> Vespula vulgaris
<400> 1053
   ccaattttat ggcaaaatcc aaaaagcaaa cgtattttcc ttctt 45
<210> 1054
   <211> 45
   <212> DNA
   <213> Vespula vulgaris
<400> 1054
   tcttttgcag tggaatcaat agcatcgaaa atgactataa aattc 45
<210> 1055
   <211> 45
   <212> DNA
   <213> Vespula vulgaris
<400> 1055
   gcaacgcgtt ttctctattg cggtgcaact ataataactc cgcaa 45
<210> 1056
   <211> 45
   <212> DNA
   <213> Vespula vulgaris
<400> 1056
   tgtcttccgt tctattacat gcagcgaaac tttgtagaca ctgtt 45
<210> 1057
   <211> 45
   <212> DNA
   <213> Vespula vulgaris
<400> 1057
   gttgcgaggt ataaaaggat tttatatatt ctaggagttg ttgtt 45
<210> 1058
   <211> 45
   <212> DNA
   <213> Vespula vulgaris
<400> 1058
   gtgaatgaat atcctatgat ggctggtata atacatcttg caacg 45
<210> 1059
   <211> 45
   <212> DNA
   <213> Vespula vulgaris
<400> 1059
   aattgtcggt ggagtgctag gagcaatact cgaattaaat tgaat 45
<210> 1060
   <211> 45
   <212> DNA
   <213> Vespula vulgaris
<400> 1060
   tgcgtcaagt atcactttct ggctacacct aagcaacttt gtaca 45
<210> 1061
   <211> 45
   <212> DNA
   <213> Vespula vulgaris
<400> 1061
   catccgaatt ataggccaaa attaaacgat ttagctgtta taaaa 45
<210> 1062
   <211> 45
   <212> DNA
   <213> Vespula vulgaris
<400> 1062
   aggccaaaat taaacgattt agctgttata aaattgcaga agagg 45
<210> 1063
   <211> 45
   <212> DNA
   <213> Vespula vulgaris
<400> 1063
   gtaggatggg gtcttacgaa tttttatggt gtcaagtctg aagtt 45
<210> 1064
   <211> 45
   <212> DNA
   <213> Vespula vulgaris
<400> 1064
   acatgggcaa ttaacgatac aaaggcaact caactttatc ttatt 45
<210> 1065
   <211> 45
   <212> DNA
   <213> Vespula vulgaris
<400> 1065
   gagtacgttt tctgtggatt aaattctttt gcagtggaat caata 45
<210> 1066
   <211> 45
   <212> DNA
   <213> Vespula vulgaris
<400> 1066
   cttacgaatt tttatggtgt caagtctgaa gttttgagaa aagtc 45
<210> 1067
   <211> 45
   <212> DNA
   <213> Vespula vulgaris
<400> 1067
   tataaaagga ttttatatat tctaggagtt gttgttggag aacat 45
<210> 1068
   <211> 45
   <212> DNA
   <213> Vespula vulgaris
<400> 1068
   atggctggta taatacatct tgcaacgcgt tttctctatt gcggt 45
<210> 1069
   <211> 45
   <212> DNA
   <213> Vespula vulgaris
<400> 1069
   ccaattttat ggcaaaatcc aaaaagcaaa cgtattttcc ttctt 45
<210> 1070
   <211> 45
   <212> DNA
   <213> Vespula vulgaris
<400> 1070
   gcgaggtata aaaggatttt atatattcta ggagttgttg ttgga 45
<210> 1071
   <211> 45
   <212> DNA
   <213> Vespula vulgaris
<400> 1071
   gcaacgcgtt ttctctattg cggtgcaact ataataactc cgcaa 45
<210> 1072
   <211> 45
   <212> DNA
   <213> Vespula vulgaris
<400> 1072
   tcttttgcag tggaatcaat agcatcgaaa atgactataa aattc 45
<210> 1073
   <211> 45
   <212> DNA
   <213> Vespula vulgaris
<400> 1073
   tgtcttccgt tctattacat gcagcgaaac tttgtagaca ctgtt 45
<210> 1074
   <211> 45
   <212> DNA
   <213> Vespula vulgaris
<400> 1074
   tgcgtcaagt atcactttct ggctacacct aagcaacttt gtaca 45
<210> 1075
   <211> 45
   <212> DNA
   <213> Vespula vulgaris
<400> 1075
   aattgtcggt ggagtgctag gagcaatact cgaattaaat tgaat 45
<210> 1076
   <211> 45
   <212> DNA
   <213> Vespula vulgaris
<400> 1076
   catccgaatt ataggccaaa attaaacgat ttagctgtta taaaa 45
<210> 1077
   <211> 45
   <212> DNA
   <213> Vespula vulgaris
<400> 1077
   gtgaatgaat atcctatgat ggctggtata atacatcttg caacg 45
<210> 1078
   <211> 45
   <212> DNA
   <213> Vespula vulgaris
<400> 1078
   cagcgaaact ttgtagacac tgttgttaca gctgtaggat ggggt 45
<210> 1079
   <211> 45
   <212> DNA
   <213> Vespula vulgaris
<400> 1079
   acatgggcaa ttaacgatac aaaggcaact caactttatc ttatt 45
<210> 1080
   <211> 45
   <212> DNA
   <213> Vespula vulgaris
<400> 1080
   aggccaaaat taaacgattt agctgttata aaattgcaga agagg 45
<210> 1081
   <211> 45
   <212> DNA
   <213> Vespula vulgaris
<400> 1081
   gtaggatggg gtcttacgaa tttttatggt gtcaagtctg aagtt 45
<210> 1082
   <211> 45
   <212> DNA
   <213> Vespula vulgaris
<400> 1082
   gagtacgttt tctgtggatt aaattctttt gcagtggaat caata 45
<210> 1083
   <211> 45
   <212> DNA
   <213> Vespula vulgaris
<400> 1083
   cttacgaatt tttatggtgt caagtctgaa gttttgagaa aagtc 45
<210> 1084
   <211> 45
   <212> DNA
   <213> Vespula vulgaris
<400> 1084
   atggctggta taatacatct tgcaacgcgt tttctctatt gcggt 45
<210> 1085
   <211> 45
   <212> DNA
   <213> Vespula vulgaris
<400> 1085
   tataaaagga ttttatatat tctaggagtt gttgttggag aacat 45
<210> 1086
   <211> 45
   <212> DNA
   <213> Vespula vulgaris
<400> 1086
   ccaattttat ggcaaaatcc aaaaagcaaa cgtattttcc ttctt 45
<210> 1087
   <211> 45
   <212> DNA
   <213> Vespula vulgaris
<400> 1087
   gcaacgcgtt ttctctattg cggtgcaact ataataactc cgcaa 45
<210> 1088
   <211> 45
   <212> DNA
   <213> Vespula vulgaris
<400> 1088
   tcttttgcag tggaatcaat agcatcgaaa atgactataa aattc 45
<210> 1089
   <211> 45
   <212> DNA
   <213> Vespula vulgaris
<400> 1089
   tgtcttccgt tctattacat gcagcgaaac tttgtagaca ctgtt 45
<210> 1090
   <211> 45
   <212> DNA
   <213> Vespula vulgaris
<400> 1090
   gcgaggtata aaaggatttt atatattcta ggagttgttg ttgga 45
<210> 1091
   <211> 45
   <212> DNA
   <213> Vespula vulgaris
<400> 1091
   tgcgtcaagt atcactttct ggctacacct aagcaacttt gtaca 45
<210> 1092
   <211> 45
   <212> DNA
   <213> Vespula vulgaris
<400> 1092
   gtcaagtatc actttctggc tacacctaag caactttgta cattc 45
<210> 1093
   <211> 45
   <212> DNA
   <213> Vespula vulgaris
<400> 1093
   acatgggcaa ttaacgatac aaaggcaact caactttatc ttatt 45
<210> 1094
   <211> 45
   <212> DNA
   <213> Vespula vulgaris
<400> 1094
   cagcgaaact ttgtagacac tgttgttaca gctgtaggat ggggt 45
<210> 1095
   <211> 45
   <212> DNA
   <213> Vespula vulgaris
<400> 1095
   aattgtcggt ggagtgctag gagcaatact cgaattaaat tgaat 45
<210> 1096
   <211> 45
   <212> DNA
   <213> Vespula vulgaris
<400> 1096
   catccgaatt ataggccaaa attaaacgat ttagctgtta taaaa 45
<210> 1097
   <211> 45
   <212> DNA
   <213> Vespula vulgaris
<400> 1097
   gtgaatgaat atcctatgat ggctggtata atacatcttg caacg 45
<210> 1098
   <211> 45
   <212> DNA
   <213> Vespula vulgaris
<400> 1098
   ggtgcaacta taataactcc gcaacatgta ttaacggctg ctcat 45
<210> 1099
   <211> 45
   <212> DNA
   <213> Vespula vulgaris
<400> 1099
   gagtacgttt tctgtggatt aaattctttt gcagtggaat caata 45
<210> 1100
   <211> 45
   <212> DNA
   <213> Vespula vulgaris
<400> 1100
   cttacgaatt tttatggtgt caagtctgaa gttttgagaa aagtc 45
<210> 1101
   <211> 45
   <212> DNA
   <213> Vespula vulgaris
<400> 1101
   atggctggta taatacatct tgcaacgcgt tttctctatt gcggt 45
<210> 1102
   <211> 45
   <212> DNA
   <213> Vespula vulgaris
<400> 1102
   tataaaagga ttttatatat tctaggagtt gttgttggag aacat 45
<210> 1103
   <211> 45
   <212> DNA
   <213> Vespula vulgaris
<400> 1103
   gcgaggtata aaaggatttt atatattcta ggagttgttg ttgga 45
<210> 1104
   <211> 45
   <212> DNA
   <213> Vespula vulgaris
<400> 1104
   gcaacgcgtt ttctctattg cggtgcaact ataataactc cgcaa 45
<210> 1105
   <211> 45
   <212> DNA
   <213> Vespula vulgaris
<400> 1105
   tcttttgcag tggaatcaat agcatcgaaa atgactataa aattc 45
<210> 1106
   <211> 45
   <212> DNA
   <213> Vespula vulgaris
<400> 1106
   tgtcttccgt tctattacat gcagcgaaac tttgtagaca ctgtt 45
<210> 1107
   <211> 45
   <212> DNA
   <213> Vespula vulgaris
<400> 1107
   ccaattttat ggcaaaatcc aaaaagcaaa cgtattttcc ttctt 45
<210> 1108
   <211> 45
   <212> DNA
   <213> Vespula vulgaris
<400> 1108
   tgcgtcaagt atcactttct ggctacacct aagcaacttt gtaca 45
<210> 1109
   <211> 45
   <212> DNA
   <213> Vespula vulgaris
<400> 1109
   gtgaatgaat atcctatgat ggctggtata atacatcttg caacg 45
<210> 1110
   <211> 45
   <212> DNA
   <213> Vespula vulgaris
<400> 1110
   acatgggcaa ttaacgatac aaaggcaact caactttatc ttatt 45
<210> 1111
   <211> 45
   <212> DNA
   <213> Vespula vulgaris
<400> 1111
   catccgaatt ataggccaaa attaaacgat ttagctgtta taaaa 45
<210> 1112
   <211> 45
   <212> DNA
   <213> Vespula vulgaris
<400> 1112
   aattgtcggt ggagtgctag gagcaatact cgaattaaat tgaat 45
<210> 1113
   <211> 45
   <212> DNA
   <213> Vespula vulgaris
<400> 1113
   ggtgcaacta taataactcc gcaacatgta ttaacggctg ctcat 45
<210> 1114
   <211> 45
   <212> DNA
   <213> Vespula vulgaris
<400> 1114
   aggccaaaat taaacgattt agctgttata aaattgcaga agagg 45
<210> 1115
   <211> 45
   <212> DNA
   <213> Vespula vulgaris
<400> 1115
   gtaggatggg gtcttacgaa tttttatggt gtcaagtctg aagtt 45
<210> 1116
   <211> 45
   <212> DNA
   <213> Vespula vulgaris
<400> 1116
   tttgtagaca ctgttgttac agctgtagga tggggtctta cgaat 45
<210> 1117
   <211> 45
   <212> DNA
   <213> Vespula vulgaris
<400> 1117
   cagcgaaact ttgtagacac tgttgttaca gctgtaggat ggggt 45
<210> 1118
   <211> 45
   <212> DNA
   <213> Vespula vulgaris
<400> 1118
   tgtggattaa attcttttgc agtggaatca atagcatcga aaatg 45
<210> 1119
   <211> 45
   <212> DNA
   <213> Vespula vulgaris
<400> 1119
   ttaaaatatt ctatgagaat tggtccagct tgtcttccgt tctat 45
<210> 1120
   <211> 45
   <212> DNA
   <213> Vespula vulgaris
<400> 1120
   gagtacgttt tctgtggatt aaattctttt gcagtggaat caata 45
<210> 1121
   <211> 45
   <212> DNA
   <213> Vespula vulgaris
<400> 1121
   cttacgaatt tttatggtgt caagtctgaa gttttgagaa aagtc 45
<210> 1122
   <211> 45
   <212> DNA
   <213> Vespula vulgaris
<400> 1122
   tataaaagga ttttatatat tctaggagtt gttgttggag aacat 45
<210> 1123
   <211> 45
   <212> DNA
   <213> Vespula vulgaris
<400> 1123
   atggctggta taatacatct tgcaacgcgt tttctctatt gcggt 45
<210> 1124
   <211> 45
   <212> DNA
   <213> Vespula vulgaris
<400> 1124
   ccaattttat ggcaaaatcc aaaaagcaaa cgtattttcc ttctt 45
<210> 1125
   <211> 45
   <212> DNA
   <213> Vespula vulgaris
<400> 1125
   tcttttgcag tggaatcaat agcatcgaaa atgactataa aattc 45
<210> 1126
   <211> 45
   <212> DNA
   <213> Vespula vulgaris
<400> 1126
   tgcgtcaagt atcactttct ggctacacct aagcaacttt gtaca 45
<210> 1127
   <211> 45
   <212> DNA
   <213> Vespula vulgaris
<400> 1127
   tgtcttccgt tctattacat gcagcgaaac tttgtagaca ctgtt 45
<210> 1128
   <211> 45
   <212> DNA
   <213> Vespula vulgaris
<400> 1128
   cgtattttcc ttcttggagt catcaattat ggaagaacat gtgcc 45
<210> 1129
   <211> 45
   <212> DNA
   <213> Vespula vulgaris
<400> 1129
   cttccgttct attacatgca gcgaaacttt gtagacactg ttgtt 45
<210> 1130
   <211> 45
   <212> DNA
   <213> Vespula vulgaris
<400> 1130
   gttgcgaggt ataaaaggat tttatatatt ctaggagttg ttgtt 45
<210> 1131
   <211> 45
   <212> DNA
   <213> Vespula vulgaris
<400> 1131
   gcaacgcgtt ttctctattg cggtgcaact ataataactc cgcaa 45
<210> 1132
   <211> 45
   <212> DNA
   <213> Vespula vulgaris
<400> 1132
   gtgaatgaat atcctatgat ggctggtata atacatcttg caacg 45
<210> 1133
   <211> 45
   <212> DNA
   <213> Vespula vulgaris
<400> 1133
   cagcgaaact ttgtagacac tgttgttaca gctgtaggat ggggt 45
<210> 1134
   <211> 45
   <212> DNA
   <213> Vespula vulgaris
<400> 1134
   aattgtcggt ggagtgctag gagcaatact cgaattaaat tgaat 45
<210> 1135
   <211> 45
   <212> DNA
   <213> Vespula vulgaris
<400> 1135
   gtaggatggg gtcttacgaa tttttatggt gtcaagtctg aagtt 45
<210> 1136
   <211> 45
   <212> DNA
   <213> Vespula vulgaris
<400> 1136
   catccgaatt ataggccaaa attaaacgat ttagctgtta taaaa 45
<210> 1137
   <211> 45
   <212> DNA
   <213> Vespula vulgaris
<400> 1137
   acatgggcaa ttaacgatac aaaggcaact caactttatc ttatt 45
<210> 1138
   <211> 45
   <212> DNA
   <213> Vespula vulgaris
<400> 1138
   actataaaat tccattcaag atacaatact tatggaggca aattt 45
<210> 1139
   <211> 45
   <212> DNA
   <213> Vespula vulgaris
<400> 1139
   tttgtagaca ctgttgttac agctgtagga tggggtctta cgaat 45
<210> 1140
   <211> 45
   <212> DNA
   <213> Vespula vulgaris
<400> 1140
   tacacccaaa aattaaaatc agatgttaat tattgcgtgt ataat 45
<210> 1141
   <211> 45
   <212> DNA
   <213> Vespula vulgaris
<400> 1141
   ggtgcaacta taataactcc gcaacatgta ttaacggctg ctcat 45
<210> 1142
   <211> 45
   <212> DNA
   <213> Vespula vulgaris
<400> 1142
   aggttaaaat attctatgag aattggtcca gcttgtcttc cgttc 45
<210> 1143
   <211> 45
   <212> DNA
   <213> Vespula vulgaris
<400> 1143
   tataatcctg attttccgaa ttattacatg ggagaacata attgt 45
<210> 1144
   <211> 45
   <212> DNA
   <213> Vespula vulgaris
<400> 1144
   gagtacgttt tctgtggatt aaattctttt gcagtggaat caata 45
<210> 1145
   <211> 45
   <212> DNA
   <213> Vespula vulgaris
<400> 1145
   atggctggta taatacatct tgcaacgcgt tttctctatt gcggt 45
<210> 1146
   <211> 45
   <212> DNA
   <213> Vespula vulgaris
<400> 1146
   cttacgaatt tttatggtgt caagtctgaa gttttgagaa aagtc 45
<210> 1147
   <211> 45
   <212> DNA
   <213> Vespula vulgaris
<400> 1147
   tataaaagga ttttatatat tctaggagtt gttgttggag aacat 45
<210> 1148
   <211> 45
   <212> DNA
   <213> Vespula vulgaris
<400> 1148
   gcaacgcgtt ttctctattg cggtgcaact ataataactc cgcaa 45
<210> 1149
   <211> 45
   <212> DNA
   <213> Vespula vulgaris
<400> 1149
   tcttttgcag tggaatcaat agcatcgaaa atgactataa aattc 45
<210> 1150
   <211> 45
   <212> DNA
   <213> Vespula vulgaris
<400> 1150
   acatgggcaa ttaacgatac aaaggcaact caactttatc ttatt 45
<210> 1151
   <211> 45
   <212> DNA
   <213> Vespula vulgaris
<400> 1151
   tgtcttccgt tctattacat gcagcgaaac tttgtagaca ctgtt 45
<210> 1152
   <211> 45
   <212> DNA
   <213> Vespula vulgaris
<400> 1152
   ccaattttat ggcaaaatcc aaaaagcaaa cgtattttcc ttctt 45
<210> 1153
   <211> 45
   <212> DNA
   <213> Vespula vulgaris
<400> 1153
   tgcgtcaagt atcactttct ggctacacct aagcaacttt gtaca 45
<210> 1154
   <211> 45
   <212> DNA
   <213> Vespula vulgaris
<400> 1154
   tgtggattaa attcttttgc agtggaatca atagcatcga aaatg 45
<210> 1155
   <211> 45
   <212> DNA
   <213> Vespula vulgaris
<400> 1155
   cagcgaaact ttgtagacac tgttgttaca gctgtaggat ggggt 45
<210> 1156
   <211> 45
   <212> DNA
   <213> Vespula vulgaris
<400> 1156
   tttgtagaca ctgttgttac agctgtagga tggggtctta cgaat 45
<210> 1157
   <211> 45
   <212> DNA
   <213> Vespula vulgaris
<400> 1157
   aggccaaaat taaacgattt agctgttata aaattgcaga agagg 45
<210> 1158
   <211> 45
   <212> DNA
   <213> Vespula vulgaris
<400> 1158
   tgcgttgcga ggtataaaag gattttatat attctaggag ttgtt 45
<210> 1159
   <211> 45
   <212> DNA
   <213> Vespula vulgaris
<400> 1159
   aattgtcggt ggagtgctag gagcaatact cgaattaaat tgaat 45
<210> 1160
   <211> 45
   <212> DNA
   <213> Vespula vulgaris
<400> 1160
   gtgaatgaat atcctatgat ggctggtata atacatcttg caacg 45
<210> 1161
   <211> 45
   <212> DNA
   <213> Vespula vulgaris
<400> 1161
   actccgcaac atgtattaac ggctgctcat tgcgttgcga ggtat 45
<210> 1162
   <211> 45
   <212> DNA
   <213> Vespula vulgaris
<400> 1162
   ttaaaatatt ctatgagaat tggtccagct tgtcttccgt tctat 45
<210> 1163
   <211> 45
   <212> DNA
   <213> Vespula vulgaris
<400> 1163
   cttacgaatt tttatggtgt caagtctgaa gttttgagaa aagtc 45
<210> 1164
   <211> 45
   <212> DNA
   <213> Vespula vulgaris
<400> 1164
   gagtacgttt tctgtggatt aaattctttt gcagtggaat caata 45
<210> 1165
   <211> 45
   <212> DNA
   <213> Vespula vulgaris
<400> 1165
   tataaaagga ttttatatat tctaggagtt gttgttggag aacat 45
<210> 1166
   <211> 45
   <212> DNA
   <213> Vespula vulgaris
<400> 1166
   atggctggta taatacatct tgcaacgcgt tttctctatt gcggt 45
<210> 1167
   <211> 45
   <212> DNA
   <213> Vespula vulgaris
<400> 1167
   ccaattttat ggcaaaatcc aaaaagcaaa cgtattttcc ttctt 45
<210> 1168
   <211> 45
   <212> DNA
   <213> Vespula vulgaris
<400> 1168
   tcttttgcag tggaatcaat agcatcgaaa atgactataa aattc 45
<210> 1169
   <211> 45
   <212> DNA
   <213> Vespula vulgaris
<400> 1169
   gcaacgcgtt ttctctattg cggtgcaact ataataactc cgcaa 45
<210> 1170
   <211> 45
   <212> DNA
   <213> Vespula vulgaris
<400> 1170
   aattgtcggt ggagtgctag gagcaatact cgaattaaat tgaat 45
<210> 1171
   <211> 45
   <212> DNA
   <213> Vespula vulgaris
<400> 1171
   tgtcttccgt tctattacat gcagcgaaac tttgtagaca ctgtt 45
<210> 1172
   <211> 45
   <212> DNA
   <213> Vespula vulgaris
<400> 1172
   tgcgttgcga ggtataaaag gattttatat attctaggag ttgtt 45
<210> 1173
   <211> 45
   <212> DNA
   <213> Vespula vulgaris
<400> 1173
   gtgaatgaat atcctatgat ggctggtata atacatcttg caacg 45
<210> 1174
   <211> 45
   <212> DNA
   <213> Vespula vulgaris
<400> 1174
   aggccaaaat taaacgattt agctgttata aaattgcaga agagg 45
<210> 1175
   <211> 45
   <212> DNA
   <213> Vespula vulgaris
<400> 1175
   catccgaatt ataggccaaa attaaacgat ttagctgtta taaaa 45
<210> 1176
   <211> 45
   <212> DNA
   <213> Vespula vulgaris
<400> 1176
   acatgggcaa ttaacgatac aaaggcaact caactttatc ttatt 45
<210> 1177
   <211> 45
   <212> DNA
   <213> Vespula vulgaris
<400> 1177
   gtaggatggg gtcttacgaa tttttatggt gtcaagtctg aagtt 45
<210> 1178
   <211> 45
   <212> DNA
   <213> Vespula vulgaris
<400> 1178
   ccaattttat ggcaaaatcc aaaaagcaaa cgtattttcc ttctt 45
<210> 1179
   <211> 45
   <212> DNA
   <213> Vespula vulgaris
<400> 1179
   cttacgaatt tttatggtgt caagtctgaa gttttgagaa aagtc 45
<210> 1180
   <211> 45
   <212> DNA
   <213> Vespula vulgaris
<400> 1180
   gagtacgttt tctgtggatt aaattctttt gcagtggaat caata 45
<210> 1181
   <211> 45
   <212> DNA
   <213> Vespula vulgaris
<400> 1181
   tacacccaaa aattaaaatc agatgttaat tattgcgtgt ataat 45
<210> 1182
   <211> 45
   <212> DNA
   <213> Vespula vulgaris
<400> 1182
   aattgtcggt ggagtgctag gagcaatact cgaattaaat tgaat 45
<210> 1183
   <211> 45
   <212> DNA
   <213> Vespula vulgaris
<400> 1183
   gagtacgttt tctgtggatt aaattctttt gcagtggaat caata 45
<210> 1184
   <211> 45
   <212> DNA
   <213> Vespula vulgaris
<400> 1184
   cttacgaatt tttatggtgt caagtctgaa gttttgagaa aagtc 45
<210> 1185
   <211> 45
   <212> DNA
   <213> Vespula vulgaris
<400> 1185
   atggctggta taatacatct tgcaacgcgt tttctctatt gcggt 45
<210> 1186
   <211> 45
   <212> DNA
   <213> Vespula vulgaris
<400> 1186
   tataaaagga ttttatatat tctaggagtt gttgttggag aacat 45
<210> 1187
   <211> 45
   <212> DNA
   <213> Vespula vulgaris
<400> 1187
   ccaattttat ggcaaaatcc aaaaagcaaa cgtattttcc ttctt 45
<210> 1188
   <211> 45
   <212> DNA
   <213> Vespula vulgaris
<400> 1188
   gcgaggtata aaaggatttt atatattcta ggagttgttg ttgga 45
<210> 1189
   <211> 45
   <212> DNA
   <213> Vespula vulgaris
<400> 1189
   tcttttgcag tggaatcaat agcatcgaaa atgactataa aattc 45
<210> 1190
   <211> 45
   <212> DNA
   <213> Vespula vulgaris
<400> 1190
   tgtcttccgt tctattacat gcagcgaaac tttgtagaca ctgtt 45
<210> 1191
   <211> 45
   <212> DNA
   <213> Vespula vulgaris
<400> 1191
   gcaacgcgtt ttctctattg cggtgcaact ataataactc cgcaa 45
<210> 1192
   <211> 45
   <212> DNA
   <213> Vespula vulgaris
<400> 1192
   acatgggcaa ttaacgatac aaaggcaact caactttatc ttatt 45
<210> 1193
   <211> 45
   <212> DNA
   <213> Vespula vulgaris
<400> 1193
   aattgtcggt ggagtgctag gagcaatact cgaattaaat tgaat 45
<210> 1194
   <211> 45
   <212> DNA
   <213> Vespula vulgaris
<400> 1194
   tgcgtcaagt atcactttct ggctacacct aagcaacttt gtaca 45
<210> 1195
   <211> 45
   <212> DNA
   <213> Vespula vulgaris
<400> 1195
   catccgaatt ataggccaaa attaaacgat ttagctgtta taaaa 45
<210> 1196
   <211> 45
   <212> DNA
   <213> Vespula vulgaris
<400> 1196
   gtgaatgaat atcctatgat ggctggtata atacatcttg caacg 45
<210> 1197
   <211> 45
   <212> DNA
   <213> Vespula vulgaris
<400> 1197
   aggccaaaat taaacgattt agctgttata aaattgcaga agagg 45
<210> 1198
   <211> 45
   <212> DNA
   <213> Vespula vulgaris
<400> 1198
   cagcgaaact ttgtagacac tgttgttaca gctgtaggat ggggt 45
<210> 1199
   <211> 45
   <212> DNA
   <213> Vespula vulgaris
<400> 1199
   tgtggattaa attcttttgc agtggaatca atagcatcga aaatg 45
<210> 1200
   <211> 45
   <212> DNA
   <213> Vespula vulgaris
<400> 1200
   ggtgcaacta taataactcc gcaacatgta ttaacggctg ctcat 45
<210> 1201
   <211> 45
   <212> DNA
   <213> Vespula vulgaris
<400> 1201
   gtaggatggg gtcttacgaa tttttatggt gtcaagtctg aagtt 45
<210> 1202
   <211> 45
   <212> DNA
   <213> Vespula vulgaris
<400> 1202
   tttgtagaca ctgttgttac agctgtagga tggggtctta cgaat 45
<210> 1203
   <211> 45
   <212> DNA
   <213> Vespula vulgaris
<400> 1203
   ttaaaatatt ctatgagaat tggtccagct tgtcttccgt tctat 45
<210> 1204
   <211> 45
   <212> DNA
   <213> Vespula vulgaris
<400> 1204
   actccgcaac atgtattaac ggctgctcat tgcgttgcga ggtat 45
<210> 1205
   <211> 45
   <212> DNA
   <213> Vespula vulgaris
<400> 1205
   aggttaaaat attctatgag aattggtcca gcttgtcttc cgttc 45
<210> 1206
   <211> 45
   <212> DNA
   <213> Vespula vulgaris
<400> 1206
   gagtacgttt tctgtggatt aaattctttt gcagtggaat caata 45
<210> 1207
   <211> 45
   <212> DNA
   <213> Vespula vulgaris
<400> 1207
   cttacgaatt tttatggtgt caagtctgaa gttttgagaa aagtc 45
<210> 1208
   <211> 45
   <212> DNA
   <213> Vespula vulgaris
<400> 1208
   atggctggta taatacatct tgcaacgcgt tttctctatt gcggt 45
<210> 1209
   <211> 45
   <212> DNA
   <213> Vespula vulgaris
<400> 1209
   tataaaagga ttttatatat tctaggagtt gttgttggag aacat 45
<210> 1210
   <211> 45
   <212> DNA
   <213> Vespula vulgaris
<400> 1210
   tgtcttccgt tctattacat gcagcgaaac tttgtagaca ctgtt 45
<210> 1211
   <211> 45
   <212> DNA
   <213> Vespula vulgaris
<400> 1211
   gcaacgcgtt ttctctattg cggtgcaact ataataactc cgcaa 45
<210> 1212
   <211> 45
   <212> DNA
   <213> Vespula vulgaris
<400> 1212
   gttgcgaggt ataaaaggat tttatatatt ctaggagttg ttgtt 45
<210> 1213
   <211> 45
   <212> DNA
   <213> Vespula vulgaris
<400> 1213
   tgcgtcaagt atcactttct ggctacacct aagcaacttt gtaca 45
<210> 1214
   <211> 45
   <212> DNA
   <213> Vespula vulgaris
<400> 1214
   tcttttgcag tggaatcaat agcatcgaaa atgactataa aattc 45
<210> 1215
   <211> 45
   <212> DNA
   <213> Vespula vulgaris
<400> 1215
   gtgaatgaat atcctatgat ggctggtata atacatcttg caacg 45
<210> 1216
   <211> 45
   <212> DNA
   <213> Vespula vulgaris
<400> 1216
   ccaattttat ggcaaaatcc aaaaagcaaa cgtattttcc ttctt 45
<210> 1217
   <211> 45
   <212> DNA
   <213> Vespula vulgaris
<400> 1217
   gtaggatggg gtcttacgaa tttttatggt gtcaagtctg aagtt 45
<210> 1218
   <211> 45
   <212> DNA
   <213> Vespula vulgaris
<400> 1218
   aggccaaaat taaacgattt agctgttata aaattgcaga agagg 45
<210> 1219
   <211> 45
   <212> DNA
   <213> Vespula vulgaris
<400> 1219
   acatgggcaa ttaacgatac aaaggcaact caactttatc ttatt 45
<210> 1220
   <211> 45
   <212> DNA
   <213> Vespula vulgaris
<400> 1220
   ggtgcaacta taataactcc gcaacatgta ttaacggctg ctcat 45
<210> 1221
   <211> 45
   <212> DNA
   <213> Vespula vulgaris
<400> 1221
   gagtacgttt tctgtggatt aaattctttt gcagtggaat caata 45
<210> 1222
   <211> 45
   <212> DNA
   <213> Vespula vulgaris
<400> 1222
   cttacgaatt tttatggtgt caagtctgaa gttttgagaa aagtc 45
<210> 1223
   <211> 45
   <212> DNA
   <213> Vespula vulgaris
<400> 1223
   atggctggta taatacatct tgcaacgcgt tttctctatt gcggt 45
<210> 1224
   <211> 45
   <212> DNA
   <213> Vespula vulgaris
<400> 1224
   tataaaagga ttttatatat tctaggagtt gttgttggag aacat 45
<210> 1225
   <211> 45
   <212> DNA
   <213> Vespula vulgaris
<400> 1225
   gcgaggtata aaaggatttt atatattcta ggagttgttg ttgga 45
<210> 1226
   <211> 45
   <212> DNA
   <213> Vespula vulgaris
<400> 1226
   gcaacgcgtt ttctctattg cggtgcaact ataataactc cgcaa 45
<210> 1227
   <211> 45
   <212> DNA
   <213> Vespula vulgaris
<400> 1227
   tcttttgcag tggaatcaat agcatcgaaa atgactataa aattc 45
<210> 1228
   <211> 45
   <212> DNA
   <213> Vespula vulgaris
<400> 1228
   tgtcttccgt tctattacat gcagcgaaac tttgtagaca ctgtt 45
<210> 1229
   <211> 45
   <212> DNA
   <213> Vespula vulgaris
<400> 1229
   ccaattttat ggcaaaatcc aaaaagcaaa cgtattttcc ttctt 45
<210> 1230
   <211> 45
   <212> DNA
   <213> Vespula vulgaris
<400> 1230
   tgcgtcaagt atcactttct ggctacacct aagcaacttt gtaca 45
<210> 1231
   <211> 45
   <212> DNA
   <213> Vespula vulgaris
<400> 1231
   gtgaatgaat atcctatgat ggctggtata atacatcttg caacg 45
<210> 1232
   <211> 45
   <212> DNA
   <213> Vespula vulgaris
<400> 1232
   acatgggcaa ttaacgatac aaaggcaact caactttatc ttatt 45
<210> 1233
   <211> 45
   <212> DNA
   <213> Vespula vulgaris
<400> 1233
   catccgaatt ataggccaaa attaaacgat ttagctgtta taaaa 45
<210> 1234
   <211> 45
   <212> DNA
   <213> Vespula vulgaris
<400> 1234
   aattgtcggt ggagtgctag gagcaatact cgaattaaat tgaat 45
<210> 1235
   <211> 45
   <212> DNA
   <213> Vespula vulgaris
<400> 1235
   ggtgcaacta taataactcc gcaacatgta ttaacggctg ctcat 45
<210> 1236
   <211> 45
   <212> DNA
   <213> Vespula vulgaris
<400> 1236
   aggccaaaat taaacgattt agctgttata aaattgcaga agagg 45
<210> 1237
   <211> 45
   <212> DNA
   <213> Vespula vulgaris
<400> 1237
   gtaggatggg gtcttacgaa tttttatggt gtcaagtctg aagtt 45
<210> 1238
   <211> 45
   <212> DNA
   <213> Vespula vulgaris
<400> 1238
   tttgtagaca ctgttgttac agctgtagga tggggtctta cgaat 45
<210> 1239
   <211> 45
   <212> DNA
   <213> Vespula vulgaris
<400> 1239
   cagcgaaact ttgtagacac tgttgttaca gctgtaggat ggggt 45
<210> 1240
   <211> 45
   <212> DNA
   <213> Vespula vulgaris
<400> 1240
   tgtggattaa attcttttgc agtggaatca atagcatcga aaatg 45
<210> 1241
   <211> 45
   <212> DNA
   <213> Vespula vulgaris
<400> 1241
   ttaaaatatt ctatgagaat tggtccagct tgtcttccgt tctat 45
<210> 1242
   <211> 15
   <212> PRT
   <213> Vespula vulgaris
<400> 1242
   Glu Tyr Val Phe Cys Gly Leu Asn Ser Phe Ala Val Glu Ser Ile
   1 5 10 15
<210> 1243
   <211> 15
   <212> PRT
   <213> Vespula vulgaris
<400> 1243
   Leu Thr Asn Phe Tyr Gly Val Lys Ser Glu Val Leu Arg Lys Val
   1 5 10 15
<210> 1244
   <211> 15
   <212> PRT
   <213> Vespula vulgaris
<400> 1244
   Met Ala Gly Ile Ile His Leu Ala Thr Arg Phe Leu Tyr Cys Gly
   1 5 10 15
<210> 1245
   <211> 15
   <212> PRT
   <213> Vespula vulgaris
<400> 1245
   Tyr Lys Arg Ile Leu Tyr Ile Leu Gly Val Val Val Gly Glu His
   1 5 10 15
<210> 1246
   <211> 15
   <212> PRT
   <213> Vespula vulgaris
<400> 1246
   Ala Arg Tyr Lys Arg Ile Leu Tyr Ile Leu Gly Val Val Val Gly
   1 5 10 15
<210> 1247
   <211> 15
   <212> PRT
   <213> Vespula vulgaris
<400> 1247
   Ala Thr Arg Phe Leu Tyr Cys Gly Ala Thr Ile Ile Thr Pro Gln
   1 5 10 15
<210> 1248
   <211> 15
   <212> PRT
   <213> Vespula vulgaris
<400> 1248
   Ser Phe Ala Val Glu Ser Ile Ala Ser Lys Met Thr Ile Lys Phe
   1 5 10 15
<210> 1249
   <211> 15
   <212> PRT
   <213> Vespula vulgaris
<400> 1249
   Cys Leu Pro Phe Tyr Tyr Met Gln Arg Asn Phe Val Asp Thr Val
   1 5 10 15
<210> 1250
   <211> 15
   <212> PRT
   <213> Vespula vulgaris
<400> 1250
   Pro Ile Leu Trp Gln Asn Pro Lys Ser Lys Arg Ile Phe Leu Leu
   1 5 10 15
<210> 1251
   <211> 15
   <212> PRT
   <213> Vespula vulgaris
<400> 1251
   Cys Val Lys Tyr His Phe Leu Ala Thr Pro Lys Gln Leu Cys Thr
   1 5 10 15
<210> 1252
   <211> 15
   <212> PRT
   <213> Vespula vulgaris
<400> 1252
   Val Asn Glu Tyr Pro Met Met Ala Gly Ile Ile His Leu Ala Thr
   1 5 10 15
<210> 1253
   <211> 15
   <212> PRT
   <213> Vespula vulgaris
<400> 1253
   Thr Trp Ala Ile Asn Asp Thr Lys Ala Thr Gln Leu Tyr Leu Ile
   1 5 10 15
<210> 1254
   <211> 15
   <212> PRT
   <213> Vespula vulgaris
<400> 1254
   His Pro Asn Tyr Arg Pro Lys Leu Asn Asp Leu Ala Val Ile Lys
   1 5 10 15
<210> 1255
   <211> 15
   <212> PRT
   <213> Vespula vulgaris
<400> 1255
   Asn Cys Arg Trp Ser Ala Arg Ser Asn Thr Arg Ile Lys Leu Asn
   1 5 10 15
<210> 1256
   <211> 15
   <212> PRT
   <213> Vespula vulgaris
<400> 1256
   Gly Ala Thr Ile Ile Thr Pro Gln His Val Leu Thr Ala Ala His
   1 5 10 15
<210> 1257
   <211> 15
   <212> PRT
   <213> Vespula vulgaris
<400> 1257
   Arg Pro Lys Leu Asn Asp Leu Ala Val Ile Lys Leu Gln Lys Arg
   1 5 10 15
<210> 1258
   <211> 15
   <212> PRT
   <213> Vespula vulgaris
<400> 1258
   Val Gly Trp Gly Leu Thr Asn Phe Tyr Gly Val Lys Ser Glu Val
   1 5 10 15
<210> 1259
   <211> 15
   <212> PRT
   <213> Vespula vulgaris
<400> 1259
   Phe Val Asp Thr Val Val Thr Ala Val Gly Trp Gly Leu Thr Asn
   1 5 10 15
<210> 1260
   <211> 15
   <212> PRT
   <213> Vespula vulgaris
<400> 1260
   Gln Arg Asn Phe Val Asp Thr Val Val Thr Ala Val Gly Trp Gly
   1 5 10 15
<210> 1261
   <211> 15
   <212> PRT
   <213> Vespula vulgaris
<400> 1261
   Cys Gly Leu Asn Ser Phe Ala Val Glu Ser Ile Ala Ser Lys Met
   1 5 10 15
<210> 1262
   <211> 15
   <212> PRT
   <213> Vespula vulgaris
<400> 1262
   Leu Lys Tyr Ser Met Arg Ile Gly Pro Ala Cys Leu Pro Phe Tyr
   1 5 10 15
<210> 1263
   <211> 15
   <212> PRT
   <213> Vespula vulgaris
<400> 1263
   Glu Tyr Val Phe Cys Gly Leu Asn Ser Phe Ala Val Glu Ser Ile
   1 5 10 15
<210> 1264
   <211> 15
   <212> PRT
   <213> Vespula vulgaris
<400> 1264
   Tyr Lys Arg Ile Leu Tyr Ile Leu Gly Val Val Val Gly Glu His
   1 5 10 15
<210> 1265
   <211> 15
   <212> PRT
   <213> Vespula vulgaris
<400> 1265
   Met Ala Gly Ile Ile His Leu Ala Thr Arg Phe Leu Tyr Cys Gly
   1 5 10 15
<210> 1266
   <211> 15
   <212> PRT
   <213> Vespula vulgaris
<400> 1266
   Leu Thr Asn Phe Tyr Gly Val Lys Ser Glu Val Leu Arg Lys Val
   1 5 10 15
<210> 1267
   <211> 15
   <212> PRT
   <213> Vespula vulgaris
<400> 1267
   Cys Leu Pro Phe Tyr Tyr Met Gln Arg Asn Phe Val Asp Thr Val
   1 5 10 15
<210> 1268
   <211> 15
   <212> PRT
   <213> Vespula vulgaris
<400> 1268
   Arg Ile Phe Leu Leu Gly Val Ile Asn Tyr Gly Arg Thr Cys Ala
   1 5 10 15
<210> 1269
   <211> 15
   <212> PRT
   <213> Vespula vulgaris
<400> 1269
   Ser Phe Ala Val Glu Ser Ile Ala Ser Lys Met Thr Ile Lys Phe
   1 5 10 15
<210> 1270
   <211> 15
   <212> PRT
   <213> Vespula vulgaris
<400> 1270
   Ala Thr Arg Phe Leu Tyr Cys Gly Ala Thr Ile Ile Thr Pro Gln
   1 5 10 15
<210> 1271
   <211> 15
   <212> PRT
   <213> Vespula vulgaris
<400> 1271
   Cys Val Ala Arg Tyr Lys Arg Ile Leu Tyr Ile Leu Gly Val Val
   1 5 10 15
<210> 1272
   <211> 15
   <212> PRT
   <213> Vespula vulgaris
<400> 1272
   Val Asp Thr Val Val Thr Ala Val Gly Trp Gly Leu Thr Asn Phe
   1 5 10 15
<210> 1273
   <211> 15
   <212> PRT
   <213> Vespula vulgaris
<400> 1273
   Val Asn Glu Tyr Pro Met Met Ala Gly Ile Ile His Leu Ala Thr
   1 5 10 15
<210> 1274
   <211> 15
   <212> PRT
   <213> Vespula vulgaris
<400> 1274
   Glu Tyr Pro Met Met Ala Gly Ile Ile His Leu Ala Thr Arg Phe
   1 5 10 15
<210> 1275
   <211> 15
   <212> PRT
   <213> Vespula vulgaris
<400> 1275
   Cys Val Lys Tyr His Phe Leu Ala Thr Pro Lys Gln Leu Cys Thr
   1 5 10 15
<210> 1276
   <211> 15
   <212> PRT
   <213> Vespula vulgaris
<400> 1276
   Leu Lys Tyr Ser Met Arg Ile Gly Pro Ala Cys Leu Pro Phe Tyr
   1 5 10 15
<210> 1277
   <211> 15
   <212> PRT
   <213> Vespula vulgaris
<400> 1277
   Thr Trp Ala Ile Asn Asp Thr Lys Ala Thr Gln Leu Tyr Leu Ile
   1 5 10 15
<210> 1278
   <211> 15
   <212> PRT
   <213> Vespula vulgaris
<400> 1278
   Val Gly Trp Gly Leu Thr Asn Phe Tyr Gly Val Lys Ser Glu Val
   1 5 10 15
<210> 1279
   <211> 15
   <212> PRT
   <213> Vespula vulgaris
<400> 1279
   Met Ala Gly Ile Ile His Leu Ala Thr Arg Phe Leu Tyr Cys Gly
   1 5 10 15
<210> 1280
   <211> 15
   <212> PRT
   <213> Vespula vulgaris
<400> 1280
   Tyr Lys Arg Ile Leu Tyr Ile Leu Gly Val Val Val Gly Glu His
   1 5 10 15
<210> 1281
   <211> 15
   <212> PRT
   <213> Vespula vulgaris
<400> 1281
   Leu Thr Asn Phe Tyr Gly Val Lys Ser Glu Val Leu Arg Lys Val
   1 5 10 15
<210> 1282
   <211> 15
   <212> PRT
   <213> Vespula vulgaris
<400> 1282
   Glu Tyr Val Phe Cys Gly Leu Asn Ser Phe Ala Val Glu Ser Ile
   1 5 10 15
<210> 1283
   <211> 15
   <212> PRT
   <213> Vespula vulgaris
<400> 1283
   Pro Ile Leu Trp Gln Asn Pro Lys Ser Lys Arg Ile Phe Leu Leu
   1 5 10 15
<210> 1284
   <211> 15
   <212> PRT
   <213> Vespula vulgaris
<400> 1284
   Ala Thr Arg Phe Leu Tyr Cys Gly Ala Thr Ile Ile Thr Pro Gln
   1 5 10 15
<210> 1285
   <211> 15
   <212> PRT
   <213> Vespula vulgaris
<400> 1285
   Asn Cys Arg Trp Ser Ala Arg Ser Asn Thr Arg Ile Lys Leu Asn
   1 5 10 15
<210> 1286
   <211> 15
   <212> PRT
   <213> Vespula vulgaris
<400> 1286
   Ser Phe Ala Val Glu Ser Ile Ala Ser Lys Met Thr Ile Lys Phe
   1 5 10 15
<210> 1287
   <211> 15
   <212> PRT
   <213> Vespula vulgaris
<400> 1287
   Arg Pro Lys Leu Asn Asp Leu Ala Val Ile Lys Leu Gln Lys Arg
   1 5 10 15
<210> 1288
   <211> 15
   <212> PRT
   <213> Vespula vulgaris
<400> 1288
   Tyr Lys Arg Ile Leu Tyr Ile Leu Gly Val Val Val Gly Glu His
   1 5 10 15
<210> 1289
   <211> 15
   <212> PRT
   <213> Vespula vulgaris
<400> 1289
   Glu Tyr Val Phe Cys Gly Leu Asn Ser Phe Ala Val Glu Ser Ile
   1 5 10 15
<210> 1290
   <211> 15
   <212> PRT
   <213> Vespula vulgaris
<400> 1290
   Leu Thr Asn Phe Tyr Gly Val Lys Ser Glu Val Leu Arg Lys Val
   1 5 10 15
<210> 1291
   <211> 15
   <212> PRT
   <213> Vespula vulgaris
<400> 1291
   Met Ala Gly Ile Ile His Leu Ala Thr Arg Phe Leu Tyr Cys Gly
   1 5 10 15
<210> 1292
   <211> 15
   <212> PRT
   <213> Vespula vulgaris
<400> 1292
   Arg Ile Phe Leu Leu Gly Val Ile Asn Tyr Gly Arg Thr Cys Ala
   1 5 10 15
<210> 1293
   <211> 15
   <212> PRT
   <213> Vespula vulgaris
<400> 1293
   Ser Phe Ala Val Glu Ser Ile Ala Ser Lys Met Thr Ile Lys Phe
   1 5 10 15
<210> 1294
   <211> 15
   <212> PRT
   <213> Vespula vulgaris
<400> 1294
   Cys Leu Pro Phe Tyr Tyr Met Gln Arg Asn Phe Val Asp Thr Val
   1               5                   10                  15
<210> 1295
   <211> 15
   <212> PRT
   <213> Vespula vulgaris
<400> 1295
   Ala Thr Arg Phe Leu Tyr Cys Gly Ala Thr Ile Ile Thr Pro Gln
   1               5                   10                  15
<210> 1296
   <211> 15
   <212> PRT
   <213> Vespula vulgaris
<400> 1296
   Phe Val Asp Thr Val Val Thr Ala Val Gly Trp Gly Leu Thr Asn
   1               5                   10                  15
<210> 1297
   <211> 15
   <212> PRT
   <213> Vespula vulgaris
<400> 1297
   Glu Tyr Val Phe Cys Gly Leu Asn Ser Phe Ala Val Glu Ser Ile
   1               5                   10                  15
<210> 1298
   <211> 15
   <212> PRT
   <213> Vespula vulgaris
<400> 1298
   Leu Thr Asn Phe Tyr Gly Val Lys Ser Glu Val Leu Arg Lys Val
   1               5                   10                  15
<210> 1299
   <211> 15
   <212> PRT
   <213> Vespula vulgaris
<400> 1299
   Met Ala Gly Ile Ile His Leu Ala Thr Arg Phe Leu Tyr Cys Gly
   1 5 10 15
<210> 1300
   <211> 15
   <212> PRT
   <213> Vespula vulgaris
<400> 1300
   Tyr Lys Arg Ile Leu Tyr Ile Leu Gly Val Val Val Gly Glu His
   1 5 10 15
<210> 1301
   <211> 15
   <212> PRT
   <213> Vespula vulgaris
<400> 1301
   Ala Arg Tyr Lys Arg Ile Leu Tyr Ile Leu Gly Val Val Val Gly
   1 5 10 15
<210> 1302
   <211> 15
   <212> PRT
   <213> Vespula vulgaris
<400> 1302
   Ala Thr Arg Phe Leu Tyr Cys Gly Ala Thr Ile Ile Thr Pro Gln
   1 5 10 15
<210> 1303
   <211> 15
   <212> PRT
   <213> Vespula vulgaris
<400> 1303
   Ser Phe Ala Val Glu Ser Ile Ala Ser Lys Met Thr Ile Lys Phe
   1 5 10 15
<210> 1304
   <211> 15
   <212> PRT
   <213> Vespula vulgaris
<400> 1304
   Cys Leu Pro Phe Tyr Tyr Met Gln Arg Asn Phe Val Asp Thr Val
   1 5 10 15
<210> 1305
   <211> 15
   <212> PRT
   <213> Vespula vulgaris
<400> 1305
   Pro Ile Leu Trp Gln Asn Pro Lys Ser Lys Arg Ile Phe Leu Leu
   1 5 10 15
<210> 1306
   <211> 15
   <212> PRT
   <213> Vespula vulgaris
<400> 1306
   Cys Val Lys Tyr His Phe Leu Ala Thr Pro Lys Gln Leu Cys Thr
   1 5 10 15
<210> 1307
   <211> 15
   <212> PRT
   <213> Vespula vulgaris
<400> 1307
   Val Asn Glu Tyr Pro Met Met Ala Gly Ile Ile His Leu Ala Thr
   1 5 10 15
<210> 1308
   <211> 15
   <212> PRT
   <213> Vespula vulgaris
<400> 1308
   Thr Trp Ala Ile Asn Asp Thr Lys Ala Thr Gln Leu Tyr Leu Ile
   1 5 10 15
<210> 1309
   <211> 15
   <212> PRT
   <213> Vespula vulgaris
<400> 1309
   His Pro Asn Tyr Arg Pro Lys Leu Asn Asp Leu Ala Val Ile Lys
   1 5 10 15
<210> 1310
   <211> 15
   <212> PRT
   <213> Vespula vulgaris
<400> 1310
   Asn Cys Arg Trp Ser Ala Arg Ser Asn Thr Arg Ile Lys Leu Asn
   1 5 10 15
<210> 1311
   <211> 15
   <212> PRT
   <213> Vespula vulgaris
<400> 1311
   Gly Ala Thr Ile Ile Thr Pro Gln His Val Leu Thr Ala Ala His
   1 5 10 15
<210> 1312
   <211> 15
   <212> PRT
   <213> Vespula vulgaris
<400> 1312
   Arg Pro Lys Leu Asn Asp Leu Ala Val Ile Lys Leu Gln Lys Arg
   1 5 10 15
<210> 1313
   <211> 15
   <212> PRT
   <213> Vespula vulgaris
<400> 1313
   Val Gly Trp Gly Leu Thr Asn Phe Tyr Gly Val Lys Ser Glu Val
   1 5 10 15
<210> 1314
   <211> 15
   <212> PRT
   <213> Vespula vulgaris
<400> 1314
   Phe Val Asp Thr Val Val Thr Ala Val Gly Trp Gly Leu Thr Asn
   1 5 10 15
<210> 1315
   <211> 15
   <212> PRT
   <213> Vespula vulgaris
<400> 1315
   Gln Arg Asn Phe Val Asp Thr Val Val Thr Ala Val Gly Trp Gly
   1 5 10 15
<210> 1316
   <211> 15
   <212> PRT
   <213> Vespula vulgaris
<400> 1316
   Cys Gly Leu Asn Ser Phe Ala Val Glu Ser Ile Ala Ser Lys Met
   1 5 10 15
<210> 1317
   <211> 15
   <212> PRT
   <213> Vespula vulgaris
<400> 1317
   Leu Lys Tyr Ser Met Arg Ile Gly Pro Ala Cys Leu Pro Phe Tyr
   1 5 10 15
<210> 1318
   <211> 15
   <212> PRT
   <213> Vespula vulgaris
<400> 1318
   Tyr Lys Arg Ile Leu Tyr Ile Leu Gly Val Val Val Gly Glu His
   1 5 10 15
<210> 1319
   <211> 15
   <212> PRT
   <213> Vespula vulgaris
<400> 1319
   Leu Thr Asn Phe Tyr Gly Val Lys Ser Glu Val Leu Arg Lys Val
   1 5 10 15
<210> 1320
   <211> 15
   <212> PRT
   <213> Vespula vulgaris
<400> 1320
   Glu Tyr Val Phe Cys Gly Leu Asn Ser Phe Ala Val Glu Ser Ile
   1 5 10 15
<210> 1321
   <211> 15
   <212> PRT
   <213> Vespula vulgaris
<400> 1321
   Met Ala Gly Ile Ile His Leu Ala Thr Arg Phe Leu Tyr Cys Gly
   1 5 10 15
<210> 1322
   <211> 15
   <212> PRT
   <213> Vespula vulgaris
<400> 1322
   Ser Phe Ala Val Glu Ser Ile Ala Ser Lys Met Thr Ile Lys Phe
   1               5                   10                  15
<210> 1323
   <211> 15
   <212> PRT
   <213> Vespula vulgaris
<400> 1323
   Cys Leu Pro Phe Tyr Tyr Met Gln Arg Asn Phe Val Asp Thr Val
   1               5                   10                  15
<210> 1324
   <211> 15
   <212> PRT
   <213> Vespula vulgaris
<400> 1324
   Arg Ile Phe Leu Leu Gly Val Ile Asn Tyr Gly Arg Thr Cys Ala
   1               5                   10                  15
<210> 1325
   <211> 15
   <212> PRT
   <213> Vespula vulgaris
<400> 1325
   Val Asn Glu Tyr Pro Met Met Ala Gly Ile Ile His Leu Ala Thr
   1               5                   10                  15
<210> 1326
   <211> 15
   <212> PRT
   <213> Vespula vulgaris
<400> 1326
   Ala Thr Arg Phe Leu Tyr Cys Gly Ala Thr Ile Ile Thr Pro Gln
   1               5                   10                  15
<210> 1327
   <211> 15
   <212> PRT
   <213> Vespula vulgaris
<400> 1327
   Cys Val Ala Arg Tyr Lys Arg Ile Leu Tyr Ile Leu Gly Val Val
   1               5                   10                  15
<210> 1328
   <211> 15
   <212> PRT
   <213> Vespula vulgaris
<400> 1328
   Val Asp Thr Val Val Thr Ala Val Gly Trp Gly Leu Thr Asn Phe
   1 5 10 15
<210> 1329
   <211> 15
   <212> PRT
   <213> Vespula vulgaris
<400> 1329
   Val Gly Trp Gly Leu Thr Asn Phe Tyr Gly Val Lys Ser Glu Val
   1 5 10 15
<210> 1330
   <211> 15
   <212> PRT
   <213> Vespula vulgaris
<400> 1330
   Glu Tyr Val Phe Cys Gly Leu Asn Ser Phe Ala Val Glu Ser Ile
   1 5 10 15
<210> 1331
   <211> 15
   <212> PRT
   <213> Vespula vulgaris
<400> 1331
   Leu Thr Asn Phe Tyr Gly Val Lys Ser Glu Val Leu Arg Lys Val
   1 5 10 15
<210> 1332
   <211> 15
   <212> PRT
   <213> Vespula vulgaris
<400> 1332
   Met Ala Gly Ile Ile His Leu Ala Thr Arg Phe Leu Tyr Cys Gly
   1 5 10 15
<210> 1333
   <211> 15
   <212> PRT
   <213> Vespula vulgaris
<400> 1333
   Tyr Lys Arg Ile Leu Tyr Ile Leu Gly Val Val Val Gly Glu His
   1 5 10 15
<210> 1334
   <211> 15
   <212> PRT
   <213> Vespula vulgaris
<400> 1334
   Ala Arg Tyr Lys Arg Ile Leu Tyr Ile Leu Gly Val Val Val Gly
   1 5 10 15
<210> 1335
   <211> 15
   <212> PRT
   <213> Vespula vulgaris
<400> 1335
   Ala Thr Arg Phe Leu Tyr Cys Gly Ala Thr Ile Ile Thr Pro Gln
   1 5 10 15
<210> 1336
   <211> 15
   <212> PRT
   <213> Vespula vulgaris
<400> 1336
   Ser Phe Ala Val Glu Ser Ile Ala Ser Lys Met Thr Ile Lys Phe
   1 5 10 15
<210> 1337
   <211> 15
   <212> PRT
   <213> Vespula vulgaris
<400> 1337
   Cys Leu Pro Phe Tyr Tyr Met Gln Arg Asn Phe Val Asp Thr Val
   1 5 10 15
<210> 1338
   <211> 15
   <212> PRT
   <213> Vespula vulgaris
<400> 1338
   Pro Ile Leu Trp Gln Asn Pro Lys Ser Lys Arg Ile Phe Leu Leu
   1 5 10 15
<210> 1339
   <211> 15
   <212> PRT
   <213> Vespula vulgaris
<400> 1339
   Cys Val Lys Tyr His Phe Leu Ala Thr Pro Lys Gln Leu Cys Thr
   1 5 10 15
<210> 1340
   <211> 15
   <212> PRT
   <213> Vespula vulgaris
<400> 1340
   Val Asn Glu Tyr Pro Met Met Ala Gly Ile Ile His Leu Ala Thr
   1               5                   10                  15
<210> 1341
   <211> 15
   <212> PRT
   <213> Vespula vulgaris
<400> 1341
   Thr Trp Ala Ile Asn Asp Thr Lys Ala Thr Gln Leu Tyr Leu Ile
   1               5                   10                  15
<210> 1342
   <211> 15
   <212> PRT
   <213> Vespula vulgaris
<400> 1342
   His Pro Asn Tyr Arg Pro Lys Leu Asn Asp Leu Ala Val Ile Lys
   1               5                   10                  15
<210> 1343
   <211> 15
   <212> PRT
   <213> Vespula vulgaris
<400> 1343
   Asn Cys Arg Trp Ser Ala Arg Ser Asn Thr Arg Ile Lys Leu Asn
   1               5                   10                  15
<210> 1344
   <211> 15
   <212> PRT
   <213> Vespula vulgaris
<400> 1344
   Gly Ala Thr Ile Ile Thr Pro Gln His Val Leu Thr Ala Ala His
   1               5                   10                  15
<210> 1345
   <211> 15
   <212> PRT
   <213> Vespula vulgaris
<400> 1345
   Arg Pro Lys Leu Asn Asp Leu Ala Val Ile Lys Leu Gln Lys Arg
   1 5 10 15
<210> 1346
   <211> 15
   <212> PRT
   <213> Vespula vulgaris
<400> 1346
   Val Gly Trp Gly Leu Thr Asn Phe Tyr Gly Val Lys Ser Glu Val
   1 5 10 15
<210> 1347
   <211> 15
   <212> PRT
   <213> Vespula vulgaris
<400> 1347
   Phe Val Asp Thr Val Val Thr Ala Val Gly Trp Gly Leu Thr Asn
   1 5 10 15
<210> 1348
   <211> 15
   <212> PRT
   <213> Vespula vulgaris
<400> 1348
   Gln Arg Asn Phe Val Asp Thr Val Val Thr Ala Val Gly Trp Gly
   1 5 10 15
<210> 1349
   <211> 15
   <212> PRT
   <213> Vespula vulgaris
<400> 1349
   Cys Gly Leu Asn Ser Phe Ala Val Glu Ser Ile Ala Ser Lys Met
   1 5 10 15
<210> 1350
   <211> 15
   <212> PRT
   <213> Vespula vulgaris
<400> 1350
   Leu Lys Tyr Ser Met Arg Ile Gly Pro Ala Cys Leu Pro Phe Tyr
   1 5 10 15
<210> 1351
   <211> 15
   <212> PRT
   <213> Vespula vulgaris
<400> 1351
   Glu Tyr Val Phe Cys Gly Leu Asn Ser Phe Ala Val Glu Ser Ile
   1 5 10 15
<210> 1352
   <211> 15
   <212> PRT
   <213> Vespula vulgaris
<400> 1352
   Leu Thr Asn Phe Tyr Gly Val Lys Ser Glu Val Leu Arg Lys Val
   1 5 10 15
<210> 1353
   <211> 15
   <212> PRT
   <213> Vespula vulgaris
<400> 1353
   Met Ala Gly Ile Ile His Leu Ala Thr Arg Phe Leu Tyr Cys Gly
   1 5 10 15
<210> 1354
   <211> 15
   <212> PRT
   <213> Vespula vulgaris
<400> 1354
   Tyr Lys Arg Ile Leu Tyr Ile Leu Gly Val Val Val Gly Glu His
   1 5 10 15
<210> 1355
   <211> 15
   <212> PRT
   <213> Vespula vulgaris
<400> 1355
   Ala Arg Tyr Lys Arg Ile Leu Tyr Ile Leu Gly Val Val Val Gly
   1 5 10 15
<210> 1356
   <211> 15
   <212> PRT
   <213> Vespula vulgaris
<400> 1356
   Ala Thr Arg Phe Leu Tyr Cys Gly Ala Thr Ile Ile Thr Pro Gln
   1 5 10 15
<210> 1357
   <211> 15
   <212> PRT
   <213> Vespula vulgaris
<400> 1357
   Ser Phe Ala Val Glu Ser Ile Ala Ser Lys Met Thr Ile Lys Phe
   1 5 10 15
<210> 1358
   <211> 15
   <212> PRT
   <213> Vespula vulgaris
<400> 1358
   Cys Leu Pro Phe Tyr Tyr Met Gln Arg Asn Phe Val Asp Thr Val
   1 5 10 15
<210> 1359
   <211> 15
   <212> PRT
   <213> Vespula vulgaris
<400> 1359
   Pro Ile Leu Trp Gln Asn Pro Lys Ser Lys Arg Ile Phe Leu Leu
   1 5 10 15
<210> 1360
   <211> 15
   <212> PRT
   <213> Vespula vulgaris
<400> 1360
   Cys Val Lys Tyr His Phe Leu Ala Thr Pro Lys Gln Leu Cys Thr
   1 5 10 15
<210> 1361
   <211> 15
   <212> PRT
   <213> Vespula vulgaris
<400> 1361
   Val Asn Glu Tyr Pro Met Met Ala Gly Ile Ile His Leu Ala Thr
   1 5 10 15
<210> 1362
   <211> 15
   <212> PRT
   <213> Vespula vulgaris
<400> 1362
   Thr Trp Ala Ile Asn Asp Thr Lys Ala Thr Gln Leu Tyr Leu Ile
   1 5 10 15
<210> 1363
   <211> 15
   <212> PRT
   <213> Vespula vulgaris
<400> 1363
   His Pro Asn Tyr Arg Pro Lys Leu Asn Asp Leu Ala Val Ile Lys
   1 5 10 15
<210> 1364
   <211> 15
   <212> PRT
   <213> Vespula vulgaris
<400> 1364
   Asn Cys Arg Trp Ser Ala Arg Ser Asn Thr Arg Ile Lys Leu Asn
   1 5 10 15
<210> 1365
   <211> 15
   <212> PRT
   <213> Vespula vulgaris
<400> 1365
   Gly Ala Thr Ile Ile Thr Pro Gln His Val Leu Thr Ala Ala His
   1 5 10 15
<210> 1366
   <211> 15
   <212> PRT
   <213> Vespula vulgaris
<400> 1366
   Arg Pro Lys Leu Asn Asp Leu Ala Val Ile Lys Leu Gln Lys Arg
   1 5 10 15
<210> 1367
   <211> 15
   <212> PRT
   <213> Vespula vulgaris
<400> 1367
   Val Gly Trp Gly Leu Thr Asn Phe Tyr Gly Val Lys Ser Glu Val
   1 5 10 15
<210> 1368
   <211> 15
   <212> PRT
   <213> Vespula vulgaris
<400> 1368
   Phe Val Asp Thr Val Val Thr Ala Val Gly Trp Gly Leu Thr Asn
   1               5                   10                  15
<210> 1369
   <211> 15
   <212> PRT
   <213> Vespula vulgaris
<400> 1369
   Gln Arg Asn Phe Val Asp Thr Val Val Thr Ala Val Gly Trp Gly
   1               5                   10                  15
<210> 1370
   <211> 15
   <212> PRT
   <213> Vespula vulgaris
<400> 1370
   Cys Gly Leu Asn Ser Phe Ala Val Glu Ser Ile Ala Ser Lys Met
   1               5                   10                  15
<210> 1371
   <211> 15
   <212> PRT
   <213> Vespula vulgaris
<400> 1371
   Leu Lys Tyr Ser Met Arg Ile Gly Pro Ala Cys Leu Pro Phe Tyr
   1               5                   10                  15
<210> 1372
   <211> 15
   <212> PRT
   <213> Vespula vulgaris
<400> 1372
   Glu Tyr Val Phe Cys Gly Leu Asn Ser Phe Ala Val Glu Ser Ile
   1               5                   10                  15
<210> 1373
   <211> 15
   <212> PRT
   <213> Vespula vulgaris
<400> 1373
   Leu Thr Asn Phe Tyr Gly Val Lys Ser Glu Val Leu Arg Lys Val
   1               5                   10                  15
<210> 1374
   <211> 15
   <212> PRT
   <213> Vespula vulgaris
<400> 1374
   Tyr Lys Arg Ile Leu Tyr Ile Leu Gly Val Val Val Gly Glu His
   1 5 10 15
<210> 1375
   <211> 15
   <212> PRT
   <213> Vespula vulgaris
<400> 1375
   Met Ala Gly Ile Ile His Leu Ala Thr Arg Phe Leu Tyr Cys Gly
   1 5 10 15
<210> 1376
   <211> 15
   <212> PRT
   <213> Vespula vulgaris
<400> 1376
   Pro Ile Leu Trp Gln Asn Pro Lys Ser Lys Arg Ile Phe Leu Leu
   1 5 10 15
<210> 1377
   <211> 15
   <212> PRT
   <213> Vespula vulgaris
<400> 1377
   Ser Phe Ala Val Glu Ser Ile Ala Ser Lys Met Thr Ile Lys Phe
   1 5 10 15
<210> 1378
   <211> 15
   <212> PRT
   <213> Vespula vulgaris
<400> 1378
   Ala Thr Arg Phe Leu Tyr Cys Gly Ala Thr Ile Ile Thr Pro Gln
   1 5 10 15
<210> 1379
   <211> 15
   <212> PRT
   <213> Vespula vulgaris
<400> 1379
   Cys Leu Pro Phe Tyr Tyr Met Gln Arg Asn Phe Val Asp Thr Val
   1 5 10 15
<210> 1380
   <211> 15
   <212> PRT
   <213> Vespula vulgaris
<400> 1380
   Val Ala Arg Tyr Lys Arg Ile Leu Tyr Ile Leu Gly Val Val Val
   1 5 10 15
<210> 1381
   <211> 15
   <212> PRT
   <213> Vespula vulgaris
<400> 1381
   Val Asn Glu Tyr Pro Met Met Ala Gly Ile Ile His Leu Ala Thr
   1 5 10 15
<210> 1382
   <211> 15
   <212> PRT
   <213> Vespula vulgaris
<400> 1382
   Asn Cys Arg Trp Ser Ala Arg Ser Asn Thr Arg Ile Lys Leu Asn
   1 5 10 15
<210> 1383
   <211> 15
   <212> PRT
   <213> Vespula vulgaris
<400> 1383
   Cys Val Lys Tyr His Phe Leu Ala Thr Pro Lys Gln Leu Cys Thr
   1 5 10 15
<210> 1384
   <211> 15
   <212> PRT
   <213> Vespula vulgaris
<400> 1384
   His Pro Asn Tyr Arg Pro Lys Leu Asn Asp Leu Ala Val Ile Lys
   1 5 10 15
<210> 1385
   <211> 15
   <212> PRT
   <213> Vespula vulgaris
<400> 1385
   Arg Pro Lys Leu Asn Asp Leu Ala Val Ile Lys Leu Gln Lys Arg
   1 5 10 15
<210> 1386
   <211> 15
   <212> PRT
   <213> Vespula vulgaris
<400> 1386
   Val Gly Trp Gly Leu Thr Asn Phe Tyr Gly Val Lys Ser Glu Val
   1 5 10 15
<210> 1387
   <211> 15
   <212> PRT
   <213> Vespula vulgaris
<400> 1387
   Thr Trp Ala Ile Asn Asp Thr Lys Ala Thr Gln Leu Tyr Leu Ile
   1 5 10 15
<210> 1388
   <211> 15
   <212> PRT
   <213> Vespula vulgaris
<400> 1388
   Glu Tyr Val Phe Cys Gly Leu Asn Ser Phe Ala Val Glu Ser Ile
   1 5 10 15
<210> 1389
   <211> 15
   <212> PRT
   <213> Vespula vulgaris
<400> 1389
   Leu Thr Asn Phe Tyr Gly Val Lys Ser Glu Val Leu Arg Lys Val
   1 5 10 15
<210> 1390
   <211> 15
   <212> PRT
   <213> Vespula vulgaris
<400> 1390
   Tyr Lys Arg Ile Leu Tyr Ile Leu Gly Val Val Val Gly Glu His
   1 5 10 15
<210> 1391
   <211> 15
   <212> PRT
   <213> Vespula vulgaris
<400> 1391
   Met Ala Gly Ile Ile His Leu Ala Thr Arg Phe Leu Tyr Cys Gly
   1 5 10 15
<210> 1392
   <211> 15
   <212> PRT
   <213> Vespula vulgaris
<400> 1392
   Pro Ile Leu Trp Gln Asn Pro Lys Ser Lys Arg Ile Phe Leu Leu
   1 5 10 15
<210> 1393
   <211> 15
   <212> PRT
   <213> Vespula vulgaris
<400> 1393
   Ala Arg Tyr Lys Arg Ile Leu Tyr Ile Leu Gly Val Val Val Gly
   1 5 10 15
<210> 1394
   <211> 15
   <212> PRT
   <213> Vespula vulgaris
<400> 1394
   Ala Thr Arg Phe Leu Tyr Cys Gly Ala Thr Ile Ile Thr Pro Gln
   1 5 10 15
<210> 1395
   <211> 15
   <212> PRT
   <213> Vespula vulgaris
<400> 1395
   Ser Phe Ala Val Glu Ser Ile Ala Ser Lys Met Thr Ile Lys Phe
   1 5 10 15
<210> 1396
   <211> 15
   <212> PRT
   <213> Vespula vulgaris
<400> 1396
   Cys Leu Pro Phe Tyr Tyr Met Gln Arg Asn Phe Val Asp Thr Val
   1 5 10 15
<210> 1397
   <211> 15
   <212> PRT
   <213> Vespula vulgaris
<400> 1397
   Cys Val Lys Tyr His Phe Leu Ala Thr Pro Lys Gln Leu Cys Thr
   1               5                   10                  15
<210> 1398
   <211> 15
   <212> PRT
   <213> Vespula vulgaris
<400> 1398
   Asn Cys Arg Trp Ser Ala Arg Ser Asn Thr Arg Ile Lys Leu Asn
   1               5                   10                  15
<210> 1399
   <211> 15
   <212> PRT
   <213> Vespula vulgaris
<400> 1399
   His Pro Asn Tyr Arg Pro Lys Leu Asn Asp Leu Ala Val Ile Lys
   1               5                   10                  15
<210> 1400
   <211> 15
   <212> PRT
   <213> Vespula vulgaris
<400> 1400
   Val Asn Glu Tyr Pro Met Met Ala Gly Ile Ile His Leu Ala Thr
   1               5                   10                  15
<210> 1401
   <211> 15
   <212> PRT
   <213> Vespula vulgaris
<400> 1401
   Gln Arg Asn Phe Val Asp Thr Val Val Thr Ala Val Gly Trp Gly
   1               5                   10                  15
<210> 1402
   <211> 15
   <212> PRT
   <213> Vespula vulgaris
<400> 1402
   Thr Trp Ala Ile Asn Asp Thr Lys Ala Thr Gln Leu Tyr Leu Ile
   1               5                   10                  15
<210> 1403
   <211> 15
   <212> PRT
   <213> Vespula vulgaris
<400> 1403
   Arg Pro Lys Leu Asn Asp Leu Ala Val Ile Lys Leu Gln Lys Arg
   1 5 10 15
<210> 1404
   <211> 15
   <212> PRT
   <213> Vespula vulgaris
<400> 1404
   Val Gly Trp Gly Leu Thr Asn Phe Tyr Gly Val Lys Ser Glu Val
   1 5 10 15
<210> 1405
   <211> 15
   <212> PRT
   <213> Vespula vulgaris
<400> 1405
   Glu Tyr Val Phe Cys Gly Leu Asn Ser Phe Ala Val Glu Ser Ile
   1 5 10 15
<210> 1406
   <211> 15
   <212> PRT
   <213> Vespula vulgaris
<400> 1406
   Leu Thr Asn Phe Tyr Gly Val Lys Ser Glu Val Leu Arg Lys Val
   1 5 10 15
<210> 1407
   <211> 15
   <212> PRT
   <213> Vespula vulgaris
<400> 1407
   Met Ala Gly Ile Ile His Leu Ala Thr Arg Phe Leu Tyr Cys Gly
   1 5 10 15
<210> 1408
   <211> 15
   <212> PRT
   <213> Vespula vulgaris
<400> 1408
   Tyr Lys Arg Ile Leu Tyr Ile Leu Gly Val Val Val Gly Glu His
   1 5 10 15
<210> 1409
   <211> 15
   <212> PRT
   <213> Vespula vulgaris
<400> 1409
   Pro Ile Leu Trp Gln Asn Pro Lys Ser Lys Arg Ile Phe Leu Leu
   1               5                   10                  15
<210> 1410
   <211> 15
   <212> PRT
   <213> Vespula vulgaris
<400> 1410
   Ala Thr Arg Phe Leu Tyr Cys Gly Ala Thr Ile Ile Thr Pro Gln
   1               5                   10                  15
<210> 1411
   <211> 15
   <212> PRT
   <213> Vespula vulgaris
<400> 1411
   Ser Phe Ala Val Glu Ser Ile Ala Ser Lys Met Thr Ile Lys Phe
   1               5                   10                  15
<210> 1412
   <211> 15
   <212> PRT
   <213> Vespula vulgaris
<400> 1412
   Cys Leu Pro Phe Tyr Tyr Met Gln Arg Asn Phe Val Asp Thr Val
   1               5                   10                  15
<210> 1413
   <211> 15
   <212> PRT
   <213> Vespula vulgaris
<400> 1413
   Ala Arg Tyr Lys Arg Ile Leu Tyr Ile Leu Gly Val Val Val Gly
   1               5                   10                  15
<210> 1414
   <211> 15
   <212> PRT
   <213> Vespula vulgaris
<400> 1414
   Cys Val Lys Tyr His Phe Leu Ala Thr Pro Lys Gln Leu Cys Thr
   1               5                   10                  15
<210> 1415
   <211> 15
   <212> PRT
   <213> Vespula vulgaris
<400> 1415
   Val Lys Tyr His Phe Leu Ala Thr Pro Lys Gln Leu Cys Thr Phe
   1 5 10 15
<210> 1416
   <211> 15
   <212> PRT
   <213> Vespula vulgaris
<400> 1416
   Thr Trp Ala Ile Asn Asp Thr Lys Ala Thr Gln Leu Tyr Leu Ile
   1 5 10 15
<210> 1417
   <211> 15
   <212> PRT
   <213> Vespula vulgaris
<400> 1417
   Gln Arg Asn Phe Val Asp Thr Val Val Thr Ala Val Gly Trp Gly
   1 5 10 15
<210> 1418
   <211> 15
   <212> PRT
   <213> Vespula vulgaris
<400> 1418
   Asn Cys Arg Trp Ser Ala Arg Ser Asn Thr Arg Ile Lys Leu Asn
   1 5 10 15
<210> 1419
   <211> 15
   <212> PRT
   <213> Vespula vulgaris
<400> 1419
   His Pro Asn Tyr Arg Pro Lys Leu Asn Asp Leu Ala Val Ile Lys
   1 5 10 15
<210> 1420
   <211> 15
   <212> PRT
   <213> Vespula vulgaris
<400> 1420
   Val Asn Glu Tyr Pro Met Met Ala Gly Ile Ile His Leu Ala Thr
   1 5 10 15
<210> 1421
   <211> 15
   <212> PRT
   <213> Vespula vulgaris
<400> 1421
   Gly Ala Thr Ile Ile Thr Pro Gln His Val Leu Thr Ala Ala His
   1 5 10 15
<210> 1422
   <211> 15
   <212> PRT
   <213> Vespula vulgaris
<400> 1422
   Glu Tyr Val Phe Cys Gly Leu Asn Ser Phe Ala Val Glu Ser Ile
   1 5 10 15
<210> 1423
   <211> 15
   <212> PRT
   <213> Vespula vulgaris
<400> 1423
   Leu Thr Asn Phe Tyr Gly Val Lys Ser Glu Val Leu Arg Lys Val
   1 5 10 15
<210> 1424
   <211> 15
   <212> PRT
   <213> Vespula vulgaris
<400> 1424
   Met Ala Gly Ile Ile His Leu Ala Thr Arg Phe Leu Tyr Cys Gly
   1 5 10 15
<210> 1425
   <211> 15
   <212> PRT
   <213> Vespula vulgaris
<400> 1425
   Tyr Lys Arg Ile Leu Tyr Ile Leu Gly Val Val Val Gly Glu His
   1 5 10 15
<210> 1426
   <211> 15
   <212> PRT
   <213> Vespula vulgaris
<400> 1426
   Ala Arg Tyr Lys Arg Ile Leu Tyr Ile Leu Gly Val Val Val Gly
   1 5 10 15
<210> 1427
   <211> 15
   <212> PRT
   <213> Vespula vulgaris
<400> 1427
   Ala Thr Arg Phe Leu Tyr Cys Gly Ala Thr Ile Ile Thr Pro Gln
   1 5 10 15
<210> 1428
   <211> 15
   <212> PRT
   <213> Vespula vulgaris
<400> 1428
   Ser Phe Ala Val Glu Ser Ile Ala Ser Lys Met Thr Ile Lys Phe
   1 5 10 15
<210> 1429
   <211> 15
   <212> PRT
   <213> Vespula vulgaris
<400> 1429
   Cys Leu Pro Phe Tyr Tyr Met Gln Arg Asn Phe Val Asp Thr Val
   1 5 10 15
<210> 1430
   <211> 15
   <212> PRT
   <213> Vespula vulgaris
<400> 1430
   Pro Ile Leu Trp Gln Asn Pro Lys Ser Lys Arg Ile Phe Leu Leu
   1 5 10 15
<210> 1431
   <211> 15
   <212> PRT
   <213> Vespula vulgaris
<400> 1431
   Cys Val Lys Tyr His Phe Leu Ala Thr Pro Lys Gln Leu Cys Thr
   1 5 10 15
<210> 1432
   <211> 15
   <212> PRT
   <213> Vespula vulgaris
<400> 1432
   Val Asn Glu Tyr Pro Met Met Ala Gly Ile Ile His Leu Ala Thr
   1 5 10 15
<210> 1433
   <211> 15
   <212> PRT
   <213> Vespula vulgaris
<400> 1433
   Thr Trp Ala Ile Asn Asp Thr Lys Ala Thr Gln Leu Tyr Leu Ile
   1 5 10 15
<210> 1434
   <211> 15
   <212> PRT
   <213> Vespula vulgaris
<400> 1434
   His Pro Asn Tyr Arg Pro Lys Leu Asn Asp Leu Ala Val Ile Lys
   1 5 10 15
<210> 1435
   <211> 15
   <212> PRT
   <213> Vespula vulgaris
<400> 1435
   Asn Cys Arg Trp Ser Ala Arg Ser Asn Thr Arg Ile Lys Leu Asn
   1 5 10 15
<210> 1436
   <211> 15
   <212> PRT
   <213> Vespula vulgaris
<400> 1436
   Gly Ala Thr Ile Ile Thr Pro Gln His Val Leu Thr Ala Ala His
   1 5 10 15
<210> 1437
   <211> 15
   <212> PRT
   <213> Vespula vulgaris
<400> 1437
   Arg Pro Lys Leu Asn Asp Leu Ala Val Ile Lys Leu Gln Lys Arg
   1 5 10 15
<210> 1438
   <211> 15
   <212> PRT
   <213> Vespula vulgaris
<400> 1438
   Val Gly Trp Gly Leu Thr Asn Phe Tyr Gly Val Lys Ser Glu Val
   1               5                   10                  15
<210> 1439
   <211> 15
   <212> PRT
   <213> Vespula vulgaris
<400> 1439
   Phe Val Asp Thr Val Val Thr Ala Val Gly Trp Gly Leu Thr Asn
   1               5                   10                  15
<210> 1440
   <211> 15
   <212> PRT
   <213> Vespula vulgaris
<400> 1440
   Gln Arg Asn Phe Val Asp Thr Val Val Thr Ala Val Gly Trp Gly
   1               5                   10                  15
<210> 1441
   <211> 15
   <212> PRT
   <213> Vespula vulgaris
<400> 1441
   Cys Gly Leu Asn Ser Phe Ala Val Glu Ser Ile Ala Ser Lys Met
   1               5                   10                  15
<210> 1442
   <211> 15
   <212> PRT
   <213> Vespula vulgaris
<400> 1442
   Leu Lys Tyr Ser Met Arg Ile Gly Pro Ala Cys Leu Pro Phe Tyr
   1               5                   10                  15
<210> 1443
   <211> 15
   <212> PRT
   <213> Vespula vulgaris
<400> 1443
   Glu Tyr Val Phe Cys Gly Leu Asn Ser Phe Ala Val Glu Ser Ile
   1 5 10 15
<210> 1444
   <211> 15
   <212> PRT
   <213> Vespula vulgaris
<400> 1444
   Leu Thr Asn Phe Tyr Gly Val Lys Ser Glu Val Leu Arg Lys Val
   1 5 10 15
<210> 1445
   <211> 15
   <212> PRT
   <213> Vespula vulgaris
<400> 1445
   Tyr Lys Arg Ile Leu Tyr Ile Leu Gly Val Val Val Gly Glu His
   1 5 10 15
<210> 1446
   <211> 15
   <212> PRT
   <213> Vespula vulgaris
<400> 1446
   Met Ala Gly Ile Ile His Leu Ala Thr Arg Phe Leu Tyr Cys Gly
   1 5 10 15
<210> 1447
   <211> 15
   <212> PRT
   <213> Vespula vulgaris
<400> 1447
   Pro Ile Leu Trp Gln Asn Pro Lys Ser Lys Arg Ile Phe Leu Leu
   1 5 10 15
<210> 1448
   <211> 15
   <212> PRT
   <213> Vespula vulgaris
<400> 1448
   Ser Phe Ala Val Glu Ser Ile Ala Ser Lys Met Thr Ile Lys Phe
   1 5 10 15
<210> 1449
   <211> 15
   <212> PRT
   <213> Vespula vulgaris
<400> 1449
   Cys Val Lys Tyr His Phe Leu Ala Thr Pro Lys Gln Leu Cys Thr
   1 5 10 15
<210> 1450
   <211> 15
   <212> PRT
   <213> Vespula vulgaris
<400> 1450
   Cys Leu Pro Phe Tyr Tyr Met Gln Arg Asn Phe Val Asp Thr Val
   1 5 10 15
<210> 1451
   <211> 15
   <212> PRT
   <213> Vespula vulgaris
<400> 1451
   Arg Ile Phe Leu Leu Gly Val Ile Asn Tyr Gly Arg Thr Cys Ala
   1 5 10 15
<210> 1452
   <211> 15
   <212> PRT
   <213> Vespula vulgaris
<400> 1452
   Leu Pro Phe Tyr Tyr Met Gln Arg Asn Phe Val Asp Thr Val Val
   1 5 10 15
<210> 1453
   <211> 15
   <212> PRT
   <213> Vespula vulgaris
<400> 1453
   Val Ala Arg Tyr Lys Arg Ile Leu Tyr Ile Leu Gly Val Val Val
   1 5 10 15
<210> 1454
   <211> 15
   <212> PRT
   <213> Vespula vulgaris
<400> 1454
   Ala Thr Arg Phe Leu Tyr Cys Gly Ala Thr Ile Ile Thr Pro Gln
   1 5 10 15
<210> 1455
   <211> 15
   <212> PRT
   <213> Vespula vulgaris
<400> 1455
   Val Asn Glu Tyr Pro Met Met Ala Gly Ile Ile His Leu Ala Thr
   1 5 10 15
<210> 1456
   <211> 15
   <212> PRT
   <213> Vespula vulgaris
<400> 1456
   Gln Arg Asn Phe Val Asp Thr Val Val Thr Ala Val Gly Trp Gly
   1 5 10 15
<210> 1457
   <211> 15
   <212> PRT
   <213> Vespula vulgaris
<400> 1457
   Asn Cys Arg Trp Ser Ala Arg Ser Asn Thr Arg Ile Lys Leu Asn
   1 5 10 15
<210> 1458
   <211> 15
   <212> PRT
   <213> Vespula vulgaris
<400> 1458
   Val Gly Trp Gly Leu Thr Asn Phe Tyr Gly Val Lys Ser Glu Val
   1 5 10 15
<210> 1459
   <211> 15
   <212> PRT
   <213> Vespula vulgaris
<400> 1459
   His Pro Asn Tyr Arg Pro Lys Leu Asn Asp Leu Ala Val Ile Lys
   1 5 10 15
<210> 1460
   <211> 15
   <212> PRT
   <213> Vespula vulgaris
<400> 1460
   Thr Trp Ala Ile Asn Asp Thr Lys Ala Thr Gln Leu Tyr Leu Ile
   1 5 10 15
<210> 1461
   <211> 15
   <212> PRT
   <213> Vespula vulgaris
<400> 1461
   Thr Ile Lys Phe His Ser Arg Tyr Asn Thr Tyr Gly Gly Lys Phe
   1 5 10 15
<210> 1462
   <211> 15
   <212> PRT
   <213> Vespula vulgaris
<400> 1462
   Phe Val Asp Thr Val Val Thr Ala Val Gly Trp Gly Leu Thr Asn
   1 5 10 15
<210> 1463
   <211> 15
   <212> PRT
   <213> Vespula vulgaris
<400> 1463
   Tyr Thr Gln Lys Leu Lys Ser Asp Val Asn Tyr Cys Val Tyr Asn
   1 5 10 15
<210> 1464
   <211> 15
   <212> PRT
   <213> Vespula vulgaris
<400> 1464
   Gly Ala Thr Ile Ile Thr Pro Gln His Val Leu Thr Ala Ala His
   1 5 10 15
<210> 1465
   <211> 15
   <212> PRT
   <213> Vespula vulgaris
<400> 1465
   Arg Leu Lys Tyr Ser Met Arg Ile Gly Pro Ala Cys Leu Pro Phe
   1 5 10 15
<210> 1466
   <211> 15
   <212> PRT
   <213> Vespula vulgaris
<400> 1466
   Tyr Asn Pro Asp Phe Pro Asn Tyr Tyr Met Gly Glu His Asn Cys
   1 5 10 15
<210> 1467
   <211> 15
   <212> PRT
   <213> Vespula vulgaris
<400> 1467
   Glu Tyr Val Phe Cys Gly Leu Asn Ser Phe Ala Val Glu Ser Ile
   1               5                   10                  15
<210> 1468
   <211> 15
   <212> PRT
   <213> Vespula vulgaris
<400> 1468
   Met Ala Gly Ile Ile His Leu Ala Thr Arg Phe Leu Tyr Cys Gly
   1               5                   10                  15
<210> 1469
   <211> 15
   <212> PRT
   <213> Vespula vulgaris
<400> 1469
   Leu Thr Asn Phe Tyr Gly Val Lys Ser Glu Val Leu Arg Lys Val
   1               5                   10                  15
<210> 1470
   <211> 15
   <212> PRT
   <213> Vespula vulgaris
<400> 1470
   Tyr Lys Arg Ile Leu Tyr Ile Leu Gly Val Val Val Gly Glu His
   1               5                   10                  15
<210> 1471
   <211> 15
   <212> PRT
   <213> Vespula vulgaris
<400> 1471
   Ala Thr Arg Phe Leu Tyr Cys Gly Ala Thr Ile Ile Thr Pro Gln
   1               5                   10                  15
<210> 1472
   <211> 15
   <212> PRT
   <213> Vespula vulgaris
<400> 1472
   Ser Phe Ala Val Glu Ser Ile Ala Ser Lys Met Thr Ile Lys Phe
   1 5 10 15
<210> 1473
   <211> 15
   <212> PRT
   <213> Vespula vulgaris
<400> 1473
   Thr Trp Ala Ile Asn Asp Thr Lys Ala Thr Gln Leu Tyr Leu Ile
   1 5 10 15
<210> 1474
   <211> 15
   <212> PRT
   <213> Vespula vulgaris
<400> 1474
   Cys Leu Pro Phe Tyr Tyr Met Gln Arg Asn Phe Val Asp Thr Val
   1 5 10 15
<210> 1475
   <211> 15
   <212> PRT
   <213> Vespula vulgaris
<400> 1475
   Pro Ile Leu Trp Gln Asn Pro Lys Ser Lys Arg Ile Phe Leu Leu
   1 5 10 15
<210> 1476
   <211> 15
   <212> PRT
   <213> Vespula vulgaris
<400> 1476
   Cys Val Lys Tyr His Phe Leu Ala Thr Pro Lys Gln Leu Cys Thr
   1 5 10 15
<210> 1477
   <211> 15
   <212> PRT
   <213> Vespula vulgaris
<400> 1477
   Cys Gly Leu Asn Ser Phe Ala Val Glu Ser Ile Ala Ser Lys Met
   1 5 10 15
<210> 1478
   <211> 15
   <212> PRT
   <213> Vespula vulgaris
<400> 1478
   Gln Arg Asn Phe Val Asp Thr Val Val Thr Ala Val Gly Trp Gly
   1 5 10 15
<210> 1479
   <211> 15
   <212> PRT
   <213> Vespula vulgaris
<400> 1479
   Phe Val Asp Thr Val Val Thr Ala Val Gly Trp Gly Leu Thr Asn
   1 5 10 15
<210> 1480
   <211> 15
   <212> PRT
   <213> Vespula vulgaris
<400> 1480
   Arg Pro Lys Leu Asn Asp Leu Ala Val Ile Lys Leu Gln Lys Arg
   1 5 10 15
<210> 1481
   <211> 15
   <212> PRT
   <213> Vespula vulgaris
<400> 1481
   Cys Val Ala Arg Tyr Lys Arg Ile Leu Tyr Ile Leu Gly Val Val
   1 5 10 15
<210> 1482
   <211> 15
   <212> PRT
   <213> Vespula vulgaris
<400> 1482
   Asn Cys Arg Trp Ser Ala Arg Ser Asn Thr Arg Ile Lys Leu Asn
   1 5 10 15
<210> 1483
   <211> 15
   <212> PRT
   <213> Vespula vulgaris
<400> 1483
   Val Asn Glu Tyr Pro Met Met Ala Gly Ile Ile His Leu Ala Thr
   1 5 10 15
<210> 1484
   <211> 15
   <212> PRT
   <213> Vespula vulgaris
<400> 1484
   Thr Pro Gln His Val Leu Thr Ala Ala His Cys Val Ala Arg Tyr
   1 5 10 15
<210> 1485
   <211> 15
   <212> PRT
   <213> Vespula vulgaris
<400> 1485
   Leu Lys Tyr Ser Met Arg Ile Gly Pro Ala Cys Leu Pro Phe Tyr
   1 5 10 15
<210> 1486
   <211> 15
   <212> PRT
   <213> Vespula vulgaris
<400> 1486
   Leu Thr Asn Phe Tyr Gly Val Lys Ser Glu Val Leu Arg Lys Val
   1 5 10 15
<210> 1487
   <211> 15
   <212> PRT
   <213> Vespula vulgaris
<400> 1487
   Glu Tyr Val Phe Cys Gly Leu Asn Ser Phe Ala Val Glu Ser Ile
   1 5 10 15
<210> 1488
   <211> 15
   <212> PRT
   <213> Vespula vulgaris
<400> 1488
   Tyr Lys Arg Ile Leu Tyr Ile Leu Gly Val Val Val Gly Glu His
   1 5 10 15
<210> 1489
   <211> 15
   <212> PRT
   <213> Vespula vulgaris
<400> 1489
   Met Ala Gly Ile Ile His Leu Ala Thr Arg Phe Leu Tyr Cys Gly
   1 5 10 15
<210> 1490
   <211> 15
   <212> PRT
   <213> Vespula vulgaris
<400> 1490
   Pro Ile Leu Trp Gln Asn Pro Lys Ser Lys Arg Ile Phe Leu Leu
   1 5 10 15
<210> 1491
   <211> 15
   <212> PRT
   <213> Vespula vulgaris
<400> 1491
   Ser Phe Ala Val Glu Ser Ile Ala Ser Lys Met Thr Ile Lys Phe
   1 5 10 15
<210> 1492
   <211> 15
   <212> PRT
   <213> Vespula vulgaris
<400> 1492
   Ala Thr Arg Phe Leu Tyr Cys Gly Ala Thr Ile Ile Thr Pro Gln
   1 5 10 15
<210> 1493
   <211> 15
   <212> PRT
   <213> Vespula vulgaris
<400> 1493
   Asn Cys Arg Trp Ser Ala Arg Ser Asn Thr Arg Ile Lys Leu Asn
   1 5 10 15
<210> 1494
   <211> 15
   <212> PRT
   <213> Vespula vulgaris
<400> 1494
   Cys Leu Pro Phe Tyr Tyr Met Gln Arg Asn Phe Val Asp Thr Val
   1 5 10 15
<210> 1495
   <211> 15
   <212> PRT
   <213> Vespula vulgaris
<400> 1495
   Cys Val Ala Arg Tyr Lys Arg Ile Leu Tyr Ile Leu Gly Val Val
   1 5 10 15
<210> 1496
   <211> 15
   <212> PRT
   <213> Vespula vulgaris
<400> 1496
   Val Asn Glu Tyr Pro Met Met Ala Gly Ile Ile His Leu Ala Thr
   1 5 10 15
<210> 1497
   <211> 15
   <212> PRT
   <213> Vespula vulgaris
<400> 1497
   Arg Pro Lys Leu Asn Asp Leu Ala Val Ile Lys Leu Gln Lys Arg
   1 5 10 15
<210> 1498
   <211> 15
   <212> PRT
   <213> Vespula vulgaris
<400> 1498
   His Pro Asn Tyr Arg Pro Lys Leu Asn Asp Leu Ala Val Ile Lys
   1 5 10 15
<210> 1499
   <211> 15
   <212> PRT
   <213> Vespula vulgaris
<400> 1499
   Thr Trp Ala Ile Asn Asp Thr Lys Ala Thr Gln Leu Tyr Leu Ile
   1 5 10 15
<210> 1500
   <211> 15
   <212> PRT
   <213> Vespula vulgaris
<400> 1500
   Val Gly Trp Gly Leu Thr Asn Phe Tyr Gly Val Lys Ser Glu Val
   1 5 10 15
<210> 1501
   <211> 15
   <212> PRT
   <213> Vespula vulgaris
<400> 1501
   Pro Ile Leu Trp Gln Asn Pro Lys Ser Lys Arg Ile Phe Leu Leu
   1 5 10 15
<210> 1502
   <211> 15
   <212> PRT
   <213> Vespula vulgaris
<400> 1502
   Leu Thr Asn Phe Tyr Gly Val Lys Ser Glu Val Leu Arg Lys Val
   1 5 10 15
<210> 1503
   <211> 15
   <212> PRT
   <213> Vespula vulgaris
<400> 1503
   Glu Tyr Val Phe Cys Gly Leu Asn Ser Phe Ala Val Glu Ser Ile
   1 5 10 15
<210> 1504
   <211> 15
   <212> PRT
   <213> Vespula vulgaris
<400> 1504
   Tyr Thr Gln Lys Leu Lys Ser Asp Val Asn Tyr Cys Val Tyr Asn
   1 5 10 15
<210> 1505
   <211> 15
   <212> PRT
   <213> Vespula vulgaris
<400> 1505
   Asn Cys Arg Trp Ser Ala Arg Ser Asn Thr Arg Ile Lys Leu Asn
   1 5 10 15
<210> 1506
   <211> 15
   <212> PRT
   <213> Vespula vulgaris
<400> 1506
   Glu Tyr Val Phe Cys Gly Leu Asn Ser Phe Ala Val Glu Ser Ile
   1 5 10 15
<210> 1507
   <211> 15
   <212> PRT
   <213> Vespula vulgaris
<400> 1507
   Leu Thr Asn Phe Tyr Gly Val Lys Ser Glu Val Leu Arg Lys Val
   1 5 10 15
<210> 1508
   <211> 15
   <212> PRT
   <213> Vespula vulgaris
<400> 1508
   Met Ala Gly Ile Ile His Leu Ala Thr Arg Phe Leu Tyr Cys Gly
   1 5 10 15
<210> 1509
   <211> 15
   <212> PRT
   <213> Vespula vulgaris
<400> 1509
   Tyr Lys Arg Ile Leu Tyr Ile Leu Gly Val Val Val Gly Glu His
   1 5 10 15
<210> 1510
   <211> 15
   <212> PRT
   <213> Vespula vulgaris
<400> 1510
   Pro Ile Leu Trp Gln Asn Pro Lys Ser Lys Arg Ile Phe Leu Leu
   1 5 10 15
<210> 1511
   <211> 15
   <212> PRT
   <213> Vespula vulgaris
<400> 1511
   Ala Arg Tyr Lys Arg Ile Leu Tyr Ile Leu Gly Val Val Val Gly
   1 5 10 15
<210> 1512
   <211> 15
   <212> PRT
   <213> Vespula vulgaris
<400> 1512
   Ser Phe Ala Val Glu Ser Ile Ala Ser Lys Met Thr Ile Lys Phe
   1 5 10 15
<210> 1513
   <211> 15
   <212> PRT
   <213> Vespula vulgaris
<400> 1513
   Cys Leu Pro Phe Tyr Tyr Met Gln Arg Asn Phe Val Asp Thr Val
   1 5 10 15
<210> 1514
   <211> 15
   <212> PRT
   <213> Vespula vulgaris
<400> 1514
   Ala Thr Arg Phe Leu Tyr Cys Gly Ala Thr Ile Ile Thr Pro Gln
   1 5 10 15
<210> 1515
   <211> 15
   <212> PRT
   <213> Vespula vulgaris
<400> 1515
   Thr Trp Ala Ile Asn Asp Thr Lys Ala Thr Gln Leu Tyr Leu Ile
   1 5 10 15
<210> 1516
   <211> 15
   <212> PRT
   <213> Vespula vulgaris
<400> 1516
   Asn Cys Arg Trp Ser Ala Arg Ser Asn Thr Arg Ile Lys Leu Asn
   1 5 10 15
<210> 1517
   <211> 15
   <212> PRT
   <213> Vespula vulgaris
<400> 1517
   Cys Val Lys Tyr His Phe Leu Ala Thr Pro Lys Gln Leu Cys Thr
   1 5 10 15
<210> 1518
   <211> 15
   <212> PRT
   <213> Vespula vulgaris
<400> 1518
   His Pro Asn Tyr Arg Pro Lys Leu Asn Asp Leu Ala Val Ile Lys
   1 5 10 15
<210> 1519
   <211> 15
   <212> PRT
   <213> Vespula vulgaris
<400> 1519
   Val Asn Glu Tyr Pro Met Met Ala Gly Ile Ile His Leu Ala Thr
   1 5 10 15
<210> 1520
   <211> 15
   <212> PRT
   <213> Vespula vulgaris
<400> 1520
   Arg Pro Lys Leu Asn Asp Leu Ala Val Ile Lys Leu Gln Lys Arg
   1 5 10 15
<210> 1521
   <211> 15
   <212> PRT
   <213> Vespula vulgaris
<400> 1521
   Gln Arg Asn Phe Val Asp Thr Val Val Thr Ala Val Gly Trp Gly
   1 5 10 15
<210> 1522
   <211> 15
   <212> PRT
   <213> Vespula vulgaris
<400> 1522
   Cys Gly Leu Asn Ser Phe Ala Val Glu Ser Ile Ala Ser Lys Met
   1 5 10 15
<210> 1523
   <211> 15
   <212> PRT
   <213> Vespula vulgaris
<400> 1523
   Gly Ala Thr Ile Ile Thr Pro Gln His Val Leu Thr Ala Ala His
   1 5 10 15
<210> 1524
   <211> 15
   <212> PRT
   <213> Vespula vulgaris
<400> 1524
   Val Gly Trp Gly Leu Thr Asn Phe Tyr Gly Val Lys Ser Glu Val
   1 5 10 15
<210> 1525
   <211> 15
   <212> PRT
   <213> Vespula vulgaris
<400> 1525
   Phe Val Asp Thr Val Val Thr Ala Val Gly Trp Gly Leu Thr Asn
   1 5 10 15
<210> 1526
   <211> 15
   <212> PRT
   <213> Vespula vulgaris
<400> 1526
   Leu Lys Tyr Ser Met Arg Ile Gly Pro Ala Cys Leu Pro Phe Tyr
   1 5 10 15
<210> 1527
   <211> 15
   <212> PRT
   <213> Vespula vulgaris
<400> 1527
   Thr Pro Gln His Val Leu Thr Ala Ala His Cys Val Ala Arg Tyr
   1 5 10 15
<210> 1528
   <211> 15
   <212> PRT
   <213> Vespula vulgaris
<400> 1528
   Arg Leu Lys Tyr Ser Met Arg Ile Gly Pro Ala Cys Leu Pro Phe
   1 5 10 15
<210> 1529
   <211> 15
   <212> PRT
   <213> Vespula vulgaris
<400> 1529
   Glu Tyr Val Phe Cys Gly Leu Asn Ser Phe Ala Val Glu Ser Ile
   1 5 10 15
<210> 1530
   <211> 15
   <212> PRT
   <213> Vespula vulgaris
<400> 1530
   Leu Thr Asn Phe Tyr Gly Val Lys Ser Glu Val Leu Arg Lys Val
   1 5 10 15
<210> 1531
   <211> 15
   <212> PRT
   <213> Vespula vulgaris
<400> 1531
   Met Ala Gly Ile Ile His Leu Ala Thr Arg Phe Leu Tyr Cys Gly
   1 5 10 15
<210> 1532
   <211> 15
   <212> PRT
   <213> Vespula vulgaris
<400> 1532
   Tyr Lys Arg Ile Leu Tyr Ile Leu Gly Val Val Val Gly Glu His
   1 5 10 15
<210> 1533
   <211> 15
   <212> PRT
   <213> Vespula vulgaris
<400> 1533
   Cys Leu Pro Phe Tyr Tyr Met Gln Arg Asn Phe Val Asp Thr Val
   1 5 10 15
<210> 1534
   <211> 15
   <212> PRT
   <213> Vespula vulgaris
<400> 1534
   Ala Thr Arg Phe Leu Tyr Cys Gly Ala Thr Ile Ile Thr Pro Gln
   1 5 10 15
<210> 1535
   <211> 15
   <212> PRT
   <213> Vespula vulgaris
<400> 1535
   Val Ala Arg Tyr Lys Arg Ile Leu Tyr Ile Leu Gly Val Val Val
   1 5 10 15
<210> 1536
   <211> 15
   <212> PRT
   <213> Vespula vulgaris
<400> 1536
   Cys Val Lys Tyr His Phe Leu Ala Thr Pro Lys Gln Leu Cys Thr
   1 5 10 15
<210> 1537
   <211> 15
   <212> PRT
   <213> Vespula vulgaris
<400> 1537
   Ser Phe Ala Val Glu Ser Ile Ala Ser Lys Met Thr Ile Lys Phe
   1 5 10 15
<210> 1538
   <211> 15
   <212> PRT
   <213> Vespula vulgaris
<400> 1538
   Val Asn Glu Tyr Pro Met Met Ala Gly Ile Ile His Leu Ala Thr
   1 5 10 15
<210> 1539
   <211> 15
   <212> PRT
   <213> Vespula vulgaris
<400> 1539
   Pro Ile Leu Trp Gln Asn Pro Lys Ser Lys Arg Ile Phe Leu Leu
   1 5 10 15
<210> 1540
   <211> 15
   <212> PRT
   <213> Vespula vulgaris
<400> 1540
   Val Gly Trp Gly Leu Thr Asn Phe Tyr Gly Val Lys Ser Glu Val
   1 5 10 15
<210> 1541
   <211> 15
   <212> PRT
   <213> Vespula vulgaris
<400> 1541
   Arg Pro Lys Leu Asn Asp Leu Ala Val Ile Lys Leu Gln Lys Arg
   1 5 10 15
<210> 1542
   <211> 15
   <212> PRT
   <213> Vespula vulgaris
<400> 1542
   Thr Trp Ala Ile Asn Asp Thr Lys Ala Thr Gln Leu Tyr Leu Ile
   1 5 10 15
<210> 1543
   <211> 15
   <212> PRT
   <213> Vespula vulgaris
<400> 1543
   Gly Ala Thr Ile Ile Thr Pro Gln His Val Leu Thr Ala Ala His
   1 5 10 15
<210> 1544
   <211> 15
   <212> PRT
   <213> Vespula vulgaris
<400> 1544
   Glu Tyr Val Phe Cys Gly Leu Asn Ser Phe Ala Val Glu Ser Ile
   1 5 10 15
<210> 1545
   <211> 15
   <212> PRT
   <213> Vespula vulgaris
<400> 1545
   Leu Thr Asn Phe Tyr Gly Val Lys Ser Glu Val Leu Arg Lys Val
   1 5 10 15
<210> 1546
   <211> 15
   <212> PRT
   <213> Vespula vulgaris
<400> 1546
   Met Ala Gly Ile Ile His Leu Ala Thr Arg Phe Leu Tyr Cys Gly
   1 5 10 15
<210> 1547
   <211> 15
   <212> PRT
   <213> Vespula vulgaris
<400> 1547
   Tyr Lys Arg Ile Leu Tyr Ile Leu Gly Val Val Val Gly Glu His
   1 5 10 15
<210> 1548
   <211> 15
   <212> PRT
   <213> Vespula vulgaris
<400> 1548
   Ala Arg Tyr Lys Arg Ile Leu Tyr Ile Leu Gly Val Val Val Gly
   1 5 10 15
<210> 1549
   <211> 15
   <212> PRT
   <213> Vespula vulgaris
<400> 1549
   Ala Thr Arg Phe Leu Tyr Cys Gly Ala Thr Ile Ile Thr Pro Gln
   1 5 10 15
<210> 1550
   <211> 15
   <212> PRT
   <213> Vespula vulgaris
<400> 1550
   Ser Phe Ala Val Glu Ser Ile Ala Ser Lys Met Thr Ile Lys Phe
   1 5 10 15
<210> 1551
   <211> 15
   <212> PRT
   <213> Vespula vulgaris
<400> 1551
   Cys Leu Pro Phe Tyr Tyr Met Gln Arg Asn Phe Val Asp Thr Val
   1 5 10 15
<210> 1552
   <211> 15
   <212> PRT
   <213> Vespula vulgaris
<400> 1552
   Pro Ile Leu Trp Gln Asn Pro Lys Ser Lys Arg Ile Phe Leu Leu
   1 5 10 15
<210> 1553
   <211> 15
   <212> PRT
   <213> Vespula vulgaris
<400> 1553
   Cys Val Lys Tyr His Phe Leu Ala Thr Pro Lys Gln Leu Cys Thr
   1 5 10 15
<210> 1554
   <211> 15
   <212> PRT
   <213> Vespula vulgaris
<400> 1554
   Val Asn Glu Tyr Pro Met Met Ala Gly Ile Ile His Leu Ala Thr
   1 5 10 15
<210> 1555
   <211> 15
   <212> PRT
   <213> Vespula vulgaris
<400> 1555
   Thr Trp Ala Ile Asn Asp Thr Lys Ala Thr Gln Leu Tyr Leu Ile
   1 5 10 15
<210> 1556
   <211> 15
   <212> PRT
   <213> Vespula vulgaris
<400> 1556
   His Pro Asn Tyr Arg Pro Lys Leu Asn Asp Leu Ala Val Ile Lys
   1 5 10 15
<210> 1557
   <211> 15
   <212> PRT
   <213> Vespula vulgaris
<400> 1557
   Asn Cys Arg Trp Ser Ala Arg Ser Asn Thr Arg Ile Lys Leu Asn
   1 5 10 15
<210> 1558
   <211> 15
   <212> PRT
   <213> Vespula vulgaris
<400> 1558
   Gly Ala Thr Ile Ile Thr Pro Gln His Val Leu Thr Ala Ala His
   1 5 10 15
<210> 1559
   <211> 15
   <212> PRT
   <213> Vespula vulgaris
<400> 1559
   Arg Pro Lys Leu Asn Asp Leu Ala Val Ile Lys Leu Gln Lys Arg
   1 5 10 15
<210> 1560
   <211> 15
   <212> PRT
   <213> Vespula vulgaris
<400> 1560
   Val Gly Trp Gly Leu Thr Asn Phe Tyr Gly Val Lys Ser Glu Val
   1 5 10 15
<210> 1561
   <211> 15
   <212> PRT
   <213> Vespula vulgaris
<400> 1561
   Phe Val Asp Thr Val Val Thr Ala Val Gly Trp Gly Leu Thr Asn
   1 5 10 15
<210> 1562
   <211> 15
   <212> PRT
   <213> Vespula vulgaris
<400> 1562
   Gln Arg Asn Phe Val Asp Thr Val Val Thr Ala Val Gly Trp Gly
   1 5 10 15
<210> 1563
   <211> 15
   <212> PRT
   <213> Vespula vulgaris
<400> 1563
   Cys Gly Leu Asn Ser Phe Ala Val Glu Ser Ile Ala Ser Lys Met
   1 5 10 15
<210> 1564
   <211> 15
   <212> PRT
   <213> Vespula vulgaris
<400> 1564
   Leu Lys Tyr Ser Met Arg Ile Gly Pro Ala Cys Leu Pro Phe Tyr
   1 5 10 15
<210> 1565
   <211> 1
   <212> PRT
   <213> Vespula vulgaris
<400> 1565
   Thr
   1
<210> 1566
   <211> 3
   <212> PRT
   <213> Vespula vulgaris
<400> 1566
   Lys Leu Lys
   1
<210> 1567
   <211> 8
   <212> PRT
   <213> Vespula vulgaris
<400> 1567
   Pro Asp Phe Pro Asn Tyr Tyr Met
   1 5
<210> 1568
   <211> 5
   <212> PRT
   <213> Vespula vulgaris
<400> 1568
   Ser Ala Arg Ser Asn
   1 5
<210> 1569
   <211> 8
   <212> PRT
   <213> Vespula vulgaris
<400> 1569
   Asp Val Pro Pro Ser Ser Asn Cys
   1 5
<210> 1570
   <211> 1
   <212> PRT
   <213> Vespula vulgaris
<400> 1570
   Asp
   1
<210> 1571
   <211> 3
   <212> PRT
   <213> Vespula vulgaris
<400> 1571
   Asn Thr Tyr
   1
<210> 1572
   <211> 10
   <212> PRT
   <213> Vespula vulgaris
<400> 1572
   Trp Lys Asn Pro Ser Arg Ile Val Gly Gly
   1               5                   10
<210> 1573
   <211> 9
   <212> PRT
   <213> Vespula vulgaris
<400> 1573
   Thr Trp Ala Ile Asn Asp Thr Lys Ala
   1               5
<210> 1574
   <211> 5
   <212> PRT
   <213> Vespula vulgaris
<400> 1574
   Asn Tyr Arg Pro Lys
   1               5
<210> 1575
   <211> 10
   <212> PRT
   <213> Vespula vulgaris
<400> 1575
   Ala Cys Gln Phe Asp Ser Gly Gly Pro Ile
   1               5                   10
<210> 1576
   <211> 9
   <212> PRT
   <213> Vespula vulgaris
<400> 1576
   Thr Cys Ala Asp Glu Ala Pro Gly Val
   1               5
<210> 1577
   <211> 7
   <212> PRT
   <213> Vespula vulgaris
<400> 1577
   Arg Ser Thr Pro Gly Glu Ile
   1               5